(19)

**Europäisches Patentamt**
**European Patent Office**
**Office européen des brevets**

(11) **EP 4 168 034 B1**

(12) **EUROPEAN PATENT SPECIFICATION**

(45) Date of publication and mention
of the grant of the patent:
**26.11.2025 Bulletin 2025/48**

(21) Application number: **20737632.8**

(22) Date of filing: **22.06.2020**

(51) International Patent Classification (IPC):
*A61K 38/17* (2006.01)     *A61K 38/21* (2006.01)
*A61P 35/00* (2006.01)     *A61P 35/04* (2006.01)
*C07K 16/24* (2006.01)     *C07K 16/28* (2006.01)
*A61K 39/395* (2006.01)

(52) Cooperative Patent Classification (CPC):
(C-Sets available)
**A61P 35/00; A61K 38/1709; A61K 38/1793;
A61K 38/217; A61K 39/3955; A61P 35/04;
C07K 16/2818;** A61K 2039/545; C07K 2317/21;
C07K 2317/76                                    (Cont.)

(86) International application number:
**PCT/SG2020/050344**

(87) International publication number:
**WO 2021/262081 (30.12.2021 Gazette 2021/52)**

(54) **RECOMBINANT ALPHA-A-CRYSTALLIN AND COMBINATIONS FOR USE IN THE TREATMENT OF CANCER**

REKOMBINANTES ALPHA-A-CRYSTALLIN UND KOMBINATIONEN ZUR VERWENDUNG BEI DER BEHANDLUNG VON KREBS

ALPHA-A-CRYSTALLIN RECOMBINANTE ET COMBINAISONS POUR UTILISATION DANS LE TRAITEMENT DU CANCER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**26.04.2023 Bulletin 2023/17**

(73) Proprietor: **Imunami Laboratories Pte. Ltd.
Singapore 228208 (SG)**

(72) Inventors:
• **CHEN, Ya-Huei
Singapore 228208 (SG)**
• **LIN, Ting-Long
Singapore 228208 (SG)**

(74) Representative: **Haseltine Lake Kempner LLP
Cheapside House
138 Cheapside
London EC2V 6BJ (GB)**

(56) References cited:
**WO-A1-2020/093149     US-A1- 2019 209 649
US-B1- 10 675 332**

• **BERRAONDO PEDRO ET AL: "Cytokines in
clinical cancer immunotherapy", BRITISH
JOURNAL OF CANCER, NATURE PUBLISHING
GROUP, GB, vol. 120, no. 1, 9 November 2018
(2018-11-09), pages 6 - 15, XP036927199, ISSN:
0007-0920, [retrieved on 20181109], DOI: 10.1038/
S41416-018-0328-Y**

Remarks:
The complete document including Reference
Table(s) and the Sequence Listing(s) can be
downloaded from the EPO website

EP 4 168 034 B1

**(Cont. next page)**

(52) Cooperative Patent Classification (CPC): (Cont.)

C-Sets
**A61K 38/1709, A61K 2300/00;**
**A61K 38/1793, A61K 2300/00;**
**A61K 38/217, A61K 2300/00;**
**A61K 39/3955, A61K 2300/00**

**Description**

SEQUENCE LISTING

The instant application contains a Sequence Listing which has been submitted in ASCII format *via* EFS-Web and is hereby incorporated by reference in its entirety. Said ASCII copy, created on June 18, 2020, is named "IMHC-003_F01WO_SeqListing_ST25.txt" and is 41 KB in size.

BACKGROUND OF THE INVENTION

Immunogenic cell death is a form of cell death or apoptosis. Unlike traditional apoptosis, which is mostly non-immunogenic, immunogenic cell death in cancer cells can induce an effective anti-tumor immune response through the activation of dendritic cells. The pre-apoptotic state is defined as the state before the activation of Caspase 3/7, the manifestation of cell apoptosis. Immunogenic cell death is characterized by the expression of pre-apoptotic damage-associated-molecular-patterns (DAMPs) on the surface of a dying cell. There are three important pre-apoptotic DAMPs which are exposed to the cell surface during immunogenic cell death: calreticulin (CRT), HSP70 and HSP90. These three pre-apoptotic DAMP signals play an important role in dendritic cell recruitment and cell phagocytosis by CRT and dendritic cell maturation/activation by HSP70 and HSP90, resulting in effective anti-tumor immune response. Selected forms of chemotherapy and radiotherapy can induce collateral immunogenic cell death. While these therapies can induce one or two of the three pre-apoptotic DAMP signals, they do not induce the expression of all three pre-apoptotic DAMP signals. Furthermore, chemotherapy and radiotherapy are immunosuppressive therapies, which reduce numbers of lymphocytes and also cause collateral damages to surrounding non-tumor cells, resulting in poor anti-tumor immune responses and also adverse events respectively. US 10 675 332 B1 describes recombinant polypeptides and methods of use thereof.
There is a need for compositions and methods that induce immunogenic cell death with increased efficiency and potency by inducing the expression of all three pre-apoptotic DAMP signals, while minimizing adverse effects. There is a need for compositions and methods of personalized and universal cancer immunotherapies by inducing dendritic cell mediated long-term adaptive immune responses. There is a need for compositions and methods of inducing T-cell polyclonal diversity with neoantigenic breadth in order to prevent cancer occurrence, irrespective of the cancer type or the tumor phenotype. The present disclosure addresses these needs.
Tumour-infiltrating (intratumoral) CD11c+ myeloid dendritic cells (mDCs), human CD141+XCR1+DCs or murine CD103+XCR1+DCs (Roberts et. al., Cancer Cell, 324-336, 2016) are rare in tumour microenvironment(Broz et. al., Cancer Cell, 638-652, 2014). These XCR1+ DCs are responsible for capturing tumour antigens, migrating to lymph nodes via CCR7 up-regulation, and cross-presenting antigens to T cells in lymph nodes for T-cell activation/expansion. Murine CD103+XCR1+DCs highly express both CD40 and TLR4. In T-cell immunotherapy, HMGB1 is typically used to activate/maturate CD103+XCR1+DCs via TLR4 in murine model (Tesniere et. al., Oncogene, 482-491, 2010). However, human CD141+XCR1+DCs highly express CD40 and TLR3, without TLR4 expression (Jongbloed et. al., J. Exp. Med., 1247-1260, 2010). In 'human' T-cell immunotherapy, HMGB1 fails to mature, activate and expand CD141+XCR1+DCs. Furthermore, to induce DC's IL12 for effective cancer immunotherapy, CD40 is more important than Toll-like receptors (TLRs)(Chang et. al., Cancer Research, 10047-10057, 2007; Pinzon-Charry et. al., Br. J. Cancer, 1251-1259, 2007). Thus, there exists a need for alternative approaches to activate, to mature and to expand dendritic cells to overcome the current limitations of dendritic cell activation and expansion. The present invention address this unmet need in the art.
Precursor frequency (probability) of a naive CD8+T cell to recognize a specific antigen is about 1-10 per million cells (Lammermann et. al., Immunological Reviews, 26-43, 2008). The total naive CD8+T cells in human is about $4 \times 10^{10}$ cells (Boon et. al., Annu. Rev. Immunol., 175-208, 2006). Thus, assuming precursor frequency of a human naive CD8+T cell to recognize a human tumour-specific antigen is about ~5 per million cells, then the number of human naive CD8+T cells recognizing a tumour-specific antigen is about $2 \times 10^5$ cells ($4 \times 10^{10} \times 5/10^6$). One CD8+T cell has the ability to kill 2-3 target tumour cells (Wiedemann et. al., PNAS, 10985-10990, 2006, McGavern et. al., Nature Immunol, 918-925, 2002).
An Epithelial tumour size is about $1 \times 10^9$ cells/1 cm$^3$, assuming cell diameter 10 $\mu$m (Del Monte et. al., Cell Cycle, 505-506, 2009). An average cell diameter is about 5-10 $\mu$m (Del Monte et. al., Cell Cycle, 505-506, 2009). However, tumour cell diameters can reach about 20 $\mu$m (Del Monte et. al., Cell Cycle, 505-506, 2009, Backman et. al., Nature, 35-36, 2000). Therefore, the epithelial tumour size could be about $1.25 \times 10^8$ cells/1 cm$^3$, assuming cell diameter 20 $\mu$m (Del Monte et. al., Cell Cycle, 505-506, 2009). Since one CD8+T cell has the ability to kill only 2-3 target tumour cells (Wiedemann et. al., PNAS,10985-10990, 2006, McGavern et. al., Nature Immunol, 918-925, 2002) and there is a low frequency of CD8+T cells that can recognize a specific tumour antigen, there exists a need to increase both the number of CD8+T cells and the frequency at which they recognize specific tumour antigens in order to efficiently target and kill the tumor. The present invention address this unmet need in the art.
T- cell priming results in a CD8+T-cell expansion after priming is about 10000-fold (or about 50000x: LCMV/LM infections) (Blattman et. al., J. Exp. Med., 657-664, 2002, Haring et. al., Immunity,19-29, 2006, Butler et. al., Cellular Microbiology, 925-933, 2011), Arens et. al., Immunol. Rev., 190-205, 2010). However, after T-cell 'priming', humans typically require a 90-day interval in order to optimally 'boost' memory T cells for further expansion. (Sallusto, Immunity, 451-463, 2010, Epstein Science, 475-80, 2011, Walsh, J. Infect. Dis., 541-550, 2016). For cancer patients with a limited life span and/or a large tumor sizes, there exists a need for alternative approaches to efficiently prime and to boost the number of antigen-specific T-cells, thereby improving immune response and cancer treatment. The present invention provides compositions and methods that address this unmet need in the art.

## SUMMARY OF THE INVENTION

The present disclosure provides a recombinant polypeptide comprising an amino acid sequence having at least 95% sequence identity to the polypeptide of SEQ ID NO: 9, or a nucleic acid encoding the recombinant polypeptide comprising an amino acid sequence having at least 95% sequence identity to the polypeptide of SEQ ID NO: 9, for use in a method of treating cancer, wherein the method comprises administering: i) a first composition comprising at least one immune cell growth factor; ii) a second composition comprising at least one IL-10 inhibitory agent; iii) a third composition comprising at least one Tumor Necrosis Factor alpha (TNFa) inhibitory agent; and iv) a fourth composition comprising the recombinant polypeptide or the nucleic acid in accordance with the claims.

The present disclosure provides an immune cell growth factor for use in a method of treating cancer, wherein the method comprises administering: i) a first composition comprising the immune cell growth factor, and optionally at least one additional immune cell growth factor; ii) a second composition comprising at least one IL-10 inhibitory agent; iii) a third composition comprising at least one Tumor Necrosis Factor alpha (TNFa) inhibitory agent; and iv) a fourth composition comprising a recombinant polypeptide comprising an amino acid sequence having at least 95% sequence identity to the polypeptide of SEQ ID NO: 9, or a nucleic acid encoding the recombinant polypeptide comprising an amino acid sequence having at least 95% sequence identity to the polypeptide of SEQ ID NO: 9 in accordance with the claims.

The present disclosure provides an IL-10 inhibitory agent for use in a method of treating cancer, wherein the method comprises administering: i) a first composition comprising at least one immune cell growth factor; ii) a second composition comprising the IL-10 inhibitory agent, and optionally at least one additional IL-10 inhibitory agent; iii) a third composition comprising at least one Tumor Necrosis Factor alpha (TNFa) inhibitory agent; and iv) a fourth composition comprising a recombinant polypeptide comprising an amino acid sequence having at least 95% sequence identity to the polypeptide of SEQ ID NO: 9, or a nucleic acid encoding the recombinant polypeptide comprising an amino acid sequence having at least 95% sequence identity to the polypeptide of SEQ ID NO: 9 in accordance with the claims.

The present disclosure provides a Tumor Necrosis Factor alpha (TNFa) inhibitory agent for use in a method of treating cancer, wherein the method comprises administering: i) a first composition comprising at least one immune cell growth factor; ii) a second composition comprising at least one IL-10 inhibitory agent; iii) a third composition comprising the TNFa inhibitory agent; and optionally at least one additional TNFa inhibitory agent; and iv) a fourth composition comprising a recombinant polypeptide comprising an amino acid sequence having at least 95% sequence identity to the polypeptide of SEQ ID NO: 9, or a nucleic acid encoding the recombinant polypeptide comprising an amino acid sequence having at least 95% sequence identity to the polypeptide of SEQ ID NO: 9 in accordance with the claims.

The present disclosure provides an immunotherapy agent for use in a method of treating cancer, wherein the method comprises administering: i) a first composition comprising at least one immune cell growth factor; ii) a second composition comprising at least one IL-10 inhibitory agent; iii) a third composition comprising at least one Tumor Necrosis Factor alpha (TNFa) inhibitory agent; iv) a fourth composition comprising a recombinant polypeptide comprising an amino acid sequence having at least 95% sequence identity to the polypeptide of SEQ ID NO: 9, or a nucleic acid encoding the recombinant polypeptide comprising an amino acid sequence having at least 95% sequence identity to the polypeptide of SEQ ID NO: 9; and v) a fifth composition comprising the immunotherapy agent in accordance with the claims.

In some aspects, the recombinant polypeptide, nucleic acid, immune cell growth factor, IL-10 inhibitory agent, or TNFa inhibitory agent for use in a method of treating cancer further comprises administering a fifth composition comprising a immunotherapy agent.

In some aspects, the first composition is administered at least about 24 hours prior to the second composition or the third composition.

In some aspects, the first composition is administered: (a) once daily for at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days or at least 23 days; or (b) once daily for about 7 days to about 12 days; or (c) once daily for about 7 days.

In some aspects, (a) the second composition and the third composition are administered concurrently or sequentially; or (b) the second composition is administered prior to the third composition; or (c) the second composition is administered after the third composition.

In some aspects, the fourth composition is administered as multiple infusions, wherein i) a second infusion is administered at least 1 day after a first infusion; ii) a third infusion is administered at least 4 days after the first infusion; iii) a fourth infusion is administered at least 5 days after the first infusion; iv) a fifth infusion is administered at least 8 days after the first infusion; and/or v) a sixth infusion is administered at least 9 days after the first infusion.

In some aspects, the third composition and the first infusion of the fourth composition are administered concurrently or sequentially.

In some aspects, the first infusion of the fourth composition is administered: (a) at least 1 hour, at least 2 hours, at least 3 hours, at least 4 hours, at least 5 hours, at least 6 hours, at least 7 hours, at least 8 hours, at least 9 hours, at least 10 hours, at least 11 hours, at least 12 hours, at least 13 hours, at least 14 hours, at least 15 hours, at least 16 hours, at least 17 hours, at least 18 hours, at least 19 hours, at least 20 hours, at least 21 hours, at least 22 hours, at least 23 hours, at least 24 hours, at least 25 hours, at least 26 hours, at least 27 hours or at least 28 hours after the administration of the second composition; or (b) at about 3 hours or about 4 hours after the administration of the second composition.

In some aspects, the recombinant polypeptide, nucleic acid, immune cell growth factor, IL-10 inhibitory agent, TNFa inhibitory agent, or immunotherapy agent for use in a method of treating cancer, further comprises administering a therapeutic cycle comprising: i) a sixth composition comprising at least one immune cell growth factor; ii) a seventh composition comprising at least one IL-10 inhibitory agent in the subject; iii) an eighth composition comprising at least one TNFa inhibitory agent; and iv) a ninth composition comprising a recombinant polypeptide comprising an amino acid sequence having at least 95% sequence identity to the polypeptide of SEQ ID NO: 9, or a nucleic acid encoding the recombinant polypeptide comprising an amino acid sequence having at least 95% sequence identity to the polypeptide of SEQ ID NO: 9 in accordance with the claims.

In some aspects, the therapeutic cycle is administered: (a) at least 1 time, at least 2 times, at least 3 times, at least 4 times or at least 5 times; optionally wherein the therapeutic cycle is administered no more than five times; and/or (b) at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days or at least 14 days after the sixth infusion of the fourth composition; optionally wherein the first therapeutic cycle is administered at about 1 day after the sixth infusion of the fourth composition.

In some aspects, the sixth composition is administered: (a) at least about 24 hours prior to the seventh composition or the eighth composition; and/or (b) once daily for at least 5, at least 6, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days or at least 23 days; or once daily for a total of about 5 days to about 14 days or about 7 days to about 12 days.

In some aspects, (a) the seventh composition and the eighth composition are administered concurrently or sequentially; or (b) the seventh composition is administered prior to the eighth composition; or (c) the seventh composition is administered after the eighth composition.

In some aspects, the ninth composition is administered as multiple infusions, wherein i) a second infusion is administered at least 1 day after a first infusion; ii) a third infusion is administered at least 4 days after the first infusion; and iii) a fourth infusion is administered at least 5 days after the first infusion.

In some aspects, the eighth composition and the first infusion of the ninth composition are administered concurrently.

In some aspects, the first infusion of the ninth composition is administered: (a) at least 1 hour, at least 2 hours, at least 3 hours, at least 4 hours, at least 5 hours, at least 6 hours, at least 7 hours, at least 8 hours, at least 9 hours, at least 10 hours, at least 11 hours, at least 12 hours, at least 13 hours, at least 14 hours, at least 15 hours, at least 16 hours, at least 17 hours, at least 18 hours, at least 19 hours, at least 20 hours, at least 21 hours, at least 22 hours, at least 23 hours, at least 24 hours, at least 25 hours, at least 26 hours, at least 27 hours or at least 28 hours after the administration of the seventh composition; or (b) at about 3 hours or about 4 hours after the administration of the seventh composition; or (c) at about 4 hours after the administration of the seventh composition.

In some aspects, the fifth composition is administered as multiple infusions, and wherein the fifth composition is administered at least one time, at least 2 times, at least 3 times, at least 4 times, at least 5 times, at least 6 times, at least 7 times, at least 8 times, at least 9 times, at least 10 times, at least 11 times, at least 12 times, at least 13 times or at least 14 times.

In some aspects, the first infusion of the fifth composition is administered after the sixth infusion of the fourth composition and prior to the first infusion of the ninth composition.

In some aspects, the fifth composition is administered: (a) at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days after the first infusion of the fourth composition; or (b) at about 10 days to about 14 days after the first infusion of the fourth composition; or (c) at about 12 days after the first infusion of the fourth composition.

In some aspects, (a) the first composition, the second composition, the third composition, the fourth composition, the fifth composition, the sixth composition, the seventh composition, the eighth composition or the ninth composition are administered intravenously or subcutaneously; and/or (b) the first composition, the second composition, the fourth composition, the fifth composition, the sixth composition, the seventh composition and the ninth composition are administered intravenously; and/or (c) the third composition and the eighth composition are administered subcutaneously.

The at least one immune cell growth factor is FMS-like tyrosine kinase 3 ligand (FLT3L) or granulocyte-macrophage colony stimulating factor (GM-CSF). In some aspects, the at least one immune cell growth factor is FLT3L; optionally wherein the FLT3L is in an amount of: (a) about 1 $\mu$g/kg, about 2 $\mu$g/kg, about 3 $\mu$g/kg, about 4 $\mu$g/kg, about 5 $\mu$g/kg, about 6 $\mu$g/kg, about 7 $\mu$g/kg, about 8 $\mu$g/kg, about 9 $\mu$g/kg, 10 $\mu$g/kg, about 11 $\mu$g/kg, about 12 $\mu$g/kg, about 13 $\mu$g/kg, about 14 $\mu$g/kg, about 15 $\mu$g/kg, about 16 $\mu$g/kg, about 17 $\mu$g/kg, about 18 $\mu$g/kg, about 19 $\mu$g/kg, or about 20 $\mu$g/kg; or (b) about 4 $\mu$g/kg to about 13 $\mu$g/kg; or (c) about 7 $\mu$g/kg.

The at least one IL-10 inhibitory agent reprograms an M2 tumour associated macrophage (TAM) into an M1 TAM in the subject, wherein reprogramming comprises a change in surface marker expression, wherein MARCO and CD163 are surface markers expressed on M2 TAM, and wherein CD80 is a surface marker expressed on M1 TAM.

The at least one IL-10 inhibitory agent is an interferon gamma (IFNg) or an IFNg mimetic; optionally wherein the at least one IL-10 inhibitory agent is IFNg and wherein the IFN gamma is administered at a dose of about 10 $\mu$g, about 15 $\mu$g, about 20 $\mu$g, about 25$\mu$g, about 30 $\mu$g, about 35 $\mu$g, about 40 $\mu$g, about 45$\mu$g, about 50 $\mu$g, about 55 $\mu$g, about 60 $\mu$g, about 65$\mu$g, about 70 $\mu$g, about 75$\mu$g, about 80 $\mu$g, about 85 $\mu$g, about 90 $\mu$g, about 95$\mu$g or about 100 $\mu$g; or about 50 $\mu$g to about 100 $\mu$g; or about 70 $\mu$g.

In some aspects, the TNFa inhibitor agent is a TNFa receptor (TNFaR), infliximab, adalimumab, certolizumab pegol, golimumab, or etanercept; optionally wherein the TNFaR is administered: (a) at a dose of about 1 mg, about 2 mg, about 2 mg, about 3 mg, about 4 mg, about 5 mg, about 6 mg, about 7 mg, about 8 mg, about 9 mg or about 10 mg; or (b) at a dose of about 4 mg to about 6 mg; or (c) at a dose of about 5 mg,

In some aspects, (a) the first infusion, the second infusion, the third infusion, the fourth infusion, the fifth infusion and/or the sixth infusion of the fourth composition, and the first infusion, the second infusion, the third infusion and/or the fourth infusion of the ninth composition comprises about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg about 25 mg/kg, about 26 mg/kg, about 27 mg/kg, about 28 mg/kg, about 29 mg/kg, about 30 mg/kg, about 31 mg/kg, about 32 mg/kg, about 33 mg/kg, about 34 mg/kg or about 35 mg/kg of the recombinant polypeptide comprising an amino acid sequence having at least 95% sequence identity to the polypeptide of SEQ ID NO: 9; or (b) the first infusion, the second infusion, the third infusion, the fourth infusion, the fifth infusion and the sixth infusion of the fourth composition, and the first infusion, the second infusion, the third infusion and the fourth infusion of the ninth composition comprises about 10 mg/kg to about 30 mg/kg of the recombinant polypeptide comprising an amino acid sequence having at least 95% sequence identity to the polypeptide of SEQ ID NO: 9; or (c) the first infusion, the second infusion, the third infusion, the fourth infusion, the fifth infusion and the sixth infusion of the fourth composition, and the first infusion, the second infusion, the third infusion and the fourth infusion of the ninth composition comprises about 20 mg/kg of the recombinant polypeptide comprising an amino acid sequence having at least 95% sequence identity to the polypeptide of SEQ ID NO: 9.

In some aspects, the checkpoint inhibitor is an anti-PD1 antibody; optionally wherein the anti-PD1 antibody is nivolumab, pembrolizumab or cemiplimab. In some aspects, the anti-PD1 antibody is administered at a dose of: (a) about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 2 mg/kg, about 3 mg/kg, about 4 mg/kg, about 5mg/kg, about 6mg/kg, about 7 mg/kg, about 8 mg/kg, about 9 mg/kg or about 10 mg/kg; or (b) about 0.1 mg/kg to about 7 mg/kg; or (c) about 3mg/kg.

In some aspects, the cancer: (a) is a solid tumor cancer; optionally wherein the solid tumor has a diameter of at least about 0.2 cm, at least about 0.5 cm, at least about 1 cm, at least about 2 cm, at least about 3 cm, at least about 4 cm, at least about 5 cm, at least about 6 cm, at least about 7 cm, at least about 8 cm, at least about 9 cm or at least about 10 cm; and/or (b) is a recurrence of an earlier presentation of a cancer or is a metastasis of an earlier presentation of cancer.

Described is a method of treating cancer in a subject in need thereof comprising administering to the subject: i) a first composition comprising at least one immune cell growth factor; ii) a second composition comprising at least one IL-10 inhibitory agent in the subject; iii) a third composition comprising at least one Tumor Necrosis Factor alpha (TNFa) inhibitory agent; and iv) a fourth composition comprising a recombinant polypeptide comprising an amino acid sequence having at least 95% sequence identity to the polypeptide of SEQ ID NO: 9, or a nucleic acid encoding the recombinant polypeptide comprising an amino acid sequence having at least 95% sequence identity to the polypeptide of SEQ ID NO: 9. In some examples, the method further comprises administering a fifth composition comprising a immunotherapy agent.

In some examples, the first composition is administered at least about 24 hours prior to the second composition or the third composition.

In some examples, the first composition is administered once daily for at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days or at least 23 days. In some examples, the first composition is administered once daily for about 7 days to about 12 days. In a preferred aspect, the first composition is administered once daily for about 7 days.

In some examples, the second composition and the third composition are administered concurrently or sequentially. In some examples, the second composition is administered prior to the third composition. In some examples, the second composition is administered after the third composition.

In some examples, the fourth composition is administered as multiple infusions, wherein i) a second infusion is administered at least 1 day after a first infusion; ii) a third infusion is administered at least 4 days after the first infusion; iii) a fourth infusion is administered at least 5 days after the first infusion; iv) a fifth infusion is administered at least 8 days after the first infusion; and/or v) a sixth infusion is administered at least 9 days after the first infusion.

In some examples, the third composition and the first infusion of the fourth composition are administered concurrently or sequentially.

In some examples, the first infusion of the fourth composition is administered at least 1 hour, at least 2 hours, at least 3 hours, at least 4 hours, at least 5 hours, at least 6 hours, at least 7 hours, at least 8 hours, at least 9 hours, at least 10 hours, at least 11 hours, at least 12 hours, at least 13 hours, at least 14 hours, at least 15 hours, at least 16 hours, at least 17 hours, at least 18 hours, at least 19 hours, at least 20 hours, at least 21 hours, at least 22 hours, at least 23 hours, at least 24 hours, at least 25 hours, at least 26 hours, at least 27 hours or at least 28 hours after the administration of the second composition. In some examples, the first infusion of the fourth composition is administered at about 3 hours or about 4 hours after the administration of the second composition. In a preferred example, the first infusion of the fourth composition is administered at about 4 hours after the administration of the second composition.

In some example, the method of this disclosure further comprises administering a therapeutic cycle comprising: i) a sixth composition comprising at least one immune cell growth factor; ii) a seventh composition comprising at least one IL-10 inhibitory agent in the subject; iii) an eighth composition comprising at least one TNFa inhibitory agent; and iv) a ninth composition comprising a recombinant polypeptide comprising an amino acid sequence having at least 95% sequence identity to the polypeptide of SEQ ID NO: 9, or a nucleic acid encoding the recombinant polypeptide comprising an amino acid sequence having at least 95% sequence identity to the polypeptide of SEQ ID NO: 9.

In some examples, the therapeutic cycle is administered at least 1 time, at least 2 times, at least 3 times, at least 4 times or at least 5 times. In a preferred example, the therapeutic cycle is administered no more than five times.

In some examples, the first therapeutic cycle is administered at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days or at least 14 days after the sixth infusion of the fourth composition. In a preferred example, the first therapeutic cycle is administered at about 1 day after the sixth infusion of the fourth composition.

In some examples, the sixth composition is administered at least about 24 hours prior to the seventh composition or the eighth composition.

In some aspects, the sixth composition is administered once daily for at least 5, at least 6, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days or at least 23 days. In some examples, the sixth composition is administered once daily for a total of about 5 days to about 14 days. In a preferred example, the sixth composition is administered once daily for a total of about 7 days to about 12 days.

In some examples, the seventh composition and the eighth composition are administered concurrently or sequentially. In some examples, the seventh composition is administered prior to the eighth composition. In some examples, the seventh composition is administered after the eighth composition.

In some examples, the ninth composition is administered as multiple infusions, wherein i) a second infusion is administered at least 1 day after a first infusion; ii) a third infusion is administered at least 4 days after the first infusion; and iii) a fourth infusion is administered at least 5 days after the first infusion.

In some examples, the eighth composition and the first infusion of the ninth composition are administered concurrently.

In some examples, the first infusion of the ninth composition is administered at least 1 hour, at least 2 hours, at least 3 hours, at least 4 hours, at least 5 hours, at least 6 hours, at least 7 hours, at least 8 hours, at least 9 hours, at least 10 hours, at least 11 hours, at least 12 hours, at least 13 hours, at least 14 hours, at least 15 hours, at least 16 hours, at least 17 hours, at least 18 hours, at least 19 hours, at least 20 hours, at least 21 hours, at least 22 hours, at least 23 hours, at least 24 hours, at least 25 hours, at least 26 hours, at least 27 hours or at least 28 hours after the administration of the seventh composition.

In some examples, the first infusion of the ninth composition is administered at about 3 hours or about 4 hours after the administration of the seventh composition. In some examples, the first infusion of the ninth composition is administered at about 4 hours after the administration of the seventh composition.

In some examples, the fifth composition is administered as multiple infusions, and wherein the fifth composition is administered at least one time, at least 2 times, at least 3 times, at least 4 times, at least 5 times, at least 6 times, at least 7 times, at least 8 times, at least 9 times, at least 10 times, at least 11 times, at least 12 times, at least 13 times or at least 14 times.

In some examples, the first infusion of the fifth composition is administered after the sixth infusion of the fourth composition and prior to the first infusion of the ninth composition.

In some examples, the fifth composition is administered at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days after the first infusion of the fourth composition. In some examples, the fifth composition is administered at about 10 days to about 14 days after the first infusion of the fourth composition.

In a preferred example, the fifth composition is administered at about 12 days after the first infusion of the fourth composition.

In some examples, the first composition, the second composition, the third composition, the fourth composition, the fifth composition, the sixth composition, the seventh composition, the eighth composition or the ninth composition are administered intravenously or subcutaneously. In a preferred example, the first composition, the second composition, the fourth composition, the fifth composition, the sixth composition, the seventh composition and the ninth composition are administered intravenously. In a preferred example, the third composition and the eighth composition are administered subcutaneously.

In some examples, the at least one immune cell growth factor is FMS-like tyrosine kinase 3 ligand (FLT3L) or granulocyte-macrophage colony stimulating factor (GM-CSF). In a preferred example, the at least one immune cell growth factor is FLT3L.

In some examples, the FLT3L is in an amount of about 1 $\mu$g/kg, about 2 $\mu$g/kg, about 3 $\mu$g/kg, about 4 $\mu$g/kg, about 5 $\mu$g/kg, about 6 $\mu$g/kg, about 7 $\mu$g/kg, about 8 $\mu$g/kg, about 9 $\mu$g/kg, 10 $\mu$g/kg, about 11 $\mu$g/kg, about 12 $\mu$g/kg, about 13 $\mu$g/kg, about 14 $\mu$g/kg, about 15 $\mu$g/kg, about 16 $\mu$g/kg, about 17 $\mu$g/kg, about 18 $\mu$g/kg, about 19 $\mu$g/kg, 20 $\mu$g/kg. In some examples, the FLT3L is in an amount of about 4 $\mu$g/kg to about 13 $\mu$g/kg. In a preferred example, the FLT3L is in an amount of about 7 $\mu$g/kg.

In some examples, the at least one IL-10 inhibitory agent reprograms an M2 tumour associated macrophage (TAM) into an M1 TAM in the subject, wherein reprogramming comprises a change in surface marker expression, wherein MARCO and CD163 are surface markers expressed on M2 TAM, and wherein CD80 is a surface marker expressed on M1 TAM.

In some examples, the at least one IL-10 inhibitory agent is an interferon gamma (IFNg), an IFNg mimetic, an IFN agonist or a combination thereof. In a preferred example, the at least one IL-10 inhibitory agent is IFNg.

In some examples, the IFNg is administered at a dose of about 10 $\mu$g, about 15 $\mu$g, about 20 $\mu$g, about 25$\mu$g, about 30 $\mu$g, about 35 $\mu$g, about 40 $\mu$g, about 45$\mu$g, about 50 $\mu$g, about 55 $\mu$g, about 60 $\mu$g, about 65$\mu$g, about 70 $\mu$g, about 75$\mu$g, about 80 $\mu$g, about 85 $\mu$g, about 90 $\mu$g, about 95$\mu$g or about 100 $\mu$g. In some examples, the IFN gamma is administered at a dose of about 50 $\mu$g to about 100 $\mu$g. In a preferred example, the IFN gamma is administered at a dose of about 70 $\mu$g.

In some examples, TNFa inhibitor agent is a TNFa receptor (TNFaR), infliximab, adalimumab, certolizumab pegol or golimumab. In a preferred example, the TNFa inhibitor agent is TNFaR. In a preferred example, the TNFaR is etanercept.

In some examples, the TNFaR is administered at a dose of about 1 mg, about 2 mg, about 2 mg, about 3 mg, about 4 mg, about 5 mg, about 6 mg, about 7 mg, about 8 mg, about 9 mg or about 10 mg. In some examples, the TNFaR is administered at a dose of about 4 mg to about 6 mg. In a preferred example, the TNFaR is administered at a dose of about 5 mg.

In some examples, the first infusion, the second infusion, the third infusion, the fourth infusion, the fifth infusion and/or the sixth infusion of the fourth composition, and the first infusion, the second infusion, the third infusion and/or the fourth infusion of the ninth composition comprises about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg about 25 mg/kg, about 26 mg/kg, about 27 mg/kg, about 28 mg/kg, about 29 mg/kg, about 30 mg/kg, about 31 mg/kg, about 32 mg/kg, about 33 mg/kg, about 34 mg/kg or about 35 mg/kg of the recombinant polypeptide comprising an amino acid sequence having at least 95% sequence identity to the polypeptide of SEQ ID NO: 9. In some examples, the first infusion, the second infusion, the third infusion, the fourth infusion, the fifth infusion and the sixth infusion of the fourth composition, and the first infusion, the second infusion, the third infusion and the fourth infusion of the ninth composition comprises about 10 mg/kg to about 30 mg/kg of the recombinant polypeptide comprising an amino acid sequence having at least 95% sequence identity to the polypeptide of SEQ ID NO: 9. In a preferred example, the first infusion, the second infusion, the third infusion, the fourth infusion, the fifth infusion and the sixth infusion of the fourth composition, and the first infusion, the second infusion, the third infusion and the fourth infusion of the ninth composition comprises about 20 mg/kg of the recombinant polypeptide comprising an amino acid sequence having at least 95% sequence identity to the polypeptide of SEQ ID NO: 9.

In some examples, the immunotherapy agent is a checkpoint inhibitor. In a preferred example, the immunotherapy agent is an anti-PD1 antibody. In some examples, the anti-PD1 antibody is nivolumab, pembrolizumab or cemiplimab. In some examples the anti-PD1 antibody is nivolumab.

In some examples, the anti-PD1 antibody is administered at a dose of about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 2 mg/kg, about 3 mg/kg, about 4 mg/kg, about 5mg/kg, about 6mg/kg, about 7 mg/kg, about 8 mg/kg, about 9 mg/kg or about 10 mg/kg. In some examples, the anti-PD1 antibody is administered at a dose of about 0.1 mg/kg to about 7 mg/kg. In a preferred example, the anti-PD1 antibody is administered at a dose of about 3mg/kg.

In some examples, the cancer is a solid tumor cancer. In some examples, the solid tumor has a diameter of at least about 0.2 cm, at least about 0.5 cm, at least about 1 cm, at least about 2 cm, at least about 3 cm, at least about 4 cm, at least about 5 cm, at least about 6 cm, at least about 7 cm, at least about 8 cm, at least about 9 cm or at least about 10 cm.

In some examples, the cancer is a recurrence of an earlier presentation of a cancer or is a metastasis of an earlier presentation of cancer.

Any of the above aspects can be combined with any other aspect in accordance with the claims.

Unless otherwise defined, all technical and scientific terms used herein have the same meaning as commonly understood by one of ordinary skill in the art to which this disclosure belongs.

As used herein, the singular forms of a word also include the plural form of the word, unless the context clearly dictates otherwise; as examples, the terms "a," "an," and "the" are understood to be singular or plural and the term "or" is understood to be inclusive. By way of example, "an element" means one or more element.

Throughout the specification the word "comprising," or variations such as "comprises," will be understood to imply the inclusion of a stated element, integer or step, or group of elements, integers or steps, but not the exclusion of any other element, integer or step, or group of elements, integers or steps. Throughout the specification the word "consisting of," or variations such as "consists of," will be understood to imply the inclusion of a stated element, integer or step, or group of elements, integers or steps, and the exclusion of any other element, integer or step, or group of elements, integers or steps. Throughout the specification the word "consisting essentially of," or variations such as "consists essentially of," will be understood to imply the inclusion of a stated element, integer or step, or group of elements, integers or steps, and any other element, integer or step, or group of elements, integers or steps that do not materially affect the basic and novel characteristics of the claimed invention.

About can be understood as within 10%, 9%, 8%, 7%, 6%, 5%, 4%, 3%, 2%, 1%, 0.5%, 0.1%, 0.05%, or 0.01% of the stated value. Unless otherwise clear from the context, all numerical values provided herein are modified by the term "about."

Although methods and materials similar or equivalent to those described herein can be used in the practice or testing of the present disclosure and in accordance with the claims, suitable methods and materials are described below. The references cited herein are not admitted to be prior art to the claimed disclosure. In the case of conflict, the present Specification, including definitions, will control. In addition, the materials, methods, and examples are illustrative only and are not intended to be limiting. Other features and advantages of the disclosure will be apparent from the following detailed description and claim and in accordance with the claims.

BRIEF DESCRIPTION OF THE DRAWINGS

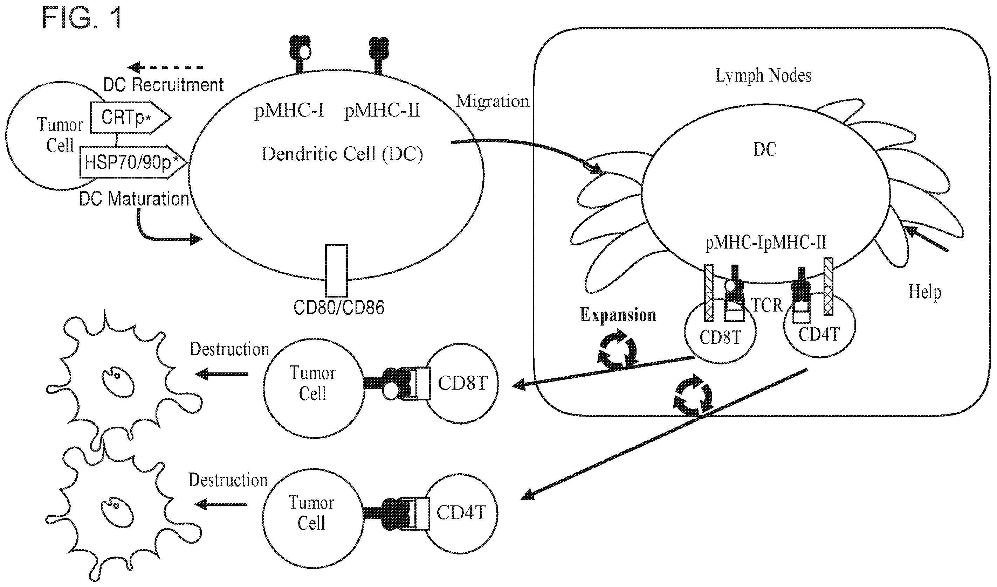

FIG. 1 is a schematic depiction of some characteristics of immunogenic cell death. Tumor cells marked for cell death have cell surface expression of pre-apoptotic Damage-Associated-Molecular-Pattern (DAMP) signals such as calreticulin (CRT), HSP70 and HSP90. Dendritic cells are activated upon the recognition of DAMP signals. Mature dendritic cells migrate to lymph nodes and can in turn prime CD4+ and CD8+ T-cells, which are important for mediating immunogenic cell death.

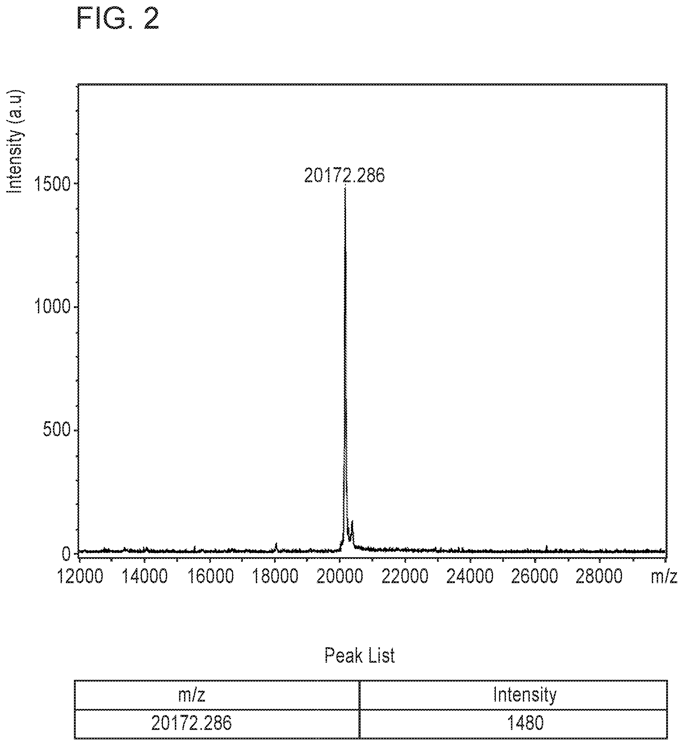

FIG. 2 shows a graph depicting the molecular weight of CRYA_1B recombinant polypeptide (SEQ ID NO: 9) of about 20kDa as determined by mass spectrometry.

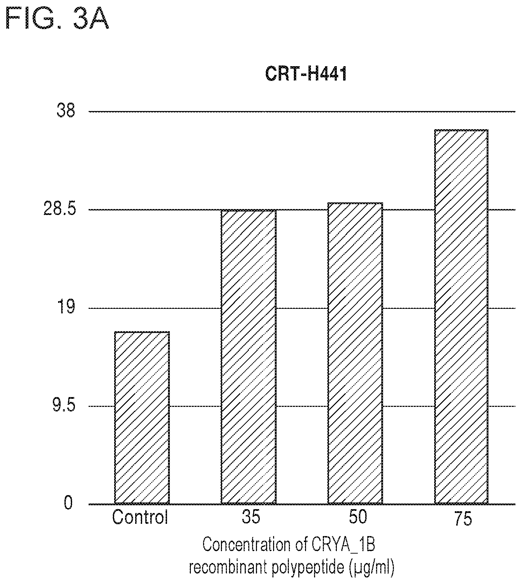

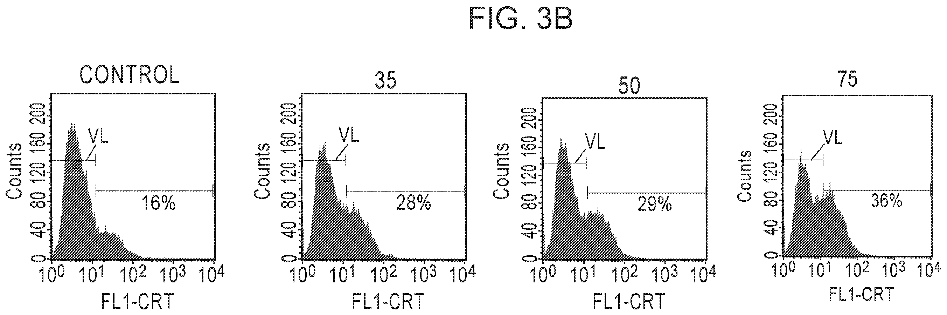

FIG. 3A shows a bar graph quantifying the percentage of cells that express CRT(Caltreticulin) following treatment of H441 human lung cancer cell lines (HTB-174, ATCC) with various concentrations of CRYA_1B recombinant polypeptide (SEQ ID NO: 9). The stock CRYA_1B recombinant polypeptide (SEQ ID NO: 9) was diluted to the final concentration of 35, 50, 75 $\mu$g/ml and the H441 cells were incubated for 1 hour at 37°C. The CRT-expressing H441 cells were determined by FACSCalibur (BD Biosciences) flow cytometry using CRT mAb (Abcam). FIG. 3B shows the flow cytometry profiles used for quantification.

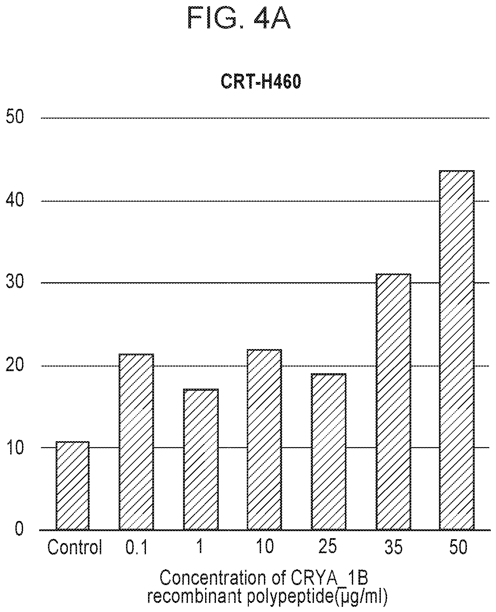

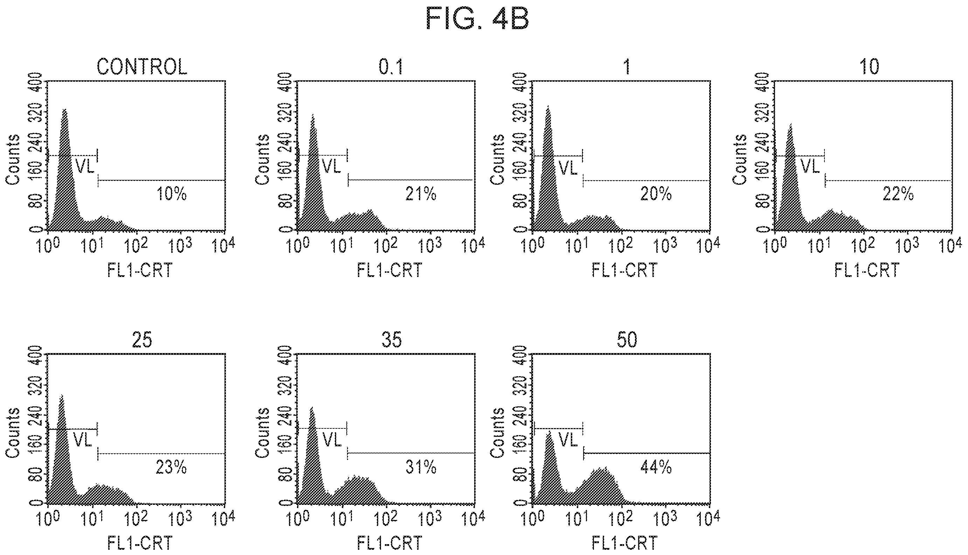

FIG. 4A shows a bar graph quantifying the percentage of cells that express CRT following treatment of H460 human lung cancer cell lines (HTB-177, ATCC) with various concentrations of CRYA_1B recombinant polypeptide (SEQ ID NO: 9). The stock CRYA_1B recombinant polypeptide (SEQ ID NO: 9) was diluted to the final concentration of 0.1, 1, 10, 25, 35, 50 $\mu$g/ml and the H460 cells were incubated for 30 minutes at 37°C. The CRT-expressing H460 cells were determined by FACSCalibur (BD Biosciences) flow cytometry using CRT mAb (Abcam). **FIG. 4B** shows the flow cytometry profiles used for quantification.

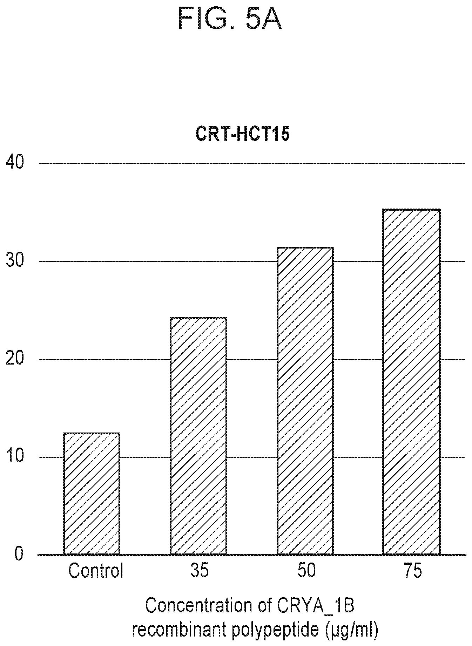

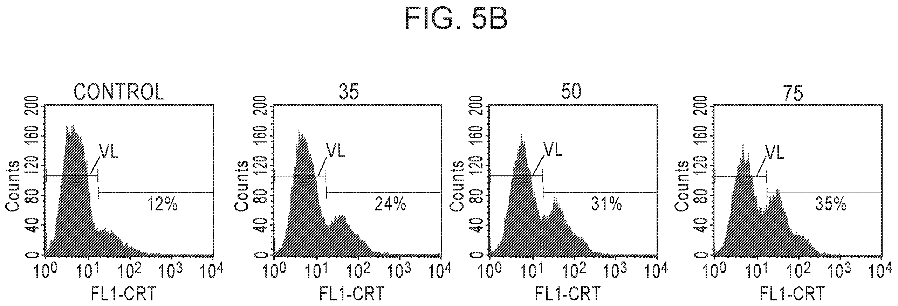

**FIG. 5A** shows a bar graph quantifying the percentage of cells that express CRT following treatment of HCT15 human colon cancer cell lines (CCL-225, ATCC) with various concentrations of CRYA_1B recombinant polypeptide (SEQ ID NO: 9). The stock CRYA_1B recombinant polypeptide (SEQ ID NO: 9) was diluted to the final concentration of 35, 50, 75 μg/ml and the HCT15 cells were incubated for 55 minutes at 37°C. The CRT-expressing HCT15 cells were determined by FACSCalibur (BD Biosciences) flow cytometry using CRT mAb (Abcam). **FIG. 5B** shows the flow cytometry profiles used for quantification.

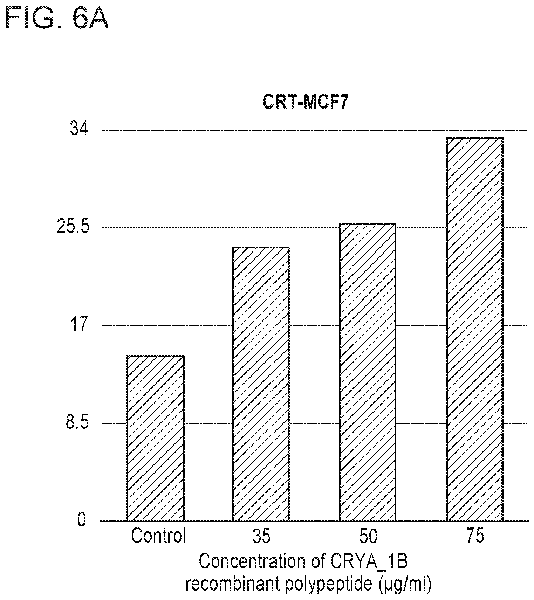

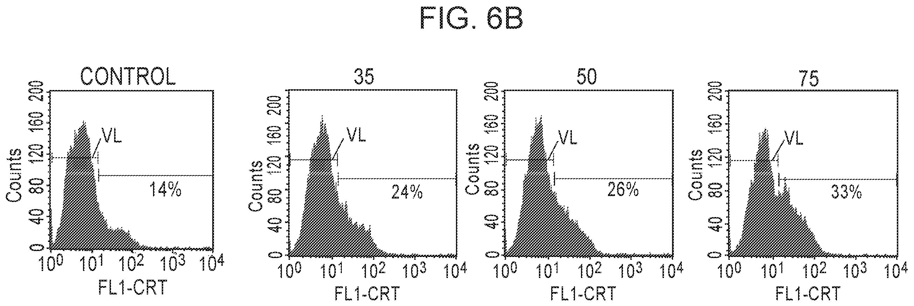

**FIG. 6A** shows a bar graph quantifying the percentage of cells that express CRT following treatment of MCF7 human breast cancer cell lines (HTB-22, ATCC) with various concentrations of CRYA_1B recombinant polypeptide (SEQ ID NO: 9). The stock CRYA_1B recombinant polypeptide (SEQ ID NO: 9) was diluted to the final concentration of 35, 50, 75 μg/ml and the MCF7 cells were incubated for 1 hour and 10 minutes at 37°C. The CRT-expressing MCF7 cells were determined by FACSCalibur (BD Biosciences) flow cytometry using CRT mAb (Abcam). **FIG. 6B** shows the flow cytometry profiles used for quantification.

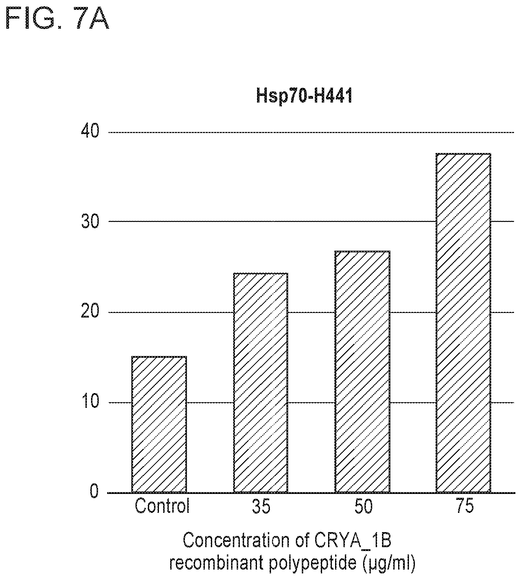

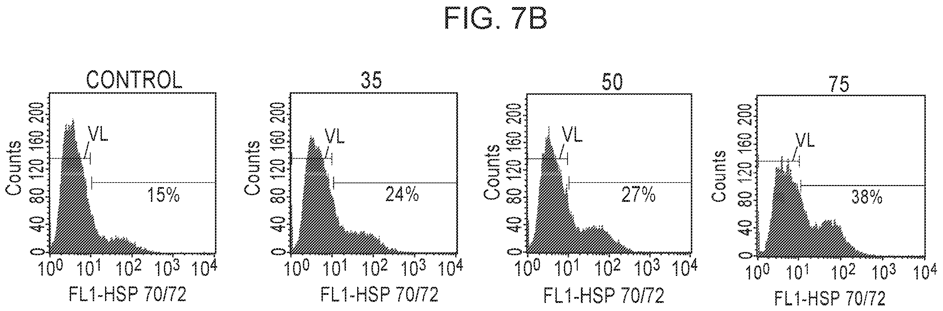

**FIG. 7A** shows a bar graph quantifying the percentage of cells that express HSP70 (70 kDa heat shock protein) following treatment of H441 human lung cancer cell lines (HTB-174, ATCC) with various concentrations of CRYA_1B recombinant polypeptide (SEQ ID NO: 9). The stock CRYA_1B recombinant polypeptide (SEQ ID NO: 9) was diluted to the final concentration of 35, 50, 75 μg/ml and the H441 cells were incubated for 1 hour and 50 minutes at 37°C. The Hsp70-expressing H441 cells were determined by FACSCalibur (BD Biosciences) flow cytometry using Hsp70 mAb (Enzo Life Sciences). **FIG. 7B** shows the flow cytometry profiles used for quantification.

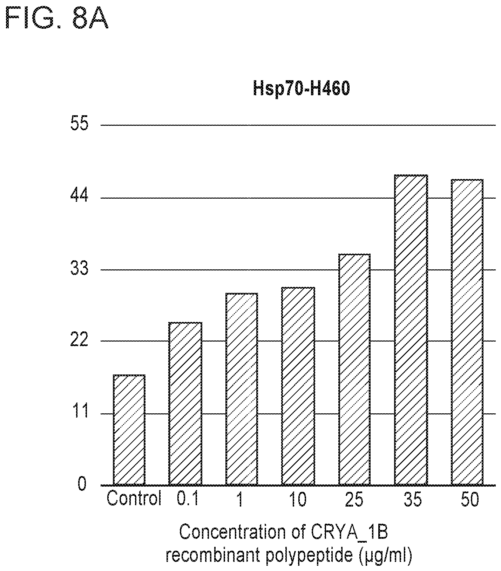

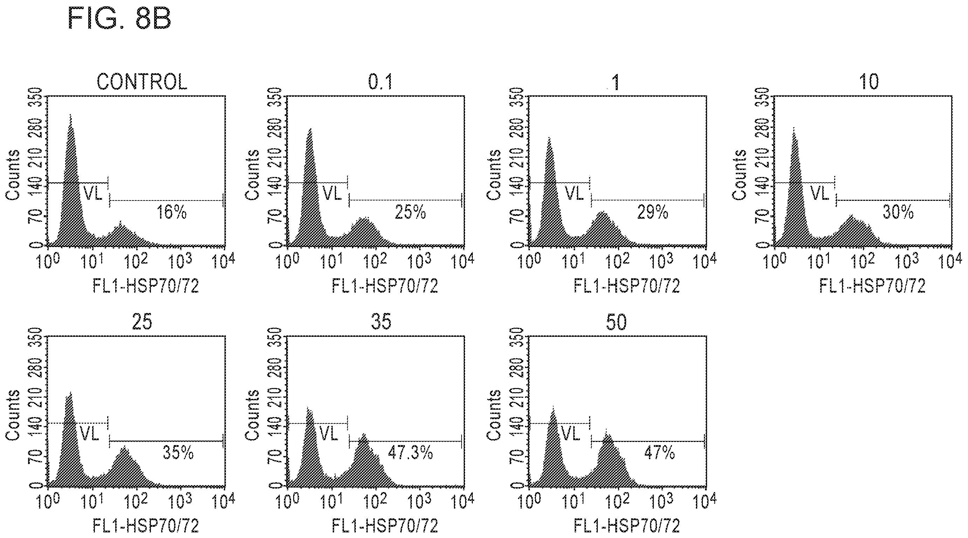

**FIG. 8A** shows a bar graph quantifying the percentage of cells that express HSP70 following treatment of H460 human lung cancer cell lines (HTB-177, ATCC) with various concentrations of CRYA_1B recombinant polypeptide (SEQ ID NO: 9). The stock CRYA_1B recombinant polypeptide (SEQ ID NO: 9) was diluted to the final concentration of 0.1, 1, 10, 25, 35, 50 μg/ml and the H460 cells were incubated for 1 hour and 15 minutes at 37°C. The Hsp70-expressing H460 cells were determined by FACSCalibur (BD Biosciences) flow cytometry using Hsp70 mAb (Enzo Life Sciences). **FIG. 8B** shows the flow cytometry profiles used for quantification.

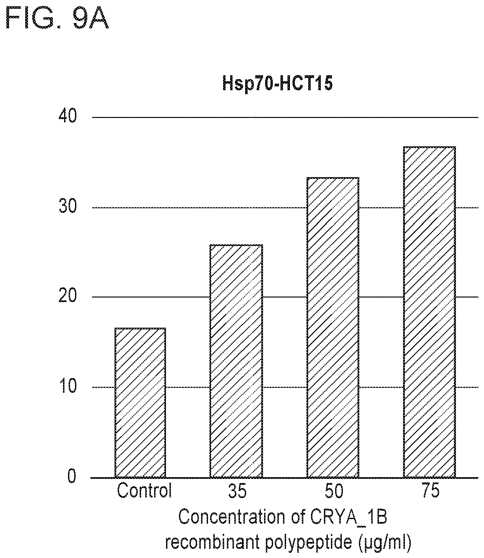

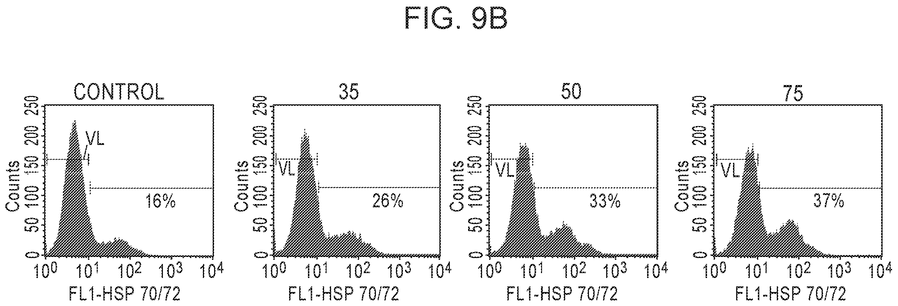

**FIG. 9A** shows a bar graph quantifying the percentage of cells that express HSP70 following treatment of HCT15 human colon cancer cell lines (CCL-225, ATCC) with various concentrations of CRYA_1B recombinant polypeptide (SEQ ID NO: 9). The stock CRYA_1B recombinant polypeptide (SEQ ID NO: 9) was diluted to the final concentration of 35, 50, 75 μg/ml and the HCT15 cells were incubated for 1 hour and 50 minutes at 37°C. The Hsp70-expressing HCT15 cells were determined by FACSCalibur (BD Biosciences) flow cytometry using Hsp70 mAb (Enzo Life Sciences). **FIG. 9B** shows the flow cytometry profiles used for quantification.

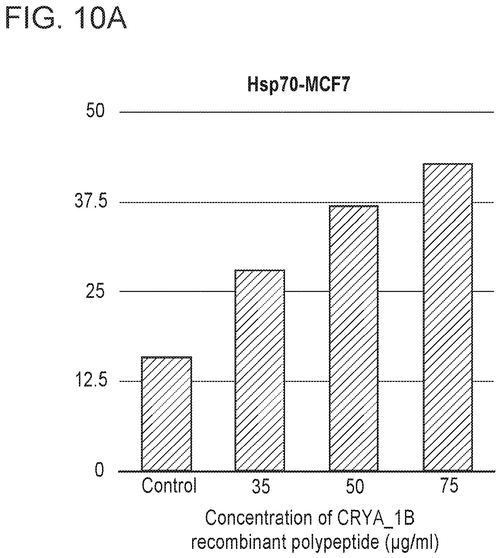

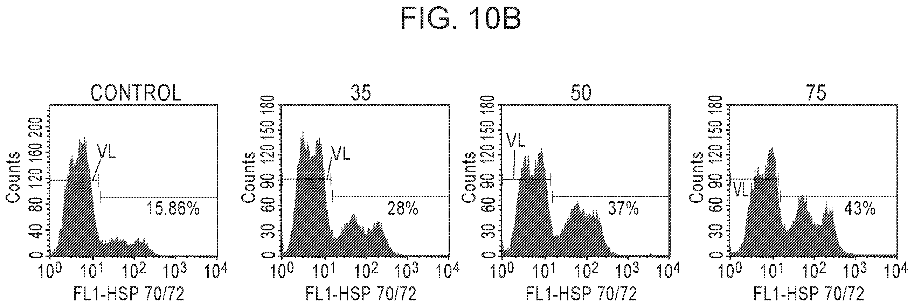

**FIG. 10A** shows a bar graph quantifying the percentage of cells that express HSP70 following treatment of MCF7 human breast cancer cell lines (HTB-22, ATCC) with various concentrations of CRYA_1B recombinant polypeptide (SEQ ID NO: 9). The stock CRYA_1B recombinant polypeptide (SEQ ID NO: 9) was diluted to the final concentration of 35, 50, 75 μg/ml and the MCF7 cells were incubated for 1 hour and 40 minutes at 37°C. The Hsp70-expressing MCF7 cells were determined by FACSCalibur (BD Biosciences) flow cytometry using Hsp70 mAb (Enzo Life Sciences). **FIG. 10B** shows the flow cytometry profiles used for quantification.

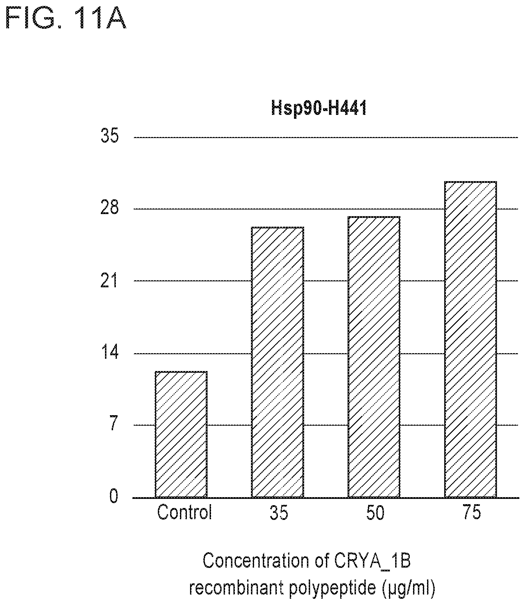

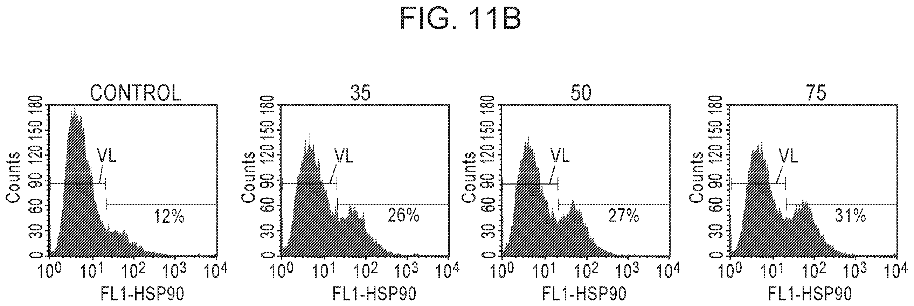

**FIG. 11A** shows a bar graph quantifying the percentage of cells that express HSP90 (90 kDa heat shock protein) following treatment of H441 human lung cancer cell lines (HTB-174, ATCC) with various concentrations of CRYA_1B recombinant polypeptide (SEQ ID NO: 9). The stock CRYA_1B recombinant polypeptide (SEQ ID NO: 9) was diluted to the final concentration of 35, 50, 75 μg/ml and the H441 cells were incubated for 1 hour and 40 minutes at 37°C. The Hsp90-expressing H441 cells were determined by FACSCalibur (BD Biosciences) flow cytometry using Hsp90 mAb (Enzo Life Sciences). **FIG. 11B** shows the flow cytometry profiles used for quantification.

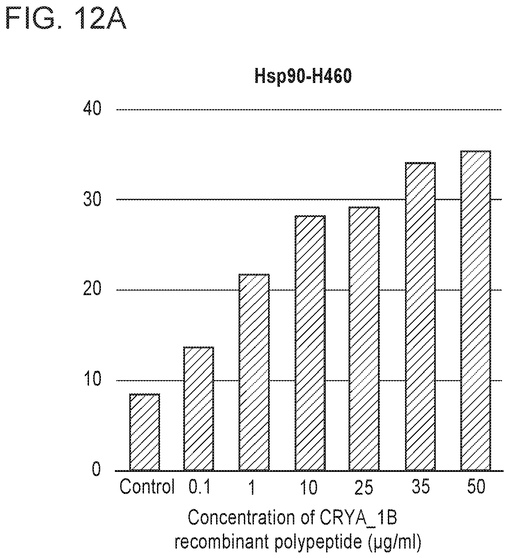

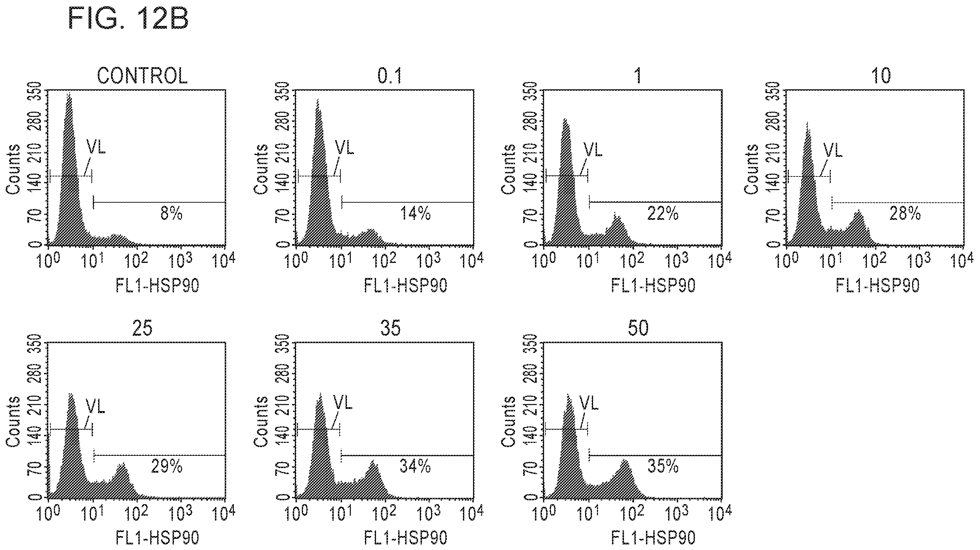

**FIG. 12A** shows a bar graph quantifying the percentage of cells that express HSP90 following treatment of H460 human lung cancer cell lines (HTB-177, ATCC) with various concentrations of CRYA_1B recombinant polypeptide (SEQ ID NO: 9). The stock CRYA_1B recombinant polypeptide (SEQ ID NO: 9) was diluted to the final concentration of 0.1, 1, 10, 25, 35, 50 μg/ml and the H460 cells were incubated for 1 hour and 5 minutes at 37°C. The Hsp90-expressing H460 cells were determined by FACSCalibur (BD Biosciences) flow cytometry using Hsp90 mAb (Enzo Life Sciences). **FIG. 12B** shows the flow cytometry profiles used for quantification.

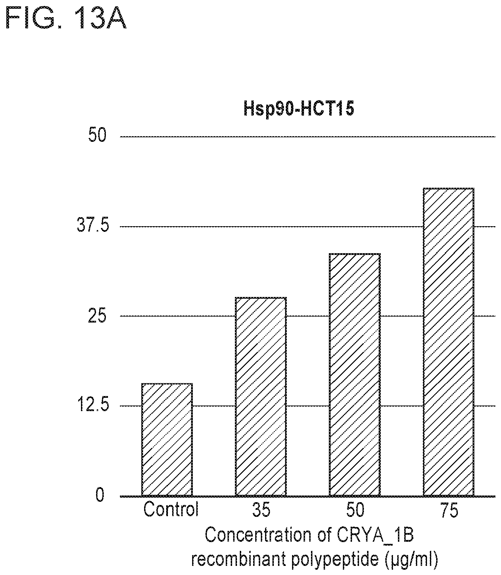

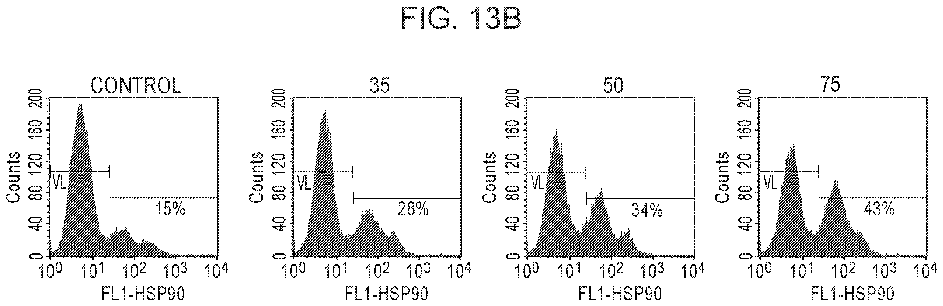

**FIG. 13A** shows a bar graph quantifying the percentage of cells that express HSP90 following treatment of HCT15 human colon cancer cell lines (CCL-225, ATCC) with various concentrations of CRYA_1B recombinant polypeptide (SEQ ID NO: 9). The stock CRYA_1B recombinant polypeptide (SEQ ID NO: 9) was diluted to the final concentration of 35, 50, 75 μg/ml and the HCT15 cells were incubated for 1 hour and 30 minutes at 37°C. The Hsp90-expressing HCT15 cells were determined by FACSCalibur (BD Biosciences) flow cytometry using Hsp90 mAb (Enzo Life Sciences). **FIG. 13B** shows the flow cytometry profiles used for quantification.

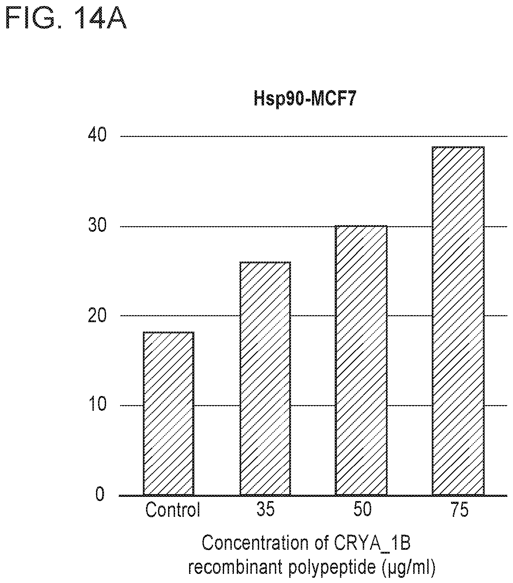

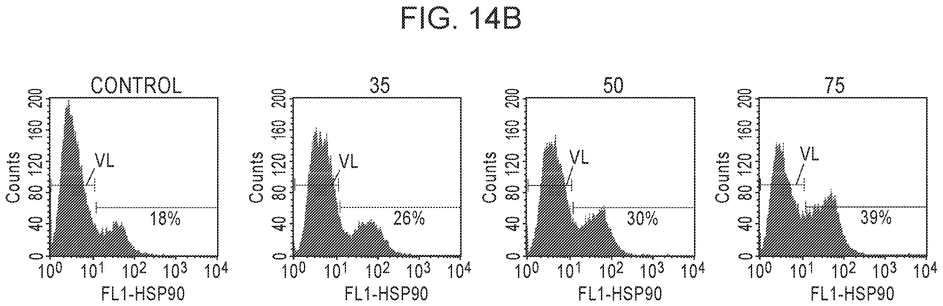

**FIG. 14A** shows a bar graph quantifying the percentage of cells that express HSP90 following treatment of MCF7 human breast cancer cell lines (HTB-22, ATCC) with various concentrations of CRYA_1B recombinant polypeptide (SEQ ID NO: 9). The stock CRYA_1B recombinant polypeptide (SEQ ID NO: 9) was diluted to the final concentration of 35, 50, 75 $\mu$g/ml and the MCF7 cells were incubated for 1 hour and 45 minutes at 37°C. The Hsp90-expressing MCF7 cells were determined by FACSCalibur (BD Biosciences) flow cytometry using Hsp90 mAb (Enzo Life Sciences). **FIG. 14B** shows the flow cytometry profiles used for quantification.

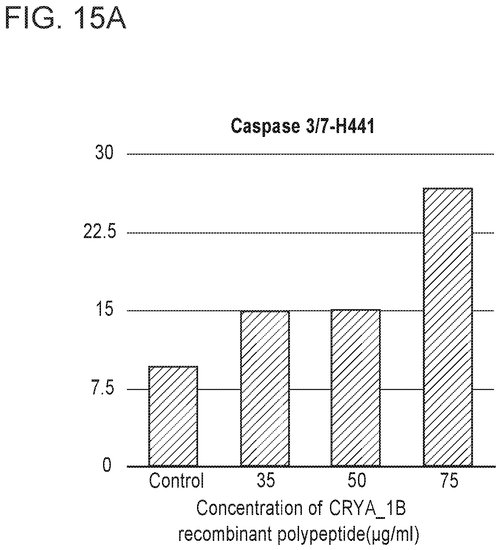

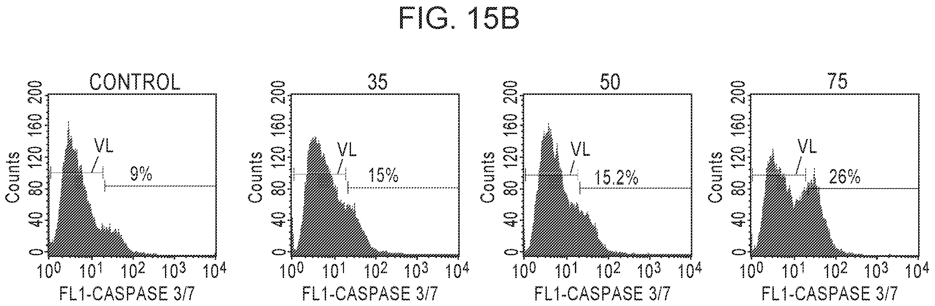

**FIG. 15A** shows a bar graph quantifying the percentage of cells that express Caspase 3/7 following treatment of H441 human lung cancer cell lines (HTB-174, ATCC) with various concentrations of CRYA_1B recombinant polypeptide (SEQ ID NO: 9). The stock CRYA_1B recombinant polypeptide (SEQ ID NO: 9) was diluted to the final concentration of 35, 50 and 75 $\mu$g/ml and the H441 cells were incubated for 2 hours and 45 minutes at 37°C. The H441 cells were determined by FACSCalibur (BD Biosciences) flow cytometry using Caspase 3/7 (Invitrogen) assay. **FIG. 15B** shows the flow cytometry profiles used for quantification.

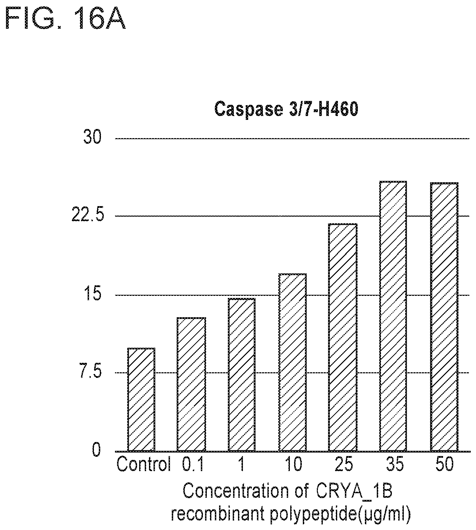

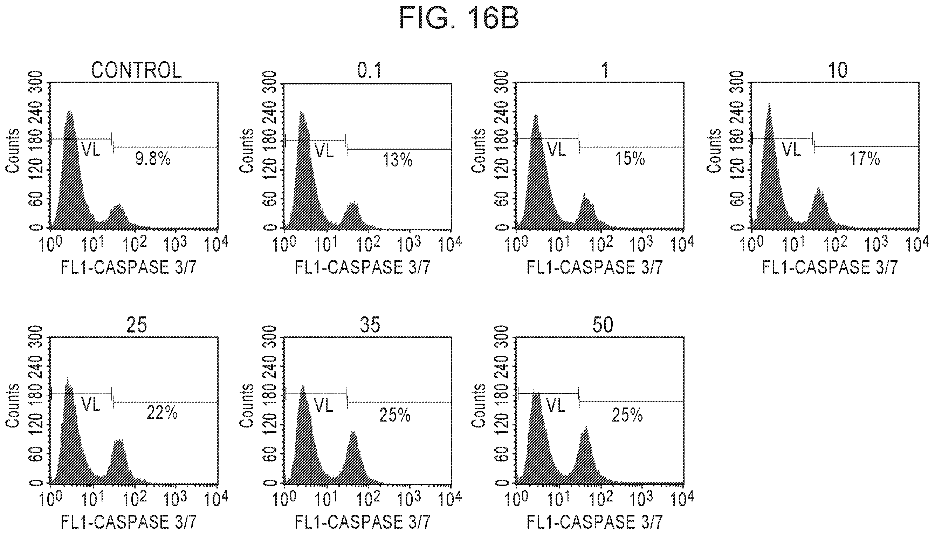

**FIG. 16A** shows a bar graph quantifying the percentage of cells that express Caspase 3/7 following treatment of H460 human lung cancer cell lines (HTB-177, ATCC) with various concentrations of CRYA_1B recombinant polypeptide (SEQ ID NO: 9). The stock CRYA_1B recombinant polypeptide (SEQ ID NO: 9) was diluted to the final concentration of 0.1, 1, 10, 25, 35 and 50 $\mu$g/ml and the H460 cells were incubated for 2 hours and 45 minutes at 37°C. The H460 cells were determined by FACSCalibur (BD Biosciences) flow cytometry using Caspase 3/7 (Invitrogen) assay. **FIG. 16B** shows the flow cytometry profiles used for quantification.

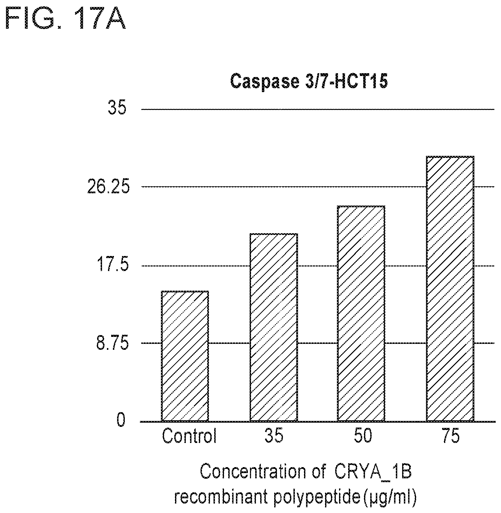

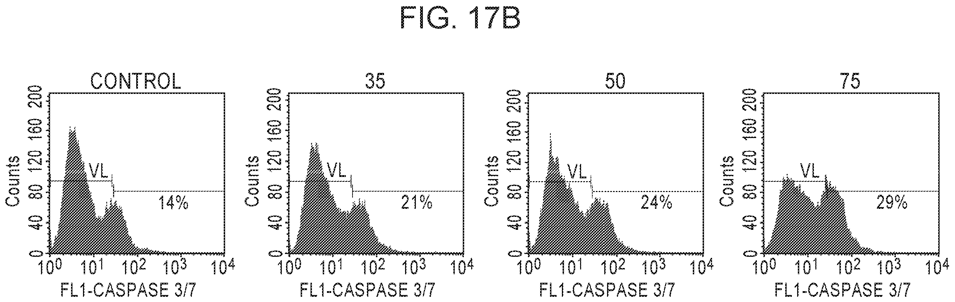

**FIG. 17A** shows a bar graph quantifying the percentage of cells that express Caspase 3/7 following treatment of HCT15 human colon cancer cell lines (CCL-225, ATCC) with various concentrations of CRYA_1B recombinant polypeptide (SEQ ID NO: 9). The stock CRYA_1B recombinant polypeptide (SEQ ID NO: 9) was diluted to the final concentration of 35, 50, 75 $\mu$g/ml and the HCT15 cells were incubated for 2 hours and 30 minutes at 37°C. The HCT15 cells were determined by FACSCalibur (BD Biosciences) flow cytometry using Caspase 3/7 (Invitrogen) assay. **FIG. 17B** shows the flow cytometry profiles used for quantification.

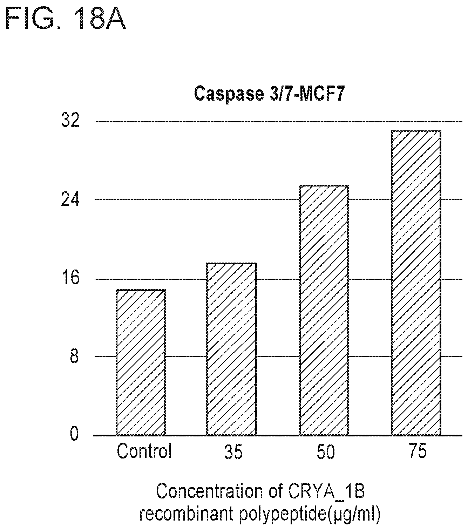

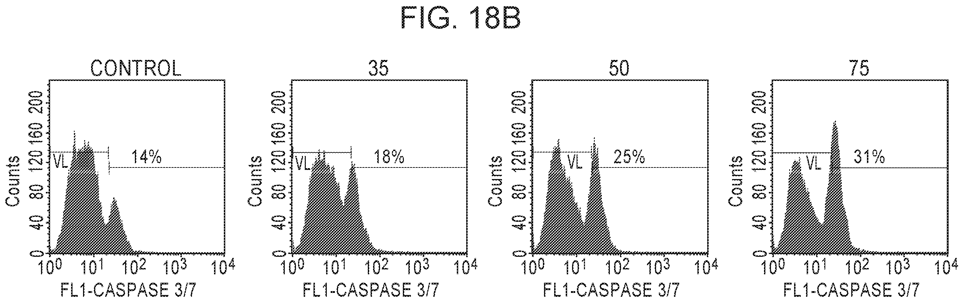

**FIG. 18A** shows a bar graph quantifying the percentage of cells that express Caspase 3/7 following treatment of MCF7 human breast cancer cell lines (HTB-22, ATCC) with various concentrations of CRYA_1B recombinant polypeptide (SEQ ID NO: 9). The stock CRYA_1B recombinant polypeptide (SEQ ID NO: 9) was diluted to the final concentration of 35, 50, 75 $\mu$g/ml and the MCF7 cells were incubated for 2 hours and 45 minutes at 37°C. The MCF7 cells were determined by FACSCalibur (BD Biosciences) flow cytometry using Caspase 3/7 (Invitrogen) assay. **FIG. 18B** shows the flow cytometry profiles used for quantification.

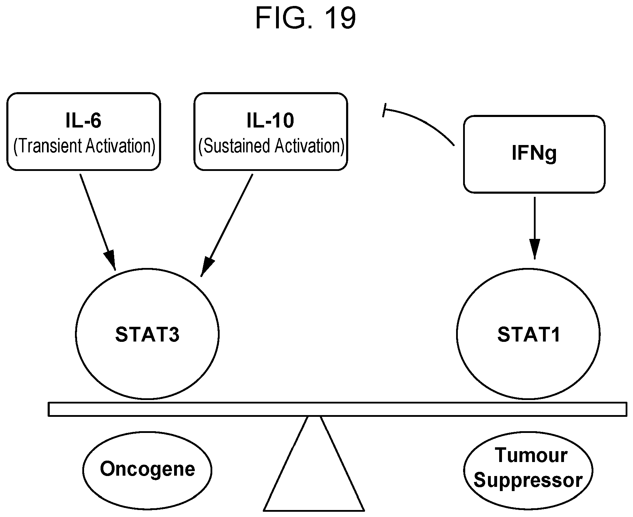

**FIG. 19** shows a schematic diagram of the effects of IL-6, IL-10 and Interferon gamma (IFNg) on STAT3 and STAT1. Human STAT1 has a half-life of ~24 hours. Murine STAT1 has a half-life of ~7.8 hours. The balance of STAT3 and STAT1 affects oncogenesis and tumor suppression, respectively.

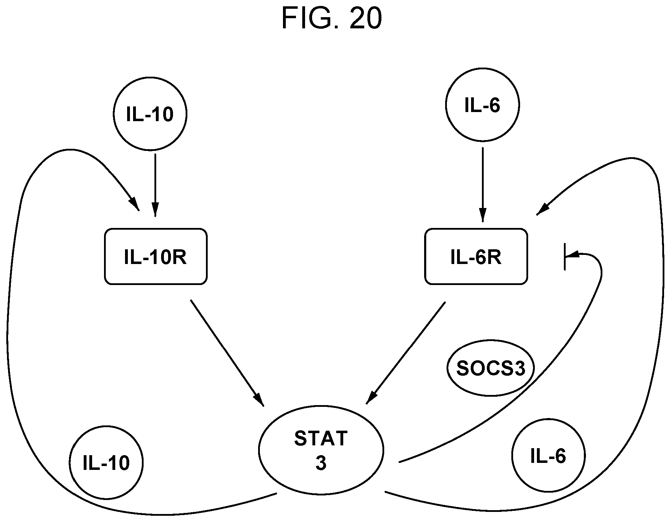

**FIG. 20** shows a schematic diagram of the IL-10 and IL-6 pathways. IL-10 sustains STAT3 activation. IL-6 transiently activates STAT3 with self-inhibiting SOCS3.

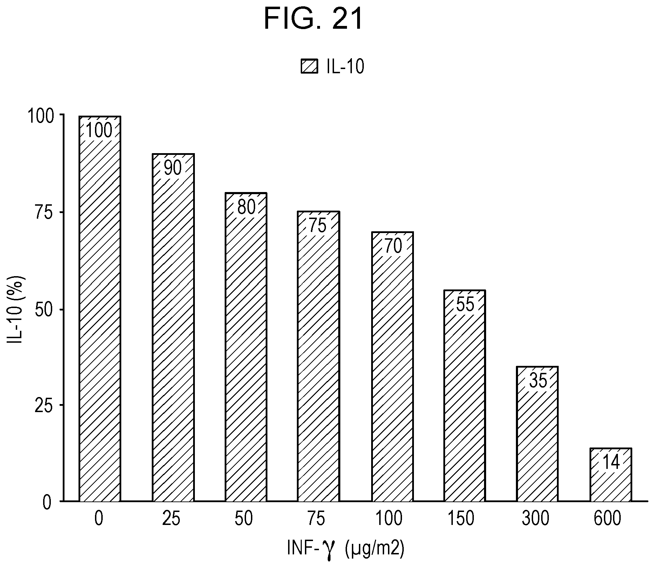

**FIG. 21** shows a bar graph of the measured % serum IL-10 versus the dose of IFN gamma. An intravenous injection of IFNg at varying doses was given to B16F10 tumor bearing mice (n = 2 for each IFNg dose). Blood was sampled 3.5 hours after IFNg injection and serum IL-10 was determined by Luminex assays.

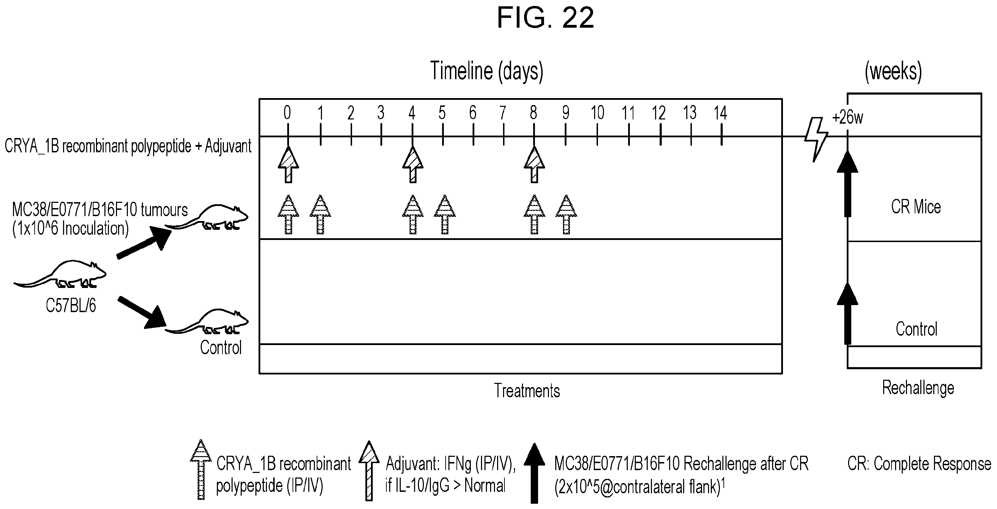

**FIG. 22** shows a schematic diagram of a murine dosage regimen for CRYA_1B recombinant polypeptide (SEQ ID NO: 9). C57BL/6 are challenged with 1x10$^6$ cell inoculation of MC38, E0771 and B16F10 cells, respectively. Horizontally shaded arrows indicate the timing of the intraperitoneal or intravenous injection of the recombinant polypeptide. Diagonally shaded arrows indicate the timing of the intraperitoneal or intravenous injection of IFNg injection. Mice with a complete response after the first treatment regimen are subjected to a rechallenge using an inoculation of 2 x 10$^5$ cells of the same cell type prior to the first treatment regimen.

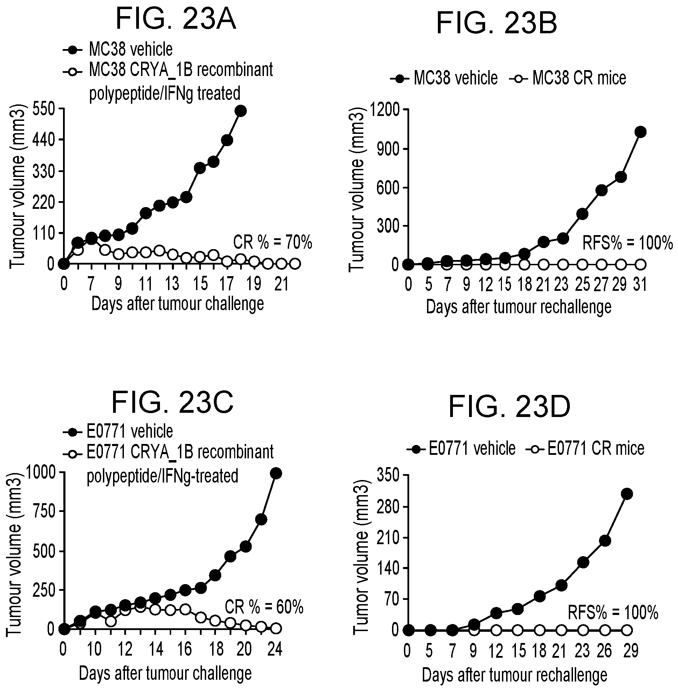

**FIG. 23A-23D** shows a series of line graphs depicting tumor volume in MC38 and E0771 tumor mice after treatment with the recombinant peptide and IFNg. **FIG. 23A** shows a MC38 tumour volume (mm$^3$) and complete response (CR) rate after recombinant polypeptide and IFNg treatment regimen (starting on day 7, n = 10 mice) or vehicle (n = 5 mice) after MC38 tumour challenge (1 x 10$^6$ cells/mouse on day 0). **FIG. 23B** shows a MC38 tumour volume and relapse-free-survival (RFS) rate (n = 12 CR mice pooled from two tumour challenge experiments) or vehicle (n = 5 mice) after MC38 tumour rechallenge (2 × 10$^5$ cells/mouse on day 0: 6 months from CR). **FIG. 23C** shows a E0771 tumour volume and CR rate after recombinant polypeptide CRYA_1B/IFNg treatments (starting on day 10, n = 10 mice) or vehicle (n = 5 mice) after E0771 tumour challenge (1 x 10$^6$ cells/mouse on day 0). **FIG. 23D** shows a E0771 tumour volume and RFS rate (n = 10 CR mice pooled from two tumour challenge experiments) or vehicle (n = 5 mice) after E0771 tumour rechallenge (2 × 10$^5$ cells/mouse on day 0: 6 months from CR). Four independent experiments are shown in FIG. 23A and 23C. Two independent experiments are shown in FIG. 23C and 23D.

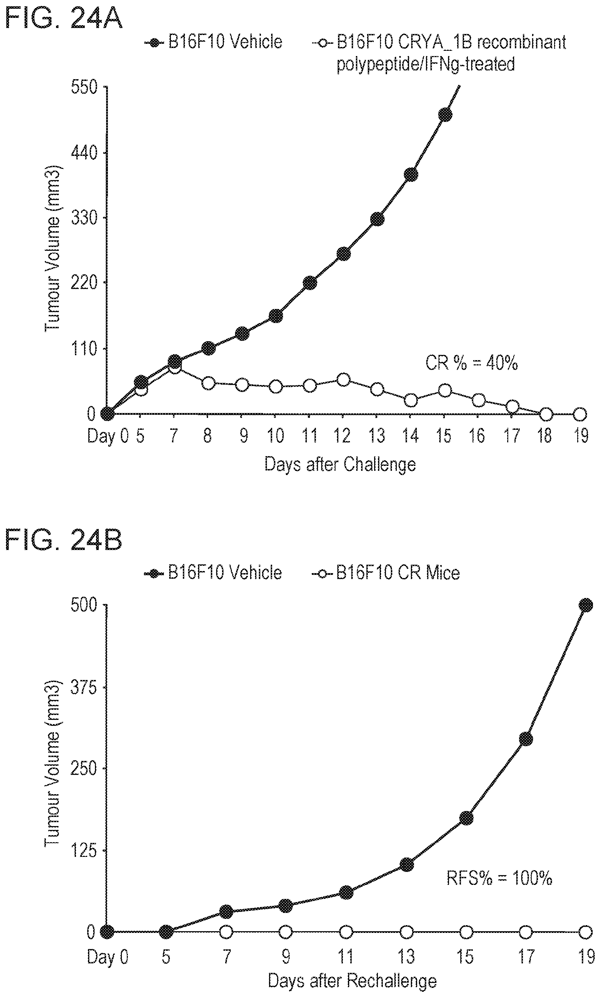

**FIG. 24A-24B** B1610 tumour challenge and rechallenge models in B6 mice. **FIG. 24A** B16F10 tumour volume and complete response (CR) rate after recombinant polypeptide CRYA_1B/IFNg treatments (starting on day 7, n = 10 mice) or vehicle (n = 5 mice) after B16F10 tumour challenge (1 x 10$^6$ cells/mouse on day 0). **FIG. 24B** B16F10 tumour volume and relapse-free-survival (RFS) rate (n = 8 CR mice pooled from two tumour challenge experiments) or vehicle (n = 5 mice) after B16F10 tumour rechallenge (2 × 10$^5$ cells/mouse on day 0: 6 months from CR). Four independent experiments are shown in FIG. 24A. Two independent experiments are shown in FIG. 24B.

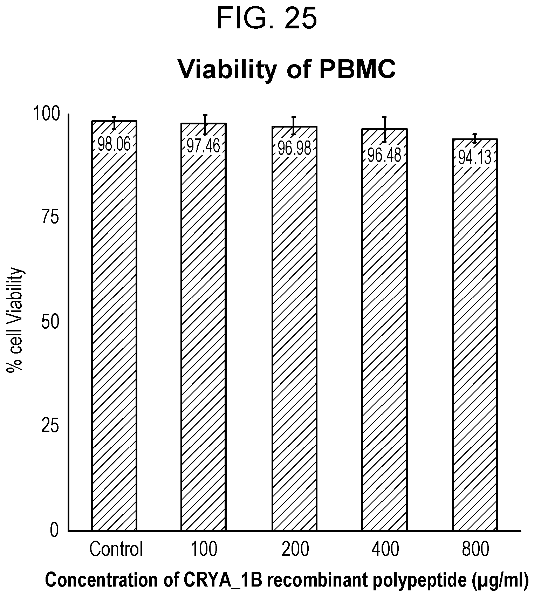

**FIG. 25** PBMC viability. PBMC were incubated in the absence or in the presence of various concentrations of recombinant polypeptide CRYA_1B for 3 hours 45 minutes. Afterwards, the cells were stained with CellEvent™ Caspase 3/7 and analysed by flow cytometry. The PBMC viability is the inverse of apoptosis. Results are one representative data from three independent experiments.

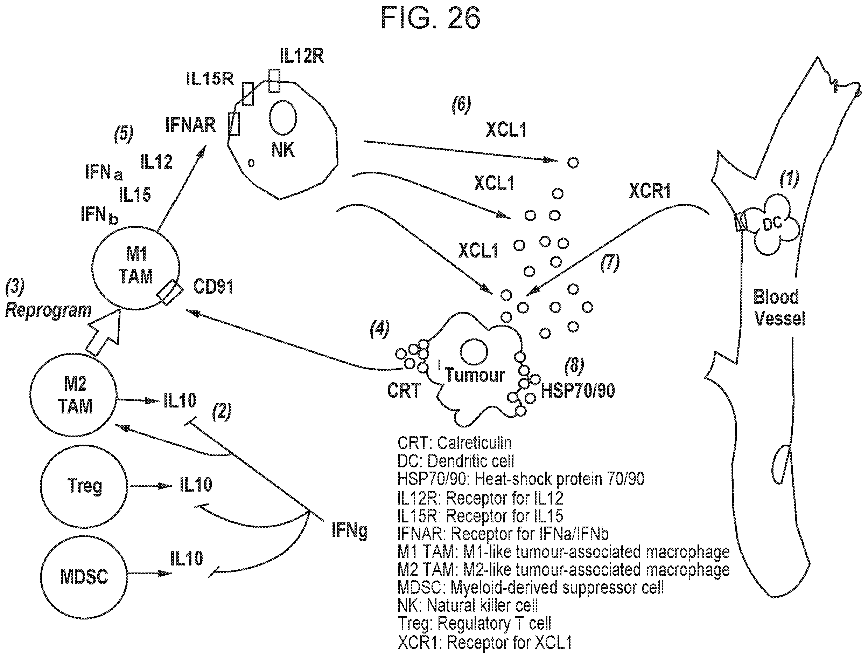

**FIG. 26** is a schematic diagram of a proposed Calreticulin (CRT)-mediated pathway for intratumoral XCR1+ dendritic cells (DC) recruitment. (1) FLT3L expands XCR1+ DC in blood. (2) IFNg reduces IL-10 to reprogram tumour microenvironment for better DC functionality. (3) IFNg simultaneously reprograms M2 tumour-associated macrophages (TAM) to become M1 TAM. (4) CRT-inducer induces CRT from tumours to activate M1 TAM via CD91. (5) Activated M1 TAM produces IL-12, IL-15, IFN alpha (IFNa), and IFN beta (IFNb). (6) IL-12/IL-15/IFNa/IFNb-activated NK produces an abundance of XCL1 (7) XCL1 serves as chemoattractant to recruit XCR1+ DCs from blood vessels to tumour sites. (8) HSP70/90-peptide complexes mature/activate intratumoral XCR1+ DCs via CD40 in order to migrate to lymph nodes with antigens and cross-present antigens to T cells in lymph nodes for T-cell priming/activation/expansion.

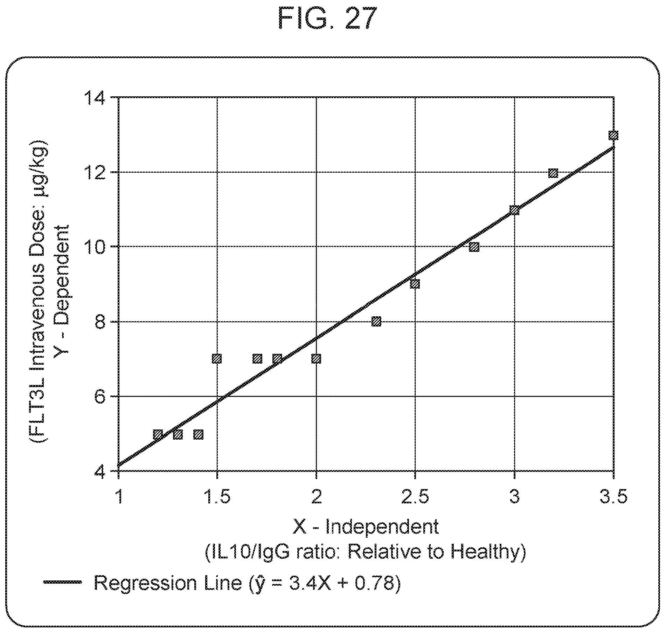

**FIG. 27** is a graph depicting the correlation of FLT3L and IL10/IgG ratio in cancer patients. The twenty advanced-stage lung and pancreatic cancer patients, with pre-treatment serum FLT3L ratio: 0.6-0.8x (relative to healthy), were treated daily with FLT3L intravenous injection for 12 consecutive days at trial doses ($\mu$g/kg) to meet the following criteria. The criteria of HLA-DR+Lin- DCs expansion ratio in PBMC from cancer patients was set as at least 12-fold (similar to that of healthy human) after FLT3L treatment for the determination of necessary FLT3L dose. The regression line was y=3.4x+0.78.

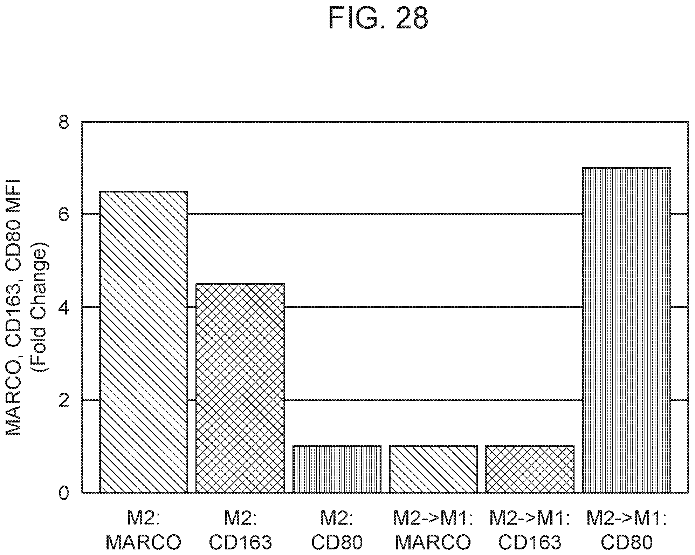

**FIG. 28** is a bar graph showing that treatment of monocytes with IFNgamma can reprogram M2 macrophages into M1 macrophages. Healthy human CD14$^+$ monocytes were cultured in presence of M-CSF (1 $\mu$g/mL), IL-4 (1 $\mu$g/mL), and IL-10 (1 $\mu$g/mL) to differentiate into M2-like macrophage cells. The differentiated M2 macrophage cells were pulsed with IFNg (1 ng/mL) for 1 hour. 4 hours later the cells were analyzed by FACS to determine the proportion of M2 macrophage cells and M1 macrophage cells. M2 macrophages highly express MARCO and CD163 whereas the M1 macrophages highly express CD80. The 3 bars on the left represent M2 macrophage population prior to treatment with IFNgamma. The 3 bars on the right represent the reprogramming to M1 macrophage population following IFNgamma treatment. Only activated M1 macrophages, not M2 macrophages, can produce pro-inflammatory cytokines such as IL-12 and IL-15.

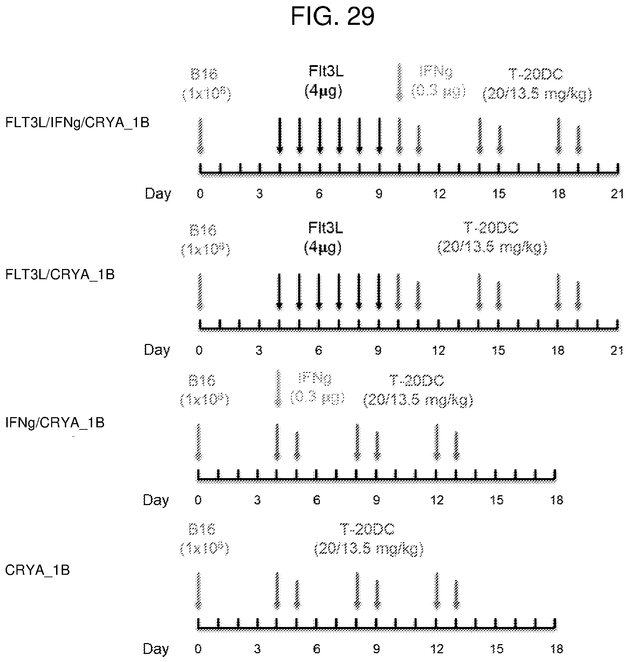

**FIG. 29** is a schematic diagram showing an in vivo dosage regimen for the combination therapy of FLT3L, IFN gamma and recombinant polypeptide CRYA_1B for treatment of melanoma. C57BL/6 mice were subcutaneously inoculated with 1 x 10$^6$ B16F10 cells. Control (n = 5), FLT3L/IFNg/CRYA_1B (n = 10), FLT3L/CRYA_1B (n = 10), IFNg/CRYA_1B (n = 10), and CRYA_1B (n = 10). The tumours reached approximately 40-50 mm3 in four days (Day 4). For FLT3L treatment, the FLT3L was then intravenously injected into mice at 4 $\mu$g (560 $\mu$g/m2) daily for six consecutive days on the days shown in the schematic. For IFNg treatment, the mice were intravenously injected with IFNg at 0.3 $\mu$g four hours before the first CRYA_1B treatment. For CRYA_1B therapy, two-day treatment (20mg/kg and 13.5 mg/kg, respectively via intravenous injections) followed by two-day non-treatment as one cycle, was repeated for three cycles.

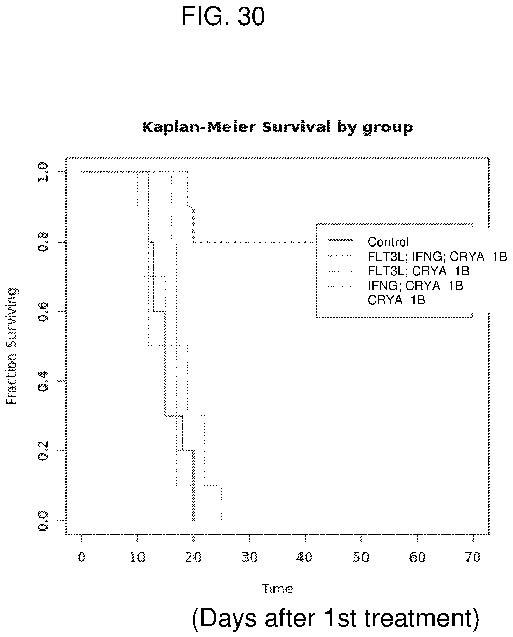

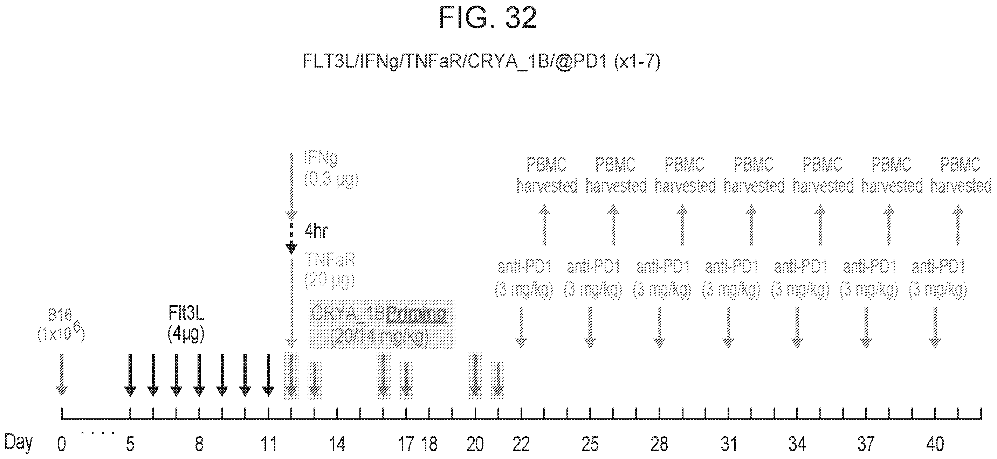

**FIG. 30** is a survival curve of mice treated with combination therapy using FLT3L, IFNg and CRYA_1B (CRT-Inducer). The C57BL/6 mice were subcutaneously inoculated with 1 x 10$^6$ B16F10 poorly immunogenic aggressive melanoma cells for each group: Control (n = 5), FLT3L/IFNg/CRYA_1B (n = 10), FLT3L/CRYA_1B (n = 10), IFNg/CRYA_1B (n = 10), and CRYA_1B (n = 10). Mice were treated according to the dosage regimen shown in FIG. 32. All mice treated with FLT3L, IFNg and CRYA_1B survived.

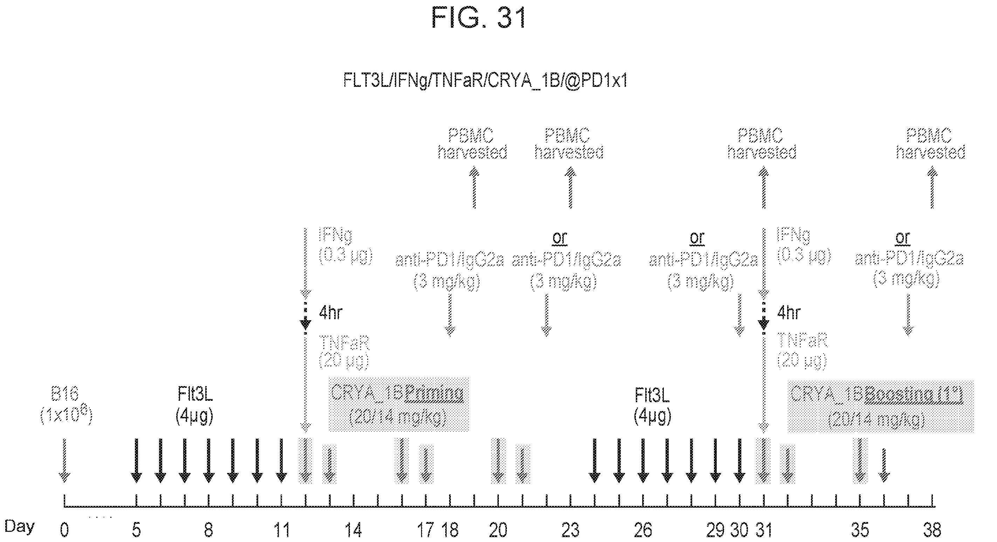

**FIG. 31** is a schematic diagram showing an in vivo dosage regimen for the combination therapy of FLT3L, IFN gamma, TNFaR and recombinant polypeptide CRYA_1B with a single anti-PD1 (nivolumab) blockade for treatment of melanoma. Female C57BL/6 mice were subcutaneously inoculated with 1 x 10$^6$ B16F10 poorly immunogenic aggressive melanoma cells for each of 8 groups: IgG2a groups: FLT3L/IFNg/TNFaR/CRYA_1B with IgG2a@Day 18 (n = 5), FLT3L/IFNg/TNFaR/CRYA_1B with IgG2a@Day 22 (n = 5), FLT3L/IFNg/TNFaR/CRYA_1B with IgG2a@Day 30 (n = 5), and FLT3L/IFNg/TNFaR/CRYA_1B with IgG2a@Day 37 (n = 5); **anti-PD1 groups:** FLT3L/IFNg/TNFaR/CRYA_1B with anti-PD1@Day 18 (n = 5), FLT3L/IFNg/TNFaR/CRYA_1B with anti-PD1@Day 22 (n = 5), FLT3L/IFNg/TNFaR/CRYA_1B with anti-PD1@Day 30 (n = 5), and FLT3L/IFNg/TNFaR/CRYA_1B with anti-PD1@Day 37 (n = 5). For anti-PD1/IgG2a treatments, the mice were administered once intravenously with murine anti-PD1 (Rat IgG2a isotype) for anti-PD1 groups, or Rat IgG2a for Control groups, all at 3 mg/kg on the specified day. The tumours reached approximately 55 mm$^3$ in five days (Day 5). For FLT3L treatment, the FLT3L was then intravenously injected into mice at 4 $\mu$g (560 $\mu$g/m$^2$) daily for seven consecutive days. For IFNg treatment, the mice were intravenously injected with IFNg at 0.3 $\mu$g* four hours before TNFaR injection (20 $\mu$g, via subcutaneous injection) immediately followed by the CRYA_1B treatment. For CRYA_1B therapy to prime T cells, the two-day treatment (20mg/kg and 14 mg/kg, respectively, via intravenous injections) followed by two-day non-treatment as one cycle, was repeated for three cycles, starting on Day 12. For CRYA_1B therapy to boost T cells, the two-day treatment (20mg/kg and 14 mg/kg, respectively via intravenous injections) followed by two-day non-treatment as one cycle, was repeated for two cycles, starting on Day 31. On Days 19, 23, 31and 38, the PBMC of mice from both anti-PD1 and IgG2a groups were harvested for T cell analysis.

**FIG. 32** is a schematic diagram showing an in vivo dosage regimen for the combination therapy of FLT3L, IFN gamma, TNFaR and recombinant polypeptide CRYA_1B with a multiple anti-PD1 (nivolumab) blockade for treatment of melanoma. Female C57BL/6 mice were subcutaneously inoculated with 1 x $10^6$ B16F10 poorly immunogenic aggressive melanoma cells for each of 7 groups: FLT3L/IFNg/TNFaR/CRYA_1B/anti-PD1x1@Day 22 (n = 5), FLT3L/IFNg/TNFaR/CRYA_1B/anti-PD1x2@Day 22 and 25 (n = 5), FLT3L/IFNg/TNFaR/CRYA_1B/anti-PD1x3@Day 22,25,28 (n = 5), FLT3L/IFNg/TNFaR/CRYA_1B/anti-PD1x4@Day 22,25,28,31 (n = 5), FLT3L/IFNg/TNFaR/CRYA_1B/anti-PD1x5@Day 22,25,28,31,34 (n = 5), FLT3L/IFNg/TNFaR/CRYA_1B/anti-PD1x6@Day 22,25,28,31,34,37 (n = 5), and FLT3L/IFNg/TNFaR/CRYA_1B/anti-PD1x7@Day 22,25,28,31,34,37,40 (n = 5). For anti-PD1 treatments, the mice were administered intravenously with murine anti-PD1 (Rat IgG2a isotype), all at 3 mg/kg on the specified day. The tumours reached approximately 55 mm3 in five days (Day 5). For FLT3L treatment, the FLT3L was then intravenously injected into mice at 4 μg (560 μg/m2) daily for seven consecutive days. For IFNg treatment, the mice were intravenously injected with IFNg at 0.3 μg* four hours before TNFaR injection (20 μg, via subcutaneous injection) immediately followed by the CRYA_1B treatment. For CRYA_1B therapy to prime T cells, the two-day treatment (20mg/kg and 14 mg/kg, respectively via intravenous injections) followed by two-day non-treatment as one cycle, was repeated for three cycles, starting on Day 12. On Days 23,26,29,32,35,38, and41, the PBMC of mice from all anti-PD1 groups were harvested for T-cell analysis.

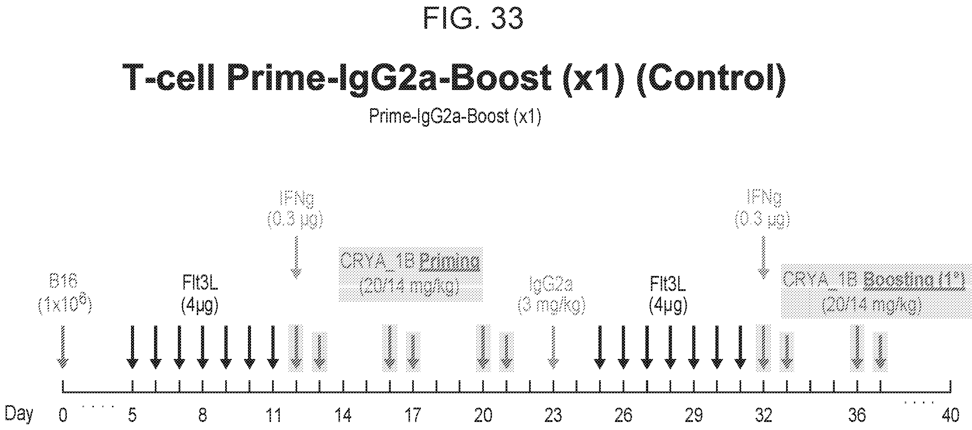

**FIG. 33** is a schematic diagram showing an in vivo dosage regimen for the combination therapy of FLT3L, IFN gamma and recombinant polypeptide CRYA_1B with a IgG2a control for treatment of melanoma. T-cell Prime-IgG2a-Boost (x1) (Control) protocol.

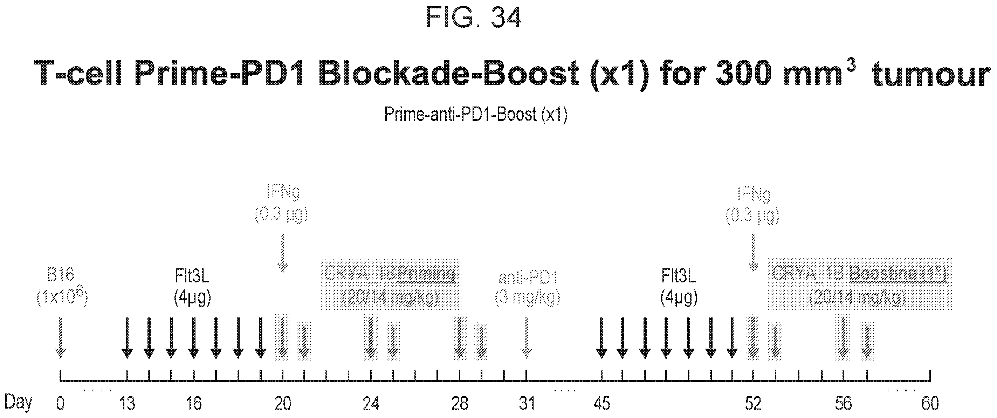

**FIG. 34** is a schematic diagram showing an in vivo dosage regimen for the combination therapy of FLT3L, IFN gamma and recombinant polypeptide CRYA_1B with a single anti-PD1 (nivolumab) blockade control for treatment of melanoma. T-cell Prime-anti-PD1-Boost (x1) protocol for 300mm$^3$.

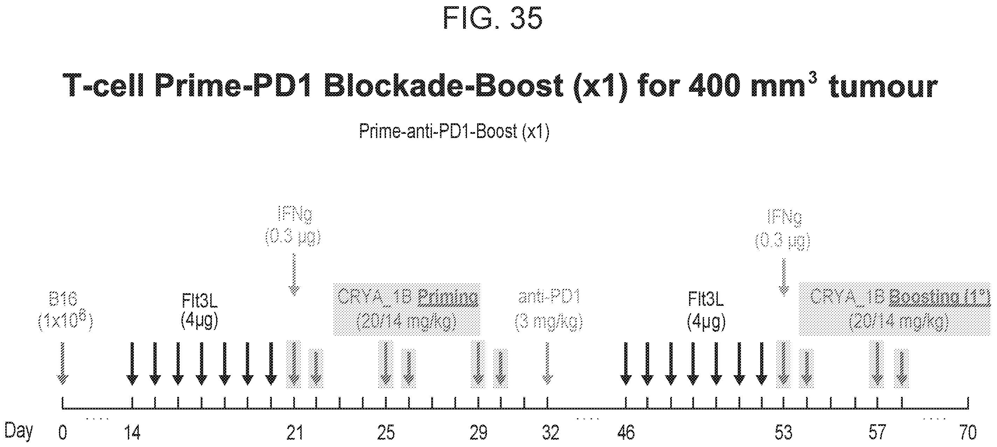

**FIG. 35** is a schematic diagram showing an in vivo dosage regimen for the combination therapy of FLT3L, IFN gamma and recombinant polypeptide CRYA_1B with a single anti-PD1 (nivolumab) blockade control for treatment of melanoma. T-cell Prime-anti-PD1-Boost (x1) protocol for 400mm$^3$.

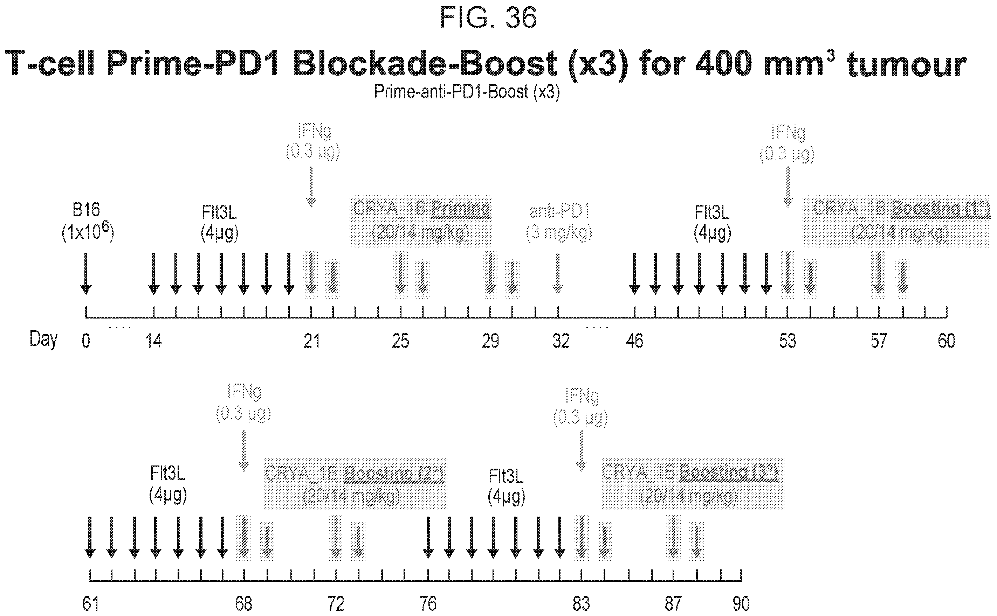

**FIG. 36** is a schematic diagram showing an in vivo dosage regimen for the combination therapy of FLT3L, IFN gamma and recombinant polypeptide CRYA_1B with a single anti-PD1 (nivolumab) blockade control for treatment of melanoma. T-cell Prime-anti-PD1-Boost (x3) protocol for 400mm$^3$.

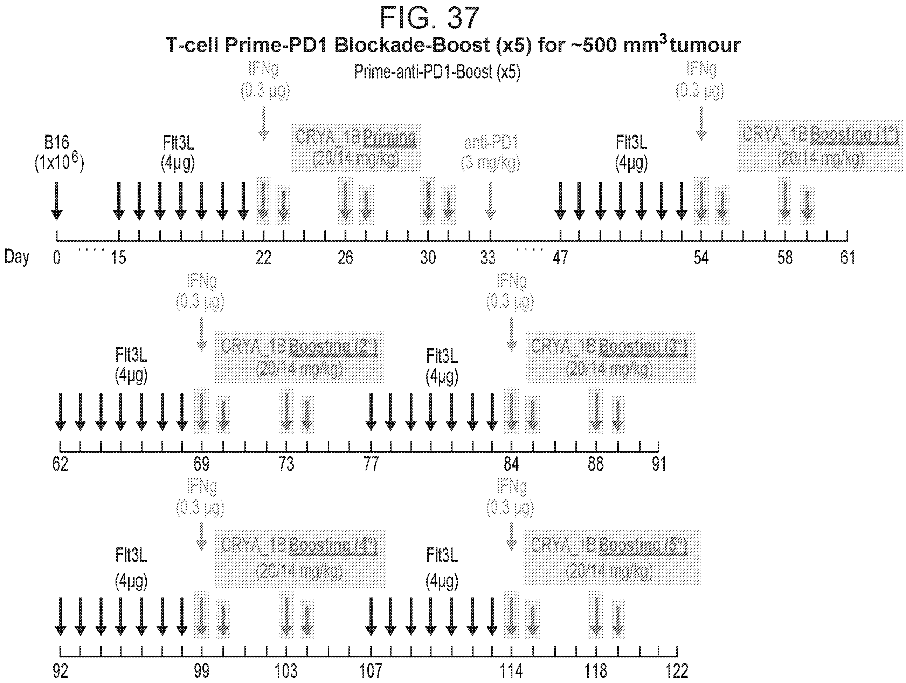

**FIG. 37** is a schematic diagram showing an in vivo dosage regimen for the combination therapy of FLT3L, IFN gamma and recombinant polypeptide CRYA_1B with a single anti-PD1 (nivolumab) blockade control for treatment of melanoma. T-cell Prime-anti-PD1-Boost (x5) protocol for 500mm$^3$.

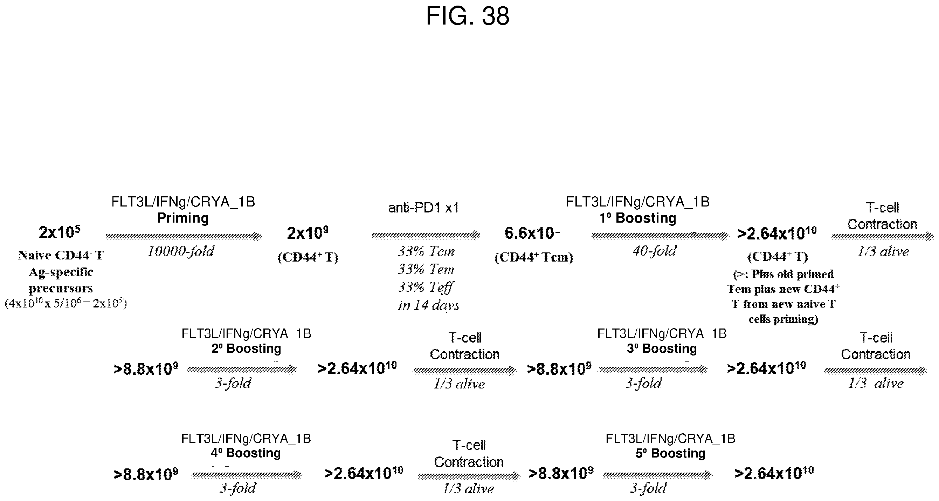

**FIG. 38** is a diagram depicting calculation of the number of antigen-experienced CD44+CD8+ T cells following treatment with the T-cell Prime-anti-PD1-Boost (x5) protocol shown in **FIG. 37.**

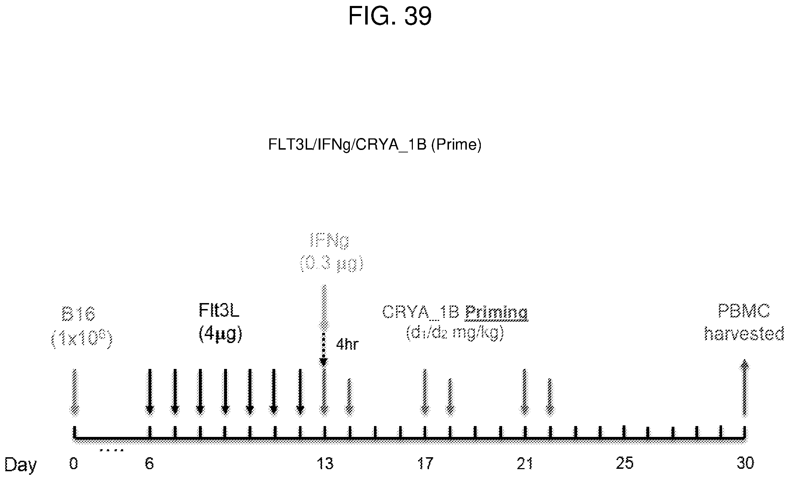

**FIG. 39** is a schematic diagram showing an in vivo dosage regimen for the combination therapy of FLT3L, IFN gamma and recombinant polypeptide CRYA_1B. Female C57BL/6 mice were subcutaneously inoculated with 1 x $10^6$ B16F10 cells (poorly immunogenic aggressive melanoma cells) for FLT3L/IFNg/CRYA_1B regimen using different CRYA_1B doses in first;second day of a treatment cycle ($Dose_{d1}$;$Dose_{d2}$): 25mg/kg;16mg/kg (n = 5), 25mg/kg;15mg/kg (n = 5), 22 mg/kg;18mg/kg (n = 5), 20mg/kg;20mg/kg (n = 5), 20mg/kg;18mg/kg (n = 5), 20mg/kg;16mg/kg (n = 5), 20mg/kg;14mg/kg (n = 5), 20mg/kg;13mg/kg (n = 5), 20mg/kg;12mg/kg (n = 5), 19mg/kg;19mg/kg (n = 5), 18mg/kg;18mg/kg (n = 5) and 18mg/kg;20 mg/kg (n = 5). The tumours reached approximately 80-90 mm$^3$ in six days (Day 6). For FLT3L treatment, the FLT3L was then intravenously injected into mice at 4 μg (560 μg/m$^2$) daily for seven consecutive days. For IFNg treatment, the mice were intravenously injected with IFNg at 0.3 μg* four hours before the first CRYA_1B treatment on Day 13. For CRYA_1B therapy to prime T cells, the two-day treatment ($Dose_{d1}$;$Dose_{d2}$ mg/kg via intravenous injections) followed by two-day non-treatment as one cycle, was repeated for three cycles. On Day 30, the PBMCs in all mice were harvested for T cell analysis.

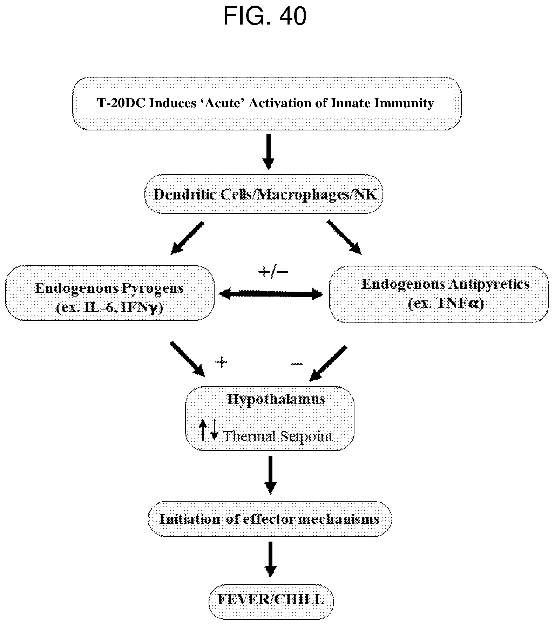

**FIG. 40** is a schematic flow chart showing the downstream effects of the activation of innate immunity by treatment with recombinant polyeptide CRYA_1B.

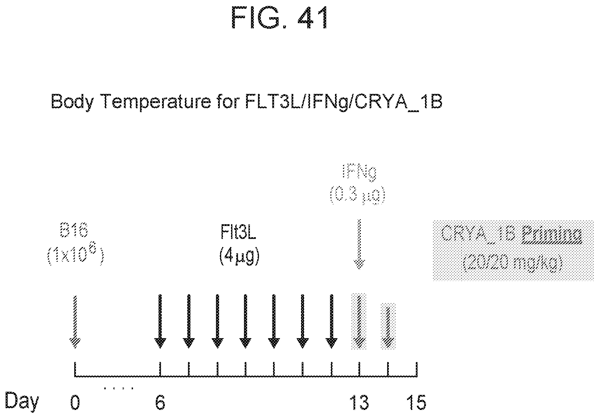

**FIG. 41** is a schematic diagram showing an in vivo dosage regimen for the combination therapy of FLT3L, IFN gamma and recombinant polypeptide CRYA_1B for treatment of melanoma. Body temperature measurement for FLT3/IFNg/-CRYA_1B regimen.

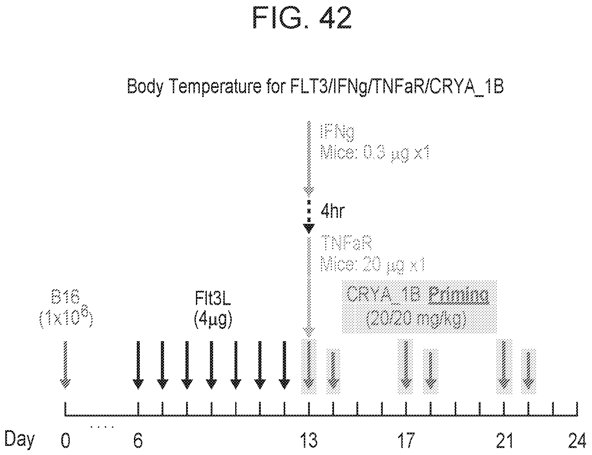

**FIG. 42** is a schematic diagram showing an in vivo dosage regimen for the combination therapy of FLT3L, IFN gamma and recombinant polypeptide CRYA_1B for treatment of melanoma. Body temperature measurement for FLT3/IFNg/TNFaR/CRYA_1B regimen.

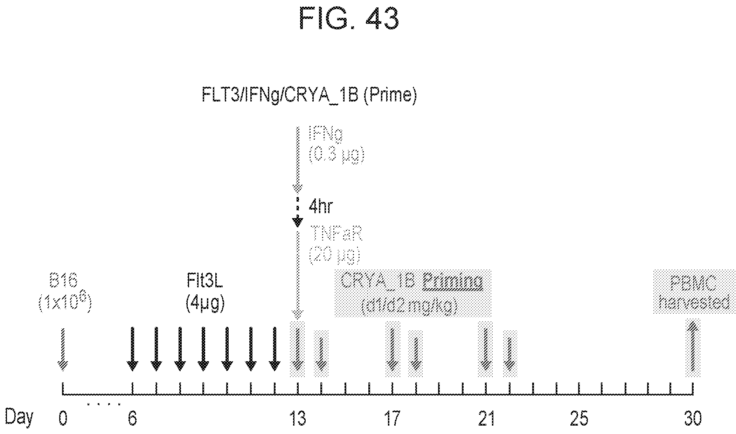

**FIG. 43** is a schematic diagram showing an in vivo dosage regimen for the combination therapy of FLT3L, IFN gamma and recombinant polypeptide CRYA_1B for treatment of melanoma. CRYA_1B dose optimisation with TNFaR. Female C57BL/6 mice were subcutaneously inoculated with $1 \times 10^6$ B16F10 poorly immunogenic aggressive melanoma cells for FLT3L, IFNg, TNFaR, and CRYA_1B regimen using different CRYA_1B doses in first;second day of a treatment cycle ($Dose_{d1}$;$Dose_{d2}$): 25mg/kg;16mg/kg (n = 5), 25 mg/kg ;15 mg/kg (n = 5), 22 mg/kg;18 mg/kg (n = 5), 20 mg/kg;20 mg/kg (n = 5), 20 mg/kg ;18 mg/kg (n = 5), 20 mg/kg ;16 mg/kg (n = 5), 20 mg/kg; 14 mg/kg (n = 5), 20 mg/kg ;13 mg/kg (n = 5), 20 mg/kg ;12 mg/kg (n = 5), 19 mg/kg ;19 mg/kg (n = 5), 18 mg/kg ;18 mg/kg (n = 5) and 18 mg/kg ;20 mg/kg (n = 5). The tumours reached approximately 80-90 mm$^3$ in six days (Day 6). Tumour size was measured in two dimensions using caliper, and the volume was determined in the following formula: $V = 0.5 \times L \times W^2$, where L and W are the long and short diameters of the tumour respectively. For FLT3L treatment, the FLT3L was then intravenously injected into mice at 4 μg (560 μg/m$^2$) daily for seven consecutive days. For IFNg treatment, the mice were intravenously injected with IFNg at 0.3 μg four hours before TNFaR injection (20 μg, via subcutaneous injection) immediately followed by the first CRYA_1B treatment on Day 13. For CRYA_1B therapy to prime T cells, the two-day treatment ($Dose_{d1}$;$Dose_{d2}$ (mg/kg) via intravenous injections) followed by two-day non-treatment as one cycle, was repeated for three cycles. On Day 30, the PBMCs in all mice were harvested for T cell analysis.

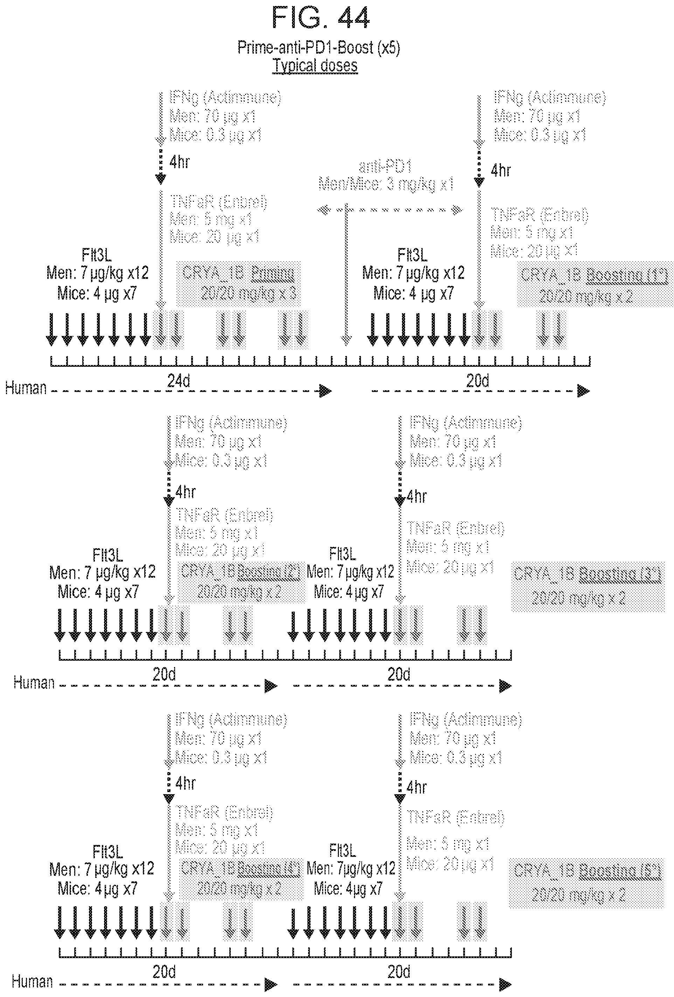

**FIG. 44** is a schematic diagram showing an in vivo dosage regimen for the combination therapy of FLT3L, IFN gamma, TNFaR and recombinant polypeptide CRYA_1B with anti-PD1 (nivolumab) blockade for treatment of melanoma. Prime-PD1 (nivolumab) blockade-Boosts (x5) regimen for metastatic large tumours. The schematic diagram shows a timeline and doses for FLT3L, IFN gamma, TNFaR and CRYA_1B. anti-PD1 is administered at least one time between the last infusion of CRYA_1B in the Priming cycle and the first infusion of FLT3L in the Boosting cycle. Optionally, anti-PD1 may be administered more than once.

## DETAILED DESCRIPTION OF THE INVENTION

The present disclosure provides preventing, delaying the progression of, treating or alleviating a symptom of, or otherwise ameliorating cancer in a subject with a recombinant polypeptide, an IL-10 inhibitory agent, a TNFa inhibitory agent, and an immune cell growth factor for use in accordance with the claims. Optionally further comprising administering an immunotherapy agent in accordance with the claims.

The present disclosure provides recombinant polypeptides that comprise, consist essentially of, or consist of, any of the amino acid sequences shown in Table 1A and in accordance with the claims. The present disclosure provides recombinant polypeptides comprising, consisting essentially of, or consisting of, an amino acid sequence that is at least 95%, about 96%, about 97%, about 98% or about 99% identical to SEQ ID NO. 9.

The present disclosure also provides acidic recombinant polypeptide variants that comprise, consist essentially of, or consist of, any of the amino acid sequences shown in Table 1A, wherein the recombinant polypeptide variant is acidic as determined by isoelectric point (pI). The present disclosure also provides acidic recombinant polypeptide variants comprising, consisting essentially of, or consisting of, an amino acid sequence that is at least 95%, about 96%, about 97%, about 98% or about 99% identical to SEQ ID NO. 9.

An "acidic variant" is a variant of a polypeptide of interest which is more acidic (*e.g.,* as determined by calculation of pI) than the parent or original polypeptide of interest. The "pI" or "isoelectric point" of a polypeptide refers to the pH at which the polypeptide's positive charge balances its negative charge. pI can be calculated by any means known in the art, for example, from the net charge of the amino acid residues of the polypeptide or can be determined by isoelectric focusing.

In some aspects, an acidic variant is derived from the original parent sequence by making amino acid substitutions. A first mutational substitution is made by substituting any basic amino acid (K, R or H), neutral non-polar amino acid (G, A, V, L, I, M, F, W or P) or neutral polar amino acid (S, T, C, Y, N or Q) of the original parent sequence with an acidic amino acid (D or E). A second mutational substitution is made by making the inverse mutational substitution of the first mutational substitution. For example, all serine (S) residues from original parent sequence are substituted with glutamic acid (E) residues (first substitution). In addition, all glutamic acid (E) residues from the original parent sequence are substituted with serine (S) residues (second substitution). In one aspect, the inverse substitutions comprise, consist essentially of or consist of the mutation of all serine (S) residues of the original parent sequence to glutamic acid (E) and the mutation of all glutamic acid (E) residues of original parent sequence to serine (S) residues; the mutation of all serine (S) residues of the original parent sequence to aspartic acid (D) and the mutation of all aspartic acid (D) residues of the original parent sequence to serine (S) residues; the mutation of all valine (V) residues of the original parent sequence to aspartic acid (D) and the mutation of all aspartic acid (D) of the original parent sequence to valine (V) residues; or the mutation of all serine (S) residues of the original parent sequence to leucine (L) residues and the mutation of all leucine (L) residues of the original parent sequence to serine (S) residues. In a preferred aspect, the amino acid substitutions result in a recombinant polypeptide where aspartic acid (D), glutamic acid (E) and leucine (L) are each independently present in an amount greater than, or equal to, the amount of any other amino acid residue present within the recombinant polypeptide sequence. In a preferred aspect, the amino acid substitutions result in a recombinant polypeptide with leucine (L), aspartic acid (D) and glutamic acid (E) as the three most abundant amino acid residues of the acidic variant or as greater than or equal to in abundance to the next most abundant amino acid residue of the acidic variant. In some aspects, multiple inverse mutational substitutions of amino acids can be made.

The present disclosure also provides acidic recombinant polypeptide variants that comprise, consist essentially of, or consist of, any of the amino acid sequences shown in Table 1A and in accordance with the claims, wherein the recombinant polypeptide variant is acidic as determined by isoelectric point (pI), wherein the pI of the recombinant peptide variant is lower than the pI of peptide sequence from which the recombinant peptide was derived, and wherein leucine (L), aspartic acid (D) and glutamic acid (E) are each independently present in an amount greater than, or equal to, the amount of any other amino acid residue present within the recombinant polypeptide sequence. The present disclosure also provides acidic recombinant polypeptide variants that comprise, consist essentially of, or consist of, any of the amino acid sequences shown in Table 1A, wherein the recombinant polypeptide variant is acidic as determined by isoelectric point (pI) and wherein leucine (L), aspartic acid (D) and glutamic acid (E) are the three most abundant amino acid residues of the acidic variant or are greater than or equal to in abundance to the next most abundant amino acid residue of the acidic variant. The present disclosure also provides acidic recombinant polypeptide variants comprising, consisting essentially of, or consisting of, an amino acid sequence that is at least 95%, about 96%, about 97%, about 98% or about 99% identical to SEQ ID NO: 9.

**Table 1A.** Recombinant Polypeptide Sequences

| Name | Amino Acid Sequence | SEQ ID NO: |
|---|---|---|
| *Anser cygnoides domesticus* CRYAA | MDITIQHPWFKRALGPLIPS RLFDQFFGEGLLEYDLLPLF SSTISPYYRQSLFRSVLESG ISEVRSDRDKFTIMLDVKHF SPEDLSVKIIDDFVEIHGKH SERQDDHGYISREFHRRYRL PANVDQSAITCSLSGDGMLT FSGPKVPSNMDPTHSERPIP VSREEKPTSAPSS | 1 |
| *Rhea americana* CRYAA | MDITIQHPWFKRALGPLIPS RLFDQFFGEGLLEYDLLPLF SSTISPYYRQSLFRSVLESG ISEVRSDREKFTIMLDVKHF SPEDLSVKIIDDFVEIHGKH SERQDDHGYISREFHRRYRL PSNVDQSAITCSLSSDGMLT FSGPKVQANMDPSHSERPIP VSREEKPTSAPSS | 2 |
| *Anas platyrhynchos* CRYAA | RALGPLIPSRLFDQFFGEGL LEYDLLPLFSSTISPYYRQS LFRSVLESGISEVRSDRDKF TIMLDVKHFSPEDLSVKIID DFVEIHGKHSERQDDHGYIS REFHRRYRLPANVDQSAITC SLSGDGMLTFSGPKVPSNMD PTHSERPIP | 3 |
| *Anas platyrhynchos* CRYAB | MDITIHNPLIRRPLFSWLAP SRIFDQIFGEHLQESELLPA SPSLSPFLMRSPIFRMPSWL ETGLSEMRLEKDKFSVNLDV KHFSPEELKVKVLGDMVEIH GKHEERQDEHGFIAREFNRK YRIPADVDPLTITSSLSLDG VLTVSAPRKQSDVPERSIPI TREEKPAIAGAQRK | 4 |

(continued)

| Name | Amino Acid Sequence | SEQ ID NO: |
|---|---|---|
| *Homo sapiens* CRYAA | MDVTIQHPWFKRTLGPFYPS RLFDQFFGEGLFEYDLLPFL SSTISPYYRQSLFRTVLDSG ISEVRSDRDKFVIFLDVKHF SPEDLTVKVQDDFVEIHGKH NERQDDHGYISREFHRRYRL PSNVDQSALSCSLSADGMLT FCGPKIQTGLDATHAERAIP VSREEKPTSAPSS | 5 |
| *Drosophila melanogaster* HSP23 | MANIPLLLSLADDLGRMSMV PFYEPYYCQRQRNPYLALVG PMEQQLRQLEKQVGASSGSS GAVSKIGKDGFQVCMDVSHF KPSELVVKVQDNSVLVEGNH EEREDDHGFITRHFVRRYAL PPGYEADKVASTLSSDGVLT IKVPKPPAIEDKGNERIVQI QQVGPAHLNVKENPKEAVEQ DNGNDK | 6 |
| *Drosophila melanogaster* HSP22 | MRSLPMFWRMAEEMARMPRL SSPFHAFFHEPPVWSVALPR NWQHIARWQEQELAPPATVN KDGYKLTLDVKDYSELKVKV LDESVVLVEAKSEQQEAEQG GYSSRHFLGRYVLPDGYEAD KVSSSLSDDGVLTISVPNPP GVQETLKEREVTIEQTGEPA KKSAEEPKDKTASQ | 7 |
| *Anser cygnoides domesticus* CRYAB | MDITIHNPLIRRPLFSWLAP SRIFDQIFGEHLQESELLPA SPSLSPFLMRSPIFRMPSWL ETGLSEMRLEKDKFSVNLDV KHFSPEELKVKVLGDMVEIH GKHEERQDEHGFIAREFNRK YRIPADVDPLTITSSLSLDG VLTVSAPRKQSDVPERSIPI TREEKPAIAGAQRK | 8 |

In a preferred aspect, the present disclosure provides recombinant polypeptides that comprise, consist essentially of, or consist of, any of the amino acid sequences shown in Table 1B and in accordance with the claims. The present disclosure provides recombinant polypeptides that have an amino acid sequence that is at least 95%, about 96%, about 97%, about 98% or about 99% identical to SEQ ID NO: 9 in Table 1B.

**Table 1B.** Recombinant Polypeptide Sequences

| Name | Amino Acid Sequence | SEQ ID NO: |
|---|---|---|
| CRYA_1B<br>*Anser cygnoides domesticus*<br>CRYAA | MDITIQHPWFKRALGPLIPE<br>RLFDQFFGSGLLSYDLLPLF<br>EETIEPYYRQELFREVLSEG<br>IESVREDRDKFTIMLDVKHF<br>EPSDLEVKIIDDFVSIHGKH<br>ESRQDDHGYIERSFHRRYRL<br>PANVDQEAITCELEGDGMLT<br>FEGPKVPENMDPTHESRPIP<br>VERSSKPTEAPEE | 9 |
| *Rhea americana*<br>CRYAA | MDITIQHPWFKRALGPLIPE<br>RLFDQFFGSGLLSYDLLPLF<br>EETIEPYYRQELFREVLSEG<br>IESVREDRSKFTIMLDVKHF<br>EPSDLEVKIIDDFVSIHGKH<br>ESRQDDHGYIERSFHRRYRL<br>PENVDQEAITCELEEDGMLT<br>FEGPKVQANMDPEHESRPIP<br>VERSSKPTEAPEE | 10 |
| *Anas platyrhynchos*<br>CRYAA | RALGPLIPERLFDQFFGSGL<br>LSYDLLPLFEETIEPYYRQE<br>LFREVLSEGIESVREDRDKF<br>TIMLDVKHFEPSDLEVKIID<br>DFVSIHGKHESRQDDHGYIE<br>RSFHRRYRLPANVDQEAITC<br>ELEGDGMLTFEGPKVPENMD<br>PTHESRPIP | 11 |
| *Anas platyrhynchos*<br>CRYAB | MSITIHNPLIRRPLFDWLAP<br>DRIFSQIFGEHLQEDELLPA<br>DPDLDPFLMRDPIFRMPDWL<br>ETGLDEMRLEKSKFDVNLSV<br>KHFDPEELKVKVLGSMVEIH<br>GKHEERQSEHGFIAREFNRK<br>YRIPASVSPLTITDDLDLSG<br>VLTVDAPRKQDSVPERDIPI<br>TREEKPAIAGAQRK | 12 |
| *Homo sapiens*<br>CRYAA | MDVTIQHPWFKRTLGPFYPE<br>RLFDQFFGSGLFSYDLLPFL<br>EETIEPYYRQELFRTVLDEG<br>IESVREDRDKFVIFLDVKHF<br>EPSDLTVKVQDDFVSIHGKH<br>NSRQDDHGYIERSFHRRYRL<br>PENVDQEALECELEADGMLT<br>FCGPKIQTGLDATHASRAIP<br>VERSSKPTEAPEE | 13 |

(continued)

| Name | Amino Acid Sequence | SEQ ID NO: |
|---|---|---|
| *Drosophila melanogaster* HSP23 | MANIPLLLSLAVVLGRMSMD PFYEPYYCQRQRNPYLALDG PMEQQLRQLEKQDGASSGSS GADSKIGKVGFQDCMVDSHF KPSELDDKDQVNSDLDEGNH EEREVVHGFITRHFDRRYAL PPGYEAVKDASTLSSVGDLT IKDPKPPAIEVKGNERIDQI QQDGPAHLNDKENPKEADEQ VNGNVK | 14 |
| *Drosophila melanogaster* HSP22 | MRLSPMFWRMAEEMARMPRS LLPFHAFFHEPPDWLDASPR NWQHIARWQEQESAPPATDN KVGYKSTSVDKVYLESKDKD SVELDDSDEAKLEQQEAEQG GYLLRHFSGRYDSPVGYEAV KDLLLSLVVGDSTILDPNPP GDQETSKEREDTIEQTGEPA KKLAEEPKVKTALQ | 15 |
| *Anser cygnoides domesticus* CRYAB | MSITIHNPLIRRPLFDWLAP DRIFSQIFGEHLQEDELLPA DPDLDPFLMRDPIFRMPDWL ETGLDEMRLEKSKFDVNLSV KHFDPEELKVKVLGSMVEIH GKHEERQSEHGFIAREFNRK YRIPASVSPLTITDDLDLSG VLTVDAPRKQDSVPERDIPI TREEKPAIAGAQRK | 16 |

Described is an alpha crystallin recombinant polypeptide sequence or amino acid sequence derived from *Anser cygnoides domesticus* alpha-A-crystallin (CRYAA) (GenBank # XP_013036875.1) comprising, consisting essentially of, or consisting of, the amino acid sequence of SEQ ID NO: 1 or a recombinant polypeptide comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 1.

Described is an acidic alpha crystallin recombinant polypeptide variant sequence or amino acid sequence derived from *Anser cygnoides domesticus* alpha-A-crystallin (CRYAA) (GenBank # XP_013036875.1) comprising, consisting essentially of, or consisting of, the amino acid sequence of SEQ ID NO: 1, wherein the alpha crystallin recombinant polypeptide variant is acidic as determined by isoelectric point (pI), or an acidic alpha crystallin recombinant polypeptide variant comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 1, wherein the alpha crystallin recombinant polypeptide variant is acidic as determined by isoelectric point (pI). In some examples, the pI of the recombinant polypeptide is lower than the pI of SEQ ID NO: 1. In some examples, leucine (L), aspartic acid (D) and glutamic acid (E) are each independently present in an amount greater than, or equal to, the amount of any other amino acid residue present within the recombinant polypeptide sequence. In some examples, leucine (L), aspartic acid (D) and glutamic acid (E) are the three most abundant amino acid residues in the acidic alpha crystallin recombinant polypeptide variant or are greater than or equal to in abundance to the next most abundant amino acid residue of the acidic alpha crystallin recombinant polypeptide variant.

The recombinant polypeptide is an acidic variant derived from *Anser cygnoides domesticus* alpha-A-crystallin (CRYAA) (GenBank # XP_013036875.1) comprising, consisting essentially of, or consisting of, the amino acid sequence of SEQ ID NO: 9 or a recombinant polypeptide comprising, consisting essentially of, or consisting of, an amino acid sequence that is at least 95%, about 96%, about 97%, about 98% or about 99% identical to the amino acid sequence of SEQ ID NO: 9. For example, SEQ ID NO:9 has at least 80% sequence identity to the polypeptide of SEQ ID NO: 1, SEQ ID NO:9 is acidic as determined by pI, the pI of SEQ ID NO:9 is lower than the pI of SEQ ID NO: 1, and aspartic acid (D), glutamic acid (E) and leucine (L) are each independently present in an amount greater than, or equal to, the amount of any other amino acid residue present within SED ID NO:9 (that is, glutamic acid (E) is 23 residues, leucine (L) is 15 residues, and aspartic acid (D) is 14 residues of the 173 amino acid sequence of SEQ ID NO:9, with proline (P) (14 residues) being the next most present amino acid residue within SEQ ID NO:9).

Described is an alpha crystallin recombinant polypeptide sequence or amino acid sequence derived from *Rhea Americana* alpha-A-crystallin (CRYAA) (GenBank # P02505.1) comprising, consisting essentially of, or consisting of, the amino acid sequence of SEQ ID NO: 2 or a recombinant polypeptide comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 2.

Described is an acidic alpha crystallin recombinant polypeptide variant sequence or amino acid sequence derived from *Rhea Americana* alpha-A-crystallin (CRYAA) (GenBank # P02505.1) comprising, consisting essentially of, or consisting of, the amino acid sequence of SEQ ID NO: 2, wherein the alpha crystallin recombinant polypeptide variant is acidic as determined by isoelectric point (pI), or an acidic alpha crystallin recombinant polypeptide variant comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 2, wherein the alpha crystallin recombinant polypeptide variant is acidic as determined by isoelectric point (pI). In some examples, the pI of the recombinant polypeptide is lower than the pI of SEQ ID NO: 2. In some examples, leucine (L), aspartic acid (D) and glutamic acid (E) are each independently present in an amount greater than, or equal to, the amount of any other amino acid residue present within the recombinant polypeptide sequence. In some examples, leucine (L), aspartic acid (D) and glutamic acid (E) are the three most abundant amino acid residues in the acidic alpha crystallin recombinant polypeptide variant or are greater than or equal to in abundance to the next most abundant amino acid residue of the acidic alpha crystallin recombinant polypeptide variant.

In a preferred example, the recombinant polypeptide is an acidic variant derived from *Rhea Americana* alpha-A-crystallin (CRYAA) (GenBank # P02505.1) comprising, consisting essentially of, or consisting of, the amino acid sequence of SEQ ID NO: 10 or a recombinant polypeptide comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 10. For example, SEQ ID NO: 10 has at least 75% sequence identity to the polypeptide of SEQ ID NO:2, SEQ ID NO: 10 is acidic as determined by pI, the pI of SEQ ID NO: 10 is lower than the pI of SEQ ID NO:2, and aspartic acid (D), glutamic acid (E) and leucine (L) are each independently present in an amount greater than, or equal to, the amount of any other amino acid residue present within SED ID NO: 10.

The present disclosure provides an alpha crystallin recombinant polypeptide sequence or amino acid sequence derived from *Anas platyrhynchos* alpha-A-crystallin (CRYAA) (GenBank # O12984.1) comprising, consisting essentially of, consisting of, the amino acid sequence of SEQ ID NO: 3 or a recombinant polypeptide comprising, consisting essentially of, consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 3.

The present disclosure provides an acidic alpha crystallin recombinant polypeptide variant sequence or amino acid sequence derived from *Anas platyrhynchos* alpha-A-crystallin (CRYAA) (GenBank # O12984.1) comprising, consisting essentially of, or consisting of, the amino acid sequence of SEQ ID NO: 3, wherein the alpha crystallin recombinant polypeptide variant is acidic as determined by isoelectric point (pI), or an acidic alpha crystallin recombinant polypeptide variant comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 3, wherein the alpha crystallin recombinant polypeptide variant is acidic as determined by isoelectric point (pI). In some examples, the pI of the recombinant polypeptide is lower than the pI of SEQ ID NO: 3. In some examples, leucine (L), aspartic acid (D) and glutamic acid (E) are each independently present in an amount greater than, or equal to, the amount of any other amino acid residue present within the recombinant polypeptide sequence. In some examples, leucine (L), aspartic acid (D) and glutamic acid (E) are the three most abundant amino acid residues in the acidic alpha crystallin recombinant polypeptide variant or are greater than or equal to in abundance to the next most abundant amino acid residue of the acidic alpha crystallin recombinant polypeptide variant.

In a preferred aspect, the recombinant polypeptide is an acidic variant derived from *Anas platyrhynchos* alpha-A-crystallin (CRYAA) (GenBank # O12984.1) comprising, consisting essentially of, or consisting of, the amino acid sequence of SEQ ID NO: 11. For example, SEQ ID NO:11 has at least 80% sequence identity to the polypeptide of SEQ ID NO:3, SEQ ID NO:11 is acidic as determined by pI, the pI of SEQ ID NO:11 is lower than the pI of SEQ ID NO:3, and aspartic acid (D), glutamic acid (E) and leucine (L) are each independently present in an amount greater than, or equal to, the amount of any other amino acid residue present within SED ID NO: 11.

Also described is an alpha crystallin recombinant polypeptide sequence or amino acid sequence derived from *Anas platyrhynchos* alpha-B-crystallin (CRYAB) (GenBank # Q05557.1) comprising, consisting essentially of, consisting of, the amino acid sequence of SEQ ID NO: 4 or a recombinant polypeptide comprising, consisting essentially of, consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 4.

Also described is an acidic alpha crystallin recombinant polypeptide variant sequence or amino acid sequence derived from *Anas platyrhynchos* alpha-B-crystallin (CRYAB) (GenBank # Q05557.1) comprising, consisting essentially of, or consisting of, the amino acid sequence of SEQ ID NO: 4, wherein the alpha crystallin recombinant polypeptide variant is acidic as determined by isoelectric point (pI), or an acidic alpha crystallin recombinant polypeptide variant comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 4, wherein the alpha crystallin recombinant polypeptide variant is acidic as determined by isoelectric point (pI). In some examples, the pI of the recombinant polypeptide is lower than the pI of SEQ ID NO: 4. In some examples, leucine (L), aspartic acid (D) and glutamic acid (E) are each independently present in an amount greater than, or equal to, the amount of any other amino acid residue present within the recombinant polypeptide sequence. In some examples, leucine (L), aspartic acid (D) and glutamic acid (E) are the three most abundant amino acid residues in the acidic alpha crystallin recombinant polypeptide variant or are greater than or equal to in abundance to the next most abundant amino acid residue of the acidic alpha crystallin recombinant polypeptide variant.

Also described is a recombinant polypeptide is an acidic variant derived from *Anas platyrhynchos* alpha-B-crystallin (CRYAB) (GenBank # Q05557.1) comprising, consisting essentially of, or consisting of, the amino acid sequence of SEQ ID NO: 12 or a recombinant polypeptide comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 12. For example, SEQ ID NO: 12 has at least 80% sequence identity to the polypeptide of SEQ ID NO:4, SEQ ID NO: 12 is acidic as determined by pI, the pI of SEQ ID NO: 12 is lower than the pI of SEQ ID NO:4, and aspartic acid (D), glutamic acid (E) and leucine (L) are each independently present in an amount greater than, or equal to, the amount of any other amino acid residue present within SED ID NO: 12.

Also described is an alpha crystallin recombinant polypeptide sequence or amino acid sequence derived from *Homo sapiens* alpha-A-crystallin (CRYAA) (GenBank # AAH69528.1) comprising, consisting essentially of, or consisting of, the amino acid sequence of SEQ ID NO: 5 or a recombinant polypeptide comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 5.

Also described is an acidic alpha crystallin recombinant polypeptide variant sequence or amino acid sequence derived from *Homo sapiens* alpha-A-crystallin (CRYAA) (GenBank # AAH69528.1) comprising, consisting essentially of, or consisting of, the amino acid sequence of SEQ ID NO: 5, wherein the alpha crystallin recombinant polypeptide variant is acidic as determined by isoelectric point (pI), or an acidic alpha crystallin recombinant polypeptide variant comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 5, wherein the alpha crystallin recombinant polypeptide variant is acidic as determined by isoelectric point (pI). In some examples, the pI of the recombinant polypeptide is lower than the pI of SEQ ID NO: 5. In some examples, leucine (L), aspartic acid (D) and glutamic acid (E) are each independently present in an amount greater than, or equal to, the amount of any other amino acid residue present within the recombinant polypeptide sequence. In some aspects, leucine (L), aspartic acid (D) and glutamic acid (E) are the three most abundant amino acid residues in the acidic alpha crystallin recombinant polypeptide variant or are greater than or equal to in abundance to the next most abundant amino acid residue of the acidic alpha crystallin recombinant polypeptide variant.

Also described is where the recombinant polypeptide is an acidic variant derived from *Homo sapiens* alpha-A-crystallin (CRYAA) (GenBank # AAH69528.1) comprising, consisting essentially of, or consisting of, the amino acid sequence of SEQ ID NO: 13 or a recombinant polypeptide comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 13. For example, SEQ ID NO: 13 has at least 80% sequence identity to the polypeptide of SEQ ID NO: 5, SEQ ID NO: 13 is acidic as determined by pI, the pI of SEQ ID NO: 13 is lower than the pI of SEQ ID NO:5, and aspartic acid (D), glutamic acid (E) and leucine (L) are each independently present in an amount greater than, or equal to, the amount of any other amino acid residue present within SED ID NO: 13.

Also described is an HSP23 recombinant polypeptide sequence or amino acid sequence derived from *Drosophila melanogaster* HSP23 (GenBank # AAA28637.1) comprising, consisting essentially of, or consisting of, the amino acid sequence of SEQ ID NO: 6 or a recombinant polypeptide comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 6.

ALso described is an HSP23 recombinant polypeptide variant sequence or amino acid sequence derived from *Drosophila melanogaster* HSP23 (GenBank # AAA28637.1) comprising, consisting essentially of, or consisting of, the amino acid sequence of SEQ ID NO: 6, wherein the HSP23 recombinant polypeptide variant is acidic as determined by isoelectric point (pI), or an acidic HSP23 recombinant polypeptide variant comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 6, wherein the HSP23 recombinant polypeptide variant is acidic as determined by isoelectric point (pI). In some examples, the pI of the recombinant polypeptide is lower than the pI of SEQ ID NO: 6. In some examples, leucine (L), aspartic acid (D) and glutamic acid (E) are each independently present in an amount greater than, or equal to, the amount of any other amino acid residue present within the recombinant polypeptide sequence. In some examples, leucine (L), aspartic acid (D) and glutamic acid (E) are the three most abundant amino acid residues in the acidic HSP23 recombinant polypeptide variant or are greater than or equal to in abundance to the next most abundant amino acid residue of the acidic HSP23 recombinant polypeptide variant.

Also described is where the recombinant polypeptide is an acidic variant derived from *Drosophila melanogaster* HSP23 (GenBank # AAA28637.1) comprising, consisting essentially of, or consisting of, the amino acid sequence of SEQ ID NO: 14 or a recombinant polypeptide comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 14. For example, SEQ ID NO: 14 has at least 80% sequence identity to the polypeptide of SEQ ID NO:6, SEQ ID NO: 14 is acidic as determined by pI, the pI of SEQ ID NO: 14 is lower than the pI of SEQ ID NO:6, and aspartic acid (D), glutamic acid (E) and leucine (L) are each independently present in an amount greater than, or equal to, the amount of any other amino acid residue present within SED ID NO: 14.

Also described is an HSP22 recombinant polypeptide sequence or amino acid sequence derived from *Drosophila melanogaster* HSP22 (GenBank # AAA28635.1) comprising, consisting essentially of, or consisting of, the amino acid sequence of SEQ ID NO: 7 or a recombinant polypeptide having an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 7.

Also described is an acidic HSP22 recombinant polypeptide variant sequence or amino acid sequence derived from *Drosophila melanogaster* HSP22 (GenBank # AAA28635.1) comprising, consisting essentially of, or consisting of, the amino acid sequence of SEQ ID NO: 7, wherein the HSP22 recombinant polypeptide variant is acidic as determined by isoelectric point (pI), or an acidic HSP22 recombinant polypeptide variant comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 7, wherein the HSP22 recombinant polypeptide variant is acidic as determined by isoelectric point (pI). In some examples, the pI of the recombinant polypeptide is lower than the pI of SEQ ID NO: 7. In some examples, leucine (L), aspartic acid (D) and glutamic acid (E) are each independently present in an amount greater than, or equal to, the amount of any other amino acid residue present within the recombinant polypeptide sequence. In some examples, leucine (L), aspartic acid (D) and glutamic acid (E) are the three most abundant amino acid residues in the acidic HSP22 recombinant polypeptide variant or are greater than or equal to in abundance to the next most abundant amino acid residue of the acidic HSP22 recombinant polypeptide variant.

Also described is where the recombinant polypeptide is an acidic variant derived from *Drosophila melanogaster* HSP22 (GenBank # AAA28635.1) comprising, consisting essentially of, or consisting of, the amino acid sequence of SEQ ID NO: 15 or a recombinant polypeptide comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 15. For example, SEQ ID NO: 15 has at least 65% sequence identity to the polypeptide of SEQ ID NO:7, SEQ ID NO: 15 is acidic as determined by pI, the pI of SEQ ID NO: 15 is lower than the pI of SEQ ID NO:7, and aspartic acid (D), glutamic acid (E) and leucine (L) are each independently present in an amount greater than, or equal to, the amount of any other amino acid residue present within SED ID NO:15.

Also described is an alpha crystallin recombinant polypeptide sequence or amino acid sequence derived from *Anser cygnoides domesticus* alpha-B-crystallin (CRYAB) (GenBank # XP_013042703.1) comprising, consisting essentially of, or consisting of, the amino acid sequence of SEQ ID NO: 8 or a recombinant polypeptide comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 8.

Also described is an acidic alpha crystallin recombinant polypeptide variant sequence or amino acid sequence derived from *Anser cygnoides domesticus* alpha-B-crystallin (CRYAB) (GenBank # XP_013042703.1) comprising, consisting essentially of, or consisting of, the amino acid sequence of SEQ ID NO: 8, wherein the alpha crystallin recombinant polypeptide variant is acidic as determined by isoelectric point (pI), or an acidic alpha crystallin recombinant polypeptide variant comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 8, wherein the alpha crystallin recombinant polypeptide variant is acidic as determined by isoelectric point (pI). In some examples, the pI of the recombinant polypeptide is lower than the pI of SEQ ID NO: 8. In some examples, leucine (L), aspartic acid (D) and glutamic acid (E) are each independently present in an amount greater than, or equal to, the amount of any other amino acid residue present within the recombinant polypeptide sequence. In some examples, leucine (L), aspartic acid (D) and glutamic acid (E) are the three most abundant amino acid residues in the acidic alpha crystallin recombinant polypeptide variant or are greater than or equal to in abundance to the next most abundant amino acid residue of the acidic alpha crystallin recombinant polypeptide variant.

Also described is where the recombinant polypeptide is an acidic variant derived from *Anser cygnoides domesticus* alpha-B-crystallin (CRYAB) (GenBank # XP_013042703.1) comprising, consisting essentially of, or consisting of, the amino acid sequence of SEQ ID NO: 16 or a recombinant polypeptide comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to the amino acid sequence of SEQ ID NO: 16. For example, SEQ ID NO: 16 has at least 80% sequence identity to the polypeptide of SEQ ID NO:8, SEQ ID NO: 16 is acidic as determined by pI, the pI of SEQ ID NO: 16 is lower than the pI of SEQ ID NO: 8, and aspartic acid (D), glutamic acid (E) and leucine (L) are each independently present in an amount greater than, or equal to, the amount of any other amino acid residue present within SED ID NO: 16.

The present disclosure provides an isolated nucleic acid molecule encoding a recombinant polypeptide that comprises, consists essentially of, or consists of, an amino acid sequence having at least 95% sequence identity to the polypeptide of SEQ ID NO: 9. The present disclosure also provides isolated nucleic acid molecules encoding recombinant polypeptides comprising, consisting essentially of, or consisting of, an amino acid sequence that is at least about 96%, about 97%, about 98% or about 99% identical to SEQ ID NO: 9.

Described is an isolated nucleic acid molecule encoding a recombinant polypeptide variant that comprises, consists essentially of, or consists of, any of the amino acid sequences shown in Table 1A, wherein the recombinant polypeptide variant is acidic as determined by isoelectric point (pI). Also described are isolated nucleic acid molecules encoding a recombinant polypeptide variants comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to any of the amino acid sequences shown in Table 1A.

Also described are isolated nucleic acid molecules encoding acidic recombinant polypeptide variants that comprise, consist essentially of, or consist of, any of the amino acid sequences shown in Table 1A, wherein the recombinant polypeptide variant is acidic as determined by isoelectric point (pI), wherein the pI of the recombinant peptide variant is lower than the pI of peptide sequence from which the recombinant peptide was derived, and wherein leucine (L), aspartic acid (D) and glutamic acid (E) are each independently present in an amount greater than, or equal to, the amount of any other amino acid residue present within the recombinant polypeptide sequence. Also described are isolated nucleic acid molecules encoding acidic recombinant polypeptide variants that comprise, consist essentially of, or consist of, any of the amino acid sequences shown in Table 1A, wherein the recombinant polypeptide variant is acidic as determined by isoelectric point (pI) and wherein leucine (L), aspartic acid (D) and glutamic acid (E) are the three most abundant amino acid sequences of the acidic variant or are greater than or equal to in abundance to the next most abundant amino acid residue of the acidic alpha crystallin recombinant polypeptide variant. Also described are isolated nucleic acid molecules encoding acid recombinant polypeptide variants comprising, consisting essentially of, or consisting of, an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to any of the amino acid sequences shown in Table 1A.

The present disclosure also provides isolated nucleic acid molecules that comprise, consist essentially of, or consist of, any of the nucleic acid sequences shown in Table 2A in accordance with the claims. The present disclosure provides nucleic acid molecules comprising, consisting essentially of, or consisting of, a nucleic acid sequence that encodes a recombinant polypeptide comprising an amino acid sequence that is at least 95%, about 96%, about 97%, about 98% or about 99% identical to the polypeptide of SEQ ID NO: 9.

**Table 2A.** Nucleic Acid Sequences

| Name | NucleicAcidSequence | SEQ ID NO: |
|---|---|---|
| *Anser cygnoides domesticus* CRYAA | ATGGATATTACCATTCAGCATCCGTGGTTTAAACGCGCGCTGG GCCCGCTGATTCCGAGCCGCCTGTTTGATCAGTTTTTTGGCGA AGGCCTGCTGGAATATGATCTGCTGCCGCTGTTTAGCAGCACC ATTAGCCCGTATTATCGCCAGAGCCTGTTTCGCAGCGTGCTGG AAAGCGGCATTAGCGAAGTGCGCAGCGATCGCGATAAATTTAC CATTATGCTGGATGTGAAACATTTTAGCCCGGAAGATCTGAGC GTGAAAATTATTGATGATTTTGTGGAAATTCATGGCAAACATA GCGAACGCCAGGATGATCATGGCTATATTAGCCGCGAATTTCA TCGCCGCTATCGCCTGCCGGCGAACGTGGATCAGAGCGCGATT ACCTGCAGCCTGAGCGGCGATGGCATGCTGACCTTTAGCGGCC CGAAAGTGCCGAGCAACATGGATCCGACCCATAGCGAACGCCC GATTCCGGTGAGCCGCGAAGAAAAACCGACCAGCGCGCCGAGC AGC | 17 |
| *Rhea americana* CRYAA | ATGGATATTACCATTCAGCATCCGTGGTTTAAACGCGCGCTGG GCCCGCTGATTCCGAGCCGCCTGTTTGATCAGTTTTTTGGCGA AGGCCTGCTGGAATATGATCTGCTGCCGCTGTTTAGCAGCACC ATTAGCCCGTATTATCGCCAGAGCCTGTTTCGCAGCGTGCTGG AAAGCGGCATTAGCGAAGTGCGCAGCGATCGCGAAAAATTTAC CATTATGCTGGATGTGAAACATTTTAGCCCGGAAGATCTGAGC GTGAAAATTATTGATGATTTTGTGGAAATTCATGGCAAACATA GCGAACGCCAGGATGATCATGGCTATATTAGCCGCGAATTTCA TCGCCGCTATCGCCTGCCGAGCAACGTGGATCAGAGCGCGATT ACCTGCAGCCTGAGCAGCGATGGCATGCTGACCTTTAGCGGCC CGAAAGTGCAGGCGAACATGGATCCGAGCCATAGCGAACGCCC GATTCCGGTGAGCCGCGAAGAAAAACCGACCAGCGCGCCGAGC AGC | 18 |
| *Anas platyrhynchos* CRYAA | CGCGCGCTGGGCCCGCTGATTCCGAGCCGCCTGTTTGATCAGT TTTTTGGCGAAGGCCTGCTGGAATATGATCTGCTGCCGCTGTT TAGCAGCACCATTAGCCCGTATTATCGCCAGAGCCTGTTTCGC AGCGTGCTGGAAAGCGGCATTAGCGAAGTGCGCAGCGATCGCG ATAAATTTACCATTATGCTGGATGTGAAACATTTTAGCCCGGA AGATCTGAGCGTGAAAATTATTGATGATTTTGTGGAAATTCAT GGCAAACATAGCGAACGCCAGGATGATCATGGCTATATTAGCC GCGAATTTCATCGCCGCTATCGCCTGCCGGCGAACGTGGATCA GAGCGCGATTACCTGCAGCCTGAGCGGCGATGGCATGCTGACC TTTAGCGGCCCGAAAGTGCCGAGCAACATGGATCCGACCCATA GCGAACGCCCGATTCCG | 19 |

(continued)

| Name | NucleicAcidSequence | SEQ ID NO: |
|---|---|---|
| *Anas platyrhynchos* CRYAB | ATGGATATTACCATTCATAACCCGCTGATTCGCCGCCCGCTGT TTAGCTGGCTGGCGCCGAGCCGCATTTTTGATCAGATTTTTGG CGAACATCTGCAGGAAAGCGAACTGCTGCCGGCGAGCCCGAGC CTGAGCCCGTTTCTGATGCGCAGCCCGATTTTTCGCATGCCGA GCTGGCTGGAAACCGGCCTGAGCGAAATGCGCCTGGAAAAAGA TAAATTTAGCGTGAACCTGGATGTGAAACATTTTAGCCCGGAA GAACTGAAAGTGAAAGTGCTGGGCGATATGGTGGAAATTCATG GCAAACATGAAGAACGCCAGGATGAACATGGCTTTATTGCGCG CGAATTTAACCGCAAATATCGCATTCCGGCGGATGTGGATCCG CTGACCATTACCAGCAGCCTGAGCCTGGATGGCGTGCTGACCG TGAGCGCGCCGCGCAAACAGAGCGATGTGCCGGAACGCAGCAT TCCGATTACCCGCGAAGAAAACCGGCGATTGCGGGCGCGCAG CGCA AA | 20 |
| *Homo sapiens* CRYAA | ATGGATGTGACCATTCAGCATCCGTGGTTTAAACGCACCCTGG GCCCGTTTTATCCGAGCCGCCTGTTTGATCAGTTTTTTGGCGA AGGCCTGTTTGAATATGATCTGCTGCCGTTTCTGAGCAGCACC ATTAGCCCGTATTATCGCCAGAGCCTGTTTCGCACCGTGCTGG ATAGCGGCATTAGCGAAGTGCGCAGCGATCGCGATAAATTTGT GATTTTTCTGGATGTGAAACATTTTAGCCCGGAAGATCTGACC GTGAAAGTGCAGGATGATTTTGTGGAAATTCATGGCAAACATA ACGAACGCCAGGATGATCATGGCTATATTAGCCGCGAATTTCA TCGCCGCTATCGCCTGCCGAGCAACGTGGATCAGAGCGCGCTG AGCTGCAGCCTGAGCGCGGATGGCATGCTGACCTTTTGCGGCC CGAAAATTCAGACCGGCCTGGATGCGACCCATGCGGAACGCGC GATTCCGGTGAGCCGCGAAGAAAACCGACCAGCGCGCCGAGC AGC | 21 |
| *Drosophila melanogaster* HSP23 | ATGGCGAACATTCCGCTGCTGCTGAGCCTGGCGGATGATCTGG GCCGCATGAGCATGGTGCCGTTTTATGAACCGTATTATTGCCA GCGCCAGCGCAACCCGTATCTGGCGCTGGTGGGCCCGATGGAA CAGCAGCTGCGCCAGCTGGAAAAACAGGTGGGCGCGAGCAGCG GCAGCAGCGGCGCGGTGAGCAAAATTGGCAAAGATGGCTTTCA GGTGTGCATGGATGTGAGCCATTTTAAACCGAGCGAACTGGTG GTGAAAGTGCAGGATAACAGCGTGCTGGTGGAAGGCAACCATG AAGAACGCGAAGATGATCATGGCTTTATTACCCGCCATTTTGT GCGCCGCTATGCGCTGCCGCCGGGCTATGAAGCGGATAAAGTG GCGAGCACCCTGAGCAGCGATGGCGTGCTGACCATTAAAGTGC CGAAACCGCCGGCGATTGAAGATAAAGGCAACGAACGCATTGT GCAGATTCAGCAGGTGGGCCCGGCGCATCTGAACGTGAAAGAA AACCCGAAAGAAGCGGTGGAACAGGATAACGGCAACGATAAA | 22 |
| *Drosophila melanogaster* HSP22 | ATGCGCAGCCTGCCGATGTTTTGGCGCATGGCGGAAGAAATGG CGCGCATGCCGCGCCTGAGCAGCCCGTTTCATGCGTTTTTTCA TGAACCGCCGGTGTGGAGCGTGGCGCTGCCGCGCAACTGGCAG CATATTGCGCGCTGGCAGGAACAGGAACTGGCGCCGCCGGCGA CCGTGAACAAAGATGGCTATAAACTGACCCTGGATGTGAAAGA TTATAGCGAACTGAAAGTGAAAGTGCTGGATGAAAGCGTGGTG CTGGTGGAAGCGAAAAGCGAACAGCAGGAAGCGGAACAGGGCG GCTATAGCAGCCGCCATTTTCTGGGCCGCTATGTGCTGCCGGA TGGCTATGAAGCGGATAAAGTGAGCAGCAGCCTGAGCGATGAT GGCGTGCTGACCATTAGCGTGCCGAACCCGCCGGGCGTGCAGG AAACCCTGAAAGAACGCGAAGTGACCATTGAACAGACCGGCGA ACCGGCGAAAAAAGCGCGGAAGAACCGAAAGATAAAACCGCG AGCCAG | 23 |

(continued)

| Name | NucleicAcidSequence | SEQ ID NO: |
|------|---------------------|-----------|
| *Anser cygnoides domesticus* CRYAB | ATGGATATTACCATTCATAACCCGCTGATTCGCCGCCCGCTGT TTAGCTGGCTGGCGCCGAGCCGCATTTTTGATCAGATTTTTGG CGAACATCTGCAGGAAAGCGAACTGCTGCCGGCGAGCCCGAGC CTGAGCCCGTTTCTGATGCGCAGCCCGATTTTTCGCATGCCGA GCTGGCTGGAAACCGGCCTGAGCGAAATGCGCCTGGAAAAAGA TAAATTTAGCGTGAACCTGGATGTGAAACATTTTAGCCCGGAA GAACTGAAAGTGAAAGTGCTGGGCGATATGGTGGAAATTCATG GCAAACATGAAGAACGCCAGGATGAACATGGCTTTATTGCGCG CGAATTTAACCGCAAATATCGCATTCCGGCGGATGTGGATCCG CTGACCATTACCAGCAGCCTGAGCCTGGATGGCGTGCTGACCG TGAGCGCGCCGCGCAAACAGAGCGATGTGCCGGAACGCAGCAT TCCGATTACCCGCGAAGAAAAACCGGCGATTGCGGGCGCGCAG CGCA AA | 24 |

The present disclosure provides isolated nucleic acid molecules that comprise, consist essentially of, or consist of, any of the nucleic acid sequences shown in Table 2B in accordance with the claims. The present disclosure provides nucleic acid molecules comprising, consisting essentially of, or consisting of, a nucleic acid sequence that encodes a recombinant polypeptide comprising an amino acid sequence at least 95%identical to SEQ ID NO:9.

**Table 2B.** Nucleic Acid Sequences

| Name | NucleicAcidSequence | SEQ ID NO: |
|------|---------------------|-----------|
| CRYA_1B *Anser cygnoides domesticus* CRYAA | ATGGATATTACCATTCAGCATCCGTGGTTTAAACGCGCGCTG GGCCCGCTGATTCCGGAACGCCTGTTTGATCAGTTTTTTGGC AGCGGCCTGCTGAGCTATGATCTGCTGCCGCTGTTTGAAGAA ACCATTGAACCGTATTATCGCCAGGAACTGTTTCGCGAAGTG CTGAGCGAAGGCATTGAAAGCGTGCGCGAAGATCGCGATAAA TTTACCATTATGCTGGATGTGAAACATTTTGAACCGAGCGAT CTGGAAGTGAAAATTATTGATGATTTTGTGAGCATTCATGGC AAACATGAAGCCGCCAGGATGATCATGGCTATATTGAACGC AGCTTTCATCGCCGCTATCGCCTGCCGGCGAACGTGGATCAG GAAGCGATTACCTGCGAACTGGAAGGCGATGGCATGCTGACC TTTGAAGGCCCGAAAGTGCCGGAAAACATGGATCCGACCCAT GAAAGCCGCCCGATTCCGGTGGAACGCAGCAGCAAACCGACC GAAGCGCCGGAAGAA | 25 |
| *Rhea americana* CRYAA | ATGGATATTACCATTCAGCATCCGTGGTTTAAACGCGCGCTG GGCCCGCTGATTCCGGAACGCCTGTTTGATCAGTTTTTTGGC AGCGGCCTGCTGAGCTATGATCTGCTGCCGCTGTTTGAAGAA ACCATTGAACCGTATTATCGCCAGGAACTGTTTCGCGAAGTG CTGAGCGAAGGCATTGAAAGCGTGCGCGAAGATCGCAGCAAA TTTACCATTATGCTGGATGTGAAACATTTTGAACCGAGCGAT CTGGAAGTGAAAATTATTGATGATTTTGTGAGCATTCATGGC AAACATGAAGCCGCCAGGATGATCATGGCTATATTGAACGC AGCTTTCATCGCCGCTATCGCCTGCCGGAAAACGTGGATCAG GAAGCGATTACCTGCGAACTGGAAGAAGATGGCATGCTGACC TTTGAAGGCCCGAAAGTGCAGGCGAACATGGATCCGGAACAT GAAAGCCGCCCGATTCCGGTGGAACGCAGCAGCAAACCGACC GAAGCGCCGGAAGAA | 26 |

(continued)

| Name | NucleicAcidSequence | SEQ ID NO: |
|------|---------------------|------------|
| *Anas platyrhynchos* CRYAA | CGCGCGCTGGGCCCGCTGATTCCGGAACGCCTGTTTGATCAG TTTTTTGGCAGCGGCCTGCTGAGCTATGATCTGCTGCCGCTG TTTGAAGAAACCATTGAACCGTATTATCGCCAGGAACTGTTT CGCGAAGTGCTGAGCGAAGGCATTGAAAGCGTGCGCGAAGAT CGCGATAAATTTACCATTATGCTGGATGTGAAACATTTTGAA CCGAGCGATCTGGAAGTGAAAATTATTGATGATTTTGTGAGC ATTCATGGCAAACATGAAAGCCGCCAGGATGATCATGGCTAT ATTGAACGCAGCTTTCATCGCCGCTATCGCCTGCCGGCGAAC GTGGATCAGGAAGCGATTACCTGCGAACTGGAAGGCGATGGC ATGCTGACCTTTGAAGGCCCGAAAGTGCCGGAAAACATGGAT CCGACCCATGAAAGCCGCCCGATTCCG | 27 |
| *Anas platyrhynchos* CRYAB | ATGAGCATTACCATTCATAACCCGCTGATTCGCCGCCCGCTG TTTGATTGGCTGGCGCCGGATCGCATTTTTAGCCAGATTTTT GGCAACATCTGCAGGAAGATGAACTGCTGCCGGCGGATCCG GATCTGGATCCGTTTCTGATGCGCGATCCGATTTTTCGCATG CCGGATTGGCTGGAAACCGGCCTGGATGAAATGCGCCTGGAA AAAAGCAAATTTGATGTGAACCTGAGCGTGAAACATTTTGAT CCGGAAGAACTGAAAGTGAAAGTGCTGGGCAGCATGGTGGAA ATTCATGGCAAACATGAAGAACGCCAGAGCGAACATGGCTTT ATTGCGCGCGAATTTAACCGCAAATATCGCATTCCGGCGAGC GTGAGCCCGCTGACCATTACCGATGATCTGGATCTGAGCGGC GTGCTGACCGTGGATGCGCCGCGCAAACAGGATAGCGTGCCG GAACGCGATATTCCGATTACCCGCGAAGAAAAACCGGCGATT GCGGGCGCGCAGCGCA AA | 28 |
| *Homo sapiens* CRYAA | ATGGATGTGACCATTCAGCATCCGTGGTTTAAACGCACCCTG GGCCCGTTTTATCCGGAACGCCTGTTTGATCAGTTTTTTGGC AGCGGCCTGTTTAGCTATGATCTGCTGCCGTTTCTGGAAGAA ACCATTGAACCGTATTATCGCCAGGAACTGTTTCGCACCGTG CTGGATGAAGGCATTGAAAGCGTGCGCGAAGATCGCGATAAA TTTGTGATTTTTCTGGATGTGAAACATTTTGAACCGAGCGAT CTGACCGTGAAAGTGCAGGATGATTTTGTGAGCATTCATGGC AAACATAACAGCCGCCAGGATGATCATGGCTATATTGAACGC AGCTTTCATCGCCGCTATCGCCTGCCGGAAAACGTGGATCAG GAAGCGCTGGAATGCGAACTGGAAGCGGATGGCATGCTGACC TTTTGCGGCCCGAAAATTCAGACCGGCCTGGATGCGACCCAT GCGAGCCGCGCGATTCCGGTGGAACGCAGCAGCAAACCGACC GAAGCGCCGGAAGAA | 29 |
| *Drosophila melanogaster* HSP23 | ATGGCGAACATTCCGCTGCTGCTGAGCCTGGCGGTGGTGCTG GGCCGCATGAGCATGGATCCGTTTTATGAACCGTATTATTGC CAGCGCCAGCGCAACCCGTATCTGGCGCTGGATGGCCCGATG GAACAGCAGCTGCGCCAGCTGGAAAAACAGGATGGCGCGAGC AGCGGCAGCAGCGGCGCGGATAGCAAAATTGGCAAAGTGGGC TTTCAGGATTGCATGGTGGATAGCCATTTTAAACCGAGCGAA CTGGATGATAAAGATCAGGTGAACAGCGATCTGGATGAAGGC AACCATGAAGAACGCGAAGTGGTGCATGGCTTTATTACCCGC CATTTTGATCGCCGCTATGCGCTGCCGCCGGGCTATGAAGCG GTGAAAGATGCGAGCACCCTGAGCGAGCGTGGGCGGATCTGACC ATTAAAGATCCGAAACCGCCGGCGATTGAAGTGAAAGGCAAC GAACGCATTGATCAGATTCAGCAGGATGGCCCGGCGCATCTG AACGATAAAGAAAACCCGAAAGAAGCGGATGAACAGGTGAAC GGCAACGTGAAA | 30 |

(continued)

| Name | NucleicAcidSequence | SEQ ID NO: |
|---|---|---|
| *Drosophila melanogaster* HSP22 | ATGCGCCTGAGCCCGATGTTTTGGCGCATGGCGGAAGAAATG GCGCGCATGCCGCGCAGCCTGCTGCCGTTTCATGCGTTTTTT CATGAACCGCCGGATTGGCTGGATGCGAGCCCGCGCAACTGG CAGCATATTGCGCGCTGGCAGGAACAGGAAAGCGCGCCGCCG GCGACCGATAACAAAGTGGGCTATAAAAGCACCAGCGTGGAT AAAGTGTATCTGGAAAGCAAAGATAAAGATAGCGTGGAACTG GATGATAGCGATGAAGCGAAACTGGAACAGCAGGAAGCGGAA CAGGGCGGCTATCTGCTGCGCCATTTTAGCGGCCGCTATGAT AGCCCGGTGGGCTATGAAGCGGTGAAAGATCTGCTGCTGAGC CTGGTGGTGGGCGATAGCACCATTCTGGATCCGAACCCGCCG GGCGATCAGGAAACCAGCAAAGAACGCGAAGATACCATTGAA CAGACCGGCGAACCGGCGAAAAAACTGGCGGAAGAACCGAAA GTGAAAACCGCGCTGCAG | 31 |
| *Anser cygnoides domesticus* CRYAB | ATGAGCATTACCATTCATAACCCGCTGATTCGCCGCCCGCTG TTTGATTGGCTGGCGCCGGATCGCATTTTTAGCCAGATTTTT GGCGAACATCTGCAGGAAGATGAACTGCTGCCGGCGGATCCG GATCTGGATCCGTTTCTGATGCGCGATCCGATTTTTCGCATG CCGGATTGGCTGGAAACCGGCCTGGATGAAATGCGCCTGGAA AAAAGCAAATTTGATGTGAACCTGAGCGTGAAACATTTTGAT CCGGAAGAACTGAAAGTGAAAGTGCTGGGCAGCATGGTGGAA ATTCATGGCAAACATGAAGAACGCCAGAGCGAACATGGCTTT ATTGCGCGCGAATTTAACCGCAAATATCGCATTCCGGCGAGC GTGAGCCCGCTGACCATTACCGATGATCTGGATCTGAGCGGC GTGCTGACCGTGGATGCGCCGCGCAAACAGGATAGCGTGCCG GAACGCGATATTCCGATTACCCGCGAAGAAAAACCGGCGATT GCGGGCGCGCAGCGCAAA | 32 |

The therapeutic methods involve administration of immune cell growth factors. Immune cell growth factors function as growth factors which increase the number of immune cells. In some aspects, the immune cells are dendritic cells. The immune cell growth factors are cytokines. The cytokines are FMS-related tyrosine kinase ligand (FLT3L) and Granulocyte-macrophage colony-stimulating factor (GM-CSF). In a preferred aspect, the immune cell growth factor is FLT3L.

Granulocyte/macrophage colony-stimulating factor (GM-CSF) is a cytokine that functions as a white blood cell growth factor, stimulates stems cells to produce granulocytes (neutrophils, eosinophils, and basophils) and monocytes. The polynucleotide sequences of GM-CSF are available from public databases as accession numbers MI 1734 (human); NM_009969 (mouse); EU520303 (chicken); NM_001037660 (rat Csf2ra); and NM_133555 (rat Csf2rb). The amino acid sequences of granulocyte/macrophage colony-stimulating factor (GM-CSF) are available from public databases as accession numbers AAA52122 (human); NP_034099 (mouse); ACBI 1534 (chicken); NP_001032749 (rat Csf2ra); and NP 598239 (Csf2rb).

FLT3L: FMS-like tyrosine kinase 3 ligand (FLT3L) is an endogenous small molecule that functions as a cytokine and growth factor that increases the number of immune cells, especially dendritic cells, including myeloid-derived DCs (mDC) and plasmacytoid-derived DCs(pDC). Human HLA-DR+Lin-DC include CD11c+CD123- Myeloid-derived DCs(mDC) and CD11c-CD123+ plasmacytoid-derived DCs (pDC).

The polynucleotide sequences of FLT3L, which may function as a growth factor for dendritic cells, hematopoietic stem cells, progenitor cells or any combination thereof, are available from public databases as accession numbers U04806 (human); and NM_013520 (mouse). The amino acid sequences of FLT3/FLK2 ligand (Flt3l) are available from public databases as accession numbers AAAI7999 (human); and NP_038548 (mouse).

As used herein, the term "dendritic cells" or "DCs" refers to immune cells that form part of the mammalian immune system. A primary function of DCs is to serve as "antigen-presenting cells" by processing foreign antigens and presenting antigenic epitopes on their surface to other cells of the immune system. DCs are present in small quantities in tissues that are in contact with the external environment, mainly the skin (where there is a specialized dendritic cell type called Langerhans cells) and the inner lining of the nose, lungs, stomach and intestines. DCs can also be found in an immature state in the blood. Once activated, they migrate to the lymphoid tissues where they interact with T cells and B cells to initiate the adaptive immune response. At certain development stages DCs grow branched projections (dendrites) that give the cell its name. In some embodiments, DCs can be differentiated into two sub-populations based on the expression of the cell surface marker CD11c. In some embodiments, CD11c+ DCs produce IL12 and stimulate a Th1 response in lymphocytes, while CD11c-DCs synthesize little IL12 but are a major source of alpha-interferon and stimulate lymphocytes to produce Th2 cytokines.

As used herein, the term "cytokines" refers to a category of protein, peptide, or glycoprotein molecules secreted by specific cells of the immune system that carry signals between cells. Cytokines are a critical component of both the innate and adaptive immune response, and are often secreted by immune cells that have encountered a pathogen to activate and recruit additional immune cells to increase the system's response to the pathogen. Cytokines are typically released in the general region of the pathogen-infected cells such that responding immune cells arrive at that site of infection. Each individual cytokine has a matching cell-surface receptor. Upon binding of a cytokine to its cell-surface receptor a cascade of intracellular signaling events alters the cell's function. This includes the upregulation and/or downregulation of genes involved in the production of other cytokines, an increase expression of surface receptors for other molecules, or suppression of the cytokine itself by feedback inhibition. The effect of a particular cytokine on a given cell depends on the cytokine, its extracellular abundance, the presence and abundance of the complementary receptor on the cell surface, and downstream signals activated by receptor binding. Common cytokines include interleukins that are responsible for communication between white blood cells; chemokines that promote chemotaxis; and interferons that have anti-viral effects, such as shutting down protein synthesis in the host cell. Cytokines are characterized by considerable "redundancy", in that many cytokines appear to share similar functions. For example, "granulocyte-macrophage colony-stimulating factor" (GM-CSF) and "Fms-related tyrosine kinase 3 ligand" (Flt3L) are both cytokines that promote dendritic cell growth and differentiation.

The therapeutic products for use in the methods of the disclosure involve administration of an IL-10 inhibitory agent in a subject in accordance with the claims. The agents that reduce IL-10 are interferon gamma (IFNg), or an IFNg mimeticor a combination thereof. Preferably, the IL-10 inhibitory agent is interferon gamma (IFNg). In some aspects, IFNg reduces IL-10 and reprograms M2 tumour-associated macrophages (TAM) to M1 TAM.

The therapeutic products for use in the methods of the disclosure involve administration of a TNFa inhibitory agent. In some aspects, the TNFa inhibitory agent is an inhibitor of tumor necrosis factor alpha (TNFa). Exemplary TNFa inhibitors include but are not limited to TNFa receptor (TNFaR), etanercept (Enbrel), infliximab (Remicade), adalimumab (Humira), certolizumab pegol (Cimzia), and golimumab (Simponi). In some embodiments, the TNFa inhibitor is a TNFaR. In a preferred aspect, the TNFa inhibitor is etanercept.

The therapeutic products for use in the methods of the disclosure involve administration of an immunotherapy agent. The immunotherapy agent is a checkpoint inhibitor. The term "inhibition" or "inhibitor" includes a reduction in a certain parameter, e.g., an activity, of a given molecule, e.g., an immune checkpoint inhibitor. For example, inhibition of an activity, e.g., an activity of, e.g., PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 or TGFR beta, of at least 5%, 10%, 20%, 30%, 40% or more is included by this term. The level of inhibition need not be 100%.

In some aspects, the checkpoint inhibitor is a PD-1 inhibitor. In some aspects, the PD-1 inhibitor is an anti-PD1 antibody. In some aspects, the PD-1 inhibitor is an anti PD-1 monoclonal antibody. Exemplary anti-PD-1 monoclonal antibodies include, but are not limited to cemiplimab (Libtayo), nivolumab (Opdivo), pembrolizumab (Keytruda). In some aspects, the checkpoint inhibitor is a PD-L1 inhibitor. Exemplary PD-L1inhibitors include but are not limited to avelumab (Bavencio), durvalumab (Imfinzi) and atezolizumab (Tecentriq).

The term "Programmed Death 1" or "PD-1" includes all isoforms, mammalian, e.g., human PD-1, species homologs of human PD-1, and analogs comprising at least one common epitope with PD-1. The amino acid sequence of PD-1, e.g., human PD-1, is known in the art, e.g., Shinohara T et al. (1994) Genomics 23(3):704-6; Finger L R, et al. Gene (1997) 197(1-2):177-87.

The term or "PD-Ligand 1" or "PD-L" includes all isoforms, mammalian, e.g., human PD-1, species homologs of human PD-L1, and analogs comprising at least one common epitope with PD-L1. The amino acid sequence of PD-L1, e.g., human PD-L1, is known in the art, e.g., Dong H, et al. (1999) Nat. Med. 5 (12):1365-1369; Freeman G et al. (2000) J. Exp. Med. 192 (7):1027-1034.

The present disclosure also provides pharmaceutical compositions for use in accordance with the claims comprising the recombinant polypeptides or nucleic acids disclosed herein.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

In one aspect, the pharmaceutical composition can comprise, consist essentially of, or consist of any one of the recombinant polypeptides disclosed herein in a pharmaceutically acceptable carrier. In some aspects, the pharmaceutical composition is formulated as an aqueous formulation. The aqueous formulation can comprise, consist essentially of, or consist of a salt buffer that may be selected from, but is not limited to, NaCl, KCl, and NaOAc. In one aspect, the salt buffer comprises NaCl. In one aspect, the NaCl is at a concentration from about 0.4M to about 1.0M. In one aspect, the pH of the buffer solution is between about 7.5 and about 9.0. In one aspect, the pH of the buffer solution is about 7.4.

A compound or pharmaceutical composition of the invention can be administered to a subject in many of the well-known methods currently used for chemotherapeutic treatment. For example, for the treatment of cancer, a compound of the invention may be injected directly into tumors, injected into the blood stream or body cavities, taken orally or applied through the skin with patches.

The term "therapeutically effective amount," as used herein, refers to an amount of a pharmaceutical agent to treat, ameliorate, or prevent an identified disease or condition, or to exhibit a detectable therapeutic or inhibitory effect. The effect can be detected by any assay method known in the art. The precise effective amount for a subject will depend upon the subject's body weight, size, and health; the nature and extent of the condition; and the therapeutic or combination of therapeutics selected for administration. Therapeutically effective amounts for a given situation can be determined by routine experimentation that is within the skill and judgment of the clinician. In one aspect, the disease or condition to be treated is a cell proliferative disorder. In a preferred aspect, the disease or condition to be treated is cancer.

For any compound, the therapeutically effective amount can be estimated initially either in cell culture assays, e.g., of neoplastic cells, or in animal models, usually rats, mice, rabbits, dogs, or pigs. The animal model may also be used to determine the appropriate concentration range and route of administration. Such information can then be used to determine useful doses and routes for administration in humans. Therapeutic/prophylactic efficacy and toxicity may be determined by standard pharmaceutical procedures in cell cultures or experimental animals, e.g., $ED_{50}$ (the dose therapeutically effective in 50% of the population) and $LD_{50}$ (the dose lethal to 50% of the population). The dose ratio between toxic and therapeutic effects is the therapeutic index, and it can be expressed as the ratio, $LD_{50}/ED_{50}$. Pharmaceutical compositions that exhibit large therapeutic indices are preferred. The dosage may vary within this range depending upon the dosage form employed, sensitivity of the patient, and the route of administration.

Dosage and administration are adjusted to provide sufficient levels of the active agent(s) or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy.

The pharmaceutical compositions containing active compounds of the present invention may be manufactured in a manner that is generally known, e.g., by means of conventional mixing, dissolving, granulating, dragee-making, levigating, emulsifying, encapsulating, entrapping, or lyophilizing processes. Pharmaceutical compositions may be formulated in a conventional manner using one or more pharmaceutically acceptable carriers comprising excipients and/or auxiliaries that facilitate processing of the active compounds into preparations that can be used pharmaceutically. Of course, the appropriate formulation is dependent upon the route of administration chosen.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze drying that yields a powder of the active ingredient plus any additional desired ingredient from a previously sterile filtered solution thereof.

Oral compositions generally include an inert diluent or an edible pharmaceutically acceptable carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

The pharmaceutical compositions can include co-formulations of any of the recombinant polypeptides and nucleic acids described herein.

The pharmaceutical compositions can be included in a container, pack, or dispenser together with instructions for administration.

Also described are plasmids, expression vectors and host cells comprising the recombinant polypeptides disclosed herein and the nucleic acid molecules encoding the recombinant polypeptides disclosed herein. In one example a plasmid or an expression vector comprising a nucleic acid molecule is provided, the molecule comprising a nucleotide sequence of any one of SEQ ID NO: 17-32, or a nucleic acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to any of the nucleic acid sequence of SEQ ID NO: 17-32, or a fragment thereof. In one example a host cell is provided comprising a recombinant polypeptide comprising an amino acid sequence of any one of SEQ ID NO: 1-16, or an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to any of the amino acid sequences of SEQ ID NO: 1-16, or a fragment thereof, or a host cell comprising a nucleic acid molecule comprising a nucleic acid sequence of any one of SEQ ID NO: 17-32, or a nucleic acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to any of the nucleic acid sequence of SEQ ID NO: 17-32, or a fragment thereof.

As used herein, the term "transformation," "transfection," and "transduction" refer to the transfer of nucleic acid (i.e., a nucleotide polymer) into a cell. As used herein, the term "genetic transformation" refers to the transfer and incorporation of DNA, especially recombinant DNA, into a cell. The transferred nucleic acid can be introduced into a cell via an expression vector.

Polynucleotide molecules comprising a desired polynucleotide sequence are propagated by placing the molecule in a vector. Viral and non-viral vectors can be used, including plasmids. The choice of plasmid will depend on the type of cell in which propagation is desired and the purpose of propagation. Certain vectors are useful for amplifying and making large amounts of the desired DNA sequence. Other vectors are suitable for expression in cells in culture. Still other vectors are suitable for transfer and expression in cells in a whole animal or person. The choice of appropriate vector is well within the skill of the art. Many such vectors are available commercially. The partial or full-length polynucleotide is inserted into a vector typically by means of DNA ligase attachment to a cleaved restriction enzyme site in the vector. Alternatively, the desired nucleotide sequence can be inserted by homologous recombination *in vivo.* Typically this is accomplished by attaching regions of homology to the vector on the flanks of the desired nucleotide sequence. Regions of homology are added by ligation of oligonucleotides, or by polymerase chain reaction using primers comprising both the region of homology and a portion of the desired nucleotide sequence, for example.

For expression, an expression cassette or system may be employed. To express a nucleic acid encoding a polypeptide disclosed herein, a nucleic acid molecule encoding the polypeptide, operably linked to regulatory sequences that control transcriptional expression in an expression vector, is introduced into a host cell. In addition to transcriptional regulatory sequences, such as promoters and enhancers, expression vectors can include translational regulatory sequences and a marker gene which is suitable for selection of cells that carry the expression vector. The gene product encoded by a polynucleotide of the disclosure is expressed in any convenient expression system, including, for example, bacterial, yeast, insect, amphibian and mammalian systems. In the expression vector, the polypeptide-encoding polynucleotide is linked to a regulatory sequence as appropriate to obtain the desired expression properties. These can include promoters, enhancers, terminators, operators, repressors, and inducers. The promoters can be regulated (e.g., the promoter from the steroid inducible pIND vector (Invitrogen)) or constitutive (e.g., promoters from CMV, SV40, Elongation Factor, or LTR sequences). These are linked to the desired nucleotide sequence using the techniques described above for linkage to vectors. Any techniques known in the art can be used. Accordingly, the expression vector will generally provide a transcriptional and translational initiation region, which can be inducible or constitutive, where the coding region is operably linked under the transcriptional control of the transcriptional initiation region, and a transcriptional and translational termination region.

An expression cassette ("expression unit") can be introduced into a variety of vectors, *e.g.,* plasmid, BAC, YAC, bacteriophage such as lambda, P1, M13, *etc.,* plant or animal viral vectors (e.g., retroviral-based vectors, adenovirus vectors), and the like, where the vectors are normally characterized by the ability to provide selection of cells comprising the expression vectors. The vectors can provide for extrachromosomal maintenance, particularly as plasmids or viruses, or for integration into the host chromosome. Where extrachromosomal maintenance is desired, an origin sequence is provided for the replication of the plasmid, which can be low or high copy-number. A wide variety of markers are available for selection, particularly those which protect against toxins, more particularly against antibiotics. The particular marker that is chosen is selected in accordance with the nature of the host, where, in some cases, complementation can be employed with auxotrophic hosts. Introduction of the DNA construct can use any convenient method, including, e.g., conjugation, bacterial transformation, calcium-precipitated DNA, electroporation, fusion, transfection, infection with viral vectors, biolistics, and the like.

Accordingly, polypeptides for use within the present disclosure in accordance with the claims can be produced in genetically engineered host cells according to conventional techniques. Suitable host cells are those cell types that can be transformed or transfected with exogenous DNA and grown in culture, and include bacteria, fungal cells, and cultured higher eukaryotic cells (including cultured cells of multicellular organisms), particularly cultured mammalian cells. Techniques for manipulating cloned DNA molecules and introducing exogenous DNA into a variety of host cells are disclosed by Sambrook and Russell, Molecular Cloning: A Laboratory Manual (3rd ed., Cold Spring Harbor Laboratory Press, Cold Spring Harbor, NY, 2001), and Ausubel et al., Short Protocols in Molecular Biology (4th ed., John Wiley & Sons, 1999). For example, the recombinant polypeptides of the disclosure can be expressed from bacterial *Escherichia coli* cells.

To direct a recombinant polypeptide into the secretory pathway of a host cell, a secretory signal sequence (also known as a leader sequence) can be provided in the expression vector. The secretory signal sequence can be that of the native form of the recombinant protein, or can be derived from another secreted protein or synthesized *de novo.* The secretory signal sequence is operably linked to the polypeptide-encoding DNA sequence, *i.e.,* the two sequences are joined in the correct reading frame and positioned to direct the newly synthesized polypeptide into the secretory pathway of the host cell. Secretory signal sequences are commonly positioned 5' to the DNA sequence encoding the polypeptide of interest, although certain signal sequences can be positioned elsewhere in the DNA sequence of interest *(see, e.g.,* Welch et al., U.S. Patent No. 5,037,743; Holland et al., U.S. Patent No. 5,143,830).

Cultured mammalian cells can be suitable hosts for production of recombinant polypeptides for use within the present disclosure. Methods for introducing exogenous DNA into mammalian host cells include calcium phosphate-mediated transfection (Wigler et al., Cell 14:725, 1978; Corsaro and Pearson, Somatic Cell Genetics 7:603, 1981: Graham and Van der Eb, Virology 52:456, 1973), electroporation (Neumann et al., EMBO J. 1:841-845, 1982), DEAE-dextran mediated transfection (Ausubel *et al., supra),* and liposome-mediated transfection (Hawley-Nelson et al., Focus 15:73, 1993; Ciccarone et al., Focus 15:80, 1993). The production of recombinant polypeptides in cultured mammalian cells is disclosed by, for example, Levinson et al., U.S. Patent No. 4,713,339; Hagen et al., U.S. Patent No. 4,784,950; Palmiter et al., U.S. Patent No. 4,579,821; and Ringold, U.S. Patent No. 4,656,134. Examples of suitable mammalian host cells include African green monkey kidney cells (Vero; ATCC CRL 1587), human embryonic kidney cells (293-HEK; ATCC CRL 1573), baby hamster kidney cells (BHK-21, BHK-570; ATCC CRL 8544, ATCC CRL 10314), canine kidney cells (MDCK; ATCC CCL 34), Chinese hamster ovary cells (CHO-K1; ATCC CCL61; CHO DG44; CHO DXB11 (Hyclone, Logan, UT); *see also, e.g.,* Chasin et al., Som. Cell. Molec. Genet. 12:555, 1986)), rat pituitary cells (GH1; ATCC CCL82), HeLa S3 cells (ATCC CCL2.2), rat hepatoma cells (H-4-II-E; ATCC CRL 1548) SV40-transformed monkey kidney cells (COS-1; ATCC CRL 1650) and murine embryonic cells (NIH-3T3; ATCC CRL 1658). Additional suitable cell lines are known in the art and available from public depositories such as the American Type Culture Collection, Manassas, Virginia. Strong transcription promoters can be used, such as promoters from SV-40 or cytomegalovirus. *See, e.g.,* U.S. Patent No. 4,956,288. Other suitable promoters include those from metallothionein genes (U.S. Patents Nos. 4,579,821 and 4,601,978) and the adenovirus major late promoter.

Drug selection is generally used to select for cultured mammalian cells into which foreign DNA has been inserted. Such cells are commonly referred to as "transfectants." Cells that have been cultured in the presence of the selective agent and are able to pass the gene of interest to their progeny are referred to as "stable transfectants." Exemplary selectable markers include a gene encoding resistance to the antibiotic neomycin, which allows selection to be carried out in the presence of a neomycin-type drug, such as G-418 or the like; the gpt gene for xanthine-guanine phosphoribosyl transferase, which permits host cell growth in the presence of mycophenolic acid/xanthine; and markers that provide resistance to zeocin, bleomycin, blastocidin, and hygromycin *(see, e.g.,* Gatignol et al., Mol. Gen. Genet. 207:342, 1987; Drocourt et al., Nucl. Acids Res. 18:4009, 1990). Selection systems can also be used to increase the expression level of the gene of interest, a process referred to as "amplification." Amplification is carried out by culturing transfectants in the presence of a low level of the selective agent and then increasing the amount of selective agent to select for cells that produce high levels of the products of the introduced genes. An exemplary amplifiable selectable marker is dihydrofolate reductase, which confers resistance to methotrexate. Other drug resistance genes (e.g., hygromycin resistance, multi-drug resistance, puromycin acetyltransferase) can also be used.

Other higher eukaryotic cells can also be used as hosts, including insect cells, plant cells and avian cells. The use of *Agrobacterium rhizogenes* as a vector for expressing genes in plant cells has been reviewed by Sinkar et al., J. Biosci. (Bangalore) 11:47-58, 1987. Transformation of insect cells and production of foreign polypeptides therein is disclosed by Guarino et al., US 5,162,222 and WO 94/06463.

Insect cells can be infected with recombinant baculovirus, commonly derived from *Autographa californica* nuclear polyhedrosis virus (AcNPV). *See* King and Possee, The Baculovirus Expression System: A Laboratory Guide (Chapman & Hall, London); O'Reilly et al., Baculovirus Expression Vectors: A Laboratory Manual (Oxford University Press., New York 1994); and Baculovirus Expression Protocols. Methods in Molecular Biology (Richardson ed., Humana Press, Totowa, NJ, 1995). Recombinant baculovirus can also be produced through the use of a transposon-based system described by Luckow et al. (J. Virol. 67:4566-4579, 1993). This system, which utilizes transfer vectors, is commercially available in kit form (BAC-TO-BAC kit; Life Technologies, Gaithersburg, MD). The transfer vector (e.g., PFASTBAC1; Life Technologies) contains a Tn7 transposon to move the DNA encoding the protein of interest into a baculovirus genome maintained in *E. coli* as a large plasmid called a "bacmid." *See* Hill-Perkins and Possee, J. Gen. Virol. 71:971-976, 1990; Bonning et al., J. Gen. Virol. 75:1551-1556, 1994; and Chazenbalk and Rapoport, J. Biol. Chem. 270:1543-1549, 1995. In addition, transfer vectors can include an in-frame fusion with DNA encoding a polypeptide extension or affinity tag as disclosed above. Using techniques known in the art, a transfer vector containing a protein-encoding DNA sequence is transformed into *E. coli* host cells, and the cells are screened for bacmids which contain an interrupted lacZ gene indicative of recombinant baculovirus. The bacmid DNA containing the recombinant baculovirus genome is isolated, using common techniques, and used to transfect *Spodoptera frugiperda* cells, such as Sf9 cells. Recombinant virus that expresses the protein or interest is subsequently produced. Recombinant viral stocks are made by methods commonly used in the art.

For protein production, a recombinant virus can be used to infect host cells, typically a cell line derived from the fall armyworm, *Spodoptera frugiperda* (*e.g.,* Sf9 or Sf21 cells) or *Trichoplusia ni* (*e.g.,* HIGH FIVE cells; Invitrogen, Carlsbad, CA). *See generally* Glick and Pasternak, Molecular Biotechnology, Principles & Applications of Recombinant DNA (ASM Press, Washington, D.C., 1994). *See also* U.S. Patent No. 5,300,435. Serum-free media are used to grow and maintain the cells. Suitable media formulations are known in the art and can be obtained from commercial suppliers. The cells are grown up from an inoculation density of approximately 2-5 x $10^5$ cells to a density of 1-2 x $10^6$ cells, at which time a recombinant viral stock is added at a multiplicity of infection (MOI) of 0.1 to 10, more typically near 3. Procedures used are generally described in available laboratory manuals *(see, e.g.,* King and Possee, *supra;* O'Reilly *et al., supra;* Richardson, *supra).*

Fungal cells, including yeast cells, can also be used within the present disclosure. Yeast species of in this regard include, e.g., *Saccharomyces cerevisiae, Pichia pastoris,* and *Pichia methanolica.* Methods for transforming *S. cerevisiae* cells with exogenous DNA and producing recombinant polypeptides therefrom are disclosed by, for example, Kawasaki, U.S. Patent No. 4,599,311; Kawasaki et al., U.S. Patent No. 4,931,373; Brake, U.S. Patent No. 4,870,008; Welch et al., U.S. Patent No. 5,037,743; and Murray et al., U.S. Patent No. 4,845,075. Transformed cells are selected by phenotype determined by the selectable marker, commonly drug resistance or the ability to grow in the absence of a particular nutrient (e.g., leucine). An exemplary vector system for use in *Saccharomyces cerevisiae* is the *POT1* vector system disclosed by Kawasaki et al. (U.S. Patent No. 4,931,373), which allows transformed cells to be selected by growth in glucose-containing media. Suitable promoters and terminators for use in yeast include those from glycolytic enzyme genes *(see, e.g.,* Kawasaki, U.S. Patent No. 4,599,311; Kingsman et al., U.S. Patent No. 4,615,974; and Bitter, U.S. Patent No. 4,977,092) and alcohol dehydrogenase genes. *See also* U.S. Patents Nos. 4,990,446; 5,063,154; 5,139,936; and 4,661,454. Transformation systems for other yeasts, including *Hansenula polymorpha, Schizosaccharomyces pombe, Kluyveromyces lactis, Kluyveromyces fragilis, Ustilago maydis, Pichia pastoris, Pichia methanolica, Pichia guillermondii,* and *Candida maltosa* are known in the art. *See, e.g.,* Gleeson et al., J. Gen. Microbiol. 132:3459-3465, 1986; Cregg, U.S. Patent No. 4,882,279; and Raymond et al., Yeast 14:11-23, 1998. *Aspergillus* cells can be utilized according to the methods of McKnight et al., U.S. Patent No. 4,935,349. Methods for transforming *Acremonium chrysogenum* are disclosed by Sumino et al., U.S. Patent No. 5,162,228. Methods for transforming Neurospora are disclosed by Lambowitz, U.S. Patent No. 4,486,533. Production of recombinant proteins in *Pichia methanolica* is disclosed in U.S. Patents Nos. 5,716,808; 5,736,383; 5,854,039; and 5,888,768.

Prokaryotic host cells, including strains of the bacteria *Escherichia coli, Bacillus,* and other genera are also useful host cells within the present disclosure. Techniques for transforming these hosts and expressing foreign DNA sequences cloned therein are well-known in the art *(see, e.g.,* Sambrook and Russell, *supra).* When expressing a recombinant protein in bacteria such as *E. coli,* the protein can be retained in the cytoplasm, typically as insoluble granules, or can be directed to the periplasmic space by a bacterial secretion sequence. In the former case, the cells are lysed, and the granules are recovered and denatured using, for example, guanidine isothiocyanate or urea. The denatured protein can then be refolded and dimerized by diluting the denaturant, such as by dialysis against a solution of urea and a combination of reduced and oxidized glutathione, followed by dialysis against a buffered saline solution. In the alternative, the protein can be recovered from the cytoplasm in soluble form and isolated without the use of denaturants. The protein is recovered from the cell as an aqueous extract in, for example, phosphate buffered saline. To capture the protein of interest, the extract is applied directly to a chromatographic medium, such as an immobilized antibody or heparin-Sepharose column. Secreted proteins can be recovered from the periplasmic space in a soluble and functional form by disrupting the cells (by, for example, sonication or osmotic shock) to release the contents of the periplasmic space and recovering the protein, thereby obviating the need for denaturation and refolding.

Transformed or transfected host cells are cultured according to conventional procedures in a culture medium containing nutrients and other components required for the growth of the chosen host cells. A variety of suitable media, including defined media and complex media, are known in the art and generally include a carbon source, a nitrogen source, essential amino acids, vitamins and minerals. Media can also contain such components as growth factors or serum, as required. The growth medium will generally select for cells containing the exogenously added DNA by, for example, drug selection or deficiency in an essential nutrient which is complemented by the selectable marker carried on the expression vector or co-transfected into the host cell.

The recombinant polypeptides can be purified by conventional protein purification methods, typically by a combination of chromatographic techniques. *See generally* Affinity Chromatography: Principles & Methods (Pharmacia LKB Biotechnology, Uppsala, Sweden, 1988); Scopes, Protein Purification: Principles and Practice (Springer-Verlag, New York 1994). Additional purification steps, such as gel filtration, can be used to obtain the desired level of purity or to provide for desalting, buffer exchange, and the like.

The present disclosure provides products for use in methods of preventing, delaying the progression of, treating or alleviating a symptom of, or otherwise ameliorating cancer in a subject in accordance with the claims.

The subject in need thereof can be a subject with a cell proliferation disorder. Cancers that can be treated include a primary, progressive, metastatic or recurrent tumor. Preferably, the tumor is a solid tumor. Cancers include for example, tumors of the central nervous system, a glioma tumor, renal cancer tumor, an ovarian cancer tumor, a head and neck cancer tumor, a liver cancer tumor, a pancreatic cancer tumor, a gastric cancer tumor, an esophageal cancer tumor, a bladder cancer tumor, a ureter cancer tumor, a renal pelvis cancer tumor, a urothelial cell cancer tumor, a urogenital cancer tumor, a cervical cancer tumor, a endometrial cancer tumor, a penile cancer tumor, a thyroid cancer tumor, or a prostate cancer tumor, a breast cancer tumor, a melanoma tumor, a glioma tumor, a colon cancer tumor, a lung cancer tumor, a sarcoma cancer tumor, or a squamous cell tumor, or a prostate cancer tumor. In one aspect the subject has lung cancer, colon cancer or breast cancer.

Also described are methods for enhancing or inducing an immune response in a subject in need thereof comprise administering at least one recombinant polypeptide, or a nucleic acid encoding a recombinant polypeptide. In one example, the at least one recombinant polypeptide comprises a recombinant polypeptide of SEQ ID NO: 1-8 or an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to any of the amino acid sequences of SEQ ID NO: 1-8 or an acidic variant thereof as described herein. In one example, the at least one recombinant polypeptide of the present disclosure comprises a recombinant polypeptide of SEQ ID NO: 9-16 or an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to any of the amino acid sequences of SEQ ID NO: 9-16. In a preferred example, the at least one recombinant polypeptide comprises a recombinant polypeptide of SEQ ID NO: 9.

In one example, enhancing or inducing an "immune response" can be, for example, a cytokine release response or a humoral (antigen-specific) immune response. The immune response to be enhanced for example, can be an innate immune response, a local immune response, a mucosal immune response or a systemic immune response. As used herein, the terms "enhance" or "enhancing" refer to strengthening (augmenting) of an existing immune response. The term "inducing" refers to the initiation of an immune response.

In one example, "immune response" refers to "immunogenic cell death" or "immunogenic apoptosis", which is characterized by a robust immune response against antigens expressed by dying cells (Figure 1). Dying cells, such as cancer cells, can have an increased expression of pre-apoptotic Damage-Associated-Molecular-Pattern (DAMP) signals comprising calreticulin (CRT), HSP70, HSP90, or a combination thereof. In a preferred example, the cells have increased expression of each of CRT, HSP70 and HSP90. Techniques known to one skilled in the art can be used to assess the expression of these cell surface markers. For example, the expression of the cell surface markers can be assessed using standard techniques such as flow cytometry, immunocytochemistry (e.g., staining with tissue specific or cell-marker specific antibodies), fluorescence activated cell sorting (FACS), magnetic activated cell sorting (MACS) or other similar methods known in the art. Fluorescence activated cell sorting (FACS) is a well-known method for separating particles, including cells, based on the fluorescent properties of the particles (Kamarch, 1987, Methods Enzymol, 151:150-165). Laser excitation of fluorescent moieties in the individual particles results in a small electrical charge allowing electromagnetic separation of positive and negative particles from a mixture. In one example, cell surface marker-specific antibodies or ligands are labeled with distinct fluorescent labels. Cells are processed through the flow cytometer, allowing separation of cells based on their ability to bind to the antibodies used. In one example, the method induces the expression of pre-apoptotic HSP70, HSP90 or calreticulin on a cell surface, such as a cancer cell surface.

In one example, "immunogenic cell death" or "immunogenic apoptosis" involves the interaction of dendritic cells with a cell, such as a cancer cell, leading to a more rapid rate of endogenous dendritic cell activation, dendritic cell maturation and phagocytosis. The recognition of pre-apoptotic DAMP signals comprising calreticulin (CRT), HSP70, HSP90, or a combination thereof, by the dendritic cells triggers "endogenous dendritic cell activation". This leads to "dendritic cell maturation", which comprises a redistribution of major histocompatibility complex (MHC) molecules from intracellular endocytic compartments to the dendritic cell surface, down-regulation of antigen internalization, an increase of surface expression of co-stimulatory molecules (including CD80 and CD86), cytoskeleton re-organization, secretion of chemokines, cytokines and proteases, surface expression of adhesion molecules and surface expression of chemokine receptors. Mature dendritic cells that have been exposed to cancer cells dying by immunogenic cell death can migrate to lymph nodes and induce high numbers of tumor-specific T lymphocytes (including CD4+ and CD8+ T cells). This triggers a targeted T-cell mediated response towards the cancer cell. The process of "immunogenic cell death" or "immunogenic apoptosis" is shown in Figure 1. A person skilled in the art will appreciate that not all techniques known to induce cell death will necessarily induce immunogenic cell death. Only agents inducing immunogenic cell death will elicit efficient endogenous dendritic cell activation. In one aspect an "immune response" refers to endogenous dendritic cell activation, dendritic cell maturation or T-cell mediated response or a combination thereof.

In one example, "apoptosis" is the term used to describe the cell signaling cascade known as programmed cell death. Various therapeutic indications exist for molecules that induce apoptosis (e.g. cancer). Apoptosis can be monitored by any of a number of available techniques known and available in the art including, for example, assays that measure fragmentation of DNA, alterations in membrane asymmetry, activation of apoptotic caspases and/or release of cytochrome C and AIF. In one example, apoptosis is measured by the activation and expression of Caspase 3/7.

Also described are methods for treating, preventing or alleviating at least one symptom of a cell proliferative disorder in a subject in need thereof. In one example, the method is alleviating at least one symptom of a cell proliferative disorder in a subject in need thereof. In a preferred aspect, the cancer is lung cancer, colon cancer or breast cancer.

Also described are methods for treating, preventing or alleviating at least one symptom of a cell proliferative disorder in a subject in need thereof comprise administering at least one recombinant polypeptide of the present disclosure, or a nucleic acid encoding a recombinant polypeptide. In one example, the at least one recombinant polypeptide of the present disclosure comprises a recombinant polypeptide of SEQ ID NO: 1-8 or an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to any of the amino acid sequences of SEQ ID NO: 1-8 or an acidic variant thereof as described herein. In one example, the at least one recombinant polypeptide of the present disclosure comprises a recombinant polypeptide of SEQ ID NO: 9-16. In a preferred example, the at least one recombinant polypeptide of the present disclosure comprises a recombinant polypeptide of SEQ ID NO: 9 or an amino acid sequence that is at least about 50%, about 55%, about 60%, about 65%, about 70%, about 75%, about 80%, about 85%, about 90%, about 91%, about 92%, about 93%, about 94%, about 95%, about 96%, about 97%, about 98% or about 99% identical to any of the amino acid sequences of SEQ ID NO: 9-16.

As used herein, a "subject" can be any mammal, e.g., a human, a primate, mouse, rat, dog, cat, cow, horse, pig, sheep, goat, camel. In a preferred aspect, the subject is a human. In one aspect, a "subject in need thereof" is a subject having a cell proliferative disorder, or a subject having an increased risk of developing a cell proliferative disorder relative to the population at large. In one aspect, a subject in need thereof has a precancerous condition. In a preferred aspect, a subject in need thereof has cancer.

As used herein, "treating" describes the management and care of a patient for the purpose of combating a disease, condition, or disorder and includes decreasing or alleviating the symptoms or complications, or eliminating the disease, condition or disorder. As used herein, "preventing" describes stopping the onset of the symptoms or complications of the disease, condition or disorder. As used herein, "alleviating" describes reducing the symptoms or complications of disease, condition or disorder.

As used herein, the term "cell proliferative disorder" refers to conditions in which unregulated or abnormal growth, or both, of cells can lead to the development of an unwanted condition or disease, which may or may not be cancerous. Exemplary cell proliferative disorders of the disclosure encompass a variety of conditions wherein cell division is deregulated. Exemplary cell proliferative disorder include, but are not limited to, neoplasms, benign tumors, malignant tumors, pre-cancerous conditions, in situ tumors, encapsulated tumors, metastatic tumors, liquid tumors, solid tumors, immunological tumors, hematological tumors, cancers, carcinomas, leukemias, lymphomas, sarcomas, and rapidly dividing cells. The term "rapidly dividing cell" as used herein is defined as any cell that divides at a rate that exceeds or is greater than what is expected or observed among neighboring or juxtaposed cells within the same tissue. A cell proliferative disorder includes a precancer or a precancerous condition. A cell proliferative disorder includes cancer. Preferably, the methods provided herein are used to treat or alleviate a symptom of cancer. The term "cancer" includes solid tumors, as well as, hematologic tumors and/or malignancies. A "precancer cell" or "precancerous cell" is a cell manifesting a cell proliferative disorder that is a precancer or a precancerous condition. A "cancer cell" or "cancerous cell" is a cell manifesting a cell proliferative disorder that is a cancer. Any reproducible means of measurement may be used to identify cancer cells or precancerous cells. Cancer cells or precancerous cells can be identified by histological typing or grading of a tissue sample (e.g., a biopsy sample). Cancer cells or precancerous cells can be identified through the use of appropriate molecular markers.

Exemplary non-cancerous conditions or disorders include, but are not limited to, rheumatoid arthritis; inflammation; autoimmune disease; lymphoproliferative conditions; acromegaly; rheumatoid spondylitis; osteoarthritis; gout, other arthritic conditions; sepsis; septic shock; endotoxic shock; gram-negative sepsis; toxic shock syndrome; asthma; adult respiratory distress syndrome; chronic obstructive pulmonary disease; chronic pulmonary inflammation; inflammatory bowel disease; Crohn's disease; psoriasis; eczema; ulcerative colitis; pancreatic fibrosis; hepatic fibrosis; acute and chronic renal disease; irritable bowel syndrome; pyresis; restenosis; cerebral malaria; stroke and ischemic injury; neural trauma; Alzheimer's disease; Huntington's disease; Parkinson's disease; acute and chronic pain; allergic rhinitis; allergic conjunctivitis; chronic heart failure; acute coronary syndrome; cachexia; malaria; leprosy; leishmaniasis; Lyme disease; Reiter's syndrome; acute synovitis; muscle degeneration, bursitis; tendonitis; tenosynovitis; herniated, ruptures, or prolapsed intervertebral disk syndrome; osteopetrosis; thrombosis; restenosis; silicosis; pulmonary sarcosis; bone resorption diseases, such as osteoporosis; graft-versus-host reaction; Multiple Sclerosis; lupus; fibromyalgia; AIDS and other viral diseases such as Herpes Zoster, Herpes Simplex I or II, influenza virus and cytomegalovirus; and diabetes mellitus.

Exemplary cancers include, but are not limited to, adrenocortical carcinoma, AIDS-related cancers, AIDS-related lymphoma, anal cancer, anorectal cancer, cancer of the anal canal, appendix cancer, childhood cerebellar astrocytoma, childhood cerebral astrocytoma, basal cell carcinoma, skin cancer (non-melanoma), biliary cancer, extrahepatic bile duct cancer, intrahepatic bile duct cancer, bladder cancer, urinary bladder cancer, bone and joint cancer, osteosarcoma and malignant fibrous histiocytoma, brain cancer, brain tumor, brain stem glioma, cerebellar astrocytoma, cerebral astrocytoma/malignant glioma, ependymoma, medulloblastoma, supratentorial primitive neuroectodeimal tumors, visual pathway and hypothalamic glioma, breast cancer, bronchial adenomas/carcinoids, carcinoid tumor, gastrointestinal, nervous system cancer, nervous system lymphoma, central nervous system cancer, central nervous system lymphoma, cervical cancer, childhood cancers, chronic lymphocytic leukemia, chronic myelogenous leukemia, chronic myeloproliferative disorders, colon cancer, colorectal cancer, cutaneous T-cell lymphoma, lymphoid neoplasm, mycosis fungoides, Seziary Syndrome, endometrial cancer, esophageal cancer, extracranial germ cell tumor, extragonadal germ cell tumor, extrahepatic bile duct cancer, eye cancer, intraocular melanoma, retinoblastoma, gallbladder cancer, gastric (stomach) cancer, gastrointestinal carcinoid tumor, gastrointestinal stromal tumor (GIST), germ cell tumor, ovarian germ cell tumor, gestational trophoblastic tumor glioma, head and neck cancer, hepatocellular (liver) cancer, Hodgkin lymphoma, hypopharyngeal cancer, intraocular melanoma, ocular cancer, islet cell tumors (endocrine pancreas), Kaposi's sarcoma, kidney cancer, renal cancer, laryngeal cancer, acute lymphoblastic leukemia, acute myeloid leukemia, chronic lymphocytic leukemia, chronic myelogenous leukemia, hairy cell leukemia, lip and oral cavity cancer, liver cancer, lung cancer, non-small cell lung cancer, small cell lung cancer, AIDS-related lymphoma, non-Hodgkin lymphoma, primary central nervous system lymphoma, Waldenstram macroglobulinemia, medulloblastoma, melanoma, intraocular (eye) melanoma, merkel cell carcinoma, mesothelioma malignant, mesothelioma, metastatic squamous neck cancer, mouth cancer, cancer of the tongue, multiple endocrine neoplasia syndrome, mycosis fungoides, myelodysplastic syndromes, myelodysplastic/ myeloproliferative diseases, chronic myelogenous leukemia, acute myeloid leukemia, multiple myeloma, chronic myeloproliferative disorders, nasopharyngeal cancer, neuroblastoma, oral cancer, oral cavity cancer, oropharyngeal cancer, ovarian cancer, ovarian epithelial cancer, ovarian low malignant potential tumor, pancreatic cancer, islet cell pancreatic cancer, paranasal sinus and nasal cavity cancer, parathyroid cancer, penile cancer, pharyngeal cancer, pheochromocytoma, pineoblastoma and supratentorial primitive neuroectodermal tumors, pituitary tumor, plasma cell neoplasm/multiple myeloma, pleuropulmonary blastoma, prostate cancer, rectal cancer, renal pelvis and ureter, transitional cell cancer, retinoblastoma, rhabdomyosarcoma, salivary gland cancer, ewing family of sarcoma tumors, Kaposi Sarcoma, uterine cancer, uterine sarcoma, skin cancer (non-melanoma), skin cancer (melanoma), merkel cell skin carcinoma, small intestine cancer, soft tissue sarcoma, squamous cell carcinoma, stomach (gastric) cancer, supratentorial primitive neuroectodermal tumors, testicular cancer, throat cancer, thymoma, thymoma and thymic carcinoma, thyroid cancer, transitional cell cancer of the renal pelvis and ureter and other urinary organs, gestational trophoblastic tumor, urethral cancer, endometrial uterine cancer, uterine sarcoma, uterine corpus cancer, vaginal cancer, vulvar cancer, and Wilm's Tumor.

A "lung cancer" is a cell proliferative disorder involving cells of the lung. In one aspect, lung cancer include all forms of cell proliferative disorders affecting lung cells. In one aspect, lung cancer include lung cancer, a precancer or precancerous condition of the lung, benign growths or lesions of the lung, and malignant growths or lesions of the lung, and metastatic lesions in tissue and organs in the body other than the lung. In a preferred aspect, the method of the present disclosure may be used to treat lung cancer or cell proliferative disorders of the lung. In one aspect, lung cancer includes all forms of cancer of the lung. In another aspect, lung cancer includes malignant lung neoplasms, carcinoma in situ, typical carcinoid tumors, and atypical carcinoid tumors. In another aspect, lung cancer includes small cell lung cancer ("SCLC"), non-small cell lung cancer ("NSCLC"), squamous cell carcinoma, adenocarcinoma, small cell carcinoma, large cell carcinoma, adenosquamous cell carcinoma, and mesothelioma. In another aspect, lung cancer includes "scar carcinoma," bronchioalveolar carcinoma, giant cell carcinoma, spindle cell carcinoma, and large cell neuroendocrine carcinoma. In one aspect lung cancer includes stage 0, IA, IB, IIA, IIB, IIIA, IIIB and IV lung cancer. In another aspect, lung cancer includes lung neoplasms having histologic and ultrastructual heterogeneity (e.g., mixed cell types).

In one aspect, lung cancer include all forms of cell proliferative disorders affecting lung cells. In one aspect, cell proliferative disorders of the lung include lung cancer, precancerous conditions of the lung. In one aspect, cell proliferative disorders of the lung include hyperplasia, metaplasia, and dysplasia of the lung. In another aspect, lung cancer include asbestos-induced hyperplasia, squamous metaplasia, and benign reactive mesothelial metaplasia. In another aspect, cell proliferative disorders of the lung include replacement of columnar epithelium with stratified squamous epithelium, and mucosal dysplasia. In another aspect, individuals exposed to inhaled injurious environmental agents such as cigarette smoke and asbestos may be at increased risk for developing cell proliferative disorders of the lung. In another aspect, prior lung diseases that may predispose individuals to development of cell proliferative disorders of the lung include chronic interstitial lung disease, necrotizing pulmonary disease, scleroderma, rheumatoid disease, sarcoidosis, interstitial pneumonitis, tuberculosis, repeated pneumonias, idiopathic pulmonary fibrosis, granulomata, asbestosis, fibrosing alveolitis, and Hodgkin's disease.

A "colon cancer" is a cell proliferative disorder involving cells of the colon. In a preferred aspect, the products for use in the method of the present disclosure in accordance with the claims may be used to treat colon cancer or cell proliferative disorders of the colon. In one aspect, colon cancer includes all forms of cancer of the colon. In another aspect, colon cancer includes sporadic and hereditary colon cancers. In another aspect, colon cancer includes malignant colon neoplasms, carcinoma in situ, typical carcinoid tumors, and atypical carcinoid tumors. In another aspect, colon cancer includes adenocarcinoma, squamous cell carcinoma, and adenosquamous cell carcinoma. In another aspect, colon cancer is associated with a hereditary syndrome selected from the group consisting of hereditary nonpolyposis colorectal cancer, familial adenomatous polyposis, Gardner's syndrome, Peutz-Jeghers syndrome, Turcot's syndrome and juvenile polyposis. In another aspect, colon cancer is caused by a hereditary syndrome selected from the group consisting of hereditary nonpolyposis colorectal cancer, familial adenomatous polyposis, Gardner's syndrome, Peutz-Jeghers syndrome, Turcot's syndrome and juvenile polyposis.

In one aspect, colon cancer include all forms of cell proliferative disorders affecting colon cells. In one aspect, colon cancer include colon cancer, precancerous conditions of the colon, adenomatous polyps of the colon and metachronous lesions of the colon. In one aspect colon cancer includes stage 0, I, IIA, IIB, IIC, IIIA, IIIB, IIIC, IVA, IVB and IVC colon cancer. In one aspect, a colon cancer includes adenoma. In one aspect, colon cancer is characterized by hyperplasia, metaplasia or dysplasia of the colon. In another aspect, prior colon diseases that may predispose individuals to development of cell proliferative disorders of the colon include prior colon cancer. In another aspect, current disease that may predispose individuals to development of cell proliferative disorders of the colon include Crohn's disease and ulcerative colitis. In one aspect, a cell proliferative disorder of the colon is associated with a mutation in a gene selected from the group consisting of p53, ras, FAP and DCC. In another aspect, an individual has an elevated risk of developing a cell proliferative disorder of the colon due to the presence of a mutation in a gene selected from the group consisting of p53, ras, FAP and DCC.

A "breast cancer" is a cell proliferative disorder involving cells of the breast. In a preferred aspect, breast cancer include all forms of cell proliferative disorders affecting breast cells. In one aspect, breast cancer include breast cancer, a precancer or precancerous condition of the breast, benign growths or lesions of the breast, and malignant growths or lesions of the breast, and metastatic lesions in tissue and organs in the body other than the breast. In another aspect, breast cancer include hyperplasia, metaplasia, and dysplasia of the breast.

In one aspect, breast cancer is a precancerous condition of the breast. In one aspect, the products for use in the method of the present disclosure in accordance with the claims may be used to treat a precancerous condition of the breast. In one aspect, a precancerous condition of the breast includes atypical hyperplasia of the breast, ductal carcinoma in situ (DCIS), intraductal carcinoma, lobular carcinoma in situ (LCIS), lobular neoplasia, and stage 0 or grade 0 growth or lesion of the breast (e.g., stage 0 or grade 0 breast cancer, or carcinoma in situ). In another aspect, a precancerous condition of the breast has been staged according to the TNM classification scheme as accepted by the American Joint Committee on Cancer (AJCC), where the primary tumor (T) has been assigned a stage of T0 or Tis; and where the regional lymph nodes (N) have been assigned a stage of N0; and where distant metastasis (M) has been assigned a stage of M0.

In one aspect, the products for use in the method of the present disclosure in accordance with the claims may be used to treat breast cancer. In one aspect, breast cancer includes all forms of cancer of the breast. In one aspect, breast cancer includes primary epithelial breast cancers. In another aspect, breast cancer includes cancers in which the breast is involved by other tumors such as lymphoma, sarcoma or melanoma. In another aspect, breast cancer includes carcinoma of the breast, ductal carcinoma of the breast, lobular carcinoma of the breast, undifferentiated carcinoma of the breast, cystosarcoma phyllodes of the breast, angiosarcoma of the breast, and primary lymphoma of the breast. In one aspect, breast cancer includes Stage I, II, IIIA, IIIB, IIIC and IV breast cancer. In one aspect, ductal carcinoma of the breast includes invasive carcinoma, invasive carcinoma in situ with predominant intraductal component, inflammatory breast cancer, and a ductal carcinoma of the breast with a histologic type selected from the group consisting of comedo, mucinous (colloid), medullary, medullary with lymphcytic infiltrate, papillary, scirrhous, and tubular. In one aspect, lobular carcinoma of the breast includes invasive lobular carcinoma with predominant in situ component, invasive lobular carcinoma, and infiltrating lobular carcinoma. In one aspect, breast cancer includes Paget's disease, Paget's disease with intraductal carcinoma, and Paget's disease with invasive ductal carcinoma. In another aspect, breast cancer includes breast neoplasms having histologic and ultrastructual heterogeneity (e.g., mixed cell types).

Epithelial tumour size is about 1 x $10^9$ cells/1 cm$^3$, assuming cell diameter 10 $\mu$m (Del Monte et. al., Cell Cycle, 505-506, 2009). Normal cell diameter 5-10 $\mu$m (Del Monte et. al., Cell Cycle, 505-506, 2009). Tumour cell diameters can be ~20 $\mu$m (Del Monte et. al., Cell Cycle, 505-506, 2009, Backman et. al., Nature, 35-36, 2000). Therefore, the epithelial tumour size could be about 1.25 x $10^8$ cells/1 cm$^3$, assuming cell diameter 20 $\mu$m (Del Monte et. al., Cell Cycle, 505-506, 2009). A typical ellipsoid volume can be calculated using for following formula: $\pi$/6 x (Length) x (Width) x (Height) (For simplicity, L = W = H). Exemplary calculations for number of cells correlated to tumour sizes are displayed in Table 2.

Table 2. Number of cells in tumours by tumour diameter

| Tumour diameter (cm) | Number of Cells |
|---|---|
| 1 | ~6.25 x $10^7$ cells |
| 2 | ~5 x $10^8$ cells |
| 3 | ~1.7 x $10^9$ cells |
| 4 | ~4.1 x $10^9$ cells |
| 4.3 | ~5 x $10^9$ cells |
| 5 | ~7.8 x $10^9$ cells |
| 6 | ~1.35 x $10^{10}$ cells |
| 7 | ~2.1 x $10^{10}$ cells |
| 8 | ~3.2 x $10^{10}$ cells |
| 9 | ~4.6 x $10^{10}$ cells |
| 10 | ~6.25 x $10^{10}$ cells |

In one aspect, treating cancer results in a reduction in size of a tumor. A reduction in size of a tumor may also be referred to as "tumor regression." Preferably, after treatment, tumor size is reduced by 5% or greater relative to its size prior to treatment; more preferably, tumor size is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75% or greater. Size of a tumor may be measured by any reproducible means of measurement. In a preferred aspect, size of a tumor may be measured as a diameter of the tumor.

In another aspect, treating cancer results in a reduction in tumor volume. Preferably, after treatment, tumor volume is reduced by 5% or greater relative to its size prior to treatment; more preferably, tumor volume is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75% or greater. Tumor volume may be measured by any reproducible means of measurement.

In another aspect, treating cancer results in a decrease in number of tumors. Preferably, after treatment, tumor number is reduced by 5% or greater relative to number prior to treatment; more preferably, tumor number is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75%. Number of tumors may be measured by any reproducible means of measurement. In a preferred aspect, number of tumors may be measured by counting tumors visible to the naked eye or at a specified magnification. In a preferred aspect, the specified magnification is 2x, 3x, 4x, 5x, 10x, or 50x.

In another aspect, treating cancer results in a decrease in number of metastatic lesions in other tissues or organs distant from the primary tumor site. Preferably, after treatment, the number of metastatic lesions is reduced by 5% or greater relative to number prior to treatment; more preferably, the number of metastatic lesions is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75%. The number of metastatic lesions may be measured by any reproducible means of measurement. In a preferred aspect, the number of metastatic lesions may be measured by counting metastatic lesions visible to the naked eye or at a specified magnification. In a preferred aspect, the specified magnification is 2x, 3x, 4x, 5x, 10x, or 50x.

In another aspect, treating cancer results in an increase in average survival time of a population of treated subjects in comparison to a population receiving carrier alone. Preferably, the average survival time is increased by more than 30 days; more preferably, by more than 60 days; more preferably, by more than 90 days; and most preferably, by more than 120 days. An increase in average survival time of a population may be measured by any reproducible means. In a preferred aspect, an increase in average survival time of a population may be measured, for example, by calculating for a population the average length of survival following initiation of treatment with an active compound. In another preferred aspect, an increase in average survival time of a population may also be measured, for example, by calculating for a population the average length of survival following completion of a first round of treatment with an active compound.

In another aspect, treating cancer results in an increase in average survival time of a population of treated subjects in comparison to a population of untreated subjects. Preferably, the average survival time is increased by more than 30 days; more preferably, by more than 60 days; more preferably, by more than 90 days; and most preferably, by more than 120 days. An increase in average survival time of a population may be measured by any reproducible means. In a preferred aspect, an increase in average survival time of a population may be measured, for example, by calculating for a population the average length of survival following initiation of treatment with an active compound. In another preferred aspect, an increase in average survival time of a population may also be measured, for example, by calculating for a population the average length of survival following completion of a first round of treatment with an active compound.

In another aspect, treating cancer results in increase in average survival time of a population of treated subjects in comparison to a population receiving a therapy that is not a recombinant polypeptide of the present disclosure. Preferably, the average survival time is increased by more than 30 days; more preferably, by more than 60 days; more preferably, by more than 90 days; and most preferably, by more than 120 days. An increase in average survival time of a population may be measured by any reproducible means. In a preferred aspect, an increase in average survival time of a population may be measured, for example, by calculating for a population the average length of survival following initiation of treatment with an active compound. In another preferred aspect, an increase in average survival time of a population may also be measured, for example, by calculating for a population the average length of survival following completion of a first round of treatment with an active compound.

In another aspect, treating cancer results in a decrease in the mortality rate of a population of treated subjects in comparison to a population receiving carrier alone. In another aspect, treating cancer results in a decrease in the mortality rate of a population of treated subjects in comparison to an untreated population. In a further aspect, treating cancer results in a decrease in the mortality rate of a population of treated subjects in comparison to a population receiving monotherapy with a drug that is not a recombinant polypeptide of the present disclosure. Preferably, the mortality rate is decreased by more than 2%; more preferably, by more than 5%; more preferably, by more than 10%; and most preferably, by more than 25%. In a preferred aspect, a decrease in the mortality rate of a population of treated subjects may be measured by any reproducible means. In another preferred aspect, a decrease in the mortality rate of a population may be measured, for example, by calculating for a population the average number of disease-related deaths per unit time following initiation of treatment with an active compound. In another preferred aspect, a decrease in the mortality rate of a population may also be measured, for example, by calculating for a population the average number of disease-related deaths per unit time following completion of a first round of treatment with an active compound.

In another aspect, treating cancer results in a decrease in tumor growth rate. Preferably, after treatment, tumor growth rate is reduced by at least 5% relative to number prior to treatment; more preferably, tumor growth rate is reduced by at least 10%; more preferably, reduced by at least 20%; more preferably, reduced by at least 30%; more preferably, reduced by at least 40%; more preferably, reduced by at least 50%; even more preferably, reduced by at least 50%; and most preferably, reduced by at least 75%. Tumor growth rate may be measured by any reproducible means of measurement. In a preferred aspect, tumor growth rate is measured according to a change in tumor diameter per unit time.

In another aspect, treating cancer results in a decrease in tumor regrowth. Preferably, after treatment, tumor regrowth is less than 5%; more preferably, tumor regrowth is less than 10%; more preferably, less than 20%; more preferably, less than 30%; more preferably, less than 40%; more preferably, less than 50%; even more preferably, less than 50%; and most preferably, less than 75%. Tumor regrowth may be measured by any reproducible means of measurement. In a preferred aspect, tumor regrowth is measured, for example, by measuring an increase in the diameter of a tumor after a prior tumor shrinkage that followed treatment. In another preferred aspect, a decrease in tumor regrowth is indicated by failure of tumors to reoccur after treatment has stopped.

In another aspect, treating, preventing, or alleviating a cancer results in a reduction in the rate of cellular proliferation. Preferably, after treatment, the rate of cellular proliferation is reduced by at least 5%; more preferably, by at least 10%; more preferably, by at least 20%; more preferably, by at least 30%; more preferably, by at least 40%; more preferably, by at least 50%; even more preferably, by at least 50%; and most preferably, by at least 75%. The rate of cellular proliferation may be measured by any reproducible means of measurement. In a preferred aspect, the rate of cellular proliferation is measured, for example, by measuring the number of dividing cells in a tissue sample per unit time.

In another aspect, treating, preventing, or alleviating a cancer results in a reduction in the proportion of proliferating cells. Preferably, after treatment, the proportion of proliferating cells is reduced by at least 5%; more preferably, by at least 10%; more preferably, by at least 20%; more preferably, by at least 30%; more preferably, by at least 40%; more preferably, by at least 50%; even more preferably, by at least 50%; and most preferably, by at least 75%. The proportion of proliferating cells may be measured by any reproducible means of measurement. In a preferred aspect, the proportion of proliferating cells is measured, for example, by quantifying the number of dividing cells relative to the number of nondividing cells in a tissue sample. In another preferred aspect, the proportion of proliferating cells is equivalent to the mitotic index.

In another aspect, treating, preventing, or alleviating a cancer results in a decrease in size of an area or zone of cellular proliferation. Preferably, after treatment, size of an area or zone of cellular proliferation is reduced by at least 5% relative to its size prior to treatment; more preferably, reduced by at least 10%; more preferably, reduced by at least 20%; more preferably, reduced by at least 30%; more preferably, reduced by at least 40%; more preferably, reduced by at least 50%; even more preferably, reduced by at least 50%; and most preferably, reduced by at least 75%. Size of an area or zone of cellular proliferation may be measured by any reproducible means of measurement. In a preferred aspect, size of an area or zone of cellular proliferation may be measured as a diameter or width of an area or zone of cellular proliferation.

In another aspect, treating, preventing, or alleviating a cancer results in a decrease in the number or proportion of cells having an abnormal appearance or morphology. Preferably, after treatment, the number of cells having an abnormal morphology is reduced by at least 5% relative to its size prior to treatment; more preferably, reduced by at least 10%; more preferably, reduced by at least 20%; more preferably, reduced by at least 30%; more preferably, reduced by at least 40%; more preferably, reduced by at least 50%; even more preferably, reduced by at least 50%; and most preferably, reduced by at least 75%. An abnormal cellular appearance or morphology may be measured by any reproducible means of measurement. In one aspect, an abnormal cellular morphology is measured by microscopy, e.g., using an inverted tissue culture microscope. In one aspect, an abnormal cellular morphology takes the form of nuclear pleiomorphism.

In one aspect, treating cancer or a cell proliferative disorder results in cell death, and preferably, cell death results in a decrease of at least 10% in number of cells in a population. More preferably, cell death means a decrease of at least 20%; more preferably, a decrease of at least 30%; more preferably, a decrease of at least 40%; more preferably, a decrease of at least 50%; most preferably, a decrease of at least 75%. Number of cells in a population may be measured by any reproducible means. In one aspect, number of cells in a population is measured by fluorescence activated cell sorting (FACS). In another aspect, number of cells in a population is measured by immunofluorescence microscopy. In another aspect, number of cells in a population is measured by light microscopy. In another aspect, methods of measuring cell death are as shown in Li et al., (2003) Proc Natl Acad Sci U S A. 100(5): 2674-8. In a preferred aspect, cell death occurs by immunogenic cell death.

The term "subject," or "individual" or "patient" as used herein in reference to individuals having a disease or disorder or are suspected of having a disease or disorder, and the like. Subject, individual or patent may be used interchangeably in the disclosure and encompass mammals and non-mammals. The subject is a pediatric patient or an adult patient.

Examples of mammals include, but are not limited to, any member of the Mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish and the like. In some aspects of the methods and compositions provided herein, the mammal is a human.

The therapeutic methods of the disclosure involve *in vivo* administration of recombinant polypeptide to a subject. The recombinant polypeptide may be administered to the subject administered by any suitable method, either systemically (e.g., orally, intravenously) or locally (e.g., intraperitoneally, intrathecally, intraventricularly, direct injection into the tissue or organ where the disease or disorder is occurring). Preferably, the recombinant polypeptide is administered intravenously.

The recombinant polypeptide is administered in a single dose or multiple doses, e.g. 2, 3, 4, 5, 6, 7, 8, 9, 10, 11, 12, 13, 14, 15, 16, 17, 18, 19, 20, 21, 22, 23, 24, 25, 26, 27, 28, 29 or 30 or more dose administrations. In some aspects, an effective amount of recombinant polypeptide in the first infusion, the second infusion, the third infusion, the fourth infusion, the fifth infusion and/or the sixth infusion comprises about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg about 25 mg/kg, about 26 mg/kg, about 27 mg/kg, about 28 mg/kg, about 29 mg/kg, about 30 mg/kg, about 31 mg/kg, about 32 mg/kg, about 33 mg/kg, about 34 mg/kg or about 35 mg/kg. In some aspects, the total dose of the first infusion and the second infusion is no more than 40 mg/kg. In some aspects, the total dose of the third infusion and the fourth infusion is no more than 40 mg/kg. In some aspects, the total dose of the fifth infusion and the sixth infusion is no more than 40 mg/kg. In some aspects, the dose of the first infusion, the third infusion and/or the fifth infusion is about 20 mg/kg to about 22 mg/kg. In some aspects, the dose of the second infusion, the fourth infusion and/or the sixth infusion is about 14 mg/kg to about 20 mg/kg. In some aspects, an effective amount of recombinant polypeptide in the first infusion, the second infusion, the third infusion, the fourth infusion, the fifth infusion and/or the sixth infusion comprises about 10 mg/kg to about 30 mg/kg. In a preferred aspect, an effective amount of the recombinant polypeptide the first infusion, the second infusion, the third infusion, the fourth infusion, the fifth infusion and/or the sixth infusion comprises about 20 mg/kg.

The term "infusion" or "infusions" as used herein refers to the administration of any agent or compound through a needle or a catheter. Infusion or infusions can comprise injection of any agent or compound into a vein or tissue, for example administration can be intravenously, subcutaneously, intramuscularly or epidurally.

The therapeutic methods of the disclosure involve *in vivo* administration of an IL-10 inhibitory agent to a subject in accordance with the claims. The IL-10 inhibitory agent may be administered to the subject administered by any suitable method, either systemically (e.g., orally, intravenously) or locally (e.g., intraperitoneally, intrathecally, intraventricularly, direct injection into the tissue or organ where the disease or disorder is occurring). Preferably, the IL-10 inhibitory agent is administered intravenously.

In one embodiments, recombinant polypeptides, recombinant polypeptide dosages and regimens include those known in the art, such as described in U.S. Patent Nos. 10,150,801, 10,323,071, 10,351,607, 10,301,364 and 10,370,421; U.S. Patent Application Nos. 16/551,659 and 16/443,517; and PCT application Nos. PCT/SG2019/050420 and PCT/SG2017/050648.

The IL-10 inhibitory agent may be an interferon gamma (IFNg) or an IFNg mimetic or a combination thereof. Preferably, the agent that reduces IL-10 is interferon gamma (IFNg). In some aspects, the IFNg is administered at a dose of about 10, about 15, about 20, about 25, about 30, about 35, about 40, about 45, about 50, about 55, about 60, about 65, about 70, about 75, about 80, about 85, about 90, about 95 or about 100 or more $\mu$g. In some aspects, the IFNg is administered at a dose of about 50 $\mu$g to about 100 $\mu$g. In a preferred aspect, the IFNg is administered at a dose of about 70 $\mu$g.

The products for use in the therapeutic methods in accordance with the claims involve *in vivo* administration of an immune cell growth factor to a subject. The immune cell growth factor may be administered to the subject administered by any suitable method, either systemically (e.g., orally, intravenously) or locally (e.g., intraperitoneally, intrathecally, intraventricularly, direct injection into the tissue or organ where the disease or disorder is occurring). Preferably, an immune cell growth factor is administered intravenously.

The at least one immune cell growth factor is FMS-like tyrosine kinase 3 ligand (FLT3L) or granulocyte-macrophage colony stimulating factor (GM-CSF). In some aspects, the at least one immune cell growth factor is FLT3L. In some aspects, FLT3L is administered in an amount of about 1 $\mu$g/kg, about 2 $\mu$g/kg, about 3 $\mu$g/kg, about 4 $\mu$g/kg, about 5 $\mu$g/kg, about 6 $\mu$g/kg, about 7 $\mu$g/kg, about 8 $\mu$g/kg, about 9 $\mu$g/kg, 10 $\mu$g/kg, about 11 $\mu$g/kg, about 12 $\mu$g/kg, about 13 $\mu$g/kg, about 14 $\mu$g/kg, about 15 $\mu$g/kg, about 16 $\mu$g/kg, about 17 $\mu$g/kg, about 18 $\mu$g/kg, about 19 $\mu$g/kg, 20 $\mu$g/kg. In some aspects, the FLT3L is in an amount of about 4 $\mu$g/kg to about 13 $\mu$g/kg. In some aspects, the FLT3L is in an amount of about 7 $\mu$g/kg.

The products for use in the therapeutic methods according to the claims involve *in vivo* administration of a TNFa inhibitory agent to a subject. The TNFa inhibitory agent may be administered to the subject administered by any suitable method, either systemically (e.g., orally, intravenously) or locally (e.g., intraperitoneally, intrathecally, intraventricularly, direct injection into the tissue or organ where the disease or disorder is occurring). Preferably, a TNFa inhibitory agent is administered subcutaneously.

In some aspects, the TNFa inhibitory agent is a tumor necrosis factor alpha (TNFa) inhibitor. In some aspects, the TNFa inhibitor is a TNFaR. In some aspects, the TNFa inhibitor is a TNFaR, etanercept, infliximab, adalimumab, certolizumab pegol or golimumab. In a preferred aspect, the TNFaR is etanercept. In some aspects, TNFaR is administered at a dose of about 1 mg, about 2 mg, about 2 mg, about 3 mg, about 4 mg, about 5 mg, about 6 mg, about 7 mg, about 8 mg, about 9 mg or about 10 mg. In some aspects, the TNFaR is administered at a dose of about 4 mg to about 6 mg. In a preferred aspect, the TNFaR is administered at a dose of about 5 mg.

In one embodiment, a product for use in treating a cancer subject in accordance with the claims includes administering an immunotherapy agent. The immunotherapy agent is a checkpoint inhibitor. In one embodiment, the administration of the immunotherapy agent does not cause toxic cytokine release or "cytokine release syndrome" (CRS) or "severe cytokine release syndrome" (sCRS) or "cytokine storm" that may occur in the subject. In one embodiment, the administration of the immunotherapy agent causes toxic cytokine release or "cytokine release syndrome" (CRS) or "severe cytokine release syndrome" (sCRS) or "cytokine storm" that may occur in the subject. In one embodiment, the CRS, sCRS or cytokine storm occurs as a result of administration of a immunotherapy agent. In one embodiment, a "cytokine cascade", or "hypercytokinemia" is a more severe form of cytokine release syndrome. In one embodiment, "hypercytokinemia" is a sustained 3-day cytokine storm.

In one embodiment, severe cytokine release syndrome (sCRS) is characterized by elevated levels of several inflammatory cytokines, multiple organ dysfunction and fever. In one embodiment, the cytokine measured in the cytokine release syndrome is IL-6. In one embodiment, the fever is transient. In one embodiment, the fever is sustained for a duration of at least one or more days. In one embodiment, the fever is sustained for a duration of three days. In one embodiment, the fever is at least about $38^0$C. In one embodiment, the temperature of the fever is at least about 38°C, 39°C, 40°C, 41°C or 42°C. In one embodiment, the C-Reactive Protein (CRP) level in the blood from the subject with sCRS is at least about 170, 171, 172, 173, 174, 175, 176, 177, 178, 179, 180, 181, 182, 183, 184, 185, 186, 187, 188, 189 or 190 mg/L.

In a preferred embodiment, the CRP level is at least about 180 mg/mL. In one embodiment, a single-day event may not result in sCRS. In one embodiment, a two-day cytokine storm develops into sCRS on the third day.

In one embodiment, hypercytokinemia is characterized by elevated levels of several inflammatory cytokines and fever. In one embodiment, the cytokine measured in the cytokine release syndrome is IL-6. In one embodiment, the fever is transient. In one embodiment, the fever occurs for a duration of at least one or more days. In one embodiment, the fever is at least about 40°C. In one embodiment, the temperature of the fever is at least about 38°C, 39°C, 40°C, 41°C or 42°C. In one embodiment, the C-Reactive Protein (CRP) level in the blood from a subject with hypercytokinemia is at least about 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229 or 230 mg/L. In a preferred embodiment, the CRP level is about 200 to about 220 mg/mL.

In one embodiment, a life threatening multiple organ dysfunction and adverse event is characterized by a severe cytokine storm. In one embodiment, the cytokine measured in the cytokine release syndrome is IL-6. In one embodiment, the fever is at least about 40°C. In one embodiment, the temperature of the fever is at least about 38°C, 39°C, 40°C, 41°C or 42°C. In one embodiment, the fever is sustained for a duration of at least one or more days. In one embodiment, the fever is sustained for a duration of three days. In one embodiment, the C-Reactive Protein (CRP) level in the blood from a subject with a life threatening adverse event characterized by a severe cytokine storm is at least about 220 mg/mL. In one embodiment, the C-Reactive Protein (CRP) level in the blood from a subject with a life threatening multiple organ dysfunction characterized by a severe cytokine storm is at least about 220 mg/mL.

In one embodiment, the method of treating a cancer subject may include an additional agent for decreasing cytokine release. In one embodiment, the additional agent for decreasing harmful cytokine release is a corticosteroid. In one embodiment, the additional agent for decreasing harmful cytokine release comprises apoptotic cells or a composition comprising said apoptotic cells. In another embodiment, the additional agent for decreasing harmful cytokine release comprises a CTLA-4 blocking agent. In another embodiment, the additional agent for decreasing harmful cytokine release comprises immunotherapy agent, and a CTLA-4 blocking agent. In another embodiment, the additional agent for decreasing harmful cytokine release comprises an alpha-1 antitrypsin or fragment thereof or analogue thereof. In another embodiment, the additional agent for decreasing harmful cytokine release comprises immunotherapy agent, and an alpha- 1 anti-trypsin or fragment thereof or analogue thereof. In another embodiment, the additional agent for decreasing harmful cytokine release comprises a tellurium-based compound. In another embodiment, the additional agent for decreasing harmful cytokine release comprises immunotherapy agent, and a tellurium-based compound. In another embodiment, the additional agent for decreasing harmful cytokine release comprises an immune modulating agent. In another embodiment, the additional agent for decreasing harmful cytokine release comprises immunotherapy agent, and an immune modulating agent.

A skilled artisan would appreciate that decreasing toxic cytokine release or toxic cytokine levels comprises decreasing or inhibiting production of toxic cytokine levels in a subject, or inhibiting or reducing the incidence of cytokine release syndrome or a cytokine storm in a subject. In another embodiment, toxic cytokine levels are reduced during CRS or a cytokine storm. In another embodiment, decreasing or inhibiting the production of toxic cytokine levels comprises treating CRS or a cytokine storm. In another embodiment, decreasing or inhibiting the production of toxic cytokine levels comprises preventing CRS or a cytokine storm. In another embodiment, decreasing or inhibiting the production of toxic cytokine levels comprises alleviating CRS or a cytokine storm. In another embodiment, decreasing or inhibiting the production of toxic cytokine levels comprises ameliorating CRS or a cytokine storm. In another embodiment, the toxic cytokines comprise pro-inflammatory cytokines. In another embodiment, pro-inflammatory cytokines comprise IL-6. In another embodiment, proinflammatory cytokines comprise IL-Iβ. In another embodiment, pro-inflammatory cytokines comprise TNF-a. In another embodiment, pro-inflammatory cytokine comprise IL-6, IL- 1 β, or TNF-a, or any combination thereof.

For patients treated with T-cell immunotherapies, the cytokine release syndrome (CRS)(especially of cytokines IL6, IFNg, & TNFa) is frequently observed (Suntharalingam et. al., N. Engl. J. Med., 1018-1028, 2006; Turtle et.al., J. Clin. Invest., 2123-2138, 2016). Furthermore, the cytokine release syndrome is characterized by hyperthermia ($\geq$ 38°C) (from IL6 and/or IFNg)(Davila et.al., Sci. Transl. Med., 1-10, 2014) and/or hypothermia ($\leq$ 35°C) (from TNFa)(Brady et. al., Clin. Transl. Immunology, 1-7, 2014; Alegre et. al., Eur. J. Immunol, 707-710, 1990).

In one embodiment, the dosing schedule of the immunotherapy agent and the amount of immunotherapy agent administrated to the subject is adjusted in order prevent life-threatening adverse effects, CRS, cytokine storm, sCRS and/or hypercytokinemia. In one embodiment, the C-Reactive Protein (CRP) levels in the subject are used to determine the amount of immunotherapy agent to be provided. In one embodiment, the first amount of the immunotherapy agent does not induce a life threatening adverse event within the subject. In one embodiment, the first amount of the immunotherapy agent is provided in an amount effective to obtain the C-Reactive Protein (CRP) levels in the blood from the subject to be at least about 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229 or 230 mg/L. In a preferred embodiment, the CRP level is about 200 to about 220 mg/mL. In one embodiment, the first amount of the immunotherapy agent induces a transient fever at about 38°C, 39°C, 40°C, 41$^0$C or 42°C. In one embodiment, the immunotherapy agent induces a transient fever that is for the duration of 1-2 hours. In one embodiment, the immunotherapy agent induces a transient fever that is for the duration of 1-5 hours.

In one embodiment, the second amount of the immunotherapy agent does not induce hypercytokinemia. In one embodiment, the second amount of the immunotherapy agent does not induce a life threatening adverse event within the subject. In one embodiment, a second amount of the immunotherapy agent is provided in an amount effective to obtain the C-Reactive Protein (CRP) levels in the blood from the subject to be at least about 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179 or 180 mg/mL. In a preferred embodiment, the CRP level is about 159 mg/mL to about 169 mg/mL. In one embodiment, the second amount of the immunotherapy agent does not induce hypercytokinemia nor a life threatening multiple organ dysfunction and adverse event characterized by cytokine storm.

In one embodiment, cytokine release syndrome is characterized by elevated levels of several inflammatory cytokines and adverse physical reactions in a subject such as low blood pressure, high fever and shivering. In one embodiment, the inflammatory cytokines comprise IL-6, IL-Iβ, and TNF-a. In another embodiment, CRS is characterized by elevated levels of IL-6, IL-1 β, or TNF-a, or any combination thereof. In another embodiment, CRS is characterized by elevated levels of IL-8, or IL-13, or any combination thereof. In another embodiment, a cytokine storm is characterized by increases in TNF-alpha, IFN-gamma, IL-1 beta, IL-2, IL-6, IL-8, IL-10, IL-13, GM- CSF, IL-5, fracktalkine, or a combination thereof or a subset thereof. In yet another embodiment, IL-6 comprises a marker of CRS or cytokine storm. In another embodiment, IFN-γ comprises a marker of CRS or cytokine storm. In another embodiment, patients with larger tumor burdens have higher incidence and severity of cytokine release syndrome.

In another embodiment, cytokines increased in CRS or a cytokine storm in 30 humans and mice may comprise any combination of cytokines listed in Tables 1 and 2 below.

Table 3: Panel of Cytokines Increased in CRS or Cytokine Storm in Humans and/or Mice

| Cytokine | Human | Mouse model (pre-clinical) | | | Cells secreting this | Notes / |
|---|---|---|---|---|---|---|
| | | CAR-T (H) origin | Mouse origin | Not specified | | |
| Flt-3L | * | | | | DC (?) | |
| Fractalkine | * | | | | APC, Endothelial cells (?) | = CX3CL1, Neurotactin (Mouse) |
| M-CSF | | | | | | = CSF1 |
| GM-CSF | * | | | * (in vitro) | T cell, M 0 | |
| IFN-a | * | | | | T cell, M0, Monocyte | |
| IFN-β | ? | | | ? | T cell, M0, Monocyte | |
| IFN-γ | * | * | | * (in vitro) | cytotoxic T cells, helper T cells, NK cells, M0, Monocyte, DC | |
| IL- 1 a | * | | | | Monocyte, M0, Epithel | |
| IL- 1 β | * | | | * | Macrophages, DCs, fibroblasts, endothelial cells, hepatocytes | |
| IL- 1 Ra | * | | | | | |
| IL- 2 | * | * | | * (in vitro) | T cells | |
| IL- 2Ra | * | | | | lymphocytes | |
| IL- 4 | * | * | | * (in vitro) | Th2 cells | |
| IL- 5 | * | * | | * | T cells | |

(continued)

| Cytokine | Human | Mouse model (pre-clinical) | | | Cells secreting this | Notes / |
|---|---|---|---|---|---|---|
| | | CAR-T (H) origin | Mouse origin | Not specified | | |
| IL- 6 | * | | * | * | monocytes/ macrophages, dendritic cells, T cells, fibroblasts, keratinocytes, endothelial cells, adipocytes, myocytes, mesangial cells, and osteoblasts | |
| IL- 7 | * | | | * | *In vitro* by BM stromal cells | |
| IL- 8 | * | | | | Macrophages, monocytes | |
| IL - 9 | * | * | | | T cells, T helper | |
| IL- 10 | * | * | * | * (in vitro) | monocytes/macrophages, mast cells, B cells, regulatory T cells, and helper T cells | |
| IL- 12 | * | | | * | M0, Monocyte, DC, activated lymphocytes, neutrophils | = p70 (p40+p35) |
| IL- 13 | * | * | | | T cells | |

In one embodiment, cytokines Flt-3L, Fractalkine, GM-CSF, IFN-γ, IL-Iβ, IL-2, IL-2Ra, IL-4, IL-5, IL-6, IL-7, IL-8, IL-9, IL-10, IL-12, and IL-13 of Table 3 are considered to be significant in CRS or cytokine storm. In another embodiment, IFN-a, 5 IFN-β, IL-1, and IL-1Ra of Table 3 appear to be important in CRS or cytokine storm. In another embodiment, M-CSF has unknown importance. In another embodiment, any cytokine listed in Table 3, or combination thereof, may be used as a marker of CRS or cytokine storm.

**Table 4: Panel of Cytokines Increased in CRS or Cytokine Storm in Humans and/or Mice**

| Cytokine (Analyte) | Human model (clinical trials) | Mouse model (pre-clinical) | | | Cells secreting this cytokine | Notes / other |
|---|---|---|---|---|---|---|
| | | CAR-T (H) origin | Mouse origin | Not specified | | |
| IL- 15 | * | | | * | Fibroblasts, monocytes (?) | 22 |
| IL- 17 | * | | | * | T cells | |
| IL- 18 | | | | | Macrophages | |
| IL- 21 | * | | | | T helper cells, NK cells | |
| IL- 22 | * | | | | activated DC and T cells | |
| IL- 23 | | | | | | |
| IL- 25 | | | | | | Protective? |
| IL- 27 | * | | | | APC | |
| IP-10 | * | | | | Monocytes (?) | |
| MCP-1 | * | | | | Endothel, fibroblast, epithel, monocytes | = CKCL10 |
| MCP-3 | * | | | | PBMCs, MØ (?) | = CCL2 |
| MIP-1α | * | | | * (in vitro) | T cells | = CXCL9 |
| MIP-1β | * | | | | T cells | = CCL3 |
| PAF | ? | | | | platelets, endothelial cells, neutrophils, monocytes, and macrophages, me-sangial cells | = CCL4 |

(continued)

| Cytokine (Analyte) | Human model (clinical trials) | Mouse model (pre-clinical) | | | Cells secreting this cytokine | Notes / other |
|---|---|---|---|---|---|---|
| | | CAR-T (H) origin | Mouse origin | Not specified | | |
| PGE2 | * | | | * | Gastrointestinal mucosa and other | |
| RANTES | * | | | | Monocytes | |
| TGF-β | * | | | * | MØ, lymphocytes, endothel, platelets ... | = CCL5 |
| TNF-α | * | * | * | * (in vitro) | Macrophages, NK cells, T cells | |
| TNF-αR1 | * | | | | | |
| HGF | | | | | | |
| MIG | * | | | | T cell chemoattractant, induced bv IFN-γ | |

In one embodiment, IL-15, IL-17, IL-18, IL-21, IL-22, IP-10, MCP-1, MIP-la, 5 MIP-Iβ, and TNF-a of Table 2 are considered to be significant in CRS or cytokine storm. In another embodiment, IL-27, MCP-3, PGE2, RANTES, TGF-β, TNF-aR1, and MIG of Table 4 appear to be important in CRS or cytokine storm. In another embodiment, IL-23 and IL-25 have unknown importance. In another embodiment, any cytokine listed in Table 4, or combination thereof, may be used as a marker of CRS or cytokine storm.

A skilled artisan would appreciate that the term "cytokine" may encompass cytokines (e.g., interferon gamma, granulocyte macrophage colony stimulating factor, tumor necrosis factor alpha), chemokines (e.g., MIP 1 alpha, MIP 1 beta, RANTES), and other soluble mediators of inflammation, such as reactive oxygen species and nitric oxide.

In one embodiment, increased release of a particular cytokine, whether significant, important or having unknown importance, does not a prion mean that the particular cytokine is part of a cytokine storm. In one embodiment, an increase of at least one cytokine is not the result of a cytokine storm or CRS. In another embodiment, CAR T-cells may be the source of increased levels of a particular cytokine or group of cytokines.

In another embodiment, cytokine release syndrome is characterized by any or all of the following symptoms: Fever with or without rigors, malaise, fatigue, anorexia, myalgias, arthalgias, nausea, vomiting, headache Skin Rash, Nausea, vomiting, diarrhea, Tachypnea, hypoxemia Cardiovascular Tachycardia, widened pulse pressure, hypotension, increased cardiac output (early), potentially diminished cardiac output (late), Elevated D-dimer, hypofibrinogenemia with or without bleeding, Azotemia Hepatic Transaminitis, hyperbilirubinemia, Headache, mental status changes, confusion, delirium, word finding difficulty or frank aphasia, hallucinations, tremor, dymetria, altered gait, seizures. In another embodiment, a cytokine storm is characterized by IL-2 release and lymphoproliferation. In one embodiment, the cytokine storm is characterized by increases in cytokines released from a immunotherapy agent. In another embodiment, a cytokine storm is characterized by increases in cytokines released by CAR T-cells. In another embodiment, a cytokine storm is characterized by increases in cytokines released by cells other than CAR T-cells.

In another embodiment, cytokine storm leads to potentially life-threatening complications including cardiac dysfunction, adult respiratory distress syndrome, neurologic toxicity, renal and/or hepatic failure, and disseminated intravascular coagulation.

A skilled artisan would appreciate that the characteristics of a cytokine release syndrome (CRS) or cytokine storm are estimated to occur a few days to several weeks following the trigger for the CRS or cytokine storm. In one embodiment, an immunotherapy agent is a trigger for CRS or a cytokine storm. In one embodiment, CAR T-cells are a trigger for CRS or a cytokine storm. In another embodiment, a trigger for CRS or a cytokine storm is not CAR T-cells.

In one embodiment, measurement of cytokine levels or concentration, as an indicator of cytokine storm, may be expressed as -fold increase, percent (%) increase, net increase or rate of change in cytokine levels or concentration. In another embodiment, absolute cytokine levels or concentrations above a certain level or concentration may be an indication of a subject undergoing or about to experience a cytokine storm. In another embodiment, absolute cytokine levels or concentration at a certain level or concentration, for example a level or concentration normally found in a control subject not undergoing CAR-T cell therapy, may be an indication of a method for inhibiting or reducing the incidence of a cytokine storm in a subject undergoing CAR T-cell.

A skilled artisan would appreciate that the term "cytokine level" may encompass a measure of concentration, a measure of fold change, a measure of percent (%) change, or a measure of rate change. Further, the methods for measuring cytokines in blood, saliva, serum, urine, and plasma are well known in the art.

In one embodiment, despite the recognition that cytokine storm is associated with elevation of several inflammatory cytokines, IL-6 levels may be used as a common measure of cytokine storm and/or as a common measure of the effectiveness of a treatment for cytokine storms. A skilled artisan would appreciate that other cytokines may be used as markers of a cytokine storm, for example any of TNF-a, IB- la, IL-6, IL-8, IL-13, or INF-γ, or any combination above may be used as a marker of CRS or a cytokine storm. Further, that assay methods for measuring cytokines are well known in the art. A skilled artisan would appreciate that methods affecting a cytokine storm may similarly affect cytokine release syndrome (CRS).

In one embodiment, cytokine release syndrome is graded. In another embodiment, Grade 1 describes cytokine release syndrome in which symptoms are not life threatening and require symptomatic treatment only, e.g., fever, nausea, fatigue, headache, myalgias, malaise. In another embodiment, Grade 2 symptoms require and respond to moderate intervention, such as oxygen, fluids or vasopressor for hypotension. In another embodiment, Grade 3 symptoms require and respond to aggressive intervention. In another embodiment, Grade 4 symptoms are life-threatening symptoms and require ventilator and patients display organ toxicity.

In another embodiment, a cytokine storm is characterized by IL-6 and interferon gamma release. In another embodiment, a cytokine storm is characterized by IL-6 release. In another embodiment, a cytokine storm is characterized by interferon gamma release. In another embodiment, a cytokine storm is characterized by release of any cytokine or combination thereof, listed in Tables 1 and 2. In another embodiment, a cytokine storm is characterized by release of any cytokine or combination thereof, known in the art.

In one embodiment, symptoms onset begins minutes to hours after the infusion of an immunotherapy agent begins. In another embodiment, symptoms coincide with peak cytokine levels.

In one embodiment, products for use in the method of treating a cancer comprises administering an immunotherapy agent in accordance with the claims. Described is an immunotherapy agent which is a CAR T-cell. Described is a method of inhibiting or reducing the incidence of a cytokine release syndrome (CRS) or a cytokine storm in a subject undergoing CAR T-cell cancer therapy comprises administering an additional agent. In another example, the additional agent may aid the CAR T- cell therapy. In another example, the additional agent may aid in the inhibition or reducing the incidence of the CRS or cytokine storm. In another example, the additional agent may aid in treating the CRS or cytokine storm. In another example, the additional agent may aid in preventing the CRS or cytokine storm. In another example, the additional agent may aid in ameliorating the CRS or cytokine storm. In another example, the additional agent may aid in alleviating the CRS or cytokine storm.

In one example, the additional agent for decreasing harmful cytokine release comprises apoptotic cells or a composition comprising said apoptotic cells. In another example, the additional agent for decreasing harmful cytokine release comprises an apoptotic cell supernatant or a composition comprising said supernatant. In another example, the additional agent for decreasing harmful cytokine release comprises a CTLA-4 blocking agent. In another example, the additional agent for decreasing harmful cytokine release comprises immunotherapy agent, and a CTLA-4 blocking agent. In another example, the additional agent for decreasing harmful cytokine release comprises an alpha- 1 antitrypsin or fragment thereof or analogue thereof. In another example, the additional agent for decreasing harmful cytokine release comprises immunotherapy agent, and an alpha- 1 anti-trypsin or fragment thereof or analogue thereof. In another example, the additional agent for decreasing harmful cytokine release comprises a tellurium-based compound. In another example, the additional agent for decreasing harmful cytokine release comprises immunotherapy agent, and a tellurium-based compound. In another example, the additional agent for decreasing harmful cytokine release comprises an immune modulating agent. In another example, the additional agent for decreasing harmful cytokine release comprises immunotherapy agent, and an immune modulating agent. In another example, the additional agent for decreasing harmful cytokine release comprises Treg cells. In another example, the additional agent for decreasing harmful cytokine release comprises immunotherapy agent, and Treg cells.

In another example, methods as disclosed herein utilize combination therapy of CAR T-cells with one or more CTLA-4-blocking agents such as Ipilimumab. In another example, CTLA-4 is a potent inhibitor of T-cell activation that helps to maintain self-tolerance. In another example, administration of an anti- CTLA-4 blocking agent, which in another example, is an antibody, produces a net effect of T-cell activation. In another example, compositions and methods as disclosed herein utilize combined therapy comprising apoptotic cells, CAR T-cells, and one or more CTLA-4-blocking agents.

In another example, other toxicities resulting from CAR T-cell or NK cell administration that may be treated, prevented, inhibited, ameliorated, reduced in incidence or alleviated by the compositions and methods as disclosed herein comprise B cell aplasia or tumor lysis syndrome (TLS).

In one example, a method of inhibiting or reducing the incidence of a cytokine release syndrome (CRS) or a cytokine storm in a subject undergoing CAR T- cell cancer therapy does not affect the efficacy of the CAR T-cell therapy. In another example, a method of inhibiting or reducing the incidence of CRS or a cytokine storm in a subject undergoing CAR T-cell cancer therapy, does reduce the efficacy of the CAR T-cells therapy by more than about 5%. In another example, a method of inhibiting or reducing the incidence of CRS or a cytokine storm in a subject undergoing CAR T-cell cancer therapy, does reduce the efficacy of the CAR T-cells therapy by more than about 10%. In another example, a method of inhibiting or reducing the incidence of CRS or a cytokine storm in a subject undergoing CAR T-cell cancer therapy, does reduce the efficacy of the CAR T-cells therapy by more than about 15%. In another example, a method of inhibiting or reducing the incidence of CRS or a cytokine storm in a subject undergoing CAR T-cell cancer therapy, does reduce the efficacy of the CAR T-cells therapy by more than about 20%.

Any appropriate method of quantifying cytotoxicity can be used to determine whether activity in an immune cell modified to express a CAR remains substantially unchanged. For example, cytotoxicity can be quantified using a cell culture-based assay such as the cytotoxic assays described in the Examples. Cytotoxicity assays can employ dyes that preferentially stain the DNA of dead cells. In other cases, fluorescent and luminescent assays that measure the relative number of live and dead cells in a cell population can be used. For such assays, protease activities serve as markers for cell viability and cell toxicity, and a labeled cell permeable peptide generates fluorescent signals that are proportional to the number of viable cells in the sample. Kits for various cytotoxicity assays are commercially available from manufacturers such as Promega and Life Technologies. In another example, a measure of cytotoxicity may be qualitative. In another embodiment, a measure of cytotoxicity may be quantitative. In a further example, a measure of cytotoxicity may be related to the change in expression of a cytotoxic cytokine.

In one aspect, products for use in a method of treating a cancer subject in accordance with the claims result in an increase in CD8+T cells that recognize a specific antigen on the tumour. Precursor frequency (probability) of a naive CD8+T cell to recognize a specific antigen is about 1-10 per million cells (Lammermann et. al., Immunological Reviews, 26-43, 2008). The total naive CD8+T cells in human is about $4 \times 10^{10}$ cells (Boon et. al., Annu. Rev. Immunol., 175-208, 2006). The total naive CD8 Tcells in mouse (C57BL/6) is about $3 \times 10^7$ cells (Blattman et. al., J. Exp. Med., 657-664, 2002). Thus, assuming precursor frequency of a human naive CD8+T cell to recognize a human tumour-specific antigen is about ~5 per million cells, then the number of human naive CD8+T cells recognizing a tumour-specific antigen is about $2 \times 10^5$ cells ($4 \times 10^{10} \times 5/10^6$).

CD8+T-cell expansion after priming is about >= 10000-fold (50000x: LCMV/LM infections) (Blattman et. al., J. Exp. Med., 657-664, 2002, Haring et. al., Immunity, 19-29, 2006, Butler et. al., Cellular Microbiology, 925-933, 2011), Arens et. al., Immunol. Rev., 190-205, 2010). One CD8 T cell kills 2-3 target tumour cells (Wiedemann et. al., PNAS, 10985-10990, 2006, McGavern et. al., Nature Immunol, 918-925, 2002).

In one aspect, products for use in a method of treating a cancer subject in accordance with the claims result in an increase in CD8+T cells that recognize a specific antigen on the tumour. In some embodiments, the T cells are central memory T cells ($CD8^+CD44^+CD127^+KLRGI^-CD62L^+$), effector memory T cells ($CD8^+CD44^+CD127^+ KLRG1^+CD62L^-$), and effector T cells ($CD8^+CD44^+CD127^-KLRG1^+CD62L^-$). In a preferred embodiment, the T cells are central memory T cells.

In some aspects, after treatment, the percentage of CD8+T cells that recognize a specific antigen on the tumor is increased by 1-fold, about 1.5 fold, about 2 fold, about 2.5 fold, about 3 fold, about 3.5 fold, about 4 fold, about 4.5 fold, about 5 fold, about 5.5 fold, about 6 fold, about 6.5 fold, about 7 fold, about 7.5 fold, about 8 fold, about 8.5 fold, about 9 fold, about 9.5 fold, about 10 fold, about 10.5 fold, about 11 fold, about 11.5 fold, about 12 fold, about 12.5 fold, about 13 fold, about 13.5 fold, about 14 fold, about 14.5 fold, about 15 fold, about 15.5 fold, about 16 fold, about 16.5 fold, about 17 fold, about 17.5 fold about 18 fold, about 18.5 fold, about 19 fold, about 19.5 fold or about 20 fold relative to prior to treatment.

In some aspects, after treatment, the percentage of central memory T cells ($CD8^+CD44^+CD127^+KLRGI^-CD62L^+$) is increased by 1-fold, about 1.5 fold, about 2 fold, about 2.5 fold, about 3 fold, about 3.5 fold, about 4 fold, about 4.5 fold, about 5 fold, about 5.5 fold, about 6 fold, about 6.5 fold, about 7 fold, about 7.5 fold, about 8 fold, about 8.5 fold, about 9 fold, about 9.5 fold, about 10 fold, about 10.5 fold, about 11 fold, about 11.5 fold, about 12 fold, about 12.5 fold, about 13 fold, about 13.5 fold, about 14 fold, about 14.5 fold, about 15 fold, about 15.5 fold, about 16 fold, about 16.5 fold, about 17 fold, about 17.5 fold about 18 fold, about 18.5 fold, about 19 fold, about 19.5 fold or about 20 fold relative to prior to treatment.

In some aspects, after treatment, the percentage of CD8+CD44+CD127+KLRG1+CD62L- (effector memory cells) is increased by 1-fold, about 1.5 fold, about 2 fold, about 2.5 fold, about 3 fold, about 3.5 fold, about 4 fold, about 4.5 fold, about 5 fold, about 5.5 fold, about 6 fold, about 6.5 fold, about 7 fold, about 7.5 fold, about 8 fold, about 8.5 fold, about 9 fold, about 9.5 fold, about 10 fold, about 10.5 fold, about 11 fold, about 11.5 fold, about 12 fold, about 12.5 fold, about 13 fold, about 13.5 fold, about 14 fold, about 14.5 fold, about 15 fold, about 15.5 fold, about 16 fold, about 16.5 fold, about 17 fold, about 17.5 fold about 18 fold, about 18.5 fold, about 19 fold, about 19.5 fold or about 20 fold relative to prior to treatment.

In some aspects, after treatment, the percentage of CD8+CD44+CD127-KLRG1+CD62L-(effector) is increased by 1-fold, about 1.5 fold, about 2 fold, about 2.5 fold, about 3 fold, about 3.5 fold, about 4 fold, about 4.5 fold, about 5 fold, about 5.5 fold, about 6 fold, about 6.5 fold, about 7 fold, about 7.5 fold, about 8 fold, about 8.5 fold, about 9 fold, about 9.5 fold, about 10 fold, about 10.5 fold, about 11 fold, about 11.5 fold, about 12 fold, about 12.5 fold, about 13 fold, about 13.5 fold, about 14 fold, about 14.5 fold, about 15 fold, about 15.5 fold, about 16 fold, about 16.5 fold, about 17 fold, about 17.5 fold about 18 fold, about 18.5 fold, about 19 fold, about 19.5 fold or about 20 fold relative to prior to treatment.

Described are methods which comprise an additional step that is useful in overcoming rejection of allogeneic donor cells. In one example, the methods comprise the step of full or partial lymphodepletion prior to administration of the CAR T-cells, which in one example, are allogeneic CAR T-cells. In another example, the lymphodepletion is adjusted so that it delays the host versus graft reaction for a period sufficient to allow said allogeneic T-cells to attack the tumor to which they are directed, but to an extent insufficient to require rescue of the host immune system by bone marrow transplantation. In another example, agents that delay egression of the allogeneic T-cells from lymph nodes, such as 2-amino-2-[2-(4-octylphenyl)ethyl]propane- 1 ,3-diol (FTY720), 5-[4-phenyl-5-(trifluoromethyl)thiophen- 2-yl]-3-[3-(trifluoromethyl)phenyl] I,2,4-oxadiazole (SEW2871), 3-(2-(- hexylphenylamino)-2-oxoethylamino)propanoic acid (W123), 2-ammonio-4-(2-chloro- 4-(3-phenoxyphenylthio)phenyl)-2-(hydroxymethyl)but-yl hydrogen phosphate (KRP-203 phosphate) or other agents known in the art, may be used as part of the compositions and methods as disclosed herein to allow the use of allogeneic CAR T- cells having efficacy and lacking initiation of graft vs host disease. In one example, MHC expression by the allogeneic T-cells is silenced to reduce the rejection of the allogeneic cells. In another example, the apoptotic cells prevent rejection of the allogeneic cells.

In one embodiment, products for use in a method of treating a cancer includes administering an immunotherapy agent in accordance with the claims. Also described is where the immunotherapy agent is a CAR-T cell. In one example, CAR T-cells are heterologous to the subject. In one example, CAR T-cells are derived from one or more donors. In one example, CAR T-cells are derived from one or more bone marrow donors. In another example, CAR T-cells are derived from one or more blood bank donations. In one example, the donors are matched donors. In one example, CAR T-cells are universal allogeneic CAR T-cells. In another example, CAR T-cells are syngeneic CAR T-cells. In another example, CAR T-cells are from unmatched third party donors. In another example, CAR T-cells are from pooled third party donor T-cells. In one example, the donor is a bone marrow donor. In another example, the donor is a blood bank donor. In one example, CAR T-cells of the compositions and methods as disclosed herein comprise one or more MHC unrestricted tumor-directed chimeric receptors. In one example, non-autologous T-cells may be engineered or administered according to protocols known in the art to prevent or minimize autoimmune reactions, such as described in U.S. Patent Application No. 20130156794.

In another example, CAR T-cells are autologous to the subject. In one example, the patient's own cells are used. In this example, if the patient's own cells are used, then the CAR T-cell therapy is administered after the immunotherapy agent.

According to some examples, the CAR-T cell is administered to the subject systemically. In another example, administration is via the intravenous route. Alternately, the CAR-T cell may be administered to the subject according to various other routes, including, but not limited to, the parenteral, intraperitoneal, intra-articular, intramuscular and subcutaneous routes. Each possibility represents a separate example, as disclosed herein.

According to some examples, the CAR-T cell and an additional agent that decreases cytokine storm is administered to the subject systemically. In another example, administration is via the intravenous route. Alternately, the CAR-T cell and an additional agent that decreases cytokine storm may be administered to the subject according to various other routes, including, but not limited to, the parenteral, intraperitoneal, intra-articular, intramuscular and subcutaneous routes. Each possibility represents a separate example, as disclosed herein.

In one example, the preparation is administered in a local rather than systemic manner, for example, via injection of the preparation directly into a specific region of a patient's body. In another example, a specific region comprises a tumor or cancer.

In another example, the immunotherapy agent is administered to the subject suspended in a suitable physiological buffer, such as, but not limited to, saline solution, PBS, HBSS, and the like. In addition the suspension medium may further comprise supplements conducive to maintaining the viability of the cells. In another example, the additional agent is administered to the subject suspended in a suitable physiological buffer, such as, but not limited to, saline solution, PBS, HBSS, and the like.

According to some examples the pharmaceutical composition is administered intravenously. According to another example, the pharmaceutical composition is administered in a single dose. According to alternative examples the pharmaceutical composition is administered in multiple doses. According to another example, the pharmaceutical composition is administered in two doses. According to another example, the pharmaceutical composition is administered in three doses. According to another example, the pharmaceutical composition is administered in four doses. According to another example, the pharmaceutical composition is administered in five or more doses. According to some examples, the pharmaceutical composition is formulated for intravenous injection.

In one example, any appropriate method of providing modified CAR- expressing immune cells to a subject can be used for methods described herein. In one example, methods for providing cells to a subject comprise hematopoietic cell transplantation (HCT), infusion of donor-derived NK cells into cancer patients or a combination thereof.

The disclosure provides dosing regimens for treating a subject having cancer with a recombinant polypeptide (i.e. recombinant polypeptide CRYA_1B), an IL-10 inhibitory agent in a subject (e.g. IFNg), an immune cell growth factor (e.g. FLT3L), a TNFa inhibitory agent (e.g. TNFaR) and optionally an immunotherapy agent (e.g. anti-PD1 antibody) in accordance with the claims. The dosage amounts of the recombinant polypeptide, the IL-10 inhibitory agent, the immune cell growth factor, the TNFa inhibitory agent and the immunotherapy agent are described herein *supra*.

In some aspects, the recombinant polypeptide is administered intravenously. In some aspects, the recombinant polypeptide is administered subcutaneously. In some aspects, the IL-10 inhibitory agent in a subject is administered intravenously. In some aspects, the IL-10 inhibitory agent in a subject is administered subcutaneously. In some aspects, the immune cell growth factor is administered intravenously. In some aspects, the immune cell growth factor is administered subcutaneously. In some aspects, the TNFa inhibitory agent is administered intravenously. In some aspects, the TNFa inhibitory agent is administered subcutaneously.

In some aspects, the immunotherapy agent is administered as multiple infusions. In some aspects, the immunotherapy agent is administered at least one time, at least 2 times, at least 3 times, at least 4 times, at least 5 times, at least 6 times, at least 7 times, at least 8 times, at least 9 times, at least 10 times, at least 11 times, at least 12 times, at least 13 times or at least 14 times. In some aspects, the immunotherapy agent is administered one time.

In some aspects, first infusion of the immunotherapy agent is administered after the sixth infusion of the recombinant polypeptide of a priming therapeutic cycle and prior to the first infusion of the first infusion of the recombinant polypeptide in boosting therapeutic cycle. In some aspects, the first infusion of the immunotherapy agent is administered at least 9 days, at least 10 days, at least 11 days, at least 12 days, at least 13 days, at least 14 days, at least 15 days after the first infusion of the recombinant polypeptide in the priming therapeutic cycle. In one aspect, the immunotherapy agent is administered about 10 days to about 14 days after the first infusion of the recombinant polypeptide in the priming therapeutic cycle. In a preferred aspect, the immunotherapy agent is administered about 12 days after the first infusion of the recombinant polypeptide in the priming therapeutic cycle.

In one aspect, the dosage regimen comprises a therapeutic cycle that comprises administration of a immune cell growth factor, an IL-10 inhibitory agent, a TNFa inhibitory agent and a recombinant polypeptide in accordance with the claims. In some aspects, a therapeutic cycle increases the number of T-cells in the subject. In some aspects, the therapeutic cycle increase the number of T-cells that recognize tumour associated antigens. In some aspects, the therapeutic cycle increases the number of CD8+ central memory T cells in the subject. In some aspects, the therapeutic cycle increase the number of CD8+ effector memory cells. In some aspects, the therapeutic cycle increase the number of CD8+ effector cells. In one aspect, the therapeutic cycle is a priming therapeutic cycle. In some aspects, the therapeutic cycle is a boosting cycle

In one aspect, the dosage regimen comprises at least 1, at least 2, at least 3, at least 4, at least 5 or at least 6 priming therapeutic cycles. In a preferred aspect, the dosage regimen comprises 1 priming therapeutic cycle. In one aspect, the dosage regimen comprises at least 1, at least 2, at least 3, at least 4, at least 5 boosting therapeutic cycles. In one aspect, the dosage regimen comprises no more than 5 boosting therapeutic cycles. In a preferred aspect, the dosage regimen comprises 1 priming therapeutic cycle and at least 1 boosting cycle. In a preferred aspect, the dosage regimen comprises 1 priming cycle and no more than 5 boosting therapeutic cycles.

In one aspect, the immune cell growth factor is administered at least about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 25 hours, about 26 hours, about 27 hours, about 28 hours, about 29 hours or about 30 hours prior to the IL-10 inhibitory agent or the TNFa inhibitory agent. In a preferred aspect, the immune cell growth factor is administered at least about 24 hours prior to the IL-10 inhibitory agent or the TNFa inhibitory agent.

In one aspect, the immune cell growth factor is administered at least once daily for at least 5, days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days or at least 23 days. In one aspect, the immune cell growth factor is administered once daily for about 7 days to about 12 days. In a preferred aspect, the immune cell growth factor is administered once daily for about 7 days.

In one aspect, the IL-10 inhibitory agent and the TNFa inhibitory agent are administered concurrently. In one aspect, the IL-10 inhibitory agent and the TNFa inhibitory agent are administered sequentially. In one aspect, the IL-10 inhibitory agent is administered prior to the TNFa inhibitory agent. In one aspect, the IL-10 inhibitory agent is administered after the TNFa inhibitory agent.

In one aspect, the recombinant polypeptide is administered as multiple infusions. In some embodiments, the second infusion in administered at least 1 day, at least 2 days, at least 3 days at least 4 days, at least 5 days, at least 6 days or at least 7 days after the first infusion. In a preferred embodiment, the second infusion is administered at least 1 day after the first infusion. In one aspect, the third infusion is administered at least 2 days, at least 3 days, at least 4 days, at least 5 days, at least 6 days or at least 7 days after the first infusion. In a preferred aspect, the third infusion is administered at least 4 days after the first infusion. In one aspect, the fourth infusion is administered at least 3 days, at least 4 days, at least 5 days, at least 6 days or at least 7 days, at least 8 days, at least 9 days or at least 10 days after the first infusion. In a preferred aspect, the fourth infusion is administered at least 5 days after the first infusion. In one aspect, the fifth infusion is administered at least 6 days or at least 7 days, at least 8 days, at least 9 days, at least 10 days, at least 11 days, at least 12 days or at least 13 days after the first infusion. In a preferred aspect, the fifth infusion is administered at least 8 days after the first infusion. In one aspect, the sixth infusion is administered at least 7 days, at least 8 days, at least 9 days, at least 10 days, at least 11 days, at least 12 days, at least 13 days or at least 14 days after the first infusion.

In one aspect, the TNFa inhibitory agent and the recombinant polypeptide are administered concurrently. In one aspect, the TNFa inhibitory agent and the recombinant polypeptide are administered sequentially. In one aspect, the TNFa inhibitory agent is administered prior to the recombinant polypeptide. In one aspect, the TNFa inhibitory agent is administered after the recombinant polypeptide.

In one aspect, the first infusion of the recombinant polypeptide is administered at least 1 hour, at least 2 hours, at least 3 hours, at least 4 hours, at least 5 hours, at least 6 hours, at least 7 hours, at least 8 hours, at least 9 hours, at least 10 hours, at least 11 hours, at least 12 hours, at least 13 hours, at least 14 hours, at least 15 hours, at least 16 hours, at least 17 hours, at least 18 hours, at least 19 hours, at least 20 hours, at least 21 hours, at least 22 hours, at least 23 hours, at least 24 hours, at least 25 hours, at least 26 hours, at least 27 hours or at least 28 hours after the administration of the IL-10 inhibitory agent. In some aspects, the first infusion of the recombinant polypeptide is administered at about 3 hours or about 4 hours after the administration of the IL-10 inhibitory agent. In a preferred aspect, the first infusion of the recombinant polypeptide is administered about 4 hours after the administration of the IL-10 inhibitory agent.

In one aspect, first dose of the IL-10 inhibitory agent and the first dose recombinant polypeptide is administered concurrently or sequentially. In one aspect, the first dose of recombinant polypeptide is administered at a period of time after the first dose of the IL-10 inhibitory agent. In one aspect, the first recombinant polypeptide is administered at about 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 hours after the administration of the first dose of the IL-10 inhibitory agent. In a preferred aspect, the first dose of the recombinant polypeptide is administered at about 3 or 4 hours after the administration of the first dose of the IL-10 inhibitory agent. In one aspect, a second dose of the recombinant polypeptide is administered at about 15, 16, 17, 18, 19, 20, 21, 22, 23, 24, 25, 26, 27, 28, 29 or 30 hours after the administration of the first dose of the recombinant polypeptide. In a preferred aspect, the second dose of the recombinant polypeptide is administered at about 24 hours after the administration of the first recombinant polypeptide.

In one aspect, a fourth dose of the recombinant polypeptide is administered at about 15, 16, 17, 18, 19, 20, 21, 22, 23, 24, 25, 26, 27, 28, 29 or 30 hours after the administration of the third dose of the recombinant polypeptide. In a preferred aspect, the fourth dose of the recombinant polypeptide is administered at about 24 hours after the administration of the third recombinant polypeptide.

In one aspect, a sixth dose of the recombinant polypeptide is administered at about 15, 16, 17, 18, 19, 20, 21, 22, 23, 24, 25, 26, 27, 28, 29 or 30 hours after the administration of the fifth dose of the recombinant polypeptide. In a preferred aspect, the sixth dose of the recombinant polypeptide is administered at about 24 hours after the administration of the fifth recombinant polypeptide.

In one aspect of the present disclosure, the second dose of the recombinant polypeptide is administered at about 15, 16, 17, 18, 19, 20, 21, 22, 23, 24, 25, 26, 27, 28, 29 or 30 hours after the administration of the first dose of the recombinant polypeptide. In a preferred aspect, the second dose of the recombinant polypeptide is administered at about 24 hours after the administration of the first dose of the recombinant polypeptide.

In one aspect a third dose of the recombinant polypeptide is administered at about 1, 2, 3, 4, 5, 6 or 7 days after the second dose of the recombinant polypeptide. In a preferred aspect, the third dose of the recombinant polypeptide is administered at about 3 days after the second dose of the recombinant polypeptide. In one aspect, the fourth dose of the recombinant polypeptide is administered at about 15, 16, 17, 18, 19, 20, 21, 22, 23, 24, 25, 26, 27, 28, 29 or 30 hours after the administration of the third dose of the recombinant polypeptide. In a preferred aspect, the fourth dose of the recombinant polypeptide is administered at about 24 hours after the administration of the third dose of the recombinant polypeptide.

In one aspect a fifth dose of the recombinant polypeptide is administered at about 1, 2, 3, 4, 5, 6 or 7 days after the fourth dose of the recombinant polypeptide. In a preferred aspect, the fifth dose of the recombinant polypeptide is administered at about 3 days after the fourth dose of the recombinant polypeptide. In one aspect, the sixth dose of the recombinant polypeptide is administered at about 15, 16, 17, 18, 19, 20, 21, 22, 23, 24, 25, 26, 27, 28, 29 or 30 hours after the administration of the fifth dose of the recombinant polypeptide. In a preferred aspect, the sixth dose of the recombinant polypeptide is administered at about 24 hours after the administration of the fifth dose of the recombinant polypeptide.

Any of the above aspects can be combined with any other aspect as disclosed herein in accordance with the claims.

## EXAMPLES

**Example 1: Methods of Producing Recombinant Polypeptides**

Materials and Methods

The methods of producing the recombinant polypeptides of the present disclosure utilized the PCR primers disclosed in Table 4.

**Table 4.** Primer Sequences

| Primer | Nucleotide Sequence (5' to 3') | SEQ ID NO: |
|---|---|---|
| A1 | GGGGGGCATATGGACATTACCATCCAGCACCCCTGGTTCAAGCGCGCTCT | 33 |
| A2 | GGGGGGAAGCTTTTACTCCTCAGGCGCCTCGGTGGGCTT | 34 |
| ioE1 | CCTCTGTTCGAGGAGACTATCGAGCCCTACTA | 35 |
| ioE2 | TAGTAGGGCTCGATAGTCTCCTCGAACAGAGG | 36 |
| ioE3 | ACCGGCAGGAGCTGTTCCGCGAGGTGCTGTCGGAGGGCATTGAGTCGGTGAGGGAGGACCGGGA | 37 |
| ioE4 | TCCCGGTCCTCCCTCACCGACTCAATGCCCTCCGACAGCACCTCGCGGAACAGCTCCTGCCGGT | 38 |
| ioE5 | ACTATGCTGGACGTAAAACACTTTGAGCCTTCGGACCTGGAGGTGAAGATTA | 39 |
| ioE6 | TAATCTTCACCTCCAGGTCCGAAGGCTCAAAGTGTTTTACGTCCAGCATGAT | 40 |
| ioE7 | AAGATTATCGACGACTTTGTGTCGATCCATGGC | 41 |
| ioE8 | GCCATGGATCGACACAAAGTCGTCGATAATCTT | 42 |
| ioE9 | GGCAAGCACGAGTCGAGACAGGACGACCACGGCTACATCGAGCGGTCGTTTCACCGC | 43 |
| ioE10 | GCGGTGAAACGACCGCTCGATGTAGCCGTGGTCGTCCTGTCTCGACTCGTGCTTGCC | 44 |
| ioE11 | GCGGACCAGGAGGCCATCACCTGCGAGCTGGAGGGCGACGG | 45 |
| ioE12 | CCGTCGCCCTCCAGCTCGCAGGTGATGGCCTCCTGGTCCAC | 46 |
| ioE13 | TTCGACCAGTTTTTCGGATCGGGTCTGCTGTCGTATGACCTGCTGCCTCTGTTC | 47 |
| ioE14 | GGGGACCTTGGGGCCCTCGAAGGTCAGCATGCCGTCGCC | 48 |
| ioE15 | TTCAAGCGCGCTCTGGGACCCCTGATTCCAGAGCGTCTGTTCGACCAGTTTTTCGGA | 49 |
| ioE16 | CACGGGGATGGGCCTCGACTCGTGGGTGGGGTCCATGTTCTCGGGGACCTTGGGG | 50 |
| ioE17 | ATGGACATTACCATCCAG | 51 |
| ioE18 | AAGCTTTTACTCCTCAGGCGCCTCGGTGGGCTTCGACGACCGCTCCACGGGGATGGGCCT | 52 |

Preparation of Template DNA

The full length CRYAA sequence from *Anser cygnoides domesticus* (SEQ ID NO: 17) was amplified in a PCR reaction using Pfu polymerase. A1 primer (SEQ ID NO: 33) and A2 primer (SEQ ID NO: 34) were used in the PCR reaction. The gene was cloned into NdeI and HindIII sites in a pET24a vector (Novagen) using the manufacturer's protocol. The ligation mixture was transformed into *Escherichia coli* DH5alpha cells and transformants were selected on LB ampicillin plates. Plasmid DNA was isolated from several transformants and screened by restriction digestion of NdeI and HindIII sites. A sequence verified clone containing *Anser cygnoides domesticus* CRYAA (SEQ ID NO: 17) was identified and used as template.

Cloning of Plasmid containing the CRYA 1B recombinant polypeptide sequence

The recombinant plasmid containing CRYA_1B (SEQ ID NO: 25) was prepared in the following manner. PCR was performed using the template DNA described above, forward primer IoE1 (SEQ ID NO: 35) and reverse primer IoE2 (SEQ ID NO: 36). PCR temperature and time were programmed as follows: denaturing at 95°C for 5 minutes; followed by 30 cycles of PCR reactions with denaturation at 95°C for 30 sec, annealing at 60°C for 30 sec, and elongation at 72°C for 1 minute; final elongation at 72°C for 10 minutes. All PCR amplifications were performed with Pfu Ultra polymerase (Stratagene). PCR products were separated electrophoretically using 1.0% agarose gel, and stained with ethidium bromide. The DNA fragment was extracted from the gel using GFX™ PCR DNA and Gel Bind Purification Kit (GE Healthcare) and ligated into a pET24a (Novagen) vector. The ligation mixture was transformed into the DH5alpha *Escherichia coli* strain and transformants were selected on LB plates containing ampicillin. Plasmid DNA was isolated from transformants. A sequence verified clone, Plasmid_1, was used as a template for a subsequent round of PCR amplification.

PCR amplification was performed using Plasmid_1, forward primer IoE3 (SEQ ID NO: 37) and reverse primer IoE4 (SEQ ID NO: 38). PCR amplification and cloning were performed using the procedure described above and the following PCR conditions: 95°C for 5 minutes, 32 cycles of (95°C for 30 seconds, 65°C for 30 seconds, 72°C for 1 minute), followed by 5 minutes at 72°C. The PCR product was purified and cloned into a pET24a plasmid using NdeI and HindIII restriction sites. A sequence verified clone, Plasmid_2, was used as a template for a subsequent round of PCR amplification.

PCR amplification was performed using Plasmid_2, forward primer IoE5 (SEQ ID NO: 39) and reverse primer IoE6 (SEQ ID NO: 40). PCR amplification and cloning were performed using the procedure described above and the following PCR conditions: 95°C for 5 minutes, followed by 95°C for 30 seconds, 58°C for 30 seconds, 72°C for 1 minute in 35 cycles, with a final 5 minute extension at 72°C. PCR products were separated electrophoretically using 1.0% agarose gel, and stained with ethidium bromide. The DNA fragment was excised from the gel, extracted and cloned into a pET24a plasmid. A sequence verified clone, Plasmid_3, was used as a template for a subsequent round of PCR amplification.

PCR amplification was performed using Plasmid_3, forward primer IoE7 (SEQ ID NO: 41) and reverse primer IoE8 (SEQ ID NO: 42). PCR amplification and cloning were performed using the procedure described above and the following PCR conditions: 95°C for 5 minutes, followed by 95°C for 30 seconds, 55°C for 30 seconds, 72°C for 1 minute in 28 cycles, with a final 5 minute extension at 72°C. PCR products were separated electrophoretically using 1.0% agarose gel, and stained with ethidium bromide. The DNA fragment was excised from the gel, extracted and cloned into a pET24a plasmid. A sequence verified clone, Plasmid_4, was used as a template for a subsequent round of PCR amplification.

PCR amplification was performed using Plasmid_4, forward primer IoE9 (SEQ ID NO: 43) and reverse primer IoE10 (SEQ ID NO: 44). PCR amplification and cloning were performed using the procedure described above and the following PCR conditions: 95°C for 5 minutes, followed by 95°C for 30 seconds, 53°C for 30 seconds, 72°C for 1 minute in 33 cycles, with a final 5 minute extension at 72°C. PCR products were separated electrophoretically using 1.0% agarose gel, and stained with ethidium bromide. The DNA fragment was excised from the gel, extracted and cloned into a pET24a plasmid. A sequence verified clone, Plasmid_5, was used as a template for a subsequent round of PCR amplification.

PCR amplification was performed using Plasmid_5, forward primer IoE11 (SEQ ID NO: 45) and reverse primer IoE12 (SEQ ID NO: 46). PCR amplification and cloning were performed using the procedure described above and the following PCR conditions: 95°C for 5 minutes, followed by 95°C for 30 seconds, 57°C for 30 seconds, 72°C for 1 minute in 30 cycles, with a final 5 minute extension at 72°C. PCR products were separated electrophoretically using 1.0% agarose gel, and stained with ethidium bromide. The DNA fragment was excised from the gel, extracted and cloned into a pET24a plasmid. A sequence verified clone, Plasmid_6, was used as a template for a subsequent round of PCR amplification.

PCR amplification was performed using Plasmid_6, forward primer IoE13 (SEQ ID NO: 47) and reverse primer IoE14 (SEQ ID NO: 48). PCR amplification and cloning were performed using the procedure described above and the following PCR conditions: 95°C for 5 minutes, followed by 95°C for 30 seconds, 51°C for 30 seconds, 72°C for 1 minute in 32 cycles, with a final 5 minute extension at 72°C. PCR products were separated electrophoretically using 1.0% agarose gel, and stained with ethidium bromide. The DNA fragment was excised from the gel, extracted and cloned into a pET24a plasmid. A sequence verified clone, Plasmid_7, was used as a template for a subsequent round of PCR amplification.

PCR amplification was performed using Plasmid_7, forward primer IoE15 (SEQ ID NO: 49) and reverse primer IoE16 (SEQ ID NO: 50). PCR amplification and cloning were performed using the procedure described above and the following PCR conditions: 95°C for 5 minutes, followed by 95°C for 30 seconds, 54°C for 30 seconds, 72°C for 1 minute in 32 cycles, with a final 5 minute extension at 72°C. PCR products were separated electrophoretically using 1.0% agarose gel, and stained with ethidium bromide. The DNA fragment was excised from the gel, extracted and cloned into a pET24a plasmid. A sequence verified clone, Plasmid_8, was used as a template for a subsequent round of PCR amplification.

PCR amplification was performed using Plasmid_8, forward primer IoE17 (SEQ ID NO: 51) and reverse primer IoE18 (SEQ ID NO: 52). PCR amplification and cloning were performed using the procedure described above and the following PCR conditions: 95°C for 5 minutes, followed by 95°C for 30 seconds, 52°C for 30 seconds, 72°C for 1 minute in 32 cycles, with a final 5 minute extension at 72°C. PCR products were separated electrophoretically using 1.0% agarose gel, and stained with ethidium bromide. The DNA fragment was excised from the gel, extracted and cloned into a pET24a plasmid. The ligation mixture was transformed into DH5alpha strain of *Escherichia coli* cells and transformants were selected on LB plates containing ampicillin. A sequence verified clone, Plasmid_9 contains the CRYA_1B (SEQ ID NO: 25) in the correct reading frame.

Expression of Recombinant Polypeptide CRYA_1B

Plasmid_9 was transformed into the expression Escherichia coli strain BL21, and the ampicillin-resistant colonies were selected. The expected molecular weight for CRYA_1B recombinant polypeptide was 20kDa (Figure 2). A single colony from Luria-Betani (LB)-agar plate supplemented with 100 $\mu$g/ml ampicillin was selected. In this preparation, a 50 ml conical tube containing 3 ml of LB medium (10g tryptone, 10g NaCl and 5g yeast extract per L) and 100 $\mu$g/ml of ampicillin was inoculated with a single colony and grown overnight in a shaking incubator set at 37°C and 200 RPM. The culture was further expanded by adding 3 ml of the culture into a sterile 500 ml Erlenmeyer flask containing 100 ml of 2YT medium (16g tryptone, 15g yeast extract and 8g NaCl per L) and 100 $\mu$g/ml of ampicillin and grown overnight in a shaking incubator set at 37°C and 200 RPM. This resulted in a seed culture.

A 6L bioreactor was used to further expand the seed culture. 4L of 2YT medium containing 100 $\mu$g/ml of ampicillin was inoculated with 100 ml of seed culture grown overnight in a shaking incubator set at 37°C and 200 RPM. In the bioreactor, cultures were incubated at 37°C, airflow and agitation of 2 SLPM (standard liners per minute) and 200 RPM. When the OD600 reached 0.65 to 0.75, protein overexpression was induced with 1.0 mM Isopropyl-$\beta$-D-thiogalactopyranoside (IPTG). The cells were allowed to grow for 7 to 8 hours and the agitation speed, temperature and air flow were set to 400 RPM, 28°C and 4 SLPM, respectively. To control foaming, Polyglycol P-2000 antifoam was added as required. After 7 to 8 hours of induction, the cells were harvested by centrifugation at 8000 rpm for 15 minutes at 4°C. The cell pellets were frozen and stored at -80°C.

Purification of Recombinant Polypeptide CRYA_1B

In this preparation, the pellets, equivalent to 6g of CRYA_1B recombinant polypeptide (SEQ ID NO: 9) was resuspended in 40 ml of Buffer A (50 mM Tris-HCl buffer) and disrupted by sonication on ice (28 cycles of 10-s pulses with 30-s intervals, 30% amplitude using an ultrasonic cell disruptor Misonix Ultrasonic Liquid Processors S-4000, USA) to obtain the total protein extract for solubility analysis. The total protein extract was centrifuged at 14,000 rpm for 45 min at 4°C using a Sorvall RC5C Plus (USA) ultracentrifuge using a type SS-34 rotor. The supernatant was filtered through a 0.45 $\mu$m filter (Millipore) and loaded onto a Q-Sepharose anion exchange column equilibrated in the same buffer. Q-Sepharose was packed into a C 26/40 Column (GE Healthcare) to a bed height of 20 cm. A 40mL volume of supernatant containing CRYA_1B recombinant polypeptide (SEQ ID NO: 9) was loaded onto the column using AKTA FPLC (GE Healthcare) at a flow rate of 5 ml/min. CRYA_1B recombinant polypeptide (SEQ ID NO: 9) was eluted by a concentration gradient by using an equilibrium buffer containing 50 mM Tris-HCl, NaCl buffer and collected in a single peak based on $A_{280}$ absorbance for further application on the hydrophobic interaction column. The eluents collected were analyzed by 15% SDS-polyacrylamide gel electrophoresis.

Hydrophobic Interaction Chromatography

After ion exchange chromatography, the eluted CRYA_1B recombinant polypeptide (SEQ ID NO: 9) was pooled together, concentrated by Amicon Ultra 15 ml Centrifugal Filter (Merck), and subsequently added to the saturated ammonium sulphate buffer (50 mM Tris-HCl, 3.8 M ammonium sulphate, 1 mM DTT and 1 mM EDTA), resulting in a final concentration of 1.2 M ammonium sulphate. The concentrated product with adding ammonium sulphate was filtered using a 0.45 $\mu$m syringe filter (Millipore) and loaded to Hydrophobic Interaction Chromatography column (C 10/20 column, Ge Healthcare) at a flow rate of 2 ml/min. Source 15PHE (GE Healthcare) was packed into C 10/20 column (GE Healthcare) to a bed height of 10 cm and pre-equilibrated with buffer A (50 mM Tris-HCl, 1.2 M ammonium sulphate, 10% glycerol, 1 mM DTT and 1 mM EDTA). The column was washed with buffer A and the protein elution using buffer B (50 mM Tris-HCl, 10% glycerol, 1 mM DTT and 1 mM EDTA) was achieved with a linear gradient with decreasing ammonium sulphate and increasing glycerol. The eluted protein was analyzed by 15 % SDS-PAGE. The fractions were further concentrated by Amicon Ultra 15 ml Centrifugal Filter (Merck).

Buffer Exchange Using Gel Filtration

The purified recombinant polypeptide was further exchanged into PBS buffer by using a Sephadex G-25 column. Sephadex G-25 was packed into a C 26/100 column (GE Healthcare) to a bed height of 85 cm and pre-equilibrated with PBS buffer at a flow rate of 1 ml/min. The concentrated protein was eluted after 2.5 hours and analyzed by 15% SDS-PAGE. The resulting eluates were then concentrated by Amicon Ultra 15 ml Centrifugal Filter (Merck).

**Example 2: Methods of Inducing or Enhancing an Immune Response**

Cancer cell lines were treated with CRYA_1B recombinant polypeptide (SEQ ID NO: 9), CRYA_1B recombinant polypeptide was diluted to various concentrations and incubated with human cancer cell lines H441 (lung cancer, HTB-174, ATCC), H460 (lung cancer, HTB-177, ATCC), HCT15 (colon cancer, CCL-225, ATCC) and MCF7 (breast cancer, HTB-22, ATCC) all at 37°C. As of CRT, HSP70, HSP90, and Caspase 3/7 assay, the recombinant polypeptide incubation time for H441 is 60min, 1hr50min, 1hr40min, and 2hr45min respectively, for H460 is 30min, 1hr15min, 1hr5min, and 2hr30min respectively, for HCT15 is 55min, 1hr50min, 1hr30min, and 2hr30min respectively, and for MCF7 is 1hr10min, 1hr40min, 1hr45min, and 2hr45min respectively. Flow cytometry was used to assess the cell surface expression of calreticulin (CRT) (Figures 3-6), HSP70 (Figures 7-10), HSP90 (Figures 11-14) and Caspase 3/7 (Figures 15-18) in cells treated with CRYA_1B recombinant polypeptide and in untreated control cells. This was performed using a FACSCalibur (BD Biosciences) using CRT mAb (Abcam), HSP70 mAb (Enzo Life Sciences), HSP90 mAb (Enzo Life Sciences) and Caspase 3/7 (Invitrogen assay), respectively.

Cancers can undergo extensive genetic and phenotypic variations within tumours (intratumoural heterogeneity) but also between tumours (intertumoural heterogeneity). Recurrent tumour, often seeded from the covert subclones of primary tumour, is a leading cause of cancer mortality. Similarly, metastases, the lethal late stage of cancer progression, inherit multiple genetically distinct early subclones from primary tumour. However, the metastatic tumours exhibits less intratumoural heterogeneity than that of primary tumour. The main cause of intertumoural heterogeneity originates from the intratumoural heterogeneity of primary tumour. Because of cancer immunoediting process, tumours can develop an attenuated immunogenicity and create an immunosuppressive microenvironment to prevent tumour's eradication by host immune system.

Cancer cells have the ability to conceal tumor danger signals, causing chronic inflammation of the tumor microenvironment with Th2 cells which release (Interleukin (IL)-4 and IL-10) leading to a higher serum IL-10 level in cancer subjects in comparison to normal healthy subjects. IL-10 is an anti-inflammatory cytokine that induces STAT3 signaling (FIG. 19 and 20) and inhibits DC activation and functionality. IL-10 leads to a tumor microenvironment made of myeloid-derived suppressor cells (MDSC), tumour-associated macrophages (TAM) and regulatory T cells (Treg), resulting in tumour escape. This ultimately causes reduced adaptive immune activation. By contrast, bacterial and viral infections expose danger signals, which leads to acute inflammation with Th1 cells which release IL-12 and IFNg (FIG. 19) immune-stimulatory cytokines into the microenvironment, resulting in adaptive immune activation.

Calreticulin (CRT) is a danger signal that recruits dendritic cells (DC) to ingest tumours and pathogens. HSP70 and HSP90 are danger signals that induce maturation or activation of dendritic cells, which in turn produce IL-12 and IL-6. Dendritic cells can then present antigens in the context of MHC-I and MHC-II to T cells in lymph nodes. Activated DC can produce IL-12 which is a Th1 cytokine that promotes the activation of T cells, which in turn produces IL-2 and IFNg. The acute transient high IL-6 from activated-DC causes acute inflammation resulting in fever, which facilitates the immune T-cell activation. Chronic expression of IL-6 causes chronic inflammation, which impedes immune activation.

**Example 3: The Effects of Interferon gamma on Serum IL-10**

The levels of Serum IL-10 in cancer patients is increased in comparison to normal healthy subjects. For example, the serum IL-10 in human patients with lung cancer stage III-IV and a normal healthy subject is 17.7 pg/mL and 9.2 pg/mL, respectively (IL-10 ratio of 1.924). The serum IL-10 in murine C57BL/6 with B16F10 melanoma and a control healthy mouse is 95 pg/mL and 50 pg/mL, respectively (IL-10 ratio of 1.9). The serum IgG in murine C57BL/6 with B16F10 melanoma and a control healthy mouse is 1200 and 650 MFI, respectively (IgG ratio of 1.846). The serum IL-10 in murine C57BL/6 with MC38 (colon cancer) and a control healthy mouse is 70 pg/mL and 50 pg/mL respectively (IL-10 ratio of 1.4). The serum IL-10 in murine C57BL/6 with E0771 (breast cancer) and a control healthy mouse is 70 pg/mL and 50 pg/mL respectively (IL-10 ratio of 1.4).

As IFNg inhibits the levels of Serum IL-10, intravenous injections of IFNg were administered to B16F10 tumor bearing mice to determine the effects on serum IL-10. Blood was sampled 3.5 hours after IFNg injection and serum IL-10 was determined by Luminex assays. Table 5 and FIG. 21 show that an increased dosage of IFNg results in decreased serum IL-10 levels in mice.

**Table 5. Percent Serum IL-10 Levels after following injection of IFNg**

| In vivo IFNg dose ($\mu$g/m$^2$) | Serum IL-10 Levels remaining |
|---|---|
| 600 | 14% |
| 300 | 35% |
| 150 | 55% |
| 100 | 70% |
| 75 | 75% |
| 50 | 80% |
| 25 | 90% |
| 0 | 100% |

**Example 4: MC38, E0771 and B16F10 *in vivo* Tumor Challenge and Re-Challenge Methods and Dosage Regimen**

Methods. Female10-12 weeks old C57BL/6 mice (Charles River Laboratories, USA) were housed and maintained under pathogen-free conditions in accordance with the guidelines of the Association for Assessment and Accreditation of Laboratory Animal International Care.

The C57BL/6 mice were inoculated subcutaneously with $1\times10^6$ MC38/E0771/B16F10 cells in 100$\mu$l PBS into the flank. Tumour volume was measured with a caliper and calculated using the formula $(A \times B2)/2$ [*A:* the largest diameter; *B:* the smallest diameter of tumour]. When the tumour reached approximately 100 mm3, we began recombinant polypeptide and IFN gamma treatment via intraperitoneal injection according to the treatment schedule of FIG. 22.

Results. MC38 is a chemical-induced Grade-III SMAD4-loss metastatic colorectal adenocarcinoma for the model of metastases, and E0771, a triple-negative cancer claudin-low self-renewal spontaneous mammary adenocarcinoma for the model of relapse. The hallmark of adaptive immunity to cancer is for long-lived neoantigen-specific Tcells with immunologic memory to respond faster and robustly upon re-encountering the same neoantigen in order to prevent cancer relapses. For the proof of long-term adaptive immunity memory, we pooled together the complete-response (CR) mice from the tumour challenge experiments, and then rechallenged these CR mice with live tumour cells, mimicking the spontaneous tumour recurrence by externally forced live tumour cells injection, at the opposite site of primary tumour 6-month after the complete rejection of primary tumour. When there is no sign of tumour growth, it indicates the successful murine 6-month adaptive immunity corresponding to approximately 15-20 years in human. The MC38 has the phenotype of SMAD4-loss, which is associated with colon cancer patient's metastases and poor survival. MC38 tumour challenge experiments show that primary tumour variants began to grow aggressively after the initial spontaneous partial tumour regression and approximately 40% of mice would develop 1 or 2 locoregional metastases. For the CR mice after recombinant polypeptide CRYA_1B (SEQ ID NO: 9) treatment, the metastases would undergo complete regression first whereas the primary tumour would first undergo partial regression and then eventually complete regression. We hypothesize that 1) before recombinant polypeptide CRYA_1B treatment, the preexisting T-cell's immunoselection against dominant neoantigen caused the initial spontaneous partial regression of primary tumour and the persistent immune pressure eventually led to the natural selection of dominant neoantigen-loss and/or MHC-I-deficient tumour variants; 2) primary tumour variants then suddenly grew aggressively and the locoregional metastases developed (immune escape); 3) after recombinant polypeptide CRYA_1B treatment, the drug-induced neoantigens were predominantly from metastases due to higher drug concentration coverage over smaller metastases compared to larger primary tumour variants; 4) the metastases underwent complete regression by multiple metastases-only subdominant neoantigens and putatively one shared subdominant neoantigen while the primary tumour variants were in parallel undergoing limited partial regression by one shared subdominant neoantigen; 5) after the CR of metastases, the subsequent doses of recombinant polypeptide CRYA_1B induced the multiple primary-only subdominant neoantigens in addition to the shared subdominant neoantigen; 6) the primary tumour variants eventually underwent complete regression. In contrast, if the DC-mediated neoantigen-based vaccines utilise only immunodominant neoantigen, screened ex vivo from patient's TILs or peripheral blood mononuclear cells (PBMCs), without incorporating subdominant neoantigens, then it would be impossible to completely eradicate metastases and primary tumour due to the escapes of tumour variants.

The overall CR rate of MC38 tumour challenge model was 70% after recombinant polypeptide CRYA_1B treatment and the relapse-free-survival rate for CR mice was 100% with long-term immunity without tumour recurrence after tumour rechallenge (FIG. 23A and 23B). The triple-negative breast cancer, with extensive intratumoural heterogeneity, E0771 has a claudin-low tumour phenotype with the characteristic of enriched tumour-initiating (stem cell-like) CD44+CD24-/low cells especially after drug treatments for recurrence in human breast cancer patients. In light of E0771's stem-cell like tumour-initiating ability, we used it for the model of spontaneous tumour relapse. After recombinant polypeptide CRYA_1B treatments, we achieved the CR rate of 60% in E0771 tumour challenge model (FIG. 23C). We observed for 6 months and there was no sign of spontaneous E0771 tumour recurrence after CR. We hypothesized that recombinant polypeptide CRYA_1B simultaneously induced E0771-derived dominant neoantigen and multiple subdominant neoantigens, including those of cancer stem cell subclones, the neoantigen's multiplicity of which prevented E0771 from spontaneous relapse. Moreover, we rechallenged the CR mice with live E0771 tumour cells as externally forced tumour relapse, and there was no sign of tumour growth after rechallenge, indicating a successful long-lived adaptive immunity memory (FIG. 23D). Interestingly, we observed that there are two distinct tumour regression patterns after recombinant polypeptide CRYA_1B treatment for both MC38 and E0771 tumour models, one with continuous tumour regression until CR (FIG. 23A) whereas the other with temporary tumour flare and then later tumour regression until CR (FIG. 23C). The identical tumor challenge and tumor rechallenge experiments were carried out with B16F10 murine melanoma model. The overall CR rate of B16F10 tumour challenge model was 40% after treatment regimen and the relapse-free-survival rate for CR mice was 100% with long-term immunity without tumour recurrence after tumour rechallenge (FIG. 24A and 24B). We hypothesize that after immunotherapy the tumour flare may occur, since the priming of immune system is complex and can be delayed while the tumour may transiently grow and/or substantial immune cells may transiently infiltrate tumour, resulting in an increase of tumour size during this period.

Additionally, recombinant polypeptide CRYA_1B (SEQ ID NO: 9) was administered daily in healthy immunocompetent B6 mice (n = 7) at 1.6-fold clinical dose for seven consecutive days, with drug serum peak concentration at ~800 $\mu$g/ml, showed no significant changes in behaviour, body weight, food intake and key serum chemistry parameters at the end of experiment except the noticeable drop in body weight and food consumption after the first dose, yet usually recovered afterwards in 2-3 days without much influences by subsequent doses after the first dose.

In vitro toxicity was also investigated, by measuring Caspase 3/7, for normal human PBMCs (three healthy donors), made of innate and adaptive immune cells. Our results showed that the PBMCs viability was not significantly decreased even at 800 $\mu$g/mL injection of recombinant polypeptide CRYA_1B (SEQ ID NO: 9) (FIG. 25) which corroborated the in vivo safety profile without adverse events at comparable drug serum peak concentration. Together, this indicates that recombinant polypeptide CRYA_1B (SEQ ID NO: 9) potently activates long-term adaptive immunity devoid of cancer relapses and noticeable adverse events.

Discussion. Despite the clinical success of checkpoint-blockading cancer immunotherapy for solid tumours such as anti-PD-1 therapy, there were only limited subset of patients with PD-L1+ tumour phenotype responding to anti-PD-1 therapy and very significant numbers of responding patients were subject to recurrence (mean time to relapse: 624 days) largely due to the natural selection of neoantigenloss and/or MHC-I-deficient tumour variants resulting from the immune pressure of repeated immunotherapy treatments. For better therapeutic efficacy and wider patient coverage without tumour phenotype restriction, the personalised DC-mediated neoantigen-based vaccines such as ex vivo DC vaccine, long-peptide vaccine, RNA vaccine and DNA vaccine invoked the de novo polyclonal T-cell immune responses with encouraging results. Although the polyclonal T cells can mitigate the risk of tumour outgrowth from the neoantigen-loss variants, the total loss of MHC-I presentation resulting from $\beta$2-microglobulin deficiency still remains as an effective tumour escape mechanism. Moreover, the ex vivo in silico neoantigen prediction accuracy challenge from patient's resectable tumour samples, problematic for inoperable tumours, the time-consuming (about 3-5 months) and costly GMP manufacturing are still the daunting issues to be resolved. In contrast, the intratumoural oncolytic virus (OV) therapy, an antigen-agnostic vaccine without ex vivo neoantigen prediction, uses the replication-competent adapted virus to selectively infect, multiply and lyse tumour cells via necroptosis, a programmed necrosis, which causes the permeability of cell membrane and the release of the tumor antigens and DAMPs for immunogenic cell death via endogenous DCs. Nevertheless, the systemic intravenous infusion of OV is required for the effective treatment of distant metastatic tumours; however, such systemic infusion will cause OV to be rapidly diluted in the circulation, neutralised by serum factors and sequestered in the liver and spleen. Consequently, the IV infusion of OV will require substantially higher dose, subject to manufacturing technology capability, by more than two orders of magnitude in order to achieve similar efficacy as that of intratumoural injection except with a potential dose-limiting Grade 4 toxicity. Furthermore, a biosafety regulatory approval is required for each clinical grade OV and the manufacturing technology capability for the required high clinical IV dose is still a challenge. In contrast, recombinant polypeptide CRYA_1B is a recombinant protein using the standard biologic manufacturing process. Moreover, recombinant polypeptide CRYA_1B can systemically induce multiple personalised in situ neoantigens to mitigate the escape of neoantigen-loss variants, synergistically activate CD4+ T cells, via HSP70/HSP90- peptide, and NK cells, via CD4+ helper's interleukin-2 priming for effective recognition of HSP70- expressing tumour cells, to eradicate MHC-I-deficient (NK cells susceptible) variants, and potently trigger CD70-CD27 ligation for reduced PD-1 expression on T cells to minimise PD-1/ PD-L1-mediated immune peripheral tolerance. Thus, in light of attenuated immunogenicity such as neoantigen-loss, MHC-I-deficient variants and PD-1/PD-L1-mediated immune tolerance, and the immunosuppressive tumour microenvironment constituents such as myeloid-derived suppressor cells, tumour-associated macrophages and regulatory T cells, we chose to focus on how to reprogram the immunosuppressive tumour microenvironment for better DC activation in the first place. At this writing, we had screened, identified and verified an adjuvant synergistic with recombinant polypeptide CRYA_1B for reshaping tumour microenvironment, resulting in an increase of CR for both MC38 and E0771 colorectal and breast syngeneic tumour models respectively in immunocompetent mice. Together, the first-in-class recombinant $\alpha$-crystallin recombinant polypeptide CRYA_1B showed the potent, systemic, real-time and *in situ* induction of pre-apoptotic personalised tumour-derived CRT-peptide, HSP70-peptide and HSP90- peptide, irrespective of solid-tumour cancer types and tumour phenotypes, elicited anti-tumour long-term immunity with T-cell polyclonal diversity to prevent cancer relapses, and also demonstrated minimal toxicity and adverse events to normal cells and hosts respectively.

**EXAMPLE 5** - **FLT3L Expansion of Dendritic Cells (DCs)**

*FLT3L Expansion of Dendritic Cells*

There exists a need in the art for alternative approaches to improve immune response by expanding dendritic cells at tumour sites, which are responsible for capturing tumour antigens and presenting antigens to T cells for T-cell activation/expansion. The ability of FMS-like tyrosine kinase 3 ligand (FLT3L) to expand rare human CD141+XCR1+DC or murine CD103+XCR1+DC at tumour sites was tested. Six healthy volunteers were treated with either placebo (n = 2) or FLT3L (n = 4; subcutaneous injection at 20 $\mu$g/kg/d) for 14 consecutive days. The HLA-DR+Lin- DCs population (2 days after the last FLT3L/Placebo injection) was 1.5% and 18.8% in PBMC from placebo and FLT3L-treated groups respectively (Table 6). The DC expansion ratio was 12.33-fold after FLT3L treatment. Thus, subcutaneous injection of FLT3L expands HLA-DR+Lin- DCs in human PBMC from healthy volunteers.

**Table 6. HLA-DR+Lin-DC (Dendritic Cell) Population Following Treatment with FLT3L**

| | % HLA-DR+Lin-DC cells | |
|---|---|---|
| Sample | Placebo Treatment (n=2) | FLT3L Treatment (n=4) |
| Healthy human volunteer | 1.5 | 18.8 |
| Healthy rhesus macaques | 1.0 | 12.6 |
| Advanced stage lung cancer patient | 1.6 | 7.25 |

Six healthy rhesus macaques were treated with either placebo (n = 2) or FLT3L (n = 4; intravenous injection at 560 $\mu$g/m$^2$) for 7 consecutive days. The HLA-DR+Lin- DCs population (2 days after the last FLT3L/Placebo injection) was 1.0% and 12.6% in PBMC from placebo and FLT3L-treated groups respectively (Table 6). The DC expansion ratio was 12.6-fold after FLT3L treatment. Thus, intravenous injection of FLT3L expands HLA-DR+Lin- DCs in PBMC from healthy rhesus macaques.

Six advanced-stage lung cancer patients, with pre-treatment serum FLT3L ratio: 0.5-0.6x (relative to healthy), were treated with either placebo (n = 2) or FLT3L (n = 4; subcutaneous injection at 20 $\mu$g/kg/d) for 14 consecutive days. The HLA-DR+Lin- DCs population (2 days after the last FLT3L/Placebo injection) was 1.6% and 7.25% in PBMC from placebo and FLT3L-treated groups respectively. The DC expansion ratio was 4.53-fold after FLT3L treatment for cancer patients. Thus, FLT3L expands HLA-DR+Lin- DCs in human PBMC from cancer patients, but less than the fold expansion in healthy humans (12.33-fold).

*Determination of FLT3L intravenous dosing duration for cancer patients.*

Twenty advanced-stage lung cancer patients, with pre-treatment IL-10/IgG ratio: 1.45-1.6x (relative to healthy), were treated daily with FLT3L intravenous injection at 6 $\mu$g/kg for a various durations of time in order to determine the total length of dosing time required in order to achieve at least a 12-fold expansion of HLA-DR+Lin- DCs (fold increase observed in healthy humans in above experiment). The serum FLT3L level was determined by ELISA. The IL-10/IgG level was determined by Luminex assays. Table 7 shows the results of this experiment.

**Table 7. FLT3L ratios (relative to healthy)**

| Serum FLT3L Ratio (Relative to Healthy) | FLT3L Intravenous Dosing Duration (Days) |
|---|---|
| 1x | 7 |
| 0.9x | 10 |
| 0. 8x | 12 |

(continued)

| Serum FLT3L Ratio (Relative to Healthy) | FLT3L Intravenous Dosing Duration (Days) |
|---|---|
| 0.7x | 12 |
| 0. 6x | 12 |
| 0. 5x | 14 |
| 0.4x | 16 |
| 0.3x | 18 |
| 0.2x | 20 |
| 0.1x | 23 |

To determine the FLT3L intravenous dose determination for cancer patients, twenty advanced-stage lung and pancreatic cancer patients, with pre-treatment serum FLT3L ratio: 0.6-0.8x (relative to healthy), were treated daily with FLT3L intravenous injection for 12 consecutive days at trial doses ($\mu$g/kg) to meet the following criteria. The criteria of HLA-DR+Lin- DCs expansion ratio in PBMC from cancer patients was set as at least 12-fold (similar to that of healthy human) after FLT3L treatment for the determination of necessary FLT3L dose. The serum FLT3L level was determined by ELISA. The serum IL10/IgG level was determined by Luminex assays. FIG. 27 shows the correlation between FLT3L intravenous dose and IL10/IgG ratio relative to healthy, where the regression line was determined to be y=3.4X + 0.78.

From the experiments above, the conventional human FLT3L treatment (via subcutaneous injection) lasts for 14 days, using 740 $\mu$g/m$^2$ (20 $\mu$g/kg) dose. The 7-day FLT3L treatment (12.6x, via intravenous injection 560 $\mu$g/m$^2$ dose) achieves similar DC-expansion efficacy as that of 14-day (12.33x, via subcutaneous injection 740 $\mu$g/m$^2$ dose) for healthy subjects. In the case of cancer patients with limited DC expansion ability (cancer patients: 4.53x v.s. healthy: 12.33x) and limited life span, a more effective intravenous injection is used for FLT3L treatment with optimised dose and duration.

**EXAMPLE 6 - Effects of IFNgamma on IL-10 and CTLA-4 expression levels in human PBMC**

Maximum Serum interferon gamma (IFNg) concentration (Cmax) and half life was tested healthy human volunteers. Six healthy volunteers were intravenously injected with either placebo or IFNg (ACTIMMUNE®) at specified doses and the Cmax levels of IFNg was measured (Table 8). The Cmax for 50 and 70 $\mu$g dose was 1 ng/ml and 1.75 ng/ml respectively. The serum half-life of IFNg was approximately 30 min. The $C_{avg(1hr)}$, 1-hour average concentration, of 50 $\mu$g dose was 0.6 ng/ml [(1 + 0.5 + 0.25) / 3 = 0.58], whereas the $C_{avg(1hr)}$ of 70 $\mu$g dose was 1 ng/ml [(1.75 + 0.875 + 0.4375) / 3 = 1.02]. The Cmax level of serum IFNg was determined by ELISA.

**Table 8. Effects of IFNgamma on CTLA4 expression in healthy humans**

| Treatment - IFNg amount | Cmax (ng/ml) | CTLA4 Expression MFI | Fold Change relative to placebo | IL-2 (% of Placebo) |
|---|---|---|---|---|
| Placebo | 0 | 100 | 1x | 100 |
| 50$\mu$g | 1 | 300 | 3x | 100 |
| 70$\mu$g | 1.75 | 350 | 3.5x | 95 |
| 100$\mu$g | 2.25 | 700 | 7x | 75 |
| 150$\mu$g | 3 | 900 | 9x | 60 |

CTLA4 expression was measured in human PBMC from human healthy volunteers intravenously injected with IFNg. Six healthy volunteers were intravenously injected with either placebo or IFNg (ACTIMMUNE®) at specified doses and CTLA4 expression was measured (Table 8). The CTLA-4 expression MFI (Mean Fluorescence Intensity) was assessed by FACS analysis. Fold change was calculated relative to Placebo. The CTLA-4 expression in PBMC from healthy volunteers positively correlates with the injected IFNg doses.

IL-2 production was measured in PBMC isolated from human healthy volunteers intravenously injected with IFNg. Six healthy volunteers were intravenously injected with either placebo or IFNg (ACTIMMUNE®) at specified doses and IL-2 production was measured (Table 8). The PBMC cells were incubated with anti-CD3 mAb/CD80-Ig-coated beads to activate T cells for 48 hours. The IL-2 level was determined by ELISA. The IL-2 production in PBMC from healthy volunteers negatively correlates with the injected IFNg doses and the CTLA-4 expression.

To stimulate IL-10 production, the human PBMC cells were incubated with Staphylococcus aureus cowan 1 strain (SAC) at a 1:200 dilution for 48 hours. Following this incubation, IFNg was added at different concentrations for 1 hour. IL-10 expression was determined by ELISA and shown in Table 9. These results show that Interferon gamma (IFNg) reduces IL-10 in human PBMC.

**Table 9. IL-10 concentration following incubation of PBMC cells with SAC**

| Treatment | IL-10 Concentration ng/ml | IL-10 concentration as a percentage of control |
|---|---|---|
| Control | 4.1 | 100 |
| 0.6 ng/ml IFN gamma | 3.9 | 95 |
| 1.0 ng/ml IFN gamma | 2.74 | 67 |

Thus, the above experiments show that Interferon gamma (IFNg) reduces IL-10, reprogramming tumour microenvironment for better dendritic cell functionality, but simultaneously up-regulates CTLA-4, impeding T-cell activation, in human PBMC. To minimize two conflicting effects, the optimal dose of IFNg was determined to be 70 $\mu$g (a fixed dose with Cmax: 1.75 ng/mL; $C_{avg(1hr)}$: 1 ng/mL), which down-regulates IL-10 by 33% without compromising IL-2 production by activated T cells.

**EXAMPLE 7** - **IFNgamma reprograms M2 TAM**

Next, we tested if IFNgamma could induce reprogramming of M2-like tumour-associated macrophage (TAM) into M1 TAM, which are responsible for the secretion of IL-12, IL-15, IFN alpha, and IFN beta after calreticulin-mediated activation. Healthy human CD14[+] monocytes were cultured in presence of M-CSF (1 $\mu$g/mL), IL-4 (1 $\mu$g/mL), and IL-10 (1 $\mu$g/mL) to differentiate into M2-like macrophage cells. The differentiated M2 macrophage cells were pulsed with Interferon gamma (IFNg)(1 ng/mL) for 1 hour. 4 hours later, levels of M2 cells and M1 cells were measured. Up-regulation of surface expression marker CD80 (M1 macrophages) and down-regulation of MARCO and CD163 (M2 macrophages) was measured by FACS analysis and shown in FIG. 28. M2 macrophages highly expressed MARCO and CD163 whereas the M1 macrophages highly expressed CD80. Only activated M1 macrophages, not M2 macrophages, can produce pro-inflammatory cytokines such as IL-12 and IL-15. Altogether, this shows that IFNg can reprogram M2 macrophages into M1 macrophages. Specifically, it shows that Interferon gamma (IFNg), at low intravenous injection dose of 70 $\mu$g (a fixed dose with $C_{avg(1hr)}$: 1 ng/mL) could reprogram the M2-like tumour-associated macrophage (TAM) to become M1 TAM and induce secretion of IL-12, IL-15, IFN alpha, and IFN beta, following calreticulin-mediated activation. Furthermore, IFNg reduces IL-10, reprogramming tumour microenvironment for better dendritic cell functionality.

**EXAMPLE 8 - XCL1 recruits FLT3L-expanded XCR1+Dendritic Cells to tumour site**

Chemokine (C motif) ligand (XCL1) is the ligand for "C" sub-family of chemokine receptors (XCR1, also known as GPR5), serves as a chemoattractant for XCR1+ DCs to tumor sites. To test if XCL1 recruits XCR1+DCs to B16F10 or MC38 melanoma tumour sites, C57BL/6 mice were first inoculated with 2 x 10[6] B16F10 cells with or 2 x 10[6] MC38 cells, respectively to induce melanomas. Mice were transduced with XCL1 or with an empty vector and were all intravenously injected with FLT3L at 4 $\mu$g (560 $\mu$g/m[2]) for six days starting from tumour cell inoculation. Tumours were isolated 7 days after inoculation (1 day after the last FLT3L injection). Table 10 shows the recruitment of XRC1+DC cells to both types of tumour sites.

**Table 10. Recruitment of XCR1+ Dendritic cells to B16F10 and MC38 melanoma sites**

| | Number of intratumoral XCR1+DC (10[5]/g tumor) | |
|---|---|---|
| | B16F10 | MC38 |
| Empty Vector | 0.6 | 0.8 |
| XCL1 | 2.2 | 2 |

Thus, these results show that following treatment with FLT3L, expanded XCR1+ DCs remain in blood circulation system and XCL1 recruits XCR1+ DCs from blood vessels to tumour sites.

**EXAMPLE 9 - Recombinant Polypeptide CRYA_1B (SEQ ID NO: 9) activates macrophages and NK cells to release XCL1**

*Cytokine-activated NK cells release XCL1*

To determine if NK cells treated with cytokines release XCL1, NK cells were purified from splenocytes of C57BL/6 mice using an NK Cell Isolation Kit. The NK cells were activated by IL-12 (80 ng/ml), or IL-15 (80 ng/ml), or IFN alpha (1000 IU/ml), or IFN beta (1000 IU/ml) or a combination thereof for 20 min. The XCL1 MFI (Mean Fluorescence Intensity) was assessed by FACS analysis and shown in Table 11. Treatment with IL-12, IL-15, IFN alpha and IFN beta resulted in the highest fold increase (12-fold increase) in XCL1 release from NK cells relative to the control.

**Table 11. XCL1 release from NK cells following treatment with cytokines**

|  | XCL1 MFI (Fold Change) |
|---|---|
| Control | 1x |
| IL-12 | 3.5x |
| IL-15 | 3.5x |
| IFN alpha | 2.5x |
| IFN beta | 2.5x |
| IL-12 + IL-15 | 8.5x |
| IL-12 + IL-15 + IFN alpha + IFN beta | 12x |

*Calreticulin (CRT) activated macrophages produce IL12, IL15, IFNa and IFNb*

To determine if calreticulin (CRT)-activated macrophages release the cytokines IL12, IL15, IFN alpha (IFNa) and IFN beta (IFNb), RAW264.7 macrophage (M1) cells were pulsed with calreticulin (10 $\mu$g/ml) for 20 min. Mean fluorescence intensity (MFI) of IL-12, IL-15, IFNa and IFNb was assessed by FACS analysis as a measure of cytokine release (Table 12). IL-12, IL-15, IFNa and IFNb were all produced by calreticulin activated macrophages. IFNa and IFNb had the highest levels of release following treatment with calreticulin.

**Table 12. Cytokine release following treatment of M1 macrophages with calreticulin**

|  | MFI (Fold Change) | | | |
|---|---|---|---|---|
|  | IL-12 | IL-15 | IFN alpha | IFN beta |
| Control | 1x | 1x | 1x | 1x |
| CRT | 3.5x | 3.5x | 7.5x | 7x |

*Calreticulin-mediated Macrophages and NK cells synergistically recruits XCR1 +DC to tumour sites*

Next, we tested if Calreticulin (CRT)-mediated macrophages could activate NK cells in order to promote XCL1 release and recruitment of XCR1+ DCs to MC38 (colon carcinoma) or C1498 (acute myeloid leukemia) tumour sites. CRT is the ligand for CD91, which is expressed abundantly on macrophages. XCL1 is the ligand for XCR1, serves as a chemoattractant for XCR1+ DCs. C57BL/6 mice were inoculated with 2 x 10$^6$ MC38 colon carcinoma cells (n = 3) or MC38.CRT (n = 18; MC38 cells incubated with recombinant CRT at 3 $\mu$g per 10$^6$ cells in PBS for 30 min, and followed by three washes). The MC38.CRT (MC38 cells expressing surface CRT) tumour-bearing mice were further divided into six groups: MC38.CRT, MC38.CRT/anti-XCL1 (XCL1 depletion), MC38.CRT/anti-NK1.1 (NK cells depletion), MC38.CRT/anti-F4/80 (macrophage depletion), MC38.CRT/anti-CD91 (CD91 receptor blockade), and MC38.CRT/anti-CRT (CRT depletion). Additionally, all mice were intravenously injected with FLT3L (4 $\mu$g: 560 $\mu$g/m$^2$/d) for six days starting from tumour inoculation. Tumours were isolated 7 days after inoculation (1 day after the last FLT3L injection). Table 13 shows the number of XCR1+ Dendritic cells recruited to tumour sites. While MC38.CRT showed an increase in XCR1+dendritic cell recruitment, XCL1 depletion, NK cell depletion, macrophage depletion, CD91 receptor blockade, CRT depletion did not, suggesting that CRT mediates activation of NK cells in order to promote XCL1 release and recruitment of XCR1+dendritic cells to colon carcinoma tumour sites.

**Table 13. XCR1+ Dendritic cell recruitment to MC38 colon carcinoma sites following treatment with CRT.**

| | Number of intratumoral XCR1+Dendritic Cells ($10^5$/gram tumour) |
|---|---|
| MC38 | 0.8 |
| MC38.CRT | 2 |
| MC38.CRT/anti-XCL1 (XCL1 depletion) | 0.8 |
| MC38.CRT/anti-NK1.1 (NK cells depletion) | 0.8 |
| MC38.CRT/anti-F4/80 | 0.84 |
| MC38.CRT/anti-CD91 (CD91 receptor blockade) | 0.81 |
| MC38.CRT/anti-CRT (CRT depletion) | 0.8 |

Next, C57BL/6 mice were inoculated with 2 x $10^6$ C1498 acute myeloid leukemia cells (n = 3) or C1498.CRT (n = 18; C1498 cells incubated with recombinant CRT at 3 $\mu$g per $10^6$ cells in PBS for 30 min. and followed by three washes). The C1498.CRT (C1498 cells expressing surface CRT) tumour-bearing mice were further divided into six groups: C1498.CRT, C1498.CRT/anti-XCL1 (XCL1 depletion), C1498.CRT/anti-NK1.1 (NK cells depletion), C1498.CRT/anti-F4/80 (macrophage depletion), C1498.CRT/anti-CD91 (CD91 receptor blockade), and C1498.CRT/anti-CRT (CRT depletion). Additionally, all mice were subcutaneously injected with FLT3L (4 $\mu$g: 560 $\mu$g/m2/d) for six days starting from tumour inoculation. Tumours were isolated 7 days after inoculation (1 day after the last FLT3L injection). Table 14 shows the number of XCR1+ Dendritic cells recruited to tumour sites. While C1498.CRT showed an increase in XCR1+dendritic cell recruitment, XCL1 depletion, NK cell depletion, macrophage depletion, CD91 receptor blockade, CRT depletion did not, suggesting that CRT mediates activation of NK cells in order to promote XCL1 release and recruitment of XCR1+dendritic cells to AML tumour sites.

**Table 14. XCR1+ Dendritic cell recruitment to C1498 acute myeloid leukemia tumour sites following treatment with CRT**

| | Number of intratumoral XCR1+Dendritic Cells ($10^5$/gram tumour) |
|---|---|
| C1498 | 0.7 |
| C1498.CRT | 1.82 |
| C1498.CRT/anti-XCL1 (XCL1 depletion) | 0.7 |
| C1498.CRT/anti-NK1.1 (NK cells depletion) | 0.7 |
| C1498.CRT/anti-F4/80 | 0.76 |
| C1498.CRT/anti-CD91 (CD91 receptor blockade) | 0.71 |
| C1498.CRT/anti-CRT (CRT depletion) | 0.7 |

*Recombinant Polypeptide CRYA_1B (SEQ ID NO: 9) induces calreticulin (CRT) expression on cancer cell lines*

To determine if Recombinant Polypeptide CRYA_1B (SEQ ID NO: 9) induces calreticulin (CRT) in murine B16F10 melanoma, human HCT15 colon cancer, and human MCF7 breast cancer cell lines, 4 x $10^5$ cells were plated in 12-well plates and the next day the cells were treated with CRYA_1B for 60 min. The percentage of CRT-expressing cells was assessed by FACS analysis. Table 14 shows that CRYA_1B increases CRT expression of each type of cell in comparison to control.

**Table 15. CRT expression on cancer cell lines following treatment with recombinant polypeptide CRYA_1B**

| | % CRT | | |
|---|---|---|---|
| | B16F10 cells | HCT15 cells | MCF7 cells |
| Control | 13 | 12 | 15 |
| 35 $\mu$g/ml CRYA_1B | 25 | 22 | 25 |
| 50 $\mu$g/ml CRYA_1B | 27 | 33 | 28 |

(continued)

| | % CRT | | |
| --- | --- | --- | --- |
| | B16F10 cells | HCT15 cells | MCF7 cells |
| 70 μg/ml CRYA_1B | 35 | 38 | 35 |

*CRYA_1B induces HSP70 expression on cancer cell lines*

To determine if CRYA_1B induces HSP70 in murine B16F10 melanoma, human HCT15 colon cancer, and human MCF7 breast cancer cell lines, $4 \times 10^5$ cells were plated in 12-well plates and the next day the cells were treated with CRYA_1B for 110 min. The percentage of HSP70-expressing cells was assessed by FACS analysis. Table 16 shows that CRYA_1B increases HSP70 expression of each type of cell in comparison to control.

**Table 16. HSP70 expression on cancer cell lines following treatment with recombinant polypeptide CRYA_1B**

| | % HSP70 | | |
| --- | --- | --- | --- |
| | B16F10 cells | HCT15 cells | MCF7 cells |
| Control | 12 | 13 | 15 |
| 35 μg/ml CRYA_1B | 23 | 25 | 26 |
| 50 μg/ml CRYA_1B | 25 | 32 | 35 |
| 70 μg/ml CRYA_1B | 35 | 36 | 40 |

*CRYA_1B induces HSP90 expression on cancer cell lines*

To determine if CRYA_1B induces HSP90 in murine B16F10 melanoma, human HCT15 colon cancer, and human MCF7 breast cancer cell lines, $4 \times 10^5$ cells were plated in 12-well plates and the next day the cells were treated with CRYA_1B for 110 min. The percentage of HSP90-expressing cells was assessed by FACS analysis. Table 17 shows that CRYA_1B increases HSP90 expression of each type of cell in comparison to control.

**Table 17. HSP90 expression on cancer cell lines following treatment with recombinant polypeptide CRYA_1B**

| | % HSP90 | | |
| --- | --- | --- | --- |
| | B16F10 cells | HCT15 cells | MCF7 cells |
| Control | 12 | 13 | 17 |
| 35 μg/ml CRYA_1B | 26 | 26 | 25 |
| 50 μg/ml CRYA_1B | 27 | 32 | 28 |
| 70 μg/ml CRYA_1B | 31 | 40 | 37 |

Therefore, altogether recombinant polypeptide CRYA_1B is a potent calreticulin (CRT)-inducer for both murine and human tumours. CRYA_1B induces CRT expression on tumours. CRT in turn activates M1 tumour-associated macrophages via binding to CD91 and inducing the production of IL12, IL15, IFN alpha, and IFN beta. This combination of cytokines activate intratumoral NK cells, resulting in the production of XCL1, a ligand for XCR1 receptor. Consequently, XCL1 recruits XCR1+ DCs from blood vessels to tumour sites. Following induction of CRT expression on tumours, CRYA_1B also induces the expression of HSP70/90 on tumours, which in turn act to mature and to activate intratumoral XCR1+ DCs and induce migration of these dendritic cells with captured antigens to lymph nodes in order to prime and expand T cells. As such, treatment with CRYA_1B is a new method for inducing dendritic cells maturation and expansion, thereby priming the immune cell response.

**EXAMPLE 10 - In vivo Combination therapy for treatment of B16F10 melanoma using FLT3L, IFNgamma and Recombinant Polypeptide CRYA_1B**

To test the effects of FLT3L, IFNg and CRYA_1B in an in vivo mouse model, a combination therapy of FLT3L, IFNg and CRYA_1B (CRT-Inducer) was designed. FIG. 29 shows a schematic of the various controls and combination treatments. The C57BL/6 mice were subcutaneously inoculated with 1 x $10^6$ of B16F10 cells (poorly immunogenic and aggressive phenotype of melanoma cells) for each group: Control (n = 5), FLT3L/IFNg/CRYA_1B (n = 10), FLT3L/CRYA_1B (n = 10), IFNg/CRYA_1B (n = 10), and CRYA_1B (n = 10). The tumours reached approximately 40-50 $mm^3$ in four days (Day 4). For FLT3L treatment, the FLT3L was then intravenously injected into mice at 4 $\mu$g (560 $\mu$g/$m^2$) daily for six consecutive days. For IFNg treatment, the mice were intravenously injected with IFNg at 0.3 $\mu$g four hours before the first CRYA_1B treatment. For CRYA_1B therapy, two-day treatment (20 mg/kg and 13.5 mg/kg via intravenous injections) followed by two-day non-treatment as one cycle, was repeated for three cycles.

**EXAMPLE 11** - **In vivo Combination therapy for treatment of B16F10 melanoma using FLT3L, IFNgamma, TNFalpha Receptor, Recombinant Polypeptide CRYA_1B and PD1 (nivolumab) blockade**

We hypothesize that TNFalpha receptor (TNFaR) adjuvant could enhance the anti-PD1 treatment by relaxing the temporal window, the treatment of which can be in either priming phase or any boosting phase (1° - 5° boosting). We also hypothesize that TNFaR could also accelerate the speed in the formation of central memory T cells resulting in shortened prime-boost interval. To test the effects of FLT3L, IFNg, TNFalpha Receptor (TNFaR), CRYA_1B and a single dose of PD1 (nivolumab) blockade in an in vivo mouse model of melanoma, a combination therapy of FLT3L, IFNg, CRYA_1B (CRT-Inducer) and a single PD1 blockade was designed (FIG. 31). Female C57BL/6 mice were subcutaneously inoculated with 1 x $10^6$ B16F10 cells (poorly immunogenic aggressive melanoma cells). IgG2a groups:

1) FLT3L/IFNg/TNFaR/CRYA_1B with IgG2a@Day 18 (n = 5),
2) FLT3L/IFNg/TNFaR/CRYA_1B with IgG2a@Day 22 (n = 5),
3) FLT3L/IFNg/TNFaR/CRYA_1B with IgG2a@Day 30 (n = 5), and
4) FLT3L/IFNg/TNFaR/CRYA_1B with IgG2a@Day 37 (n = 5);

**Anti-PD1 groups:**

1) FLT3L/IFNg/TNFaR/CRYA_1B with anti-PD1@Day 18 (n = 5),
2) FLT3L/IFNg/TNFaR/CRYA_1B with anti-PD1@Day 22 (n = 5),
3) FLT3L/IFNg/TNFaR/CRYA_1B with anti-PD1@Day 30 (n = 5), and
4) FLT3L/IFNg/TNFaR/CRYA_1B with anti-PD1@Day 37 (n = 5).

For anti-PD1/IgG2a treatment groups, mice were administered once intravenously with either murine anti-PD1 (Rat IgG2a isotype) for anti-PD1 groups, or Rat IgG2a for IgG2a Control groups, all at 3 mg/kg, on the specified day. The tumours reached approximately 55 $mm^3$ in five days (Day 5). Tumour size was measured in two dimensions using caliper, and the volume was determined in the following formula: V = 0.5 x L x $W^2$, where L and W are the long and short diameters of the tumour respectively. FLT3L was then intravenously injected into mice at 4 $\mu$g (560 $\mu$g/$m^2$) daily for seven consecutive days. The mice were intravenously injected with IFNg at 0.3 $\mu$g* four hours before TNFaR injection (20 $\mu$g, via subcutaneous injection) immediately followed by the CRYA_1B treatment. *: Conversion from human fixed 70 $\mu$g dose [assuming human 60kg, 70/60 x 37 (human Km)** x 0.007 (mouse body surface area)** = 0.3 $\mu$g]. CRYA_1B was used both for priming and for boosting of T-cells. For CRYA_1B therapy to prime T-cells, the two-day treatment (20 mg/kg and 14 mg/kg, respectively was provided via intravenous injections) followed by two-day non-treatment as one cycle, was repeated for three cycles, starting on Day 12. For CRYA_1B therapy to boost T-cells, the two-day treatment (20 mg/kg and 14 mg/kg, respectively was provided via intravenous injections) followed by two-day non-treatment as one cycle, was repeated for two cycles, starting on Day 31. FIG. 31 shows a schematic of the dosing regimen. Dose conservation between animals and human was calculated according to Nair et. al., J. Basic Clin Pharm,, 27-31, 201. On Days 19,23,31, and 38, the PBMC of mice from both anti-PD1 and IgG2a groups were harvested and analyzed by flow cytometry to determine the proportion of CD8+CD44+ (antigen-experienced) T-cells in the population. For all anti-PD1 groups, the central memory T cells (CD8+CD44+CD127+KLRG1-CD62L+), effector memory T cells (CD8+CD44+CD127+ KLRG1+CD62L-), and effector T cells (CD8+CD44+CD127-KLRG1+CD62L-) were all about equally divided, with central memory T cells at 34% (Table 18). For all IgG2a groups, the dominant population were the effector memory T cells (70%), with central memory T cells at 5%.

**Table 18. T-cell population following treatment with anti-PD1 antibody or IgG2a control**

| | Percentage of T-cells | | |
|---|---|---|---|
| | CD8+CD44+ CD127+KLRG1-CD62L+ (central memory) | CD8+CD44+ CD127+KLRG1+CD62L-(effector memory) | CD8+CD44+ CD127-KLRG1+CD62L-(effector) |
| IgG2a | 5 | 70 | 25 |
| Anti-PD1 | 33.6 | 33.4 | 33 |

For the groups which were injected with a single anti-PD1 dose, the percentage of antigen-experienced central memory T cells was 6.6-fold that of without anti-PD1 treatment (33-34% v.s. 5% w/o anti-PD1). Conversion of the effector memory to central memory T cells could be via the up-regulation of CD62L and down-regulation of KLRG1.

**EXAMPLE 12** - **In vivo Combination therapy for treatment of B16F10 melanoma using FLT3L, IFNg, TNFalpha Receptor, recombinant polypeptide CRYA_1B and multiple PD1 (nivolumab) blockade doses**

To test the effects of FLT3L, IFNg, TNFalpha Receptor (TNFaR), CRYA_1B and a multiple dose of PD1 blockade in an in vivo mouse model, a combination therapy was designed (FIG. 32). Female C57BL/6 mice were subcutaneously inoculated with $1 \times 10^6$ B16F10 cells (poorly immunogenic aggressive melanoma cells). Treatment groups:

1) FLT3L/IFNg/TNFaR/CRYA_1B/anti-PD1x1@Day 22 (n = 5),
2) FLT3L/IFNg/TNFaR/CRYA_1B/anti-PD1x2@Day 22/25 (n = 5),
3) FLT3L/IFNg/TNFaR/CRYA_1B/anti-PD1x3@Day 22/25/28 (n = 5),
4) FLT3L/IFNg/TNFaR/CRYA_1B/anti-PD1x4@Day 22/25/28/31 (n = 5),
5) FLT3L/IFNg/TNFaR/CRYA_1B/anti-PD1x5@Day 22/25/28/31/34 (n = 5),
6) FLT3L/IFNg/TNFaR/CRYA_1B/anti-PD1x6@Day 22/25/28/31/34/37 (n = 5), and
7) FLT3L/IFNg/TNFaR/CRYA_1B/anti-PD1x7@Day 22/25/28/31/34/37/40 (n = 5).

For anti-PD1 treatments, the mice were administered intravenously with murine anti-PD1 (Rat IgG2a isotype), all at 3 mg/kg on the specified day(s). The tumours reached approximately 55 mm³ in five days (Day 5). Tumour size was measured in two dimensions using caliper, and the volume was determined in the following formula: $V = 0.5 \times L \times W^2$, where L and W are the long and short diameters of the tumour respectively. FLT3L was then intravenously injected into mice at 4 $\mu$g (560 $\mu$g/m²) daily for seven consecutive days. The mice were intravenously injected with IFNg at 0.3 $\mu$g* four hours before TNFaR injection (20 $\mu$g, via subcutaneous injection) immediately followed by the CRYA_1B treatment. *: Conversion from human fixed 70 $\mu$g dose [assuming human 60Kg, 70/60 x 37 (human Km)** x 0.007 (mouse body surface area)** = 0.3]. For CRYA_1B therapy to prime T cells, the two-day treatment (20 mg/kg and 14 mg/kg, respectively was provided via intravenous injections) followed by two-day non-treatment as one cycle, was repeated for three cycles, starting on Day 12. Dose conservation between animals and human was calculated according to Nair et. al., J. Basic Clin Pharm,, 27-31, 201. On Day 23,26,29,32,35,38, and 41, the PBMC of mice from all anti-PD1 groups were harvested and analyzed to determine the proportion of CD8+CD44+ (antigen-experienced) T-cells. The T-cell proportions were plotted as a relative percentage to that of single anti-PD1 treatment. Relative percentages of CD8+CD44+ cells are shown in Table 19.

**Table 19. Proportion of CD8+CD44+ T cells following multiple dose treatment using anti-PD1**

| | CD8+CD44+ T cells (relative percentage in comparison to single anti-PD1 treatment) |
|---|---|
| Anti-PD1x1 | 100 |
| Anti-PD1x2 | 90 |
| Anti-PD1x3 | 80 |
| Anti-PD1x4 | 75 |
| Anti-PD1x5 | 70 |
| Anti-PD1x6 | 65 |
| Anti-PD1x7 | 60 |

As discussed, CRYA_1B can be used both to prime and to boost T-cells. As shown by these results, an optimized T-cell prime to boost with 'shorter' interval is required for the rapid proliferation of Tcells in the boosting phase. Specifically, this allows for boosting of the previously primed central memory T cells and allow further increase the total number of antigen-experienced T cells in the hosts. Priming and boosting of T-cells that recognize tumor associated antigens, is particularly advantageous for particularly for cancer patients with shortened or limited life span and patients with large tumour sizes.

Although the percentage of CD8$^+$ central memory is all about the same (about 33%) after any single anti-PD1 treatment in either priming or any boosting phase (1° - 5° boosting), anti-PD1 treatment during the T-cell priming phase results in a higher absolute number of CD8$^+$ central memory Tcells than in T-cell boosting phase. This is because the T-cell overall expansion rate in priming phase (>= 10000-fold equivalent to >13 cell divisions) is substantially greater than that of in any boosting phase (5-8 cell divisions)(Haring et. al., Immunity,19-29, 2006, Fraser et. al., Immunity, 171-183, 2013). A maximum number of 'seven' anti-PD1 injections is tolerable. But, as shown above, a single anti-PD1 treatment achieves a higher absolute number of CD8$^+$CD44$^+$T cells and in turn more CD8$^+$ central memory T cells, than multiple anti-PD1 treatments. This is because prolonged PD1 inhibition impairs the survival of T cells. (Odorizzi et. al., J. Exp. Med.,1125-1137, 2015). Thus, we determine that an optimal time window for single anti-PD1 treatment lies between the last injection of recombinant polypeptide CRYA_1B of the priming phase and the first injection of CRYA_1B during the first boosting phase.

## EXAMPLE 13 - T-cell Priming and T-cell Boosting Protocols

*T-cell Prime-IgG2a-Boost (x1) (Control)*

Female C57BL/6 mice were subcutaneously inoculated with 1 x 10$^6$ B16F10 cells (poorly immunogenic aggressive melanoma) cells (n = 10) (FIG. 33). The tumours reached approximately 50-55 mm$^3$ in 5 days (Day 5) to start the experiments. Tumour size was measured in two dimensions using caliper, and the volume was determined in the following formula: V = 0.5 x L x W$^2$, where L and W are the long and short diameters of the tumour respectively. For FLT3L treatment to expand dendritic cells, the FLT3L was then intravenously injected into mice at 4 $\mu$g (560 $\mu$g/m$^2$) daily for seven consecutive days. For IFNg treatment to reduce IL10 and reprogram M2 tumour-associated macrophage (TAM) into M1 TAM, the mice were intravenously injected with IFNg at 0.3 $\mu$g four hours before the first CRYA_1B treatment on Day 12. For CRYA_1B to prime T cells, the two-day treatment (20 mg/kg and 14 mg/kg, respectively via intravenous injections) followed by two-day non-treatment as one cycle, was repeated for three cycles. On Day 23, the mice were intravenously injected with Rat IgG2a at 3 mg/kg as the Control. On Day 25, the FLT3L treatment was repeated to expand dendritic cells by intravenous injection into mice at 4 $\mu$g (560 $\mu$g/m$^2$) daily for seven consecutive days. On Day 32, the IFNg treatment was repeated to reduce IL10 and reprogram M2 TAM into M1 TAM by intravenous injection into mice at 0.3 $\mu$g. Four hours later, T-cell boosting was performed by introducing a CRYA_1B two-day treatment (20 mg/kg and 14 mg/kg, respectively via intravenous injections) followed by two-day non-treatment as one cycle, of which was repeated for only two cycles (v.s. three cycles for priming) (FIG. 33). On Day 40, the complete tumour regression (CR) rate was 100%. *T-cell Prime-Boost Efficacy* - The curing efficacy was determined in terms of maximum cured tumour volume at 100% CR (complete tumour regression rate) relative to that of Prime-IgG2a-Boost protocol (Control) above. The baseline tumour volume, ~50 mm$^3$ at 100% CR, was determined using Prime-IgG2a-Boost (x1, a single boost) protocol as Control.

*T-cell Prime-PD1 (nivolumab) blockade-Boost (x1) for 300 mm$^3$ tumour*

Female C57BL/6 mice were subcutaneously inoculated with 1 x 10$^6$ B16F10 cells (poorly immunogenic aggressive melanoma cells)(n = 10). The tumours reached approximately 300-310 mm$^3$ in about 13 days (Day 13). Tumour size was measured in two dimensions using caliper, and the volume was determined in the following formula: V = 0.5 x L x W$^2$, where L and W are the long and short diameters of the tumour respectively. FIG. 34 shows a schematic of the dosing regimen. For FLT3L treatment to expand dendritic cells, the FLT3L was then intravenously injected into mice at 4 $\mu$g (560 $\mu$g/m$^2$) daily for seven consecutive days. For IFNg treatment to reduce IL10 and reprogram M2 tumour-associated macrophage (TAM) into M1 TAM, the mice were intravenously injected with IFNg at 0.3 $\mu$g four hours before the first CRYA_1B treatment on Day 20. For CRYA_1B to prime T cells, the two-day treatment (20mg/kg and 14 mg/kg via intravenous injections, respectively) followed by two-day non-treatment as one cycle, was repeated for three cycles. On Day 31, the mice were intravenously injected with murine anti-PD1 at 3 mg/kg. On Day 45 (14 days from the anti-PD1 injection), the FLT3L treatment was repeated to expand dendritic cells by intravenous injection into mice at 4 $\mu$g (560 $\mu$g/m$^2$) daily for seven consecutive days. On Day 52, the IFNg treatment was repeated to reduce IL10 and reprogram M2 TAM into M1 TAM by intravenous injection into mice at 0.3 $\mu$g. Four hours later, CRYA_1B T-cell boosting regimen was introduced by use of a two-day treatment (20mg/kg and 14 mg/kg respectively via intravenous injections) followed by two-day non-treatment as one cycle, of which was repeated for only two cycles (v.s. three cycles for priming). FIG. 34 shows the dosing regimen. On Day 60, the complete tumour regression (CR) rate was 100%.

Efficacy of Prime-PD1 (nivolumab) blockade-Boost (x1) for 300 mm$^3$ tumour- The curing efficacy was determined in terms of maximum cured tumour volume at 100% CR (complete tumour regression rate) relative to ~50 mm$^3$ at 100% CR of Prime-IgG2a-Boost (Control). Using a single anti-PD1 injection in between CRYA_1B priming and CRYA_1B boost (a single boost) for treatment of ~300 mm$^3$ tumour, we achieved approximately 6-fold curing efficacy in terms of cured tumour volume (~300 mm$^3$ v.s. ~50 mm$^3$). This correlates with the 6.6-fold central memory T cells available for boosting as shown previously (33% of Prime/anti-PD1 v.s. 5% of Prime/IgG2a).

*T-cell Prime-PD1 (nivolumab) blockade-Boost (x1) for 400 mm$^3$ tumour*

The female C57BL/6 mice were subcutaneously inoculated with 1 x 10$^6$ B16F10 cells (poorly immunogenic aggressive melanoma cells) (n = 10) The dosage regimen is shown in FIG. 35. The tumours reached approximately 400-410 mm$^3$ in ~14 days (Day 14) to start the experiments. Tumour size was measured in two dimensions using caliper, and the volume was determined in the following formula: V = 0.5 x L x W$^2$, where L and W are the long and short diameters of the tumour respectively. For FLT3L treatment to expand dendritic cells, the FLT3L was then intravenously injected into mice at 4 μg (560 μg/m$^2$) daily for seven consecutive days. For IFNg treatment to reduce IL10 and reprogram M2 tumour-associated macrophage (TAM) into M1 TAM, the mice were intravenously injected with IFNg at 0.3 μg four hours before the first CRYA_1B treatment on Day 21. For CRYA_1B to prime T cells, the two-day treatment (20mg/kg and 14 mg/kg via intravenous injections) followed by two-day non-treatment as one cycle, was repeated for three cycles. On Day 32, the mice were intravenously injected with murine anti-PD1 at 3 mg/kg. On Day 46 (14 days from anti-PD1 injection), the FLT3L treatment was repeated to expand dendritic cells by intravenous injection into mice at 4 μg (560 μg/m$^2$) daily for seven consecutive days. On Day 53, the IFNg treatment was repeated to reduce IL10 and reprogram M2 TAM into M1 TAM by intravenous injection into mice at 0.3 μg. Four hours later, we started CRYA_1B T-cell's boosting by two-day treatment (20 mg/kg and 14 mg/kg, respectively via intravenous injections) followed by two-day non-treatment as one cycle, of which was repeated for only two cycles (v.s. three cycles for priming). On Day 70, the complete tumour regression (CR) rate was 50%.

The curing efficacy was determined in terms of maximum cured tumour volume at 100% CR (complete tumour regression rate) relative to ~50 mm$^3$ at 100% CR of Prime-IgG2a-Boost (Control). Using a single anti-PD1 injection in between CRYA_1B priming and CRYA_1B boost (x1, a single boost) for 400 mm$^3$ tumour, only ~50% CR was achieved instead of 100% CR. This is due to larger tumour volume, due to aggressive tumour migration, resulting in metastases in mice, and the effector T-cell natural contraction after a single boost. Thus, mice were unable to maintain high enough CD8$^+$CD44$^+$ T cells for the elimination of all tumour metastases. To avoid continuous T-cell natural contraction reaching the eventual homeostasis, administration of multiple boosts (2-5 boosts) to maintain high levels of antigen-experienced CD8$^+$CD44$^+$ T-cells. The sustained number of CD8+CD44+ T cells, without loss is important for the total eradication of tumour metastases in the hosts. However, after repetitive boosts the fully differentiated T cells are susceptible to restimulation-induced cell death (Show et. al., Immunological Reviews, 68-82, 2010). Consequently, a maximum of 5 boosting cycles can be performed to prevent T-cell overstimulation leading to abrupt T-cell death.

*T-cell Prime-PD1 (nivolumab) blockade-Boost (x3) for 400 mm$^3$ tumour*

The female C57BL/6 mice were subcutaneously inoculated with 1 x 10$^6$ of B16F10 cells (poorly immunogenic aggressive melanoma cells)(n = 10). FIG. 36 shows a schematic of the dosing regimen. The tumours reached approximately 400-410 mm$^3$ in ~14 days (Day 14) to start the experiments. Tumour size was measured in two dimensions using caliper, and the volume was determined in the following formula: V = 0.5 x L x W$^2$, where L and W are the long and short diameters of the tumour respectively. For FLT3L treatment to expand dendritic cells, the FLT3L was then intravenously injected into mice at 4 μg (560 μg/m$^2$) daily for seven consecutive days. For IFNg treatment to reduce IL10 and reprogram M2 tumour-associated macrophage (TAM) into M1 TAM, the mice were intravenously injected with IFNg at 0.3 μg four hours before the first CRYA_1B treatment on Day 21. For CRYA_1B to prime T cells, the two-day treatment (20 mg/kg and 14 mg/kg, respectively via intravenous injections) followed by two-day non-treatment as one cycle, was repeated for three cycles. On Day 32, the mice were intravenously injected with murine anti-PD1 at 3 mg/kg. On Day 46 (14 days from anti-PD1 injection), the FLT3L treatment was repeated to expand dendritic cells by intravenous injection into mice at 4 μg (560 μg/m$^2$) daily for seven consecutive days. On Day 53, the IFNg treatment was repeated to reduce IL10 and reprogram M2 TAM into M1 TAM by intravenous injection into mice at 0.3 μg. Four hours later, we started CRYA_1B T-cell's boosting by two-day treatment (20 mg/kg and 14 mg/kg, respectively via intravenous injections) followed by two-day non-treatment as one cycle, of which was repeated for only two cycles (v.s. three cycles for priming). On Day 61, the FLT3L treatment was repeated to expand dendritic cells by intravenous injection into mice at 4 μg (560 μg/m$^2$) daily for seven consecutive days. On Day 68, the IFNg treatment was repeated to reduce IL10 and reprogram M2 TAM into M1 TAM by intravenous injection into mice at 0.3 μg. Four hours later, we started CRYA_1B T-cell's boosting by two-day treatment (20 mg/kg and 14 mg/kg, respectively via intravenous injections) followed by two-day non-treatment as one cycle, of which was repeated for only two cycles (v.s. three cycles for priming). On Day 76, the FLT3L treatment was repeated to expand dendritic cells by intravenous injection into mice at 4 $\mu$g (560 $\mu$g/m$^2$) daily for seven consecutive days. On Day 83, the IFNg treatment was repeated to reduce IL10 and reprogram M2 TAM into M1 TAM by intravenous injection into mice at 0.3 $\mu$g. Four hours later, we started CRYA_1B T-cell's boosting by two-day treatment (20 mg/kg and 14 mg/kg, respectively via intravenous injections) followed by two-day non-treatment as one cycle, of which was repeated for only two cycles (v.s. three cycles for priming). On Day 90, the complete tumour regression (CR) rate was 100%.

Efficacy of Prime-PD1 (nivolumab) blockade-Boost (x3) for 400 mm$^3$ tumour - The curing efficacy was determined in terms of maximum cured tumour volume at 100% CR (complete tumour regression rate) relative to ~50 mm$^3$ at 100% CR of Prime-IgG2a-Boost (Control). Using a single anti-PD1 injection in-between CRYA_1B priming and CRYA_1B boosting, with a total of three boosts for ~400 mm$^3$ tumour, we achieved approximately 8-fold curing efficacy in terms of cured tumour volume (~400 mm$^3$ v.s. ~50 mm$^3$).

*T-cell Prime-PD1 (nivolumab) blockade-Boost (x5) for ~500 mm$^3$ tumour*

The female C57BL/6 mice were subcutaneously inoculated with 1 x 10$^6$ B16F10 cells (poorly immunogenic aggressive melanoma cells )(n = 10). A schematic of the dosing regimen is shown in FIG. 37. The tumours reached approximately 500-515 mm$^3$ in ~15 days (Day 15) to start the experiments. Tumour size was measured in two dimensions using caliper, and the volume was determined in the following formula: V = 0.5 x L x W$^2$, where L and W are the long and short diameters of the tumour respectively. For FLT3L treatment to expand dendritic cells, the FLT3L was then intravenously injected into mice at 4 $\mu$g (560 $\mu$g/m$^2$) daily for seven consecutive days. For IFNg treatment to reduce IL10 and reprogram M2 tumour-associated macrophage (TAM) into M1 TAM, the mice were intravenously injected with IFNg at 0.3 $\mu$g four hours before the first CRYA_1B treatment on Day 22. For CRYA_1B to prime T cells, the two-day treatment (20mg/kg and 14 mg/kg via intravenous injections) followed by two-day non-treatment as one cycle, was repeated for three cycles. On Day 33, the mice were intravenously injected with murine anti-PD1 at 3 mg/kg. On Day 47 (14 days from anti-PD1 injection), the FLT3L treatment was repeated to expand dendritic cells by intravenous injection into mice at 4 $\mu$g (560 $\mu$g/m$^2$) daily for seven consecutive days. On Day 54, the IFNg treatment was repeated to reduce IL10 and reprogram M2 TAM into M1 TAM by intravenous injection into mice at 0.3 $\mu$g. Four hours later, we started CRYA_1B T-cell's boosting by two-day treatment (20 mg/kg and 14 mg/kg, respectively via intravenous injections) followed by two-day non-treatment as one cycle, of which was repeated for only two cycles (v.s. three cycles for priming). On Day 62, the FLT3L treatment was repeated to expand dendritic cells by intravenous injection into mice at 4 $\mu$g (560 $\mu$g/m$^2$) daily for seven consecutive days. On Day 69, the IFNg treatment was repeated to reduce IL10 and reprogram M2 TAM into M1 TAM by intravenous injection into mice at 0.3 $\mu$g. Four hours later, we started CRYA_1B T-cell's boosting by two-day treatment (20mg/kg and 14 mg/kg via intravenous injections) followed by two-day non-treatment as one cycle, of which was repeated for only two cycles (v.s. three cycles for priming). On Day 77, the FLT3L treatment was repeated to expand dendritic cells by intravenous injection into mice at 4 $\mu$g (560 $\mu$g/m$^2$) daily for seven consecutive days. On Day 84, the IFNg treatment was repeated to reduce IL10 and reprogram M2 TAM into M1 TAM by intravenous injection into mice at 0.3 $\mu$g. Four hours later, we started CRYA_1B T-cell's boosting by two-day treatment (20mg/kg and 14 mg/kg via intravenous injections) followed by two-day non-treatment as one cycle, of which was repeated for only two cycles (v.s. three cycles for priming). On Day 92, the FLT3L treatment was repeated to expand dendritic cells by intravenous injection into mice at 4 $\mu$g (560 $\mu$g/m$^2$) daily for seven consecutive days. On Day 99, the IFNg treatment was repeated to reduce IL10 and reprogram M2 TAM into M1 TAM by intravenous injection into mice at 0.3 $\mu$g. Four hours later, we started CRYA_1B T-cell's boosting by two-day treatment (20 mg/kg and 14 mg/kg, respectively via intravenous injections) followed by two-day non-treatment as one cycle, of which was repeated for only two cycles (v.s. three cycles for priming). On Day 107, the FLT3L treatment was repeated to expand dendritic cells by intravenous injection into mice at 4 $\mu$g (560 $\mu$g/m$^2$) daily for seven consecutive days. On Day 114, the IFNg treatment was repeated to reduce IL10 and reprogram M2 TAM into M1 TAM by intravenous injection into mice at 0.3 $\mu$g. Four hours later, we started CRYA_1B T-cell's boosting by two-day treatment (20 mg/kg and 14 mg/kg, respectively via intravenous injections) followed by two-day non-treatment as one cycle, of which was repeated for only two cycles (v.s. three cycles for priming). On Day 122, the complete tumour regression (CR) rate was 100%.

The curing efficacy of the Prime-PD1 (nivolumab) blockade-Boost (x5) for ~500 mm$^3$ tumour protocol was determined in terms of maximum cured tumour volume at 100% CR (complete tumour regression rate) relative to ~50 mm$^3$ at 100% CR of Prime-IgG2a-Boost (Control). Using a single anti-PD1 injection in-between CRYA_1B priming and CRYA_1B boost protocol with a total of five boosts for ~500 mm$^3$ tumour, an approximately 10-fold curing efficacy in terms of cured tumour volume was achieved (~500 mm$^3$ v.s. ~50 mm$^3$). Of note, the curing efficacy of prime-anti-PD1-boost (x5) was about 12.5-fold relative to the priming-only protocol (~500 mm$^3$ v.s. ~40 mm$^3$)*.

**EXAMPLE 14 - Curing Efficacy Estimation of *T-cell Prime-PD1 (nivolumab) blockade-Boost (x5)* for metastatic large tumours in human**

Using the following assumptions a calculation of the total number of CD44+CD8+ T cells following the Prime-PD1 (nivolumab) blockade-Boost (x5) protocol was performed (FIG. 37). Assumptions: Human total naive CD8+ T cells (4 x $10^{10}$ cells) (Boon et. al., Annu. Rev. Immunol., 175-208, 2006). Human naive CD8+ T-cell average precursor frequency for tumour antigen (5 per million) (Lammermann et. al., Immunological Reviews, 26-43, 2008). Human naive CD8+ T-cell priming expansion (>= 10000-fold) (Haring et. al., Immunity,19-29, 2006). Human memory CD8+ T-cell 1st boosting expansion (40-fold; 5-8 cell divisions) (Williams et. al., Nature,890-893, 2006; Fraser et. al., Immunity, 171-183, 2013) Human memory CD8+ T-cell 2-5th boosting expansion (~3-fold; ~1-2 cell divisions) (Williams et. al., Nature,890-893, 2006; Rai et. al., J. of Immunol., 5652-5659, 2014). Human single CD8+ T cell kills 2-3 targets (Wiedemann et. al., PNAS,10985-10990, 2006; McGavern et. al., Nature Immunol, 918-925, 2002). CD4+ T-cell activation is excluded from curing efficacy estimation for simplicity.

Following a human *T-cell Prime-PD1 (nivolumab) blockade-Boost (x5)* treatment protocol, the sustained antigen-experienced CD44+CD8+ T cells caused by multiple boosting is ~$2.64 \times 10^{10}$, which can putatively kill ~$6.6 \times 10^{10}$ tumour cells, corresponding to approximately 10-cm diameter tumour size. However, following human Prime-Only treatment, the antigen-experienced CD44+CD8+ T cells is about ~$2 \times 10^9$, which can putatively kill ~$5 \times 10^9$ tumour cells, corresponding to approximately 4.3-cm diameter tumour size. Per efficacy of cured tumour volume, the human *T-cell Prime-PD1 (nivolumab) blockade-Boost (x5)* treatment has the 12.58-fold curing efficacy relative to human Prime-Only treatment ($10^3 / 4.3^3 = 12.58$), of which corroborated with murine model's 12.5-fold curing efficacy.

**EXAMPLE 15 - CRYA_1B dose optimization**

**1)** maximum CD8+CD44+ T-cell (antigen-experienced CD8+ T cells)(Baaten et. al., Front. Immunol., 1-12, 2012) percent of total CD3+CD8+ T cells and **2)** minimum CD8+CD44+PD1+TIM3+ T-cell (T-cell anergy or exhaustion)(Chikuma et. al., J. Immunol., 6682-6689, 2009; Martinez et. al., Immunity, 265-278, 2015; Wang et. al., J. Hepatol., 731-741, 2019) percent of total CD8+CD44+ T cells as yardsticks to determine the optimised dose regimen, the percentages of which are relatively species invariant.

An experiment was designed to determine CRYA_1B dose optimization and a schematic of the dosing strategy is shown in FIG. 39. The female C57BL/6 mice were subcutaneously inoculated with 1 x $10^6$ B16F10 cells (poorly immunogenic aggressive melanoma cells) for FLT3L/IFNg/CRYA_1B regimen using different CRYA_1B doses in first;second day of a treatment cycle (Dose$_{d1}$;Dose$_{d2}$): 25mg/kg;16mg/kg (n = 5), 25mg/kg;15mg/kg (n = 5), 22 mg/kg; 18mg/kg (n = 5), 20mg/kg;20mg/kg (n = 5), 20mg/kg;18mg/kg (n = 5), 20mg/kg;16mg/kg (n = 5), 20mg/kg;14mg/kg (n = 5), 20mg/kg;13mg/kg (n = 5), 20mg/kg;12mg/kg (n = 5), 19mg/kg;19mg/kg (n = 5), 18mg/kg;18mg/kg (n = 5) and 18mg/kg;20 mg/kg (n = 5). The tumours reached approximately 80-90 mm$^3$ in six days (Day 6). Tumour size was measured in two dimensions using caliper, and the volume was determined in the following formula: $V = 0.5 \times L \times W^2$, where L and W are the long and short diameters of the tumour respectively. For FLT3L treatment, the FLT3L was then intravenously injected into mice at 4 μg (560 μg/m$^2$) daily for seven consecutive days. For IFNg treatment, the mice were intravenously injected with IFNg at 0.3 μg* four hours before the first CRYA_1B treatment on Day 13. *: Conversion from human fixed 70 μg dose [assuming human 60Kg, 70/60 x 37 (human Km) x 0.007 (mouse body surface area) = 0.3] For CRYA_1B therapy to prime T cells, the two-day treatment (Dose$_{d1}$;Dose$_{d2}$ mg/kg via intravenous injections) followed by two-day non-treatment as one cycle, was repeated for three cycles. On Day 30, the PBMCs in all mice were harvested for CD8+CD44+ (antigen-experienced) T-cell percent assays. Table 20 shows the results of the experiment.

**Table 20. Proportion of T cells following treatment with CRYA_1B dose optimization**

| CRYA_1B doses (first;second day) | CD8+CD44+/CD3+CD8+ Tcells (Percentage) | CD8+CD44+PD1+TIM+/ CD3+CD44+ T cells (Percentage) |
|---|---|---|
| 18mg/kg; 18mg/kg | 26 | 8 |
| 18mg/kg; 20 mg/kg | 28 | |
| 19mg/kg; 19mg/kg | 28 | 8 |
| 20mg/kg; 12mg/kg | 28 | 8 |
| 20mg/kg; 13mg/kg | 30 | 8 |
| 20mg/kg; 14mg/kg | 35 | 5 |
| 20mg/kg; 16mg/kg | 35 | 5 |

(continued)

| CRYA_1B doses (first;second day) | CD8+CD44+/CD3+CD8+ Tcells (Percentage) | CD8+CD44+PD1+TIM+/ CD3+CD44+ T cells (Percentage) |
|---|---|---|
| 20mg/kg; 18mg/kg | 35 | 5 |
| 20mg/kg; 20mg/kg | 35 | 5 |
| 22mg/kg; 18mg/kg | 35 | 6 |
| 25mg/kg; 15mg/kg | 35 | 15 |
| 25mg/kg; 16mg/kg | 35 | 15 |

*CRYA_1B Dose Optimization Calculations*

Per **species-invariant** maximum $CD8^+CD44^+$/minimum $PD1^+TIM3^+$ T-cell percent assays, the rules/maximimums for CRYA_1B doses are the following,

1)

$$Dose_{day1} + Dose_{day2} \leq 40 \text{ mg/kg}$$

2)

$$Dose_{day1} \leq 22 \text{ mg/kg}$$

3)

$$Dose_{day1} \geq Dose_{day2}$$

When $Dose_{day1} + Dose_{day2} > 40$ mg/kg or $Dose_{day1} > 22$ mg/kg, the $CD8^+CD44^+PD1^+TIM3^+$ T-cell percent significantly increases (15% for **25/16** mg/kg or **25**/15 mg/kg v.s. 5% for **22/18** mg/kg), due to T-cell overstimulation, resulting in T-cell exhaustion with upregulated TOX gene leading to high expression of PD1 and TIM3. (Wang et. al., J. Hepatol., 731-741, 2019) When $Dose_{day1} < Dose_{day2}$ (e.g. 18mg/kg;20mg/kg v.s. 20mg/kg;18 mg/kg), the $CD8^+CD44^+$ T-cell percent significantly decreases (28% for 18/20 mg/kg v.s. 35% for 20/18 mg/kg) (Table 20). We obtained a range of optimal doses, such as 22mg/kg;18mg/kg, 20mg/kg;20mg/kg, 20mg/kg;18mg/kg, 20mg/kg;16mg/kg, and 20 mg/kg;14 mg/kg, respectively, all with 35% of $CD8^+CD44^+$ T cells and with only ~5% of $CD8^+CD44^+PD1^+TIM3^+$T cells after priming (Table 20).

Consequently, the optimal doses satisfy the following conditions.

1)

$$\mathbf{Dose_{day1} + Dose_{day2} \leq 40 \text{ mg/kg}}$$

2)

$$\mathbf{20 \text{ mg/kg} \leq Dose_{day1} \leq 22 \text{ mg/kg}}$$

3)

$$\mathbf{14 \text{ mg/kg} \leq Dose_{day2} \leq 20 \text{ mg/kg}}$$

When the $CD8^+CD44^+$ T-cell percent is 35%, we observed very robust therapeutic effects, such percentage of which corroborated with very effective Dryvax (human small pox) vaccine with 10-40% of activated $CD8^+$T cells. (Miller et. al., Immunity, 710-722, 2008) 20 mg/kg and 14 mg/kg, respectively, show effective therapeutic results. A range of new optimised doses were determined thereafter.

A **species-invariant** $CD8^+CD44^+$ T-cell percent dose optimization, from

1)

$$\text{Dose}_{day1} + \text{Dose}_{day2} \leq 40 \text{ mg/kg,}$$

2)

$$20 \text{ mg/kg} \leq \text{Dose}_{day1} \leq 22 \text{ mg/kg,}$$

3)

$$14 \text{ mg/kg} \leq \text{Dose}_{day2} \leq 20 \text{ mg/kg,}$$

A wider range of optimal doses such as 22mg/kg;18mg/kg, 20mg/kg;20mg/kg, 20mg/kg;18mg/kg, 20mg/kg;16mg/kg, 20mg/kg;14mg/kg, respectively, were determined.

**EXAMPLE 16 - Determination of Side Effects to Body Temperature using In Vivo Combination therapy for treatment of B16F10 melanoma using FLT3L, IFNgamma, TNFalpha Receptor and Recombinant Polypeptide CRYA_1B**

Human normal temperature is about 36.5 - 37.5 °C. C57BL/6 mouse normal temperature is about 36.5 - 38 °C. Humans and C57BL/6 mice have similar body temperature kinetics. Both humans and C57BL/6 mice can mount an acute inflammatory response with a robust upregulation of human CRP and murine SAP respectively all induced by IL6, whereas BALB/c mice can respond with only modest upregulation of murine SAP (Mortenson et. al., J. Immunol, 885-889, 1983), the deficiency of which will fail to predict the IL6 cytokine storm frequently observed in human T-cell cancer immunotherapy. CRYA_1B induces robust and acute activation of innate immunity, resulting in long-term adaptive immunity (FIG. 40). We examined the fluctuation of body temperature for CRYA_1B 20mg/kg;20 mg/kg treatment in the priming phase. The purpose is to observe any signs of potential adverse events.

*Body Temperature Time Course for In vivo Combination Treatment with FLT3L, IFNg, and Recombinant Polypeptide CRYA_1B*

The female C57BL/6 mice were subcutaneously inoculated with 1 x 10^6 B16F10 cells (poorly immunogenic aggressive melanoma cells) for FLT3L/IFNg/CRYA_1B regimen using CRYA_1B's 20mg/kg;20 mg/kg (n = 5) in first;second day of a treatment cycle (FIG. 41). The tumours reached approximately 80-90 mm^3 in six days (Day 6). Tumour size was measured in two dimensions using caliper, and the volume was determined in the following formula: V = 0.5 x L x W^2, where L and W are the long and short diameters of the tumour respectively. For FLT3L treatment, the FLT3L was then intravenously injected into mice at 4 $\mu$g (560 $\mu$g/m^2) daily for seven consecutive days. For IFNg treatment, the mice were intravenously injected with IFNg at 0.3 $\mu$g* four hours before the first CRYA_1B treatment on Day 13. To monitor the body temperature, on Day 13 we administered CRYA_1B for two-day treatment (20mg/kg;20mg/kg via intravenous injections). *: Conversion from human fixed 70 $\mu$g dose [assuming human 60Kg, 70/60 x 37 (human Km) x 0.007 (mouse body surface area) = 0.3]. Table 21 shows the results of the body temperatures. Adverse events - moderate (Grade 2) hypothermia without TNFaR adjuvant in combination treatment.

**Table 21. Body Temperature of cancer host after FLT3L, IFN gamma and CRYA_1B Treatment**

| Time point | Body temperature of cancer host after FLT3L, IFNgamma and CRYA_1B treatment |
|---|---|
| 0hr (Day 13; 20mg/kg CRYA_1B injection) | 37 |
| 4hr | 37 |
| 6hr | 37 |
| 8hr | 37 |
| 10hr | 37 |
| 12hr | 36 |
| 16hr | 36 |

(continued)

| Time point | Body temperature of cancer host after FLT3L, IFNgamma and CRYA_1B treatment |
|---|---|
| 20hr | 34 |
| 24hr (Day 14; 20mg/kg CRYA_1B injection) | 34 |
| 24.5hr | 37 |
| 28hr | 37 |
| 30hr | 37 |
| 32hr | 37 |
| 34hr | 37 |
| 36hr | 36 |
| 40hr | 36 |
| 44hr | 34 |
| 48hr | 34 |
| 48.5hr | 37 |
| 52hr | 37 |

*Body Temperature Time Course for In vivo Combination Treatment with FLT3L, IFNg, TNFaR and Recombinant Polypeptide CRYA_1B*

The female C57BL/6 mice were subcutaneously inoculated with 1 x $10^6$ B16F10 cells (poorly immunogenic aggressive melanoma cells) for FLT3L,IFNg,TNFaR and CRYA_1B regimen using CRYA_1B's 20mg/kg;20 mg/kg (n = 5) in first;second day of three-cycle treatment (FIG. 42). The tumours reached approximately 80-90 mm$^3$ in six days (Day 6). Tumour size was measured in two dimensions using caliper, and the volume was determined in the following formula: V = 0.5 x L x W$^2$, where L and W are the long and short diameters of the tumour respectively. For FLT3L treatment, the FLT3L was then intravenously injected into mice at 4 $\mu$g (560 $\mu$g/m$^2$) daily for seven consecutive days. For IFNg treatment, the mice were intravenously injected with IFNg at 0.3 $\mu$g* four hours before a fixed low-dose TNFaR (Enbrel) treatment (subcutaneous injection at 20 $\mu$g**) on Day 13. *: Conversion from human fixed 70 $\mu$g dose [assuming human 60Kg, 70 $\mu$g/60 x 37 (human Km)*** x 0.007 (mouse body surface area)*** = 0.3 $\mu$g] **: Murine 20 $\mu$g is equivalent to human 5 mg [assuming mouse 20g, 20 $\mu$g/0.02 x 3 (mouse Km)*** x 1.62 (human body surface area)*** = ~5 mg] ***: Nair, J. Basic Clin Pharm., 27-31, 201. To monitor the body temperature, on Day 13 we administered CRYA_1B immediately after TNFaR injection for the two-day treatment (20mg/kg;20 mg/kg via intravenous injections) followed by two-day non-treatment as one cycle, of which was repeated for three cycles. Table 22 shows the results of the experiments.

**Table 22. Body Temperature of cancer host after FLT3L, IFNgamma, TNFaR and CRYA_1B Treatment**

| Time point | Body Temperature | Time point | Body Temperature | Time point | Body Temperature |
|---|---|---|---|---|---|
| 0hr (Day 13; 20mg/kg CRYA_1B injection) | 37 | 0hr (Day 17; 20mg/kg CRYA_1B injection) | 37 | 0hr (Day 21; 20mg/kg CRYA_1B injection) | 37 |
| 4hr | 36.5 | 4hr | 37 | 4hr | 38 |
| 6hr | 36.5 | 6hr | 37 | 6hr | 38 |
| 8hr | 36.5 | 8hr | 37 | 8hr | 38 |
| 10hr | 36.5 | 10hr | 37 | 10hr | 38 |
| 12hr | 37.5 | 12hr | 38 | 12hr | 39 |
| 16hr | 37.5 | 16hr | 38 | 16hr | 39 |
| 20hr | 38.5 | 20hr | 39 | 20hr | 40 |

(continued)

| Time point | Body Temperature | Time point | Body Temperature | Time point | Body Temperature |
|---|---|---|---|---|---|
| 24hr (Day 14; 20mg/kg CRYA_1B injection) | 38.5 | 24hr (Day 18; 20mg/kg CRYA_1B injection) | 39 | 24hr (Day 22; 20mg/kg CRYA_1B injection) | 40 |
| 24.5hr | 37 | 24.5hr | 37 | 24.5hr | 37 |
| 28hr | 37.5 | 28hr | 37.5 | 28hr | 38.5 |
| 30hr | 37.5 | 30hr | 37.5 | 30hr | 38.5 |
| 32hr | 37.5 | 32hr | 37.5 | 32hr | 38.5 |
| 34hr | 37.5 | 34hr | 37.5 | 34hr | 38.5 |
| 36hr | 38.5 | 36hr | 38.5 | 36hr | 39.5 |
| 40hr | 38.5 | 40hr | 38.5 | 40hr | 39.5 |
| 44hr | 39.5 | 44hr | 39.5 | 44hr | 40 |
| 48hr | 39.5 | 48hr | 39.5 | 48hr | 40 |
| 48.5hr | 37 | 48.5hr | 37 | 48.5hr | 37 |
| 52hr | 37 | 52hr | 37 | 52hr | 37 |

*Determination of Adverse Events (Hypothermia v.s. Hyperthermia) due to treatment with* CRYA_1B

For patients treated with T-cell immunotherapies, the cytokine release syndrome (CRS)(especially of cytokines IL6, IFNg, & TNFa) is frequently observed (Suntharalingam et. al., N. Engl. J. Med., 1018-1028, 2006; Turtle et.al., J. Clin. Invest., 2123-2138, 2016). Furthermore, the cytokine release syndrome is characterized by hyperthermia ($\geq 38°C$) (from IL6 and/or IFNg)(Davila et.al., Sci. Transl. Med., 1-10, 2014) and/or hypothermia ($\leq 35°C$) (from TNFa)(Brady et. al., Clin. Transl. Immunology, 1-7, 2014; Alegre et. al., Eur. J. Immunol, 707-710, 1990).

As above, body temperature fluctuation of cancer hosts during CRYA_1B administration occurs in the priming phase. Without TFNaR adjuvant (Enbrel), during one-cycle administration of CRYA_1B clinical doses, the cancer hosts will experience the Grade 2 adverse event (AE) (National Cancer Institute: Common Terminology Criteria for Adverse Events (CTCAE) Version 5.0, National institute of Health, 2017), a moderate hypothermia (32 to 35°C) with a homeostasis at 37°C. With TFNaR adjuvant (Enbrel) which absorbs TNFa (a cause of hypothermia), during three-cycle administration of CRYA_1B clinical doses, the cancer hosts will experience the Grade 2 adverse event (National Cancer Institute: Common Terminology Criteria for Adverse Events (CTCAE) Version 5.0, National institute of Health, 2017), a moderate fever (39 to 40°C) with homeostasis at 37°C. This thermoregulation of was not observed without TNFaR treatment (a moderate hypothermia). A systemic natural fever-range hyperthermia (38-41C) boosts immune activation/response for cancer immunotherapy (Evans et.al., Nat. Rev. Immunol., 335-349, 2015; Fisher et.al., J. of Clin. Invest., 3846-3859, 2011). An uncontrolled severe (Grade 4 AE)(National Cancer Institute: Common Terminology Criteria for Adverse Events (CTCAE) Version 5.0, National institute of Health, 2017) hypothermia or hyperthermia for a long duration without the return to 37C will increase morbidity and mortality rate. But, hypothermia is a worse risk factor than hyperthermia for patients (Bota et.al., Intensive Care Med., 811-816, 2004; Laupland et.al., Crit. Care Med., 145-151, 2012). As such, for therapies such as T-cell immunotherapies which result in unavoidable adverse symptom such as fever or cold caused by elevated levels of various cytokines from the treatment, a fever range of hyperthermia (38 to 41°C) is preferred to hypothermia.

Regardless of moderate (Grade 2) adverse events of hypothermia or hyperthermia, in the range of optimal CRYA_1B therapeutic doses (22mg/kg;18mg/kg, 20mg/kg;20mg/kg, 20mg/kg;14mg/kg etc.) no significant changes of behaviors or signs of increased morbidity in cancer hosts were observed, mainly due to the ability of the mice to maintain body temperature's self-limiting return to 37C. However, TNFaR (Enbrel) is included as part of the overall regimen without compromising the cancer curing efficacy, to thermoregulate the temperature of the body and prevent the adverse events such as hypothermia to hyperthermia, thus allowing better management of risk of morbidity (Bota et.al., Intensive Care Med., 811-816, 2004; Laupland et.al., Crit. Care Med., 145-151, 2012) and allowing enhancement of cancer immunotherapy treatment (Evans et.al., Nat. Rev. Immunol., 335-349, 2015; Fisher et.al., J. of Clin. Invest., 3846-3859, 2011).

**EXAMPLE 17 - Combination Therapy - In vivo CRYA_1B dose optimization with TNFaR**

An dose regimen was designed to determine the CRYA_1B dose optimization with combination treatment of TNFaR (FIG. 43). Female C57BL/6 mice were subcutaneously inoculated with 1 x 10$^6$ B16F10 poorly immunogenic aggressive melanoma cells for FLT3L, IFNg, TNFaR, and CRYA_1B regimen using different CRYA_1B doses in first;second day of a treatment cycle (Dose$_{d1}$;Dose$_{d2}$): 25mg/kg;16mg/kg (n = 5), 25 mg/kg ;15 mg/kg (n = 5), 22 mg/kg;18 mg/kg (n = 5), 20 mg/kg;20 mg/kg (n = 5), 20 mg/kg ;18 mg/kg (n = 5), 20 mg/kg ;16 mg/kg (n = 5), 20 mg/kg; 14 mg/kg (n = 5), 20 mg/kg ;13 mg/kg (n = 5), 20 mg/kg ;12 mg/kg (n = 5), 19 mg/kg ;19 mg/kg (n = 5), 18 mg/kg ;18 mg/kg (n = 5) and 18 mg/kg ;20 mg/kg (n = 5). The tumours reached approximately 80-90 mm$^3$ in six days (Day 6). Tumour size was measured in two dimensions using caliper, and the volume was determined in the following formula: $V = 0.5 \times L \times W^2$, where L and W are the long and short diameters of the tumour respectively. For FLT3L treatment, the FLT3L was then intravenously injected into mice at 4 $\mu$g (560 $\mu$g/m$^2$) daily for seven consecutive days. For IFNg treatment, the mice were intravenously injected with IFNg at 0.3 $\mu$g four hours before TNFaR injection (20 $\mu$g, via subcutaneous injection) immediately followed by the first CRYA_1B treatment on Day 13. For CRYA_1B therapy to prime T cells, the two-day treatment (Dose$_{d1}$;Dose$_{d2}$ (mg/kg) via intravenous injections) followed by two-day non-treatment as one cycle, was repeated for three cycles. On Day 30, the PBMCs in all mice were harvested and analyzed to determine the proportion of CD8$^+$CD44$^+$ (antigen-experienced) T-cells in the population.

**Table 23. Proportion of T cells following treatment with CRYA_1B dose optimization**

| CRYA_1B doses (first;second day) | CD8+CD44+/CD3+CD8+ Tcells (Percentage) | CD8+CD44+PD1+TIM+/ CD3+CD44+ T cells (Percentage) |
|---|---|---|
| 18mg/kg; 18mg/kg | 25 | 8 (anergy) |
| 19mg/kg; 19mg/kg | 27 | 8 (anergy) |
| 20mg/kg; 12mg/kg | 28 | 8 (anergy) |
| 20mg/kg; 13mg/kg | 30 | 8 (anergy) |
| 20mg/kg; 14mg/kg | 35 | 5 |
| 20mg/kg; 16mg/kg | 35 | 5 |
| 20mg/kg; 18mg/kg | 35 | 5 |
| 20mg/kg; 20mg/kg | 35 | 5 |
| 22mg/kg; 18mg/kg | 35 | 5 |
| 25mg/kg; 15mg/kg | 35 | 15 (exhaustion) |
| 25mg/kg; 16mg/kg | 35 | 15 (exhaustion) |

Therefore, with additional TNFaR adjuvant, the optimal doses of CRYA_1B satisfy the following conditions.

1)

$$\text{Dose}_{\text{day1}} + \text{Dose}_{\text{day2}} \leq 40 \text{ mg/kg}$$

2)

$$20 \text{ mg/kg} \leq \text{Dose}_{\text{day1}} \leq 22 \text{ mg/kg}$$

3)

$$14 \text{ mg/kg} \leq \text{Dose}_{\text{day2}} \leq 20 \text{ mg/kg}$$

In conclusion, TNFaR adjuvant is used for the thermoregulation and to prevent adverse events such as hypothermia and natural fever-range (38-41C) of hyperthermia. It does not have negative effects on the optimised doses and overall curing efficacy.

Therefore, taking in consideration the dosage optimizations for each component, an in vivo combination immunotherapy protocol for treatment of metastatic large tumours was determined (FIG. 44). FIG. 44 shows the prime-PD-1-(nivolumab) blockade-boost (x5) regimen and the doses and timing of FLT3L, IFN gamma, TNFaR, CRYA_1B and PD-1 (nivolumab) blockade in the regimen.

All of the methods disclosed and claimed herein can be made and executed without undue experimentation in light of the present disclosure. While the compositions and methods of this invention have been described in terms of preferred embodiments, it will be apparent to those of skill in the art that variations may be applied to the methods and in the steps or in the sequence of steps of the method described herein in accordance with the claims

**Claims**

1. A recombinant polypeptide comprising an amino acid sequence having at least 95% sequence identity to the polypeptide of SEQ ID NO: 9, or a nucleic acid encoding the recombinant polypeptide comprising an amino acid sequence having at least 95% sequence identity to the polypeptide of SEQ ID NO: 9, for use in a method of treating cancer, wherein the method comprises administering:

   i) a first composition comprising at least one immune cell growth factor, wherein the at least one immune cell growth factor is FMS-like tyrosine kinase 3 ligand (FLT3L), or granulocyte-macrophage colony stimulating factor (GM-CSF);
   ii) a second composition comprising at least one IL-10 inhibitory agent, wherein the at least one IL-10 inhibitory agent is an interferon gamma (IFNg) or an IFNg mimetic;
   iii) a third composition comprising at least one Tumor Necrosis Factor alpha (TNFa) inhibitory agent; and
   iv) a fourth composition comprising the recombinant polypeptide or the nucleic acid.

2. An immune cell growth factor for use in a method of treating cancer, wherein the method comprises administering:

   i) a first composition comprising the immune cell growth factor, and optionally at least one additional immune cell growth factor, wherein the at least one immune cell growth factor is FMS-like tyrosine kinase 3 ligand (FLT3L), or granulocyte-macrophage colony stimulating factor (GM-CSF);
   ii) a second composition comprising at least one IL-10 inhibitory agent, wherein the at least one IL-10 inhibitory agent is an interferon gamma (IFNg) or an IFNg mimetic;
   iii) a third composition comprising at least one Tumor Necrosis Factor alpha (TNFa) inhibitory agent; and
   iv) a fourth composition comprising a recombinant polypeptide comprising an amino acid sequence having at least 95% sequence identity to the polypeptide of SEQ ID NO: 9, or a nucleic acid encoding the recombinant polypeptide comprising an amino acid sequence having at least 95% sequence identity to the polypeptide of SEQ ID NO: 9.

3. An IL-10 inhibitory agent for use in a method of treating cancer, wherein the method comprises administering:

   i) a first composition comprising at least one immune cell growth factor, wherein the at least one immune cell growth factor is FMS-like tyrosine kinase 3 ligand (FLT3L), or granulocyte-macrophage colony stimulating factor (GM-CSF);
   ii) a second composition comprising the IL-10 inhibitory agent, and optionally at least one additional IL-10 inhibitory agent, wherein the at least one IL-10 inhibitory agent is an interferon gamma (IFNg) or an IFNg mimetic;
   iii) a third composition comprising at least one Tumor Necrosis Factor alpha (TNFa) inhibitory agent; and
   iv) a fourth composition comprising a recombinant polypeptide comprising an amino acid sequence having at least 95% sequence identity to the polypeptide of SEQ ID NO: 9, or a nucleic acid encoding the recombinant polypeptide comprising an amino acid sequence having at least 95% sequence identity to the polypeptide of SEQ ID NO: 9.

4. A Tumor Necrosis Factor alpha (TNFa) inhibitory agent for use in a method of treating cancer, wherein the method comprises administering:

   i) a first composition comprising at least one immune cell growth factor, wherein the at least one immune cell growth factor is FMS-like tyrosine kinase 3 ligand (FLT3L), or granulocyte-macrophage colony stimulating factor (GM-CSF);
   ii) a second composition comprising at least one IL-10 inhibitory agent, wherein the at least one IL-10 inhibitory agent is an interferon gamma (IFNg) or an IFNg mimetic;
   iii) a third composition comprising the TNFa inhibitory agent; and optionally at least one additional TNFa inhibitory agent; and

iv) a fourth composition comprising a recombinant polypeptide comprising an amino acid sequence having at least 95% sequence identity to the polypeptide of SEQ ID NO: 9, or a nucleic acid encoding the recombinant polypeptide comprising an amino acid sequence having at least 95% sequence identity to the polypeptide of SEQ ID NO: 9.

5. The recombinant polypeptide, nucleic acid, immune cell growth factor, IL-10 inhibitory agent, or TNFa inhibitory agent for use according to any one of claims 1 to 4, wherein the method further comprises administering a fifth composition comprising at least one immunotherapy agent, wherein the at least one immunotherapy agent is a checkpoint inhibitor; optionally wherein the checkpoint inhibitor is an anti-PD1 antibody.

6. An immunotherapy agent for use in a method of treating cancer, wherein the method comprises administering:

i) a first composition comprising at least one immune cell growth factor, wherein the at least one immune cell growth factor is FMS-like tyrosine kinase 3 ligand (FLT3L), or granulocyte-macrophage colony stimulating factor (GM-CSF);
ii) a second composition comprising at least one IL-10 inhibitory agent, wherein the at least one IL-10 inhibitory agent is an interferon gamma (IFNg) or an IFNg mimetic;
iii) a third composition comprising at least one Tumor Necrosis Factor alpha (TNFa) inhibitory agent;
iv) a fourth composition comprising a recombinant polypeptide comprising an amino acid sequence having at least 95% sequence identity to the polypeptide of SEQ ID NO: 9, or a nucleic acid encoding the recombinant polypeptide comprising an amino acid sequence having at least 95% sequence identity to the polypeptide of SEQ ID NO: 9; and
v) a fifth composition comprising the immunotherapy agent, wherein the immunotherapy agent is a checkpoint inhibitor; optionally wherein the checkpoint inhibitor is an anti-PD1 antibody.

7. The recombinant polypeptide, nucleic acid, immune cell growth factor, IL-10 inhibitory agent, TNFa inhibitory agent, or immunotherapy agent for use according to any one of claims 1-6, wherein the first composition is administered at least about 24 hours prior to the second composition or the third composition.

8. The recombinant polypeptide, nucleic acid, immune cell growth factor, IL-10 inhibitory agent, TNFa inhibitory agent, or immunotherapy agent for use of any one of claims 1-7, wherein the first composition is administered:

(a) once daily for at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days or at least 23 days; or
(b) once daily for about 7 days to about 12 days; or
(c) once daily for about 7 days.

9. The recombinant polypeptide, nucleic acid, immune cell growth factor, IL-10 inhibitory agent, TNFa inhibitory agent, or immunotherapy agent for use according to any one of claims 1-8, wherein:

(a) the second composition and the third composition are administered concurrently or sequentially; or
(b) the second composition is administered prior to the third composition; or
(c) the second composition is administered after the third composition.

10. The recombinant polypeptide, nucleic acid, immune cell growth factor, IL-10 inhibitory agent, TNFa inhibitory agent, or immunotherapy agent for use according to any one of claims 1-9, wherein the fourth composition is administered as multiple infusions, wherein

i) a second infusion is administered at least 1 day after a first infusion;
ii) a third infusion is administered at least 4 days after the first infusion;
iii) a fourth infusion is administered at least 5 days after the first infusion;
iv) a fifth infusion is administered at least 8 days after the first infusion; and/or
v) a sixth infusion is administered at least 9 days after the first infusion.

11. The recombinant polypeptide, nucleic acid, immune cell growth factor, IL-10 inhibitory agent, TNFa inhibitory agent, or immunotherapy agent for use according to claim 10, wherein the third composition and the first infusion of the fourth composition are administered concurrently or sequentially.

12. The recombinant polypeptide, nucleic acid, immune cell growth factor, IL-10 inhibitory agent, TNFa inhibitory agent, or immunotherapy agent for use according to claim 10 or claim 11, wherein the first infusion of the fourth composition is administered:

   (a) at least 1 hour, at least 2 hours, at least 3 hours, at least 4 hours, at least 5 hours, at least 6 hours, at least 7 hours, at least 8 hours, at least 9 hours, at least 10 hours, at least 11 hours, at least 12 hours, at least 13 hours, at least 14 hours, at least 15 hours, at least 16 hours, at least 17 hours, at least 18 hours, at least 19 hours, at least 20 hours, at least 21 hours, at least 22 hours, at least 23 hours, at least 24 hours, at least 25 hours, at least 26 hours, at least 27 hours or at least 28 hours after the administration of the second composition; or
   (b) at about 3 hours or about 4 hours after the administration of the second composition.

13. The recombinant polypeptide, nucleic acid, immune cell growth factor, IL-10 inhibitory agent, TNFa inhibitory agent, or immunotherapy agent for use according to any one of claims 1 to 12, further comprising administering a therapeutic cycle comprising:

   i) a sixth composition comprising at least one immune cell growth factor, wherein the at least one immune cell growth factor is FMS-like tyrosine kinase 3 ligand (FLT3L), or granulocyte-macrophage colony stimulating factor (GM-CSF);
   ii) a seventh composition comprising at least one IL-10 inhibitory agent in the subject, wherein the at least one IL-10 inhibitory agent is an interferon gamma (IFNg) or an IFNg mimetic;
   iii) an eighth composition comprising at least one TNFa inhibitory agent; and
   iv) a ninth composition comprising a recombinant polypeptide comprising an amino acid sequence having at least 95% sequence identity to the polypeptide of SEQ ID NO: 9, or a nucleic acid encoding the recombinant polypeptide comprising an amino acid sequence having at least 95% sequence identity to the polypeptide of SEQ ID NO: 9.

14. The recombinant polypeptide, nucleic acid, immune cell growth factor, IL-10 inhibitory agent, TNFa inhibitory agent, or immunotherapy agent for use according to claim 13, wherein the therapeutic cycle is administered:

   (a) at least 1 time, at least 2 times, at least 3 times, at least 4 times or at least 5 times; optionally wherein the therapeutic cycle is administered no more than five times; and/or
   (b) at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days or at least 14 days after the sixth infusion of the fourth composition;

   optionally wherein the first therapeutic cycle is administered at about 1 day after the sixth infusion of the fourth composition.

15. The recombinant polypeptide, nucleic acid, immune cell growth factor, IL-10 inhibitory agent, TNFa inhibitory agent, or immunotherapy agent for use according to any one of claims 13 to 14, wherein the sixth composition is administered:

   (a) at least about 24 hours prior to the seventh composition or the eighth composition; and/or
   (b) once daily for at least 5, at least 6, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days or at least 23 days; or once daily for a total of about 5 days to about 14 days or about 7 days to about 12 days.

16. The recombinant polypeptide, nucleic acid, immune cell growth factor, IL-10 inhibitory agent, TNFa inhibitory agent, or immunotherapy agent for use according to any one of claims 13 to 15, wherein:

   (a) the seventh composition and the eighth composition are administered concurrently or sequentially; or
   (b) the seventh composition is administered prior to the eighth composition; or
   (c) the seventh composition is administered after the eighth composition.

17. The recombinant polypeptide, nucleic acid, immune cell growth factor, IL-10 inhibitory agent, TNFa inhibitory agent, or immunotherapy agent for use according to any one of claims 13 to 16, wherein the ninth composition is administered as multiple infusions, wherein

> i) a second infusion is administered at least 1 day after a first infusion;
> ii) a third infusion is administered at least 4 days after the first infusion; and
> iii) a fourth infusion is administered at least 5 days after the first infusion.

18. The recombinant polypeptide, nucleic acid, immune cell growth factor, IL-10 inhibitory agent, TNFa inhibitory agent, or immunotherapy agent for use according to claim 17, wherein the eighth composition and the first infusion of the ninth composition are administered concurrently.

19. The recombinant polypeptide, nucleic acid, immune cell growth factor, IL-10 inhibitory agent, TNFa inhibitory agent, or immunotherapy agent for use according to claim 17 or claim 18, wherein the first infusion of the ninth composition is administered:

> (a) at least 1 hour, at least 2 hours, at least 3 hours, at least 4 hours, at least 5 hours, at least 6 hours, at least 7 hours, at least 8 hours, at least 9 hours, at least 10 hours, at least 11 hours, at least 12 hours, at least 13 hours, at least 14 hours, at least 15 hours, at least 16 hours, at least 17 hours, at least 18 hours, at least 19 hours, at least 20 hours, at least 21 hours, at least 22 hours, at least 23 hours, at least 24 hours, at least 25 hours, at least 26 hours, at least 27 hours or at least 28 hours after the administration of the seventh composition; or
> (b) at about 3 hours or about 4 hours after the administration of the seventh composition; or
> (c) at about 4 hours after the administration of the seventh composition.

20. The recombinant polypeptide, nucleic acid, immune cell growth factor, IL-10 inhibitory agent, TNFa inhibitory agent, or immunotherapy agent for use according to any one of claims 5 to 19, wherein the fifth composition is administered as multiple infusions, and wherein the fifth composition is administered at least one time, at least 2 times, at least 3 times, at least 4 times, at least 5 times, at least 6 times, at least 7 times, at least 8 times, at least 9 times, at least 10 times, at least 11 times, at least 12 times, at least 13 times or at least 14 times.

21. The recombinant polypeptide, nucleic acid, immune cell growth factor, IL-10 inhibitory agent, TNFa inhibitory agent, or immunotherapy agent for use according to any one of claims 5 to 20, wherein the first infusion of the fifth composition is administered after the sixth infusion of the fourth composition and prior to the first infusion of the ninth composition.

22. The recombinant polypeptide, nucleic acid, immune cell growth factor, IL-10 inhibitory agent, TNFa inhibitory agent, or immunotherapy agent for use according to any one of claims 5 to 21, wherein the fifth composition is administered:

> (a) at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days after the first infusion of the fourth composition; or
> (b) at about 10 days to about 14 days after the first infusion of the fourth composition; or
> (c) at about 12 days after the first infusion of the fourth composition.

23. The recombinant polypeptide, nucleic acid, immune cell growth factor, IL-10 inhibitory agent, TNFa inhibitory agent, or immunotherapy agent for use according to any one of claims 1 to 22, wherein:

> (a) the first composition, the second composition, the third composition, the fourth composition, the fifth composition, the sixth composition, the seventh composition, the eighth composition or the ninth composition are administered intravenously or subcutaneously; and/or
> (b) the first composition, the second composition, the fourth composition, the fifth composition, the sixth composition, the seventh composition and the ninth composition are administered intravenously; and/or
> (c) the third composition and the eighth composition are administered subcutaneously.

24. The recombinant polypeptide, nucleic acid, immune cell growth factor, IL-10 inhibitory agent, TNFa inhibitory agent, or immunotherapy agent for use according to any one of claims 1 to 23, wherein the at least one immune cell growth factor is FLT3L; optionally wherein the FLT3L is in an amount of:

> (a) about 1 $\mu$g/kg, about 2 $\mu$g/kg, about 3 $\mu$g/kg, about 4 $\mu$g/kg, about 5 $\mu$g/kg, about 6 $\mu$g/kg, about 7 $\mu$g/kg, about 8 $\mu$g/kg, about 9 $\mu$g/kg, 10 $\mu$g/kg, about 11 $\mu$g/kg, about 12 $\mu$g/kg, about 13 $\mu$g/kg, about 14 $\mu$g/kg, about 15

μg/kg, about 16 μg/kg, about 17 μg/kg, about 18 μg/kg, about 19 μg/kg, or about 20 μg/kg; or
(b) about 4 μg/kg to about 13 μg/kg; or
(c) about 7 μg/kg.

25. The recombinant polypeptide, nucleic acid, immune cell growth factor, IL-10 inhibitory agent, TNFa inhibitory agent, or immunotherapy agent for use according to any one of claims 1 to 24, wherein
the at least one IL-10 inhibitory agent is IFNg, optionally wherein the IFNg is administered at a dose of about 10 μg, about 15 μg, about 20 μg, about 25μg, about 30 μg, about 35 μg, about 40 μg, about 45μg, about 50 μg, about 55 μg, about 60 μg, about 65μg, about 70 μg, about 75μg, about 80 μg, about 85 μg, about 90 μg, about 95μg or about 100 μg; or about 50 μg to about 100 μg; or about 70 μg.

26. The recombinant polypeptide, nucleic acid, immune cell growth factor, IL-10 inhibitory agent, TNFa inhibitory agent, or immunotherapy agent for use according to any one of claims 1 to 25, wherein the TNFa inhibitor agent is a TNFa receptor (TNFaR), infliximab, adalimumab, certolizumab pegol, golimumab, or etanercept; optionally wherein the TNFaR is administered:

(a) at a dose of about 1 mg, about 2 mg, about 2 mg, about 3 mg, about 4 mg, about 5 mg, about 6 mg, about 7 mg, about 8 mg, about 9 mg or about 10 mg; or
(b) at a dose of about 4 mg to about 6 mg; or
(c) at a dose of about 5 mg.

27. The recombinant polypeptide, nucleic acid, immune cell growth factor, IL-10 inhibitory agent, TNFa inhibitory agent, or immunotherapy agent for use according to any one of claims 1 to 26, wherein:

(a) the first infusion, the second infusion, the third infusion, the fourth infusion, the fifth infusion and/or the sixth infusion of the fourth composition, and the first infusion, the second infusion, the third infusion and/or the fourth infusion of the ninth composition comprises about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg about 25 mg/kg, about 26 mg/kg, about 27 mg/kg, about 28 mg/kg, about 29 mg/kg, about 30 mg/kg, about 31 mg/kg, about 32 mg/kg, about 33 mg/kg, about 34 mg/kg or about 35 mg/kg of the recombinant polypeptide comprising an amino acid sequence having at least 95% sequence identity to the polypeptide of SEQ ID NO: 9; or
(b) the first infusion, the second infusion, the third infusion, the fourth infusion, the fifth infusion and the sixth infusion of the fourth composition, and the first infusion, the second infusion, the third infusion and the fourth infusion of the ninth composition comprises about 10 mg/kg to about 30 mg/kg of the recombinant polypeptide comprising an amino acid sequence having at least 95% sequence identity to the polypeptide of SEQ ID NO: 9; or
(c) the first infusion, the second infusion, the third infusion, the fourth infusion, the fifth infusion and the sixth infusion of the fourth composition, and the first infusion, the second infusion, the third infusion and the fourth infusion of the ninth composition comprises about 20 mg/kg of the recombinant polypeptide comprising an amino acid sequence having at least 95% sequence identity to the polypeptide of SEQ ID NO: 9.

28. The recombinant polypeptide, nucleic acid, immune cell growth factor, IL-10 inhibitory agent, TNFa inhibitory agent, or immunotherapy agent for use according to any one of claims 5 to 27, wherein the checkpoint inhibitor is an anti-PD1 antibody; optionally wherein the anti-PD1 antibody is nivolumab, pembrolizumab or cemiplimab.

29. The recombinant polypeptide, nucleic acid, immune cell growth factor, IL-10 inhibitory agent, TNFa inhibitory agent, or immunotherapy agent for use according to claim 28, wherein the anti-PD1 antibody is administered at a dose of:

(a) about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 2 mg/kg, about 3 mg/kg, about 4 mg/kg, about 5mg/kg, about 6mg/kg, about 7 mg/kg, about 8 mg/kg, about 9 mg/kg or about 10 mg/kg; or
(b) about 0.1 mg/kg to about 7 mg/kg; or
(c) about 3mg/kg.

30. The recombinant polypeptide, nucleic acid, immune cell growth factor, IL-10 inhibitory agent, TNFa inhibitory agent, or immunotherapy agent for use according any one of claims 1 to 29, wherein the cancer:

(a) is a solid tumor cancer; optionally wherein the solid tumor has a diameter of at least about 0.2 cm, at least about 0.5 cm, at least about 1 cm, at least about 2 cm, at least about 3 cm, at least about 4 cm, at least about 5 cm, at least about 6 cm, at least about 7 cm, at least about 8 cm, at least about 9 cm or at least about 10 cm; and/or

(b) is a recurrence of an earlier presentation of a cancer or is a metastasis of an earlier presentation of cancer.

## Patentansprüche

1. Rekombinantes Polypeptid, umfassend eine Aminosäuresequenz mit mindestens 95 % Sequenzidentität mit dem Polypeptid von SEQ ID NO: 9, oder eine Nukleinsäure, die das rekombinante Polypeptid kodiert, umfassend eine Aminosäuresequenz mit mindestens 95 % Sequenzidentität mit dem Polypeptid von SEQ ID NO: 9, zur Verwendung in einem Verfahren zur Behandlung von Krebs, das Verfahren umfassend Verabreichen von:

   i) einer ersten Zusammensetzung, umfassend mindestens einen Immunzellwachstumsfaktor, wobei der mindestens eine Immunzellwachstumsfaktor FMS-ähnlicher Tyrosinkinase-3-Ligand (FMS-like tyrosine kinase 3 ligand - FLT3L) oder Granulozyten-Makrophagen-Kolonie-stimulierender Faktor (granulocytemacrophage colony stimulating factor - GM-CSF) ist;
   ii) einer zweiten Zusammensetzung, umfassend mindestens einen IL-10-Hemmer, wobei der mindestens eine IL-10-Hemmer ein Interferon-Gamma (IFNg) oder ein IFNg-Mimetikum ist;
   iii) einer dritten Zusammensetzung, umfassend mindestens einen Tumornekrosefaktor-alpha-(TNFa-)Hemmer; und
   iv) einer vierten Zusammensetzung, umfassend das rekombinante Polypeptid oder die Nukleinsäure.

2. Immunzellwachstumsfaktor zur Verwendung in einem Verfahren zur Behandlung von Krebs, das Verfahren umfassend Verabreichen von:

   i) einer ersten Zusammensetzung, umfassend den Immunzellwachstumsfaktor und gegebenenfalls mindestens einen weiteren Immunzellwachstumsfaktor, wobei der mindestens eine Immunzellwachstumsfaktor FMS-ähnlicher Tyrosinkinase-3-Ligand (FLT3L) oder Granulozyten-Makrophagen-Kolonie-stimulierender Faktor (GM-CSF) ist;
   ii) einer zweiten Zusammensetzung, umfassend mindestens einen IL-10-Hemmer, wobei der mindestens eine IL-10-Hemmer ein Interferon-Gamma (IFNg) oder ein IFNg-Mimetikum ist;
   iii) einer dritten Zusammensetzung, umfassend mindestens einen Tumornekrosefaktor-alpha-(TNFa-)Hemmer; und
   iv) einer vierten Zusammensetzung, umfassend ein rekombinantes Polypeptid, umfassend eine Aminosäuresequenz mit mindestens 95 % Sequenzidentität mit dem Polypeptid von SEQ ID NO: 9, oder eine Nukleinsäure, die das rekombinante Polypeptid kodiert, umfassend eine Aminosäuresequenz mit mindestens 95 % Sequenzidentität mit dem Polypeptid von SEQ ID NO: 9.

3. IL-10-Hemmer zur Verwendung in einem Verfahren zur Behandlung von Krebs, das Verfahren umfassend Verabreichen von:

   i) einer ersten Zusammensetzung, umfassend mindestens einen Immunzellwachstumsfaktor, wobei der mindestens eine Immunzellwachstumsfaktor FMS-ähnlicher Tyrosinkinase-3-Ligand (FLT3L) oder Granulozyten-Makrophagen-Kolonie-stimulierender Faktor (GM-CSF) ist;
   ii) einer zweiten Zusammensetzung, umfassend den IL-10-Hemmer und gegebenenfalls mindestens einen weiteren IL-10-Hemmer, wobei der mindestens eine IL-10-Hemmer ein Interferon-Gamma (IFNg) oder ein IFNg-Mimetikum ist;
   iii) einer dritten Zusammensetzung, umfassend mindestens einen Tumornekrosefaktor-alpha-(TNFa-)Hemmer; und
   iv) einer vierten Zusammensetzung, umfassend ein rekombinantes Polypeptid, umfassend eine Aminosäuresequenz mit mindestens 95 % Sequenzidentität mit dem Polypeptid von SEQ ID NO: 9, oder eine Nukleinsäure, die das rekombinante Polypeptid kodiert, umfassend eine Aminosäuresequenz mit mindestens 95 % Sequenzidentität mit dem Polypeptid von SEQ ID NO: 9.

4. Tumornekrosefaktor-alpha-(TNFa-)Hemmer zur Verwendung in einem Verfahren zur Behandlung von Krebs, das Verfahren umfassend Verabreichen von:

i) einer ersten Zusammensetzung, umfassend mindestens einen Immunzellwachstumsfaktor, wobei der mindestens eine Immunzellwachstumsfaktor FMS-ähnlicher Tyrosinkinase-3-Ligand (FLT3L) oder Granulozyten-Makrophagen-Kolonie-stimulierender Faktor (GM-CSF) ist;

ii) einer zweiten Zusammensetzung, umfassend mindestens einen IL-10-Hemmer, wobei der mindestens eine IL-10-Hemmer ein Interferon-Gamma (IFNg) oder ein IFNg-Mimetikum ist;

iii) einer dritten Zusammensetzung, umfassend den TNFa-Hemmer; und gegebenenfalls mindestens einen weiteren TNFa-Hemmer; und

iv) einer vierten Zusammensetzung, umfassend ein rekombinantes Polypeptid, umfassend eine Aminosäuresequenz mit mindestens 95 % Sequenzidentität mit dem Polypeptid von SEQ ID NO: 9, oder eine Nukleinsäure, die das rekombinante Polypeptid kodiert, umfassend eine Aminosäuresequenz mit mindestens 95 % Sequenzidentität mit dem Polypeptid von SEQ ID NO: 9.

5. Rekombinantes Polypeptid, Nukleinsäure, Immunzellwachstumsfaktor, IL-10-Hemmer oder TNFa-Hemmer zur Verwendung nach einem der Ansprüche 1 bis 4, das Verfahren ferner umfassend Verabreichen einer fünften Zusammensetzung, umfassend mindestens ein Immuntherapiemittel, wobei das mindestens eine Immuntherapiemittel ein Checkpoint-Inhibitor ist; wobei der Checkpoint-Inhibitor gegebenenfalls ein Anti-PD1-Antikörper ist.

6. Immuntherapiemittel zur Verwendung in einem Verfahren zur Behandlung von Krebs, das Verfahren umfassend Verabreichen von:

i) einer ersten Zusammensetzung, umfassend mindestens einen Immunzellwachstumsfaktor, wobei der mindestens eine Immunzellwachstumsfaktor FMS-ähnlicher Tyrosinkinase-3-Ligand (FLT3L) oder Granulozyten-Makrophagen-Kolonie-stimulierender Faktor (GM-CSF) ist;

ii) einer zweiten Zusammensetzung, umfassend mindestens einen IL-10-Hemmer, wobei der mindestens eine IL-10-Hemmer ein Interferon-Gamma (IFNg) oder ein IFNg-Mimetikum ist;

iii) einer dritten Zusammensetzung, umfassend mindestens einen Tumornekrosefaktor-alpha-(TNFa-)Hemmer;

iv) einer vierten Zusammensetzung, umfassend ein rekombinantes Polypeptid, umfassend eine Aminosäuresequenz mit mindestens 95 % Sequenzidentität mit dem Polypeptid von SEQ ID NO: 9, oder eine Nukleinsäure, die das rekombinante Polypeptid kodiert, umfassend eine Aminosäuresequenz mit mindestens 95 % Sequenzidentität mit dem Polypeptid von SEQ ID NO: 9; und

v) einer fünften Zusammensetzung, umfassend das Immuntherapiemittel, wobei das Immuntherapiemittel ein Checkpoint-Inhibitor ist; wobei der Checkpoint-Inhibitor gegebenenfalls ein Anti-PD1-Antikörper ist.

7. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 1-6, wobei die erste Zusammensetzung mindestens etwa 24 Stunden vor der zweiten Zusammensetzung oder der dritten Zusammensetzung verabreicht wird.

8. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 1-7, wobei die erste Zusammensetzung wie folgt verabreicht wird:

(a) einmal täglich während mindestens 5 Tagen, mindestens 6 Tagen, mindestens 7 Tagen, mindestens 8 Tagen, mindestens 9 Tagen, mindestens 10 Tagen, mindestens 11 Tagen, mindestens 12 Tagen, mindestens 13 Tagen, mindestens 14 Tagen, mindestens 15 Tagen, mindestens 16 Tagen, mindestens 17 Tagen, mindestens 18 Tagen, mindestens 19 Tagen, mindestens 20 Tagen, mindestens 21 Tagen, mindestens 22 Tagen oder mindestens 23 Tagen; oder

(b) einmal täglich für etwa 7 Tage bis etwa 12 Tage; oder

(c) einmal täglich für etwa 7 Tage.

9. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 1-8, wobei:

(a) die zweite Zusammensetzung und die dritte Zusammensetzung gleichzeitig oder nacheinander verabreicht werden; oder

(b) die zweite Zusammensetzung vor der dritten Zusammensetzung verabreicht wird; oder

(c) die zweite Zusammensetzung nach der dritten Zusammensetzung verabreicht wird.

10. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 1-9, wobei die vierte Zusammensetzung als Mehrfachinfusionen verabreicht wird, wobei

i) eine zweite Infusion mindestens 1 Tag nach einer ersten Infusion verabreicht wird;
ii) eine dritte Infusion mindestens 4 Tage nach der ersten Infusion verabreicht wird;
iii) eine vierte Infusion mindestens 5 Tage nach der ersten Infusion verabreicht wird;
iv) eine fünfte Infusion mindestens 8 Tage nach der ersten Infusion verabreicht wird; und/oder
v) eine sechste Infusion mindestens 9 Tage nach der ersten Infusion verabreicht wird.

11. Rekombinantes Polypeptid, Nukleinsäure, Immunzellwachstumsfaktor, IL-10-Hemmer, TNFa-Hemmer oder Immuntherapiemittel zur Verwendung nach Anspruch 10, wobei die dritte Zusammensetzung und die erste Infusion der vierten Zusammensetzung gleichzeitig oder nacheinander verabreicht werden.

12. Rekombinantes Polypeptid, Nukleinsäure, Immunzellwachstumsfaktor, IL-10-Hemmer, TNFa-Hemmer oder Immuntherapiemittel zur Verwendung nach Anspruch 10 oder Anspruch 11, wobei die erste Infusion der vierten Zusammensetzung wie folgt verabreicht wird:

(a) mindestens 1 Stunde, mindestens 2 Stunden, mindestens 3 Stunden, mindestens 4 Stunden, mindestens 5 Stunden, mindestens 6 Stunden, mindestens 7 Stunden, mindestens 8 Stunden, mindestens 9 Stunden, mindestens 10 Stunden, mindestens 11 Stunden, mindestens 12 Stunden, mindestens 13 Stunden, mindestens 14 Stunden, mindestens 15 Stunden, mindestens 16 Stunden, mindestens 17 Stunden, mindestens 18 Stunden, mindestens 19 Stunden, mindestens 20 Stunden, mindestens 21 Stunden, mindestens 22 Stunden, mindestens 23 Stunden, mindestens 24 Stunden, mindestens 25 Stunden, mindestens 26 Stunden, mindestens 27 Stunden oder mindestens 28 Stunden nach der Verabreichung der zweiten Zusammensetzung; oder
(b) etwa 3 Stunden oder etwa 4 Stunden nach der Verabreichung der zweiten Zusammensetzung.

13. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 1 bis 12, ferner umfassend Verabreichen eines therapeutischen Zyklus, umfassend:

i) eine sechste Zusammensetzung, umfassend mindestens einen Immunzellwachstumsfaktor, wobei der mindestens eine Immunzellwachstumsfaktor FMS-ähnlicher Tyrosinkinase-3-Ligand (FLT3L) oder Granulozyten-Makrophagen-Kolonie-stimulierender Faktor (GM-CSF) ist;
ii) eine siebente Zusammensetzung, umfassend mindestens einen IL-10-Hemmer in dem Subjekt, wobei der mindestens eine IL-10-Hemmer ein Interferon-Gamma (IFNg) oder ein IFNg-Mimetikum ist;
iii) eine achte Zusammensetzung, umfassend mindestens einen TNFa-Hemmer; und
iv) eine neunte Zusammensetzung, umfassend ein rekombinantes Polypeptid, umfassend eine Aminosäuresequenz mit mindestens 95 % Sequenzidentität mit dem Polypeptid von SEQ ID NO: 9, oder eine Nukleinsäure, die das rekombinante Polypeptid kodiert, umfassend eine Aminosäuresequenz mit mindestens 95 % Sequenzidentität mit dem Polypeptid von SEQ ID NO: 9.

14. Rekombinantes Polypeptid, Nukleinsäure, Immunzellwachstumsfaktor, IL-10-Hemmer, TNFa-Hemmer oder Immuntherapiemittel zur Verwendung nach Anspruch 13, wobei der therapeutische Zyklus wie folgt verabreicht wird:

(a) mindestens 1 Mal, mindestens 2 Mal, mindestens 3 Mal, mindestens 4 Mal oder mindestens 5 Mal; wobei der therapeutische Zyklus gegebenenfalls nicht mehr als fünf Mal verabreicht wird; und/oder
(b) mindestens 1 Tag, mindestens 2 Tage, mindestens 3 Tage, mindestens 4 Tage, mindestens 5 Tage, mindestens 6 Tage, mindestens 7 Tage, mindestens 8 Tage, mindestens 9 Tage, mindestens 10 Tage, mindestens 11 Tage, mindestens 12 Tage, mindestens 13 Tage oder mindestens 14 Tage nach der sechsten Infusion der vierten Zusammensetzung; wobei der erste therapeutische Zyklus gegebenenfalls etwa 1 Tag nach der sechsten Infusion der vierten Zusammensetzung verabreicht wird.

15. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 13 bis 14, wobei die sechste Zusammensetzung wie folgt verabreicht wird:

(a) mindestens etwa 24 Stunden vor der siebten oder achten Zusammensetzung; und/oder
(b) einmal täglich für mindestens 5, mindestens 6, mindestens 7 Tage, mindestens 8 Tage, mindestens 9 Tage, mindestens 10 Tage, mindestens 11 Tage, mindestens 12 Tage, mindestens 13 Tage, mindestens 14 Tage, mindestens 15 Tage, mindestens 16 Tage, mindestens 17 Tage, mindestens 18 Tage, mindestens 19 Tage, mindestens 20 Tage, mindestens 21 Tage, mindestens 22 Tage oder mindestens 23 Tage; oder einmal täglich für insgesamt etwa 5 Tage bis etwa 14 Tage oder etwa 7 Tage bis etwa 12 Tage.

16. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 13 bis 15, wobei:

   (a) die siebente Zusammensetzung und die achte Zusammensetzung gleichzeitig oder nacheinander verabreicht werden; oder
   (b) die siebente Zusammensetzung vor der achten Zusammensetzung verabreicht wird; oder
   (c) die siebente Zusammensetzung nach der achten Zusammensetzung verabreicht wird.

17. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 13 bis 16, wobei die neunte Zusammensetzung als Mehrfachinfusionen verabreicht wird, wobei

   i) eine zweite Infusion mindestens 1 Tag nach einer ersten Infusion verabreicht wird;
   ii) eine dritte Infusion mindestens 4 Tage nach der ersten Infusion verabreicht wird; und
   iii) eine vierte Infusion mindestens 5 Tage nach der ersten Infusion verabreicht wird.

18. Rekombinantes Polypeptid, Nukleinsäure, Immunzellwachstumsfaktor, IL-10-Hemmer, TNFa-Hemmer oder Immuntherapiemittel zur Verwendung nach Anspruch 17, wobei die achte Zusammensetzung und die erste Infusion der neunten Zusammensetzung gleichzeitig verabreicht werden.

19. Rekombinantes Polypeptid, Nukleinsäure, Immunzellwachstumsfaktor, IL-10-Hemmer, TNFa-Hemmer oder Immuntherapiemittel zur Verwendung nach Anspruch 17 oder Anspruch 18, wobei die erste Infusion der neunten Zusammensetzung wie folgt verabreicht wird:

   (a) mindestens 1 Stunde, mindestens 2 Stunden, mindestens 3 Stunden, mindestens 4 Stunden, mindestens 5 Stunden, mindestens 6 Stunden, mindestens 7 Stunden, mindestens 8 Stunden, mindestens 9 Stunden, mindestens 10 Stunden, mindestens 11 Stunden, mindestens 12 Stunden, mindestens 13 Stunden, mindestens 14 Stunden, mindestens 15 Stunden, mindestens 16 Stunden, mindestens 17 Stunden, mindestens 18 Stunden, mindestens 19 Stunden, mindestens 20 Stunden, mindestens 21 Stunden, mindestens 22 Stunden, mindestens 23 Stunden, mindestens 24 Stunden, mindestens 25 Stunden, mindestens 26 Stunden, mindestens 27 Stunden oder mindestens 28 Stunden nach der Verabreichung der siebenten Zusammensetzung; oder
   (b) etwa 3 Stunden oder etwa 4 Stunden nach der Verabreichung der siebenten Zusammensetzung; oder
   (c) etwa 4 Stunden nach der Verabreichung der siebenten Zusammensetzung.

20. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 5 bis 19, wobei die fünfte Zusammensetzung als Mehrfachinfusion verabreicht wird und wobei die fünfte Zusammensetzung mindestens einmal, mindestens 2 Mal, mindestens 3 Mal, mindestens 4 Mal, mindestens 5 Mal, mindestens 6 Mal, mindestens 7 Mal, mindestens 8 Mal, mindestens 9 Mal, mindestens 10 Mal, mindestens 11 Mal, mindestens 12 Mal, mindestens 13 Mal oder mindestens 14 Mal verabreicht wird.

21. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 5 bis 20, wobei die erste Infusion der fünften Zusammensetzung nach der sechsten Infusion der vierten Zusammensetzung und vor der ersten Infusion der neunten Zusammensetzung verabreicht wird.

22. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 5 bis 21, wobei die fünfte Zusammensetzung wie folgt verabreicht wird:

   (a) mindestens 1 Tag, mindestens 2 Tage, mindestens 3 Tage, mindestens 4 Tage, mindestens 5 Tage, mindestens 6 Tage, mindestens 7 Tage, mindestens 8 Tage, mindestens 9 Tage, mindestens 10 Tage, mindestens 11 Tage, mindestens 12 Tage, mindestens 13 Tage, mindestens 14 Tage, mindestens 15 Tage nach der ersten Infusion der vierten Zusammensetzung; oder

(b) etwa 10 Tage bis etwa 14 Tage nach der ersten Infusion der vierten Zusammensetzung; oder

(c) etwa 12 Tage nach der ersten Infusion der vierten Zusammensetzung.

23. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 1 bis 22, wobei:

(a) die erste Zusammensetzung, die zweite Zusammensetzung, die dritte Zusammensetzung, die vierte Zusammensetzung, die fünfte Zusammensetzung, die sechste Zusammensetzung, die siebte Zusammensetzung, die achte Zusammensetzung oder die neunte Zusammensetzung intravenös oder subkutan verabreicht werden; und/oder

(b) die erste Zusammensetzung, die zweite Zusammensetzung, die vierte Zusammensetzung, die fünfte Zusammensetzung, die sechste Zusammensetzung, die siebte Zusammensetzung und die neunte Zusammensetzung intravenös verabreicht werden; und/oder

(c) die dritte Zusammensetzung und die achte Zusammensetzung subkutan verabreicht werden.

24. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 1 bis 23, wobei der mindestens eine Immunzellwachstumsfaktor FLT3L ist; wobei das FLT3L gegebenenfalls in einer Menge wie folgt vorliegt:

(a) etwa 1 $\mu$g/kg, etwa 2 $\mu$g/kg, etwa 3 $\mu$g/kg, etwa 4 $\mu$g/kg, etwa 5 $\mu$g/kg, etwa 6 $\mu$g/kg, etwa 7 $\mu$g/kg, etwa 8 $\mu$g/kg, etwa 9 $\mu$g/kg, 10 $\mu$g/kg, etwa 11 $\mu$g/kg, etwa 12 $\mu$g/kg, etwa 13 $\mu$g/kg, etwa 14 $\mu$g/kg, etwa 15 $\mu$g/kg, etwa 16 $\mu$g/kg, etwa 17 $\mu$g/kg, etwa 18 $\mu$g/kg, etwa 19 $\mu$g/kg, oder etwa 20 $\mu$g/kg; oder

(b) etwa 4 $\mu$g/kg bis etwa 13 $\mu$g/kg; oder

(c) etwa 7 $\mu$g/kg.

25. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 1 bis 24, wobei der mindestens eine IL-10-Hemmer IFNg ist, wobei das IFNg gegebenenfalls in einer Dosis von etwa 10 $\mu$g, etwa 15 $\mu$g, etwa 20 $\mu$g, etwa 25 $\mu$g, etwa 30 $\mu$g, etwa 35 $\mu$g verabreicht wird, etwa 40 $\mu$g, etwa 45 $\mu$g, etwa 50 $\mu$g, etwa 55 $\mu$g, etwa 60 $\mu$g, etwa 65 $\mu$g, etwa 70 $\mu$g, etwa 75 $\mu$g, etwa 80 $\mu$g, etwa 85 $\mu$g, etwa 90 $\mu$g, etwa 95 $\mu$g oder etwa 100 $\mu$g; oder etwa 50 $\mu$g bis etwa 100 $\mu$g; oder etwa 70 $\mu$g.

26. Rekombinantes Polypeptid, die Nukleinsäure, der Immunzellwachstumsfaktor, IL-10-Hemmer, TNFa-Hemmer oder Immuntherapiemittel zur Verwendung nach einem der Ansprüche 1 bis 25, wobei der TNFa-Hemmer ein TNFa-Rezeptor (TNFaR), Infliximab, Adalimumab, Certolizumab Pegol, Golimumab oder Etanercept ist; wobei der TNFaR gegebenenfalls wie folgt verabreicht wird:

(a) in einer Dosis von etwa 1 mg, etwa 2 mg, etwa 2 mg, etwa 3 mg, etwa 4 mg, etwa 5 mg, etwa 6 mg, etwa 7 mg, etwa 8 mg, etwa 9 mg oder etwa 10 mg; oder

(b) in einer Dosis von etwa 4 mg bis etwa 6 mg; oder

(c) in einer Dosis von etwa 5 mg.

27. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 1 bis 26, wobei:

(a) die erste Infusion, die zweite Infusion, die dritte Infusion, die vierte Infusion, die fünfte Infusion und/oder die sechste Infusion der vierten Zusammensetzung und die erste Infusion, die zweite Infusion, die dritte Infusion und/oder die vierte Infusion der neunten Zusammensetzung etwa 5 mg/kg, etwa 6 mg/kg, etwa 7 mg/kg, etwa 8 mg/kg, etwa 9 mg/kg, etwa 10 mg/kg, etwa 11 mg/kg, etwa 12 mg/kg, etwa 13 mg/kg, 14 mg/kg, etwa 15 mg/kg, etwa 16 mg/kg, etwa 17 mg/kg, etwa 18 mg/kg, etwa 19 mg/kg, etwa 20 mg/kg, etwa 21 mg/kg, etwa 22 mg/kg, etwa 23 mg/kg, etwa 24 mg/kg, etwa 25 mg/kg, etwa 26 mg/kg, etwa 27 mg/kg, etwa 28 mg/kg, etwa 29 mg/kg, etwa 30 mg/kg, etwa 31 mg/kg, etwa 32 mg/kg, etwa 33 mg/kg, etwa 34 mg/kg oder etwa 35 mg/kg des rekombinanten Polypeptids umfasst, das eine Aminosäuresequenz mit mindestens 95 % Sequenzidentität mit dem Polypeptid von SEQ ID NO: 9 umfasst; oder

(b) die erste Infusion, die zweite Infusion, die dritte Infusion, die vierte Infusion, die fünfte Infusion und die sechste Infusion der vierten Zusammensetzung und die erste Infusion, die zweite Infusion, die dritte Infusion und die vierte Infusion der neunten Zusammensetzung etwa 10 mg/kg bis etwa 30 mg/kg des rekombinanten Polypeptids umfasst, das eine Aminosäuresequenz mit mindestens 95 % Sequenzidentität mit dem Polypeptid von SEQ ID NO: 9 umfasst; oder

(c) die erste Infusion, die zweite Infusion, die dritte Infusion, die vierte Infusion, die fünfte Infusion und die sechste Infusion der vierten Zusammensetzung und die erste Infusion, die zweite Infusion, die dritte Infusion und die vierte Infusion der neunten Zusammensetzung etwa 20 mg/kg des rekombinanten Polypeptids umfasst, das eine Aminosäuresequenz mit mindestens 95 % Sequenzidentität mit dem Polypeptid von SEQ ID NO: 9 umfasst.

28. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 5 bis 27, wobei der Checkpoint-Inhibitor ein Anti-PD1-Antikörper ist; wobei der Anti-PD1-Antikörper gegebenenfalls Nivolumab, Pembrolizumab oder Cemiplimab ist.

29. Rekombinantes Polypeptid, Nukleinsäure, Immunzellwachstumsfaktor, IL-10-Hemmer, TNFa-Hemmer oder Immuntherapiemittel zur Verwendung nach Anspruch 28, wobei der Anti-PD1-Antikörper in einer Dosis wie folgt verabreicht wird:

(a) etwa 0,1 mg/kg, etwa 0,2 mg/kg, etwa 0,3 mg/kg, etwa 0,4 mg/kg, etwa 0,5 mg/kg, etwa 0,6 mg/kg, etwa 0,7 mg/kg, etwa 0,8 mg/kg, etwa 0,9 mg/kg, etwa 1 mg/kg, etwa 2 mg/kg, etwa 3 mg/kg, etwa 4 mg/kg, etwa 5 mg/kg, etwa 6 mg/kg, etwa 7 mg/kg, etwa 8 mg/kg, etwa 9 mg/kg oder etwa 10 mg/kg; oder
(b) etwa 0,1 mg/kg bis etwa 7 mg/kg; oder
(c) etwa 3 mg/kg.

30. Rekombinantes Polypeptid, Nukleinsäure, Immunzellwachstumsfaktor, IL-10-Hemmer, TNFa-Hemmer oder Immuntherapiemittel zur Verwendung nach einem der Ansprüche 1 bis 29, wobei der Krebs:

(a) ein solider Tumorkrebs ist; wobei der solide Tumor gegebenenfalls einen Durchmesser von mindestens etwa 0,2 cm, mindestens etwa 0,5 cm, mindestens etwa 1 cm, mindestens etwa 2 cm, mindestens etwa 3 cm, mindestens etwa 4 cm, mindestens etwa 5 cm, mindestens etwa 6 cm, mindestens etwa 7 cm, mindestens etwa 8 cm, mindestens etwa 9 cm oder mindestens etwa 10 cm aufweist; und/oder
(b) ein Wiederauftreten einer früheren Krebserkrankung oder eine Metastase einer früheren Krebserkrankung ist.

**Revendications**

1. Polypeptide recombinant comprenant une séquence d'acides aminés ayant au moins 95 % d'identité de séquence avec le polypeptide de SEQ ID NO: 9, ou un acide nucléique codant pour le polypeptide recombinant comprenant une séquence d'acides aminés ayant au moins 95 % d'identité de séquence avec le polypeptide de SEQ ID NO: 9, pour utilisation dans un procédé de traitement du cancer, dans lequel le procédé comprend l'administration :

i) d'une première composition comprenant au moins un facteur de croissance des cellules immunitaires, dans lequel l'au moins un facteur de croissance des cellules immunitaires est le ligand de la tyrosine kinase 3 de type FMS (FLT3L) ou le facteur de stimulation des colonies de granulocytes et de macrophages (GM-CSF) ;
ii) d'une deuxième composition comprenant au moins un agent inhibiteur de l'IL-10, dans lequel l'au moins un agent inhibiteur de l'IL-10 est un interféron gamma (IFNg) ou un mimétique de l'IFNg ;
iii) d'une troisième composition comprenant au moins un agent inhibiteur du facteur de nécrose tumorale alpha (TNFa) ; et
iv) d'une quatrième composition comprenant le polypeptide recombinant ou l'acide nucléique.

2. Facteur de croissance des cellules immunitaires pour utilisation dans un procédé de traitement du cancer, dans lequel le procédé comprend l'administration :

i) d'une première composition comprenant le facteur de croissance des cellules immunitaires, et éventuellement au moins un facteur de croissance des cellules immunitaires supplémentaire, dans lequel l'au moins un facteur de croissance des cellules immunitaires est le ligand de la tyrosine kinase 3 de type FMS (FLT3L) ou le facteur de stimulation des colonies de granulocytes et de macrophages (GM-CSF) ;
ii) d'une deuxième composition comprenant au moins un agent inhibiteur de l'IL-10, dans lequel l'au moins un agent inhibiteur de l'IL-10 est un interféron gamma (IFNg) ou un mimétique de l'IFNg ;
iii) d'une troisième composition comprenant au moins un agent inhibiteur du facteur de nécrose tumorale alpha (TNFa) ; et

iv) d'une quatrième composition comprenant un polypeptide recombinant comprenant une séquence d'acides aminés ayant au moins 95 % d'identité de séquence avec le polypeptide de SEQ ID NO: 9, ou un acide nucléique codant pour le polypeptide recombinant comprenant une séquence d'acides aminés ayant au moins 95 % d'identité de séquence avec le polypeptide de SEQ ID NO: 9.

3. Agent inhibiteur de l'IL-10 pour utilisation dans un procédé de traitement du cancer, dans lequel le procédé comprend l'administration :

i) d'une première composition comprenant au moins un facteur de croissance des cellules immunitaires, dans lequel l'au moins un facteur de croissance des cellules immunitaires est le ligand de la tyrosine kinase 3 de type FMS (FLT3L) ou le facteur de stimulation des colonies de granulocytes et de macrophages (GM-CSF) ;

ii) d'une deuxième composition comprenant l'agent inhibiteur de l'IL-10, et éventuellement au moins un agent inhibiteur de l'IL-10 supplémentaire, dans lequel l'au moins un agent inhibiteur de l'IL-10 est un interféron gamma (IFNg) ou un mimétique de l'IFNg ;

iii) d'une troisième composition comprenant au moins un agent inhibiteur du facteur de nécrose tumorale alpha (TNFa) ; et

iv) d'une quatrième composition comprenant un polypeptide recombinant comprenant une séquence d'acides aminés ayant au moins 95 % d'identité de séquence avec le polypeptide de SEQ ID NO: 9, ou un acide nucléique codant pour le polypeptide recombinant comprenant une séquence d'acides aminés ayant au moins 95 % d'identité de séquence avec le polypeptide de SEQ ID NO: 9.

4. Agent inhibiteur du facteur de nécrose tumorale alpha (TNFa) pour utilisation dans un procédé de traitement du cancer, dans lequel le procédé comprend l'administration :

i) d'une première composition comprenant au moins un facteur de croissance des cellules immunitaires, dans lequel l'au moins un facteur de croissance des cellules immunitaires est le ligand de la tyrosine kinase 3 de type FMS (FLT3L) ou le facteur de stimulation des colonies de granulocytes et de macrophages (GM-CSF) ;

ii) d'une deuxième composition comprenant au moins un agent inhibiteur de l'IL-10, dans lequel l'au moins un agent inhibiteur de l'IL-10 est un interféron gamma (IFNg) ou un mimétique de l'IFNg ;

iii) d'une troisième composition comprenant l'agent inhibiteur du TNFa ; et éventuellement au moins un agent inhibiteur du TNFa supplémentaire ; et

iv) d'une quatrième composition comprenant un polypeptide recombinant comprenant une séquence d'acides aminés ayant au moins 95 % d'identité de séquence avec le polypeptide de SEQ ID NO: 9, ou un acide nucléique codant pour le polypeptide recombinant comprenant une séquence d'acides aminés ayant au moins 95 % d'identité de séquence avec le polypeptide de SEQ ID NO: 9.

5. Polypeptide recombinant, acide nucléique, facteur de croissance des cellules immunitaires, agent inhibiteur de l'IL-10 ou agent inhibiteur du TNFa pour utilisation selon l'une quelconque des revendications 1 à 4, dans lequel le procédé comprend également l'administration d'une cinquième composition comprenant au moins un agent d'immunothérapie, dans lequel l'au moins un agent d'immunothérapie est un inhibiteur de point de contrôle ; éventuellement dans lequel l'inhibiteur de point de contrôle est un anticorps anti-PD1.

6. Agent d'immunothérapie pour utilisation dans un procédé de traitement du cancer, dans lequel le procédé comprend l'administration :

i) d'une première composition comprenant au moins un facteur de croissance des cellules immunitaires, dans lequel l'au moins un facteur de croissance des cellules immunitaires est le ligand de la tyrosine kinase 3 de type FMS (FLT3L) ou le facteur de stimulation des colonies de granulocytes et de macrophages (GM-CSF) ;

ii) d'une deuxième composition comprenant au moins un agent inhibiteur de l'IL-10, dans lequel l'au moins un agent inhibiteur de l'IL-10 est un interféron gamma (IFNg) ou un mimétique de l'IFNg ;

iii) d'une troisième composition comprenant au moins un agent inhibiteur du facteur de nécrose tumorale alpha (TNFa) ;

iv) d'une quatrième composition comprenant un polypeptide recombinant comprenant une séquence d'acides aminés ayant au moins 95 % d'identité de séquence avec le polypeptide de SEQ ID NO: 9, ou un acide nucléique codant pour le polypeptide recombinant comprenant une séquence d'acides aminés ayant au moins 95 % d'identité de séquence avec le polypeptide de SEQ ID NO: 9 ; et

v) d'une cinquième composition comprenant l'agent d'immunothérapie, dans lequel l'agent d'immunothérapie est un inhibiteur de point de contrôle ; éventuellement dans lequel l'inhibiteur de point de contrôle est un anticorps anti-PD1.

7. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 1 à 6, dans lequel la première composition est administrée au moins environ 24 heures avant la deuxième composition ou la troisième composition.

8. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 1 à 7, dans lequel la première composition est administrée :

(a) une fois par jour pendant au moins 5 jours, au moins 6 jours, au moins 7 jours, au moins 8 jours, au moins 9 jours, au moins 10 jours, au moins 11 jours, au moins 12 jours, au moins 13 jours, au moins 14 jours, au moins 15 jours, au moins 16 jours, au moins 17 jours, au moins 18 jours, au moins 19 jours, au moins 20 jours, au moins 21 jours, au moins 22 jours ou au moins 23 jours ; ou
(b) une fois par jour pendant environ 7 jours à environ 12 jours ; ou
(c) une fois par jour pendant environ 7 jours.

9. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 1 à 8, dans lequel :

(a) la deuxième composition et la troisième composition sont administrées simultanément ou séquentiellement ; ou
(b) la deuxième composition est administrée avant la troisième composition ; ou
(c) la deuxième composition est administrée après la troisième composition.

10. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 1 à 9, dans lequel la quatrième composition est administrée sous forme de multiples perfusions, dans lequel

i) une deuxième perfusion est administrée au moins 1 jour après une première perfusion ;
ii) une troisième perfusion est administrée au moins 4 jours après la première perfusion ;
iii) une quatrième perfusion est administrée au moins 5 jours après la première perfusion ;
iv) une cinquième perfusion est administrée au moins 8 jours après la première perfusion ; et/ou
v) une sixième perfusion est administrée au moins 9 jours après la première perfusion.

11. Polypeptide recombinant, acide nucléique, facteur de croissance des cellules immunitaires, agent inhibiteur de l'IL-10, agent inhibiteur du TNFa ou agent d'immunothérapie pour utilisation selon la revendication 10, dans lequel la troisième composition et la première perfusion de la quatrième composition sont administrées simultanément ou séquentiellement.

12. Polypeptide recombinant, acide nucléique, facteur de croissance des cellules immunitaires, agent inhibiteur de l'IL-10, agent inhibiteur du TNFa ou agent d'immunothérapie pour utilisation selon la revendication 10 ou la revendication 11, dans lequel la première perfusion de la quatrième composition est administrée :

(a) au moins 1 heure, au moins 2 heures, au moins 3 heures, au moins 4 heures, au moins 5 heures, au moins 6 heures, au moins 7 heures, au moins 8 heures, au moins 9 heures, au moins 10 heures, au moins 11 heures, au moins 12 heures, au moins 13 heures, au moins 14 heures, au moins 15 heures, au moins 16 heures, au moins 17 heures, au moins 18 heures, au moins 19 heures, au moins 20 heures, au moins 21 heures, au moins 22 heures, au moins 23 heures, au moins 24 heures, au moins 25 heures, au moins 26 heures, au moins 27 heures ou au moins 28 heures après l'administration de la deuxième composition ; ou
(b) environ 3 heures ou environ 4 heures après l'administration de la deuxième composition.

13. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 1 à 12, comprenant également l'administration d'un cycle thérapeutique comprenant :

i) une sixième composition comprenant au moins un facteur de croissance des cellules immunitaires, dans lequel l'au moins un facteur de croissance des cellules immunitaires est le ligand de la tyrosine kinase 3 de type FMS (FLT3L) ou le facteur de stimulation des colonies de granulocytes et de macrophages (GM-CSF) ;

ii) une septième composition comprenant au moins un agent inhibiteur de l'IL-10 chez le sujet, dans lequel l'au moins un agent inhibiteur de l'IL-10 est un interféron gamma (IFNg) ou un mimétique de l'IFNg ;

iii) une huitième composition comprenant au moins un agent inhibiteur du TNFa ; et

iv) une neuvième composition comprenant un polypeptide recombinant comprenant une séquence d'acides aminés ayant au moins 95 % d'identité de séquence avec le polypeptide de SEQ ID NO: 9, ou un acide nucléique codant pour le polypeptide recombinant comprenant une séquence d'acides aminés ayant au moins 95 % d'identité de séquence avec le polypeptide de SEQ ID NO: 9.

14. Polypeptide recombinant, acide nucléique, facteur de croissance des cellules immunitaires, agent inhibiteur de l'IL-10, agent inhibiteur du TNFa ou agent d'immunothérapie pour utilisation selon la revendication 13, dans lequel le cycle thérapeutique est administré :

(a) au moins 1 fois, au moins 2 fois, au moins 3 fois, au moins 4 fois ou au moins 5 fois ; éventuellement dans lequel le cycle thérapeutique est administré au plus cinq fois ; et/ou

(b) au moins 1 jour, au moins 2 jours, au moins 3 jours, au moins 4 jours, au moins 5 jours, au moins 6 jours, au moins 7 jours, au moins 8 jours, au moins 9 jours, au moins 10 jours, au moins 11 jours, au moins 12 jours, au moins 13 jours ou au moins 14 jours après la sixième perfusion de la quatrième composition ; éventuellement dans lequel le premier cycle thérapeutique est administré environ 1 jour après la sixième perfusion de la quatrième composition.

15. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 13 et 14, dans lequel la sixième composition est administrée :

(a) au moins environ 24 heures avant la septième composition ou la huitième composition ; et/ou

(b) une fois par jour pendant au moins 5, au moins 6, au moins 7 jours, au moins 8 jours, au moins 9 jours, au moins 10 jours, au moins 11 jours, au moins 12 jours, au moins 13 jours, au moins 14 jours, au moins 15 jours, au moins 16 jours, au moins 17 jours, au moins 18 jours, au moins 19 jours, au moins 20 jours, au moins 21 jours, au moins 22 jours ou au moins 23 jours ; ou une fois par jour pendant un total d'environ 5 jours à environ 14 jours ou d'environ 7 jours à environ 12 jours.

16. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 13 à 15, dans lequel :

(a) la septième composition et la huitième composition sont administrées simultanément ou séquentiellement ; ou

(b) la septième composition est administrée avant la huitième composition ; ou

(c) la septième composition est administrée après la huitième composition.

17. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 13 à 16, dans lequel la neuvième composition est administrée sous forme de multiples perfusions, dans lequel

i) une deuxième perfusion est administrée au moins 1 jour après une première perfusion ;

ii) une troisième perfusion est administrée au moins 4 jours après la première perfusion ; et

iii) une quatrième perfusion est administrée au moins 5 jours après la première perfusion.

18. Polypeptide recombinant, acide nucléique, facteur de croissance des cellules immunitaires, agent inhibiteur de l'IL-10, agent inhibiteur du TNFa ou agent d'immunothérapie pour utilisation selon la revendication 17, dans lequel la huitième composition et la première perfusion de la neuvième composition sont administrées simultanément.

19. Polypeptide recombinant, acide nucléique, facteur de croissance des cellules immunitaires, agent inhibiteur de l'IL-10, agent inhibiteur du TNFa ou agent d'immunothérapie pour utilisation selon la revendication 17 ou la revendication 18, dans lequel la première perfusion de la neuvième composition est administrée :

(a) au moins 1 heure, au moins 2 heures, au moins 3 heures, au moins 4 heures, au moins 5 heures, au moins 6 heures, au moins 7 heures, au moins 8 heures, au moins 9 heures, au moins 10 heures, au moins 11 heures, au moins 12 heures, au moins 13 heures, au moins 14 heures, au moins 15 heures, au moins 16 heures, au moins 17 heures, au moins 18 heures, au moins 19 heures, au moins 20 heures, au moins 21 heures, au moins 22 heures, au moins 23 heures, au moins 24 heures, au moins 25 heures, au moins 26 heures, au moins 27 heures ou au moins 28 heures après l'administration de la septième composition ; ou
(b) environ 3 heures ou environ 4 heures après l'administration de la septième composition ; ou
(c) environ 4 heures après l'administration de la septième composition.

20. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 5 à 19, dans lequel la cinquième composition est administrée sous forme de multiples perfusions, et dans lequel la cinquième composition est administrée au moins une fois, au moins 2 fois, au moins 3 fois, au moins 4 fois, au moins 5 fois, au moins 6 fois, au moins 7 fois, au moins 8 fois, au moins 9 fois, au moins 10 fois, au moins 11 fois, au moins 12 fois, au moins 13 fois ou au moins 14 fois.

21. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 5 à 20, dans lequel la première perfusion de la cinquième composition est administrée après la sixième perfusion de la quatrième composition et avant la première perfusion de la neuvième composition.

22. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 5 à 21, dans lequel la cinquième composition est administrée :

(a) au moins 1 jour, au moins 2 jours, au moins 3 jours, au moins 4 jours, au moins 5 jours, au moins 6 jours, au moins 7 jours, au moins 8 jours, au moins 9 jours, au moins 10 jours, au moins 11 jours, au moins 12 jours, au moins 13 jours, au moins 14 jours, au moins 15 jours après la première perfusion de la quatrième composition ; ou
(b) environ 10 jours à environ 14 jours après la première perfusion de la quatrième composition ; ou
(c) environ 12 jours après la première perfusion de la quatrième composition.

23. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 1 à 22, dans lequel :

(a) la première composition, la deuxième composition, la troisième composition, la quatrième composition, la cinquième composition, la sixième composition, la septième composition, la huitième composition ou la neuvième composition sont administrées par voie intraveineuse ou sous-cutanée ; et/ou
(b) la première composition, la deuxième composition, la quatrième composition, la cinquième composition, la sixième composition, la septième composition et la neuvième composition sont administrées par voie intraveineuse ; et/ou
(c) la troisième composition et la huitième composition sont administrées par voie sous-cutanée.

24. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 1 à 23, dans lequel l'au moins un facteur de croissance des cellules immunitaires est le FLT3L ; éventuellement dans lequel le FLT3L est présent en une quantité :

(a) d'environ 1 $\mu$g/kg, d'environ 2 $\mu$g/kg, d'environ 3 $\mu$g/kg, d'environ 4 $\mu$g/kg, d'environ 5 $\mu$g/kg, d'environ 6 $\mu$g/kg, d'environ 7 $\mu$g/kg, d'environ 8 $\mu$g/kg, d'environ 9 $\mu$g/kg, 10 $\mu$g/kg, d'environ 11 $\mu$g/kg, d'environ 12 $\mu$g/kg, d'environ 13 $\mu$g/kg, d'environ 14 $\mu$g/kg, d'environ 15 $\mu$g/kg, d'environ 16 $\mu$g/kg, d'environ 17 $\mu$g/kg, d'environ 18 $\mu$g/kg, d'environ 19 $\mu$g/kg, ou d'environ 20 $\mu$g/kg ; ou
(b) d'environ 4 $\mu$g/kg à environ 13 $\mu$g/kg ; ou
(c) d'environ 7 $\mu$g/kg.

25. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 1 à 24, dans lequel

l'au moins un agent inhibiteur de l'IL-10 est l'IFNg, éventuellement dans lequel l'IFNg est administré à une dose d'environ 10 μg, d'environ 15 μg, d'environ 20 μg, d'environ 25 μg, d'environ 30 μg, d'environ 35 μg, d'environ 40 μg, d'environ 45 μg, d'environ 50 μg, d'environ 55 μg, d'environ 60 μg, d'environ 65 μg, d'environ 70 μg, d'environ 75 μg, d'environ 80 μg, d'environ 85 μg, d'environ 90 μg, d'environ 95 μg ou d'environ 100 μg ; ou d'environ 50 μg à environ 100 μg ; ou d'environ 70 μg.

26. Polypeptide recombinant, acide nucléique, facteur de croissance des cellules immunitaires, agent inhibiteur de l'IL-10, agent inhibiteur du TNFa ou agent d'immunothérapie, pour utilisation selon l'une quelconque des revendications 1 à 25, dans lequel l'agent inhibiteur du TNFa est un récepteur du TNFa (TNFaR), l'infliximab, l'adalimumab, le certolizumab pégol, le golimumab ou l'étanercept ; éventuellement dans lequel le TNFaR est administré :

(a) à une dose d'environ 1 mg, d'environ 2 mg, d'environ 2 mg, d'environ 3 mg, d'environ 4 mg, d'environ 5 mg, d'environ 6 mg, d'environ 7 mg, d'environ 8 mg, d'environ 9 mg ou d'environ 10 mg ; ou
(b) à une dose d'environ 4 mg à environ 6 mg ; ou
(c) à une dose d'environ 5 mg.

27. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 1 à 26, dans lequel :

(a) la première perfusion, la deuxième perfusion, la troisième perfusion, la quatrième perfusion, la cinquième perfusion et/ou la sixième perfusion de la quatrième composition, et la première perfusion, la deuxième perfusion, la troisième perfusion et/ou la quatrième perfusion de la neuvième composition comprennent environ 5 mg/kg, environ 6 mg/kg, environ 7 mg/kg, environ 8 mg/kg, environ 9 mg/kg, environ 10 mg/kg, environ 11 mg/kg, environ 12 mg/kg, environ 13 mg/kg, 14 mg/kg, environ 15 mg/kg, environ 16 mg/kg, environ 17 mg/kg, environ 18 mg/kg, environ 19 mg/kg, environ 20 mg/kg, environ 21 mg/kg, environ 22 mg/kg, environ 23 mg/kg, environ 24 mg/kg, environ 25 mg/kg, environ 26 mg/kg, environ 27 mg/kg, environ 28 mg/kg, environ 29 mg/kg, environ 30 mg/kg, environ 31 mg/kg, environ 32 mg/kg, environ 33 mg/kg, environ 34 mg/kg ou environ 35 mg/kg du polypeptide recombinant comprenant une séquence d'acides aminés ayant au moins 95 % d'identité de séquence avec le polypeptide de SEQ ID NO: 9 ; ou
(b) la première perfusion, la deuxième perfusion, la troisième perfusion, la quatrième perfusion, la cinquième perfusion et la sixième perfusion de la quatrième composition, et la première perfusion, la deuxième perfusion, la troisième perfusion et la quatrième perfusion de la neuvième composition comprennent environ 10 mg/kg à environ 30 mg/kg du polypeptide recombinant comprenant une séquence d'acides aminés ayant au moins 95 % d'identité de séquence avec le polypeptide de SEQ ID NO: 9 ; ou
(c) la première perfusion, la deuxième perfusion, la troisième perfusion, la quatrième perfusion, la cinquième perfusion et la sixième perfusion de la quatrième composition, et la première perfusion, la deuxième perfusion, la troisième perfusion et la quatrième perfusion de la neuvième composition comprennent environ 20 mg/kg du polypeptide recombinant comprenant une séquence d'acides aminés ayant au moins 95 % d'identité de séquence avec le polypeptide de SEQ ID NO : 9.

28. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 5 à 27, dans lequel l'inhibiteur de point de contrôle est un anticorps anti-PD1 ; éventuellement dans lequel l'anticorps anti-PD1 est le nivolumab, le pembrolizumab ou le cemiplimab.

29. Polypeptide recombinant, acide nucléique, facteur de croissance des cellules immunitaires, agent inhibiteur de l'IL-10, agent inhibiteur du TNFa ou agent d'immunothérapie pour utilisation selon la revendication 28, dans lequel l'anticorps anti-PD1 est administré à une dose :

(a) d'environ 0,1 mg/kg, d'environ 0,2 mg/kg, d'environ 0,3 mg/kg, d'environ 0,4 mg/kg, d'environ 0,5 mg/kg, d'environ 0,6 mg/kg, d'environ 0,7 mg/kg, d'environ 0,8 mg/kg, d'environ 0,9 mg/kg, d'environ 1 mg/kg, d'environ 2 mg/kg, d'environ 3 mg/kg, d'environ 4 mg/kg, d'environ 5 mg/kg, d'environ 6 mg/kg, d'environ 7 mg/kg, d'environ 8 mg/kg, d'environ 9 mg/kg ou d'environ 10 mg/kg ; ou
(b) d'environ 0,1 mg/kg à environ 7 mg/kg ; ou
(c) d'environ 3 mg/kg.

30. Polypeptide recombinant, acide nucléique, facteur de croissance des cellules immunitaires, agent inhibiteur de l'IL-10, agent inhibiteur du TNFa ou agent d'immunothérapie pour utilisation selon l'une quelconque des revendications 1 à 29, dans lequel le cancer :

(a) est un cancer à tumeur solide ; éventuellement dans lequel la tumeur solide a un diamètre d'au moins environ 0,2 cm, d'au moins environ 0,5 cm, d'au moins environ 1 cm, d'au moins environ 2 cm, d'au moins environ 3 cm, d'au moins environ 4 cm, d'au moins environ 5 cm, d'au moins environ 6 cm, d'au moins environ 7 cm, d'au moins environ 8 cm, d'au moins environ 9 cm ou d'au moins environ 10 cm ; et/ou
(b) est une récidive d'une présentation antérieure d'un cancer ou est une métastase d'une présentation antérieure d'un cancer.

FIG. 1

FIG. 2

Peak List

| m/z | Intensity |
|-----|-----------|
| 20172.286 | 1480 |

## FIG. 3A

CRT-H441

Concentration of CRYA_1B
recombinant polypeptide (µg/ml)

# FIG. 3B

EP 4 168 034 B1

# FIG. 4A

**CRT-H460**

Concentration of CRYA_1B
recombinant polypeptide(μg/ml)

# FIG. 4B

EP 4 168 034 B1

# FIG. 5A

**CRT-HCT15**

Concentration of CRYA_1B
recombinant polypeptide (μg/ml)

FIG. 5B

FIG. 6A

CRT-MCF7

Concentration of CRYA_1B
recombinant polypeptide (µg/ml)

# FIG. 7A

**Hsp70-H441**

Concentration of CRYA_1B
recombinant polypeptide (µg/ml)

FIG. 7B

# FIG. 8A

**Hsp70-H460**

Concentration of CRYA_1B
recombinant polypeptide (μg/ml)

FIG. 8B

EP 4 168 034 B1

# FIG. 9A

Hsp70-HCT15

Concentration of CRYA_1B
recombinant polypeptide (μg/ml)

# FIG. 10A

**Hsp70-MCF7**

Concentration of CRYA_1B
recombinant polypeptide (µg/ml)

EP 4 168 034 B1

## FIG. 10B

# FIG. 11A

**Hsp90-H441**

Concentration of CRYA_1B
recombinant polypeptide (µg/ml)

EP 4 168 034 B1

# FIG. 11B

# FIG. 12A

**Hsp90-H460**

Concentration of CRYA_1B
recombinant polypeptide (µg/ml)

## FIG. 12B

# FIG. 13A

**Hsp90-HCT15**

Concentration of CRYA_1B
recombinant polypeptide (µg/ml)

## FIG. 13B

## FIG. 14A

**Hsp90-MCF7**

Concentration of CRYA_1B
recombinant polypeptide (μg/ml)

FIG. 14B

EP 4 168 034 B1

# FIG. 15A

**Caspase 3/7-H441**

FIG. 15B

# FIG. 16A

Caspase 3/7-H460

Concentration of CRYA_1B
recombinant polypeptide(μg/ml)

FIG. 16B

# FIG. 17A

Caspase 3/7-HCT15

Concentration of CRYA_1B
recombinant polypeptide (µg/ml)

FIG. 17B

FIG. 18A

Caspase 3/7-MCF7

Concentration of CRYA_1B
recombinant polypeptide(μg/ml)

# FIG. 18B

# FIG. 19

# FIG. 20

# FIG. 21

⊡ IL-10

# FIG. 22

Timeline (days) — (weeks)

CRYA_1B recombinant polypeptide + Adjuvant

0 1 2 3 4 5 6 7 8 9 10 11 12 13 14 +26w

MC38/E0771/B16F10 tumours (1x10^6 Inoculation)

C57BL/6

Control

CR Mice

Control

Treatments

Rechallenge

CRYA_1B recombinant polypeptide (IP/IV)

Adjuvant: IFNg (IP/IV), if IL-10/IgG > Normal

MC38/E0771/B16F10 Rechallenge after CR (2x10^5@contralateral flank)[1]

CR: Complete Response

# FIG. 23A

# FIG. 23B

# FIG. 23C

# FIG. 23D

FIG. 24A

FIG. 24B

# FIG. 25

## Viability of PBMC

% cell Viability vs Concentration of CRYA_1B recombinant polypeptide (µg/ml)

Control: 98.06
100: 97.46
200: 96.98
400: 96.48
800: 94.13

FIG. 26

CRT: Calreticulin
DC: Dendritic cell
HSP70/90: Heat-shock protein 70/90
IL12R: Receptor for IL12
IL15R: Receptor for IL15
IFNAR: Receptor for IFNa/IFNb
M1 TAM: M1-like tumour-associated macrophage
M2 TAM: M2-like tumour-associated macrophage
MDSC: Myeloid-derived suppressor cell
NK: Natural killer cell
Treg: Regulatory T cell
XCR1: Receptor for XCL1

EP 4 168 034 B1

FIG. 27

FIG. 28

EP 4 168 034 B1

EP 4 168 034 B1

# FIG. 29

FIG. 30

Kaplan-Meier Survival by group

Legend:
- Control
- FLT3L; IFNG; CRYA_1B
- FLT3L; CRYA_1B
- IFNG; CRYA_1B
- CRYA_1B

Fraction Surviving

Time

(Days after 1st treatment)

# FIG. 31

FLT3L/IFNg/TNFaR/CRYA_1B/@PD1x1

# FIG. 32

FLT3L/IFNg/TNFaR/CRYA_1B/@PD1 (x1-7)

EP 4 168 034 B1

# FIG. 33

## T-cell Prime-IgG2a-Boost (x1) (Control)

Prime-IgG2a-Boost (x1)

FIG. 34

# T-cell Prime-PD1 Blockade-Boost (x1) for 300 mm³ tumour

Prime-anti-PD1-Boost (x1)

FIG. 35

**T-cell Prime-PD1 Blockade-Boost (x1) for 400 mm³ tumour**

Prime-anti-PD1-Boost (x1)

# FIG. 36

## T-cell Prime-PD1 Blockade-Boost (x3) for 400 mm³ tumour

Prime-anti-PD1-Boost (x3)

FIG. 37
T-cell Prime-PD1 Blockade-Boost (x5) for ~500 mm³ tumour

FIG. 38

FLT3L/IFNg/CRYA_1B
**Priming**

**2x10⁵**   ⟶   **2x10⁹**

Naïve CD44 T          *10000-fold*          (CD44⁺ T)
Ag-specific
precursors
(4x10¹⁶ x 5/10⁶ = 2x10⁵)

anti-PD1 x1

⟶   **6.6x10⁻**

33% Tcm          (CD44⁺ Tcm)
33% Tem
33% Teff
in 14 days

FLT3L/IFNg/CRYA_1B
**1º Boosting**

⟶   **>2.64x10¹⁰**

*40-fold*          (CD44⁺ T)

(>: Plus old primed
Tem plus new CD44⁺
T from new naïve T
cells priming)

T-cell
Contraction

⟶

*1/3 alive*

FLT3L/IFNg/CRYA_1B
**2º Boosting**

**>8.8x10⁹**   ⟶   **>2.64x10¹⁰**

*3-fold*

T-cell
Contraction

⟶   **>8.8x10⁹**

*1/3 alive*

FLT3L/IFNg/CRYA_1B
**3º Boosting**

⟶   **>2.64x10¹⁰**

*3-fold*

T-cell
Contraction

⟶

*1/3 alive*

FLT3L/IFNg/CRYA_1B
**4º Boosting**

**>8.8x10⁹**   ⟶   **>2.64x10¹⁰**

*3-fold*

T-cell
Contraction

⟶   **>8.8x10⁹**

*1/3 alive*

FLT3L/IFNg/CRYA_1B
**5º Boosting**

⟶   **>2.64x10¹⁰**

*3-fold*

# FIG. 39

FLT3L/IFNg/CRYA_1B (Prime)

# FIG. 40

T-20DC Induces 'Acute' Activation of Innate Immunity

↓

Dendritic Cells/Macrophages/NK

Endogenous Pyrogens (ex. IL-6, IFNγ) ←+/-→ Endogenous Antipyretics (ex. TNFα)

+ −

Hypothalamus
↑↓ Thermal Setpoint

↓

Initiation of effector mechanisms

↓

FEVER/CHILL

EP 4 168 034 B1

# FIG. 41

Body Temperature for FLT3L/IFNg/CRYA_1B

EP 4 168 034 B1

FIG. 42

EP 4 168 034 B1

## FIG. 43

FLT3/IFNg/CRYA_1B (Prime)

# FIG. 44

## Prime-anti-PD1-Boost (x5)
## Typical doses

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 10675332 B1
- US 5037743 A, Welch
- US 5143830 A, Holland
- US 4713339 A, Levinson
- US 4784950 A, Hagen
- US 4579821 A, Palmiter
- US 4656134 A, Ringold
- US 4956288 A
- US 4601978 A
- US 5162222 A, Guarino
- WO 9406463 A
- US 5300435 A
- US 4599311 A, Kawasaki
- US 4931373 A, Kawasaki
- US 4870008 A, Brake
- US 4845075 A, Murray
- US 4615974 A, Kingsman
- US 4977092 A, Bitter
- US 4990446 A
- US 5063154 A
- US 5139936 A
- US 4661454 A
- US 4882279 A, Cregg
- US 4935349 A, McKnight
- US 5162228 A, Sumino
- US 4486533 A, Lambowitz
- US 5716808 A
- US 5736383 A
- US 5854039 A
- US 5888768 A
- US 10150801 B
- US 10323071 B
- US 10351607 B
- US 10301364 B
- US 10370421 B
- US 551659
- US 16443517 B
- SG 2019050420 W
- SG 2017050648 W
- US 20130156794 A

### Non-patent literature cited in the description

- **ROBERTS**. *Cancer Cell*, 2016, 324-336
- **BROZ**. *Cancer Cell*, 2014, 638-652
- **TESNIERE**. *Oncogene*, 2010, 482-491
- **JONGBLOED**. *J. Exp. Med.*, 2010, 1247-1260
- **CHANG**. *Cancer Research*, 2007, 10047-10057
- **PINZON-CHARRY**. *Br. J. Cancer*, 2007, 1251-1259
- **LAMMERMANN**. *Immunological Reviews*, 2008, 26-43
- **BOON**. *Annu. Rev. Immunol.*, 2006, 175-208
- **WIEDEMANN**. *PNAS*, 2006, 10985-10990
- **MCGAVERN**. *Nature Immunol*, 2002, 918-925
- **DEL MONTE**. *Cell Cycle*, 2009, 505-506
- **BACKMAN**. *Nature*, 2000, 35-36
- **BLATTMAN**. *J. Exp. Med.*, 2002, 657-664
- **HARING**. *Immunity*, 2006, 19-29
- **BUTLER**. *Cellular Microbiology*, 2011, 925-933
- **ARENS**. *Immunol. Rev.*, 2010, 190-205
- **SALLUSTO**. *Immunity*, 2010, 451-463
- **EPSTEIN**. *Science*, 2011, 475-80
- **WALSH**. *J. Infect. Dis.*, 2016, 541-550
- **SHINOHARA T et al.** *Genomics*, 1994, vol. 23 (3), 704-6
- **FINGER L R et al.** *Gene*, 1997, vol. 197, 177-87
- **DONG H et al.** *Nat. Med.*, 1999, vol. 5 (12), 1365-1369
- **FREEMAN G et al.** *J. Exp. Med.*, 2000, vol. 192 (7), 1027-1034
- **SAMBROOK** ; **RUSSELL**. Molecular Cloning: A Laboratory Manual. Cold Spring Harbor Laboratory Press, 2001
- **AUSUBEL et al.** Short Protocols in Molecular Biology. John Wiley & Sons, 1999
- **WIGLER et al.** *Cell*, 1978, vol. 14, 725
- **CORSARO** ; **PEARSON**. *Somatic Cell Genetics*, 1981, vol. 7, 603
- **GRAHAM** ; **VAN DER EB**. *Virology*, 1973, vol. 52, 456

- **NEUMANN et al.** *EMBO J.*, 1982, vol. 1, 841-845
- **HAWLEY-NELSON et al.** *Focus*, 1993, vol. 15, 73
- **CICCARONE et al.** *Focus*, 1993, vol. 15, 80
- **CHASIN et al.** *Som. Cell. Molec. Genet.*, 1986, vol. 12, 555
- **GATIGNOL et al.** *Mol. Gen. Genet.*, 1987, vol. 207, 342
- **DROCOURT et al.** *Nucl. Acids Res.*, 1990, vol. 18, 4009
- **SINKAR et al.** *J. Biosci. (Bangalore)*, 1987, vol. 11, 47-58
- **KING AND POSSEE.** *The Baculovirus Expression System: A Laboratory Guide (Chapman & Hall, London)*
- **O'REILLY et al.** Baculovirus Expression Vectors: A Laboratory Manual. Oxford University Press, 1994
- Baculovirus Expression Protocols. Methods in Molecular Biology. Humana Press, 1995
- **LUCKOW et al.** *J. Virol.*, 1993, vol. 67, 4566-4579
- **HILL-PERKINS** ; **POSSEE.** *J. Gen. Virol.*, 1990, vol. 71, 971-976
- **BONNING et al.** *J. Gen. Virol.*, 1994, vol. 75, 1551-1556
- **CHAZENBALK** ; **RAPOPORT.** *J. Biol. Chem.*, 1995, vol. 270, 1543-1549
- **GLICK** ; **PASTERNAK.** Molecular Biotechnology, Principles & Applications of Recombinant DNA. ASM Press, 1994
- **GLEESON et al.** *J. Gen. Microbiol.*, 1986, vol. 132, 3459-3465
- **RAYMOND et al.** *Yeast*, 1998, vol. 14, 11-23
- Affinity Chromatography: Principles & Methods. Pharmacia LKB Biotechnology, 1988
- **SCOPES.** Protein Purification: Principles and Practice. Springer-Verlag, 1994

- **KAMARCH.** *Methods Enzymol*, 1987, vol. 151, 150-165
- **LI et al.** *Proc Natl Acad Sci U S A.*, 2003, vol. 100 (5), 2674-8
- **SUNTHARALINGAM.** *N. Engl. J. Med.*, 2006, 1018-1028
- **TURTLE.** *J. Clin. Invest.*, 2016, 2123-2138
- **DAVILA.** *Sci. Transl. Med.*, 2014, 1-10
- **BRADY.** *Clin. Transl. Immunology*, 2014, 1-7
- **ALEGRE.** *Eur. J. Immunol*, 1990
- **NAIR.** *J. Basic Clin Pharm*, vol. 201, 27-31
- **FRASER.** *Immunity*, 2013, 171-183
- **ODORIZZI.** *J. Exp. Med.*, 2015, 1125-1137
- **SHOW.** *Immunological Reviews*, 2010, 68-82
- **WILLIAMS.** *Nature*, 2006, 890-893
- **RAI.** *J. of Immunol.*, 2014, 5652-5659
- **BAATEN.** *Front. Immunol.*, 2012, 1-12
- **CHIKUMA.** *J. Immunol.*, 2009, 6682-6689
- **MARTINEZ.** *Immunity*, 2015, 265-278
- **WANG.** *J. Hepatol.*, 2019, 731-741
- **MILLER.** *Immunity*, 2008, 710-722
- **MORTENSON.** *J. Immunol*, 1983, 885-889
- **NAIR.** *J. Basic Clin Pharm.*, vol. 201, 27-31
- **ALEGRE.** *Eur. J. Immunol*, 1990, 707-710
- *National Cancer Institute: Common Terminology Criteria for Adverse Events (CTCAE) Version 5.0, National institute of Health*, 2017
- **EVANS.** *Nat. Rev. Immunol.*, 2015, 335-349
- **FISHER.** *J. of Clin. Invest.*, 2011, 3846-3859
- **BOTA.** *Intensive Care Med.*, 2004, 811-816
- **LAUPLAND.** *Crit. Care Med.*, 2012, 145-151